# EUROPEAN PATENT APPLICATION

(11) **EP 1 522 580 A1**
(43) Date of publication of application: **13.04.2005**
(21) Application number: 03023136.9
(22) Date of filing: 10.10.2003
(51) Int. Cl.: C12N 9/12, C07K 14/47, C07D 209/42

(54) **Crystals of an aurora-a tpx2 complex, tpx2 binding site of aurora-a, aurora-a ligands and their use**

(71) Applicant: EMBL, D-69117 Heidelberg (DE)
(72) Inventor: Conti, Elena, 69126 Heidelberg (DE); Bayliss, Richard, London NW4 1DJ (GB); Schultz, Carsten, 69117 Heidelberg (DE); Vernos, Isabelle, 69115 Heidelberg (DE); Sardon, Teresa, 69115 Heidelberg (DE)
(74) Representative: Wolter, Thomas, Dr.

(57) **Abstract**

The present invention relates to crystals of phosphorylated Aurora-A kinase fragment alone and in complex with a ligand, amino acid residues 1-43 of human TPX2. This invention also relates to methods for designing and selecting ligands, in particular allosteric inhibitors of Aurora-A, that bind to the Aurora-A kinase and their use. Further, the present invention relates to certain indene and indole derivatives.The present invention relates to crystals of phosphorylated Aurora-A kinase alone and in complex with a ligand, amino acid residues 1-43 of human TPX2. This invention also relates to methods for designing and selecting ligands that bind to the Aurora-A kinase and their use. Further, the present invention relates to certain indene and indole derivatives.

## Description

The present invention relates to crystals of phosphorylated Aurora-A kinase fragment alone and in complex with a ligand, amino acid residues 1-43 of human TPX2. This invention also relates to methods for designing and selecting ligands, in particular allosteric inhibitors of Aurora-A, that bind to the Aurora-A kinase and their use. Further, the present invention relates to certain indene and indole derivatives.

At the beginning of mitosis, eukaryotic cells undergo a dramatic reorganization. The nuclear envelope breaks down and microtubules rearrange around chromatin into a bipolar spindle structure that carries out the duty of separating sister chromatids. Chromatin signals to the spindle assembly machinery through Ran, a small Ras-like GTPase that is concentrated in its GTP-bound form around chromatin. Ran function in spindle assembly is connected to its role in nucleocytoplasmic transport. RanGTP releases crucial spindle assembly factors such as TPX2 and NuMA from the transport factors that mediate their import into the nucleus at interphase. After nuclear envelope breakdown, the presence of free TPX2 in the vicinity of chromatin is thought to nucleate microtubules that are subsequently organised into a spindle by microtubule motors. Additionally, TPX2 localises an essential mitotic kinase, Aurora-A, to spindle microtubules ( Kufer, T. A., Sillje, H. H., Korner, R., Gruss, O. J., Meraldi, P., and Nigg, E. A. (2002). Human TPX2 is required for targeting Aurora-A kinase to the spindle. J. Cell Biol. *158*, 617-623).

Aurora kinases constitute a family of serine-threonine protein kinases whose localization and activities are precisely choreographed as a cell progresses through mitosis. Aurora-A is a cell-cycle regulated serine-threonine kinase involved in chromosome segregation and cytokinesis (Bischoff, J.R. and Plowman, G.D. (1999). The Aurora/lpl1p kinase family: regulators of chromosome segregation and cytokinesis. Trends Cell Biol. 9, 454-459). It plays a major role in cell-cycle progression and has also been described as an oncogene. It maps to a chromosome region frequently amplified in tumours (Dutertre, S. et al. (2002). On the role of Aurora A in centrosome function. Oncogene 21, 6175-6183). It is overexpressed in a variety of human tumours, in particular breast and colon cancer, but has limited expression in normal tissues (Sen, S. et al. (1997). A putative serine/threonine kinase encoding gene BTAK on chromosome 20q13 is amplified and overexpressed in human breast cancer cell lines. Oncogene 14, 2195-2200; Bischoff, J.R. et al. (1998). A homologue of Drosophila Aurora kinase is oncogenic and amplified in human colorectal cancers. EMBO J. 17, 3052-3065). Overexpression of active Aurora-A transforms rat fibroblasts so that they are capable of growing tumours in nude mice, while an inactive mutant is unable to cause oncogenic transformations. As an oncogenic protein kinase, Aurora-A is a target for the development of specific inhibitors that may be useful as cancer therapeutics. Despite the importance of Aurora-A for both cell division and cancer perspectives, little is known at present about its downstream targets and activation/deactivation mechanisms.

Several factors contribute to the activity of a serine/threonine kinase. These include the proper positioning of active site residues and the correct organisation of the substrate-binding site (the "activation segment"). Phosphorylation of a threonine residue within the activation segment is often required to elicit kinase activity and Aurora-A is no exception. Phosphorylation of a threonine in the Aurora-A activation segment (Thr288^{AUR}, human numbering) is crucial for activity, although it is unclear as to whether it is catalysed *in vivo* by an upstream kinase or by Aurora-A itself (Bischoff, et al. (1999) *supra*). Structural studies of c-AMP dependent protein kinase (cAPK) have shown that when the corresponding threonine residue (Thr197^{cAPK}) is phosphorylated, the activation segment is in an active conformation. Cyclin-dependent kinases (CDKs) require not only phosphorylation of the equivalent threonine (Thr160^{cDK}) but also the binding by a partner protein, cyclin-A, to be fully activated. Aurora-A might also rely on a similar mechanism. It has recently been reported that in vertebrates the interaction of Aurora-A with a partner protein, TPX2, leads to a strong activation of the kinase. Upon TPX2 binding, the *in vitro* autophosphorylation activity of Aurora-A is increased and dephosphorylation is prevented (Kufer et al., (2002) *supra).*

ATP competitive inhibitors that are specific for different kinases are used as therapeutic agents in cancer treatment (Garcia-Echeverria, C., et al. (2000). ATP site-directed competitive and irreversible inhibitors of protein kinases. Med. Res. Rev. 20, 28-57). Although ATP-binding sites at the moment are the most common targets for the design of kinase inhibitors, it is difficult to achieve selectivity of such inhibitors due to the similarity in kinase active sites, which only have minor differences of surrounding amino-acid residues (Cheetham, G.M.T. et al. (2002). Crystal structure of Aurora-2, an oncogenic serine-threonine kinase. J. Biol. Chem. 277, 42419-42422).

The structure of unphosphorylated Aurora-A has been previously reported (Cheetham, G.M.T. et al. (2002). Crystal structure of Aurora-2, an oncogenic serine-threonine kinase. J. Biol. Chem. 277, 42419-42422; Nowakowski, J. et al. (2002). Structures of the cancer-related Aurora-A, FAK, and EphA2 protein kinases from nanovolume crystallography. Structure 10, 1659-1667). Aurora-A has the typical three-dimensional structure of protein kinases, with the active site situated between the N- and C-terminal lobe. Binding of ATP involves amino-acid residues that are conserved among all kinases. Extensive structural work has shown that kinases in their active state all assume a similar structural framework, with the 'activation segment' in a similar conformation competent for substrate binding (Huse, M. and Kuriyan, J. (2002). The conformational plasticity of protein kinases. Cell 109, 275-282). However, they differ in the molecular mechanisms to achieve such an active form. In the case of Abl kinase, subtle differences in its activation mechanisms have been exploited with the development of the Abl-specific inhibitor Gleevec, which is used as a leukaemia therapeutic agent (Capdeville, R. et al. (2002). Glivec (ST1571, imatinib), a rationally developed, targeted anticancer drug. Nat. Rev. Drug Discov. 1, 493-502).

In the case of Aurora-A, activation is achieved by both phosphorylation and by the binding of a specific activator, the protein TPX2 (Eyers, P.A., et al. (2003). A novel mechanism for activation of the protein kinase Aurora-A. Curr. Biol. 13, 691-697). Blocking this activator-binding site would provide a means to downregulate this kinase specifically.

In view thereof, it was an object of the present invention to elucidate the structure of the Aurora-A TPX2 binding site, to provide means for identifying compounds that bind to Aurora-A and preferably modulate Aurora-A activity, and to provide such compounds.

The present invention relates to (a) crystals of a fragment of phosphorylated human Aurora-A kinase alone (amino acid residues 122-403; hereinafter referred to as Aurora-A(ΔN)), and (b) crystals of said fragment of phosphorylated human Aurora-A kinase in complex with a ligand, i. e. an Aurora-A ligand complex. The Aurora-A ligand is a fragment of TPX2 which is a minimal activating domain of TPX2. The minimal activating domain of TPX2 consists of amino acid residues 1-43 of human TPX2 and hereinafter is referred to as TPX2(1-43).

The present invention provides the structure coordinates of the phosphorylated human Aurora-A(ΔN) kinase. The complete coordinates are listed in Table A.

The present invention also provides the structure coordinates of the phosphorylated human Aurora-A(ΔN)/TPX2(1-43) complex. The complete coordinates are listed in Table B.

The present invention also describes a method for determining at least a portion of the three-dimensional structure of molecules or molecular complexes which contain at least some structurally similar features to the Aurora-A TPX2 binding domain. It is preferred that these molecules or molecular complexes comprise at least a part of the ligand binding site defined by structure coordinates of Aurora-A amino acids Q127, W128, R126, L159, F157, E170, L169, V206, Y199, H187, R179, L178, V182, Y199, L188, 1184, V252, K250. P282, H280 according to Table B, or a mutant or homologue thereof. The numbering system as used herein refers to the protein sequences for human Aurora-A.

The present invention also provides a machine-readable data storage medium which comprises a data storage material encoded with machine readable data defined by the structure coordinates of phosphorylated human Aurora-A(ΔN) kinase according to Table A or a homologue thereof, or of the phosphorylated human Aurora-A(ΔN)/TPX2(1-43) complex according to Table B.

The present invention further provides a binding site in Aurora-A for an Aurora-A ligand such as TPX2 or fragments thereof, as well as methods for designing or selecting further Aurora-A ligands and in particular Aurora-A modulators including agonists, partial agonists, antagonists, partial antagonists of Aurora-A using information about the crystal structures disclosed herein.

The present invention further provides allosteric inhibitors of Aurora-A, wherein at least a portion of the inhibitor binds with any portion or all of residues Q127, W128, R126, L159, F157, E170, L169, V206, Y199, H187, R179, L178, V182, Y199, L188, I184, V252, K250, P282, H280 of Aurora-A according to Table B.

The present invention in particular relates to indole and indene derivatives of formula (I) 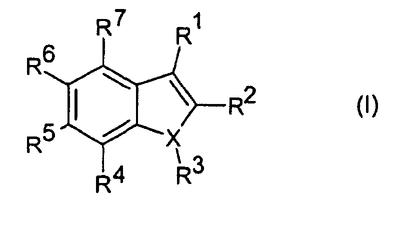 wherein
- R¹: represents hydrogen, alkylene-NHR⁸, alkylene-OR⁸, or alkylene-SR⁸;
- R²: represents hydrogen, alkylene-NHR⁸, alkylene-OR⁸, or alkylene-SR⁸;
- R³: represents hydrogen, alkyl, alkylene-R⁹, alkenylene-R⁹, alkynylene-R⁹, or arylene-R⁹;
- R⁴: represents hydrogen;
- R⁵: represents hydrogen, alkyl, OR¹⁰, NHR¹⁰, SR¹⁰, alkylene-R¹⁰, alkenylene-R¹⁰, alkynylene-R¹⁰, or arylene-R¹⁰;
- R⁶: represents hydrogen, alkyl, OR¹⁰, NHR¹⁰, SR¹⁰, alkylene-R¹⁰, alkenylene-R¹⁰, alkynylene-R¹⁰, or arylene-R¹⁰;
- R⁷: represents hydrogen;
- R⁸: represents hydrogen, CO-alkyl, (aa)ₘasp(aa)ₙ, (aa)ₘglu(aa)ₙ, or (aa)ₘcys(aa)ₙ;
- R⁹: represents NH-alkyl, N(alkyl)₂, N⁺(alkyl)₃, aryl, or heteroaryl;
- R¹⁰: represents hydrogen, aryl, or substituted aryl;
- X: represents a nitrogen atom or CH;
- aa: represents an amino acid residue; and
- n: is zero or an integer of 1 to 10;
- m: is zero or an integer of 1 to 10.
provided that R¹ and R² are not both hydrogen and that R⁵ and R⁶ are not both hydrogen, and optical isomers, physiologically acceptable salts and prodrugs thereof.

The present invention also relates to the pharmaceutical compostions containing Aurora-A ligands, such as said indole and indene derivatives, and the use of Aurora-A ligands, such as said indole and indene derivatives, in therapy, in particular in cancer treatment.

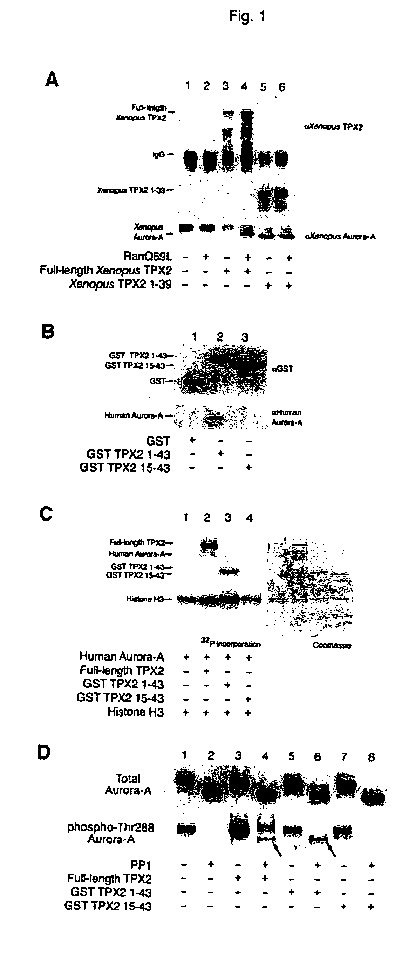

In the drawings the figures show as
- (figure 1): (A) anti-*Xenopus* TPX2 Western blot (upper panel) and anti-*Xenopus* Aurora-A Western blot (lower panel) of GST (glutathione S transferase) (lanes 1 and 2), GST *Xenopus* TPX2 (lanes 3 and 4) or GST *Xenopus* TPX2(1-39) (lanes 5 and 6) which proteins were incubated in *Xenopus* cytostatic factor (CSF) arrested extracts in the presence or absence of RanQ69L-GTP and immunoprecipitated with GST antibody-coated beads;
(B) anti-GST Western blot (upper panel) and anti-human Aurora-A Western blot (lower panel) of GST (lane 1), GST TPX2(1-43) (lane 2) or GST TPX2(15-43) (lane 3) proteins that were incubated in mitotic HeLa cell extract and immunoprecipitated with GST antibody-coated beads;
(C) autoradiography of the SDS-PAGE gel (left panel) and the corresponding Coomassie-stained gel (right panel) after *in vitro* phosphorylation (γ³²P-ATP) of histone H3 by human Aurora-A in the presence of full-length TPX2, GST TPX2(1-43) or GST TPX2(15-43) (lanes 2, 3 and 4 respectively);
(D) much of the phosphorylation signal in GST after *in vitro* phosphorylation (γ³²P-ATP) of TPX2(1-43) by Aurora-A (lane 1) followed by TEV cleavage (lane 2);
(E) an anti-human Aurora-A Western blot (upper panel) and an anti-phosphoAurora-A Western blot (lower panel) after phosphatase PP1 treatment of human Aurora-A in the absence or presence of full-length TPX2,
GST TPX2(1-43) or GST TPX2(15-43) followed by detection of Aurora-A by a polyclonal antibody (upper panel) and an antibody specific for Aurora-A phosphorylated at Thr288^{AUR} (lower panel);

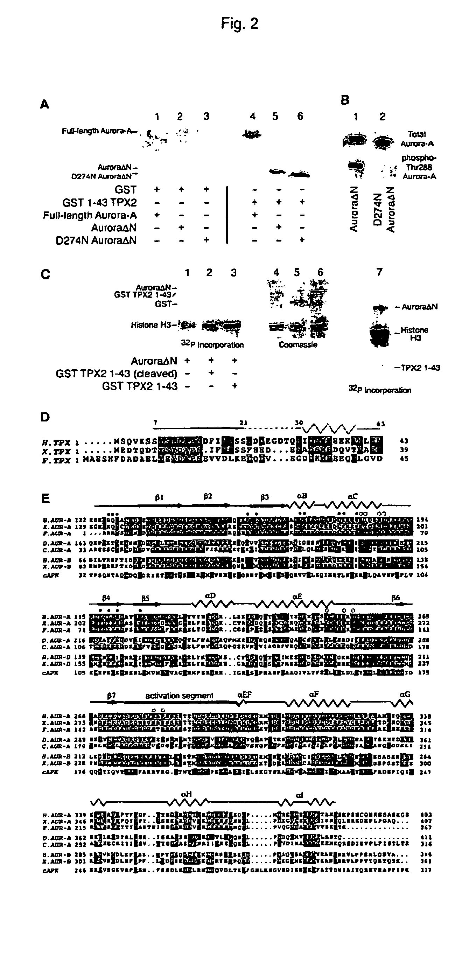

- (figure 2): (A) an *in vitro* pull-down assay with respect to the binding of full-length Aurora-A, AuroraΔN or AuroraΔN(D274N) to GST TPX2(1-43) (lanes 4, 5 and 6), and to GST (lanes 1, 2 and 3);
(B) an anti-phospho Aurora-A Western blot of wild-type Aurora(ΔN) and the D274N mutant when expressed in *E. coli.*
(C) autoradiograph for detecting *in vitro* phosphorylation of histone H3 by Aurora(ΔN) in the presence or absense of TPX2(1-43) (lane 2 compared to lane 1), the cleaved (lane 2) or uncleaved (lane 3) GST TPX2(1-43) fusion protein;
(D) Sequence alignment of TPX2 N-terminal domain from human *(H), Xenopus* (*X*) and puffer fish (*F*), secondary structure elements being shown above the sequences *in red* (upstream extended stretch) and *pink* (downstream helical stretch), and intervening residues not modelled being marked with a dotted line;
(E) Sequence alignment of Aurora-A kinase catalytic domain from three vertebrate species that contain TPX2 (human, *H.AUR-A; Xenopus, X.AUR-A;* puffer fish, *F AUR-A),* two invertebrates that do not contain TPX2 *(Drosophila, D.AUR-A; C.elegans*, *C.AUR-A)* together with human and *Xenopus* Aurora-B *(H.AUR-B, X.AUR-B)* and vertebrate cAPK, wherein Aurora-A secondary structural elements are labelled above the alignment, the phosphorylated Thr288 (human numbering) is shown, and residues that interact with 7-21^{TPX} or 30-43^{TPX} being indicated by filled or open circles respectively;

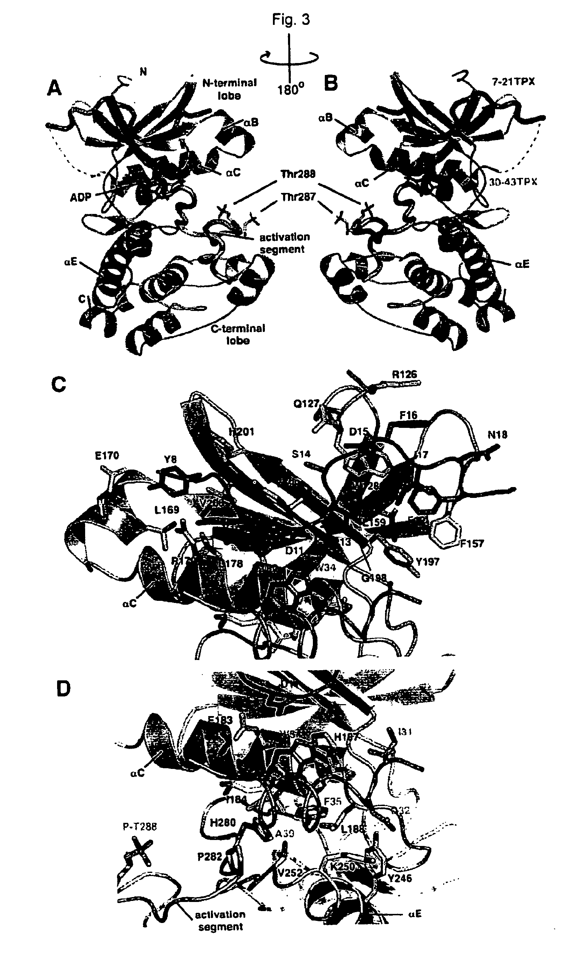

- (figure 3): ribbon style drawings of the structure of Aurora-A bound to TPX2 as
(A) a view of the complex between the catalytic domain of human Aurora (AuroraΔN) and the N-terminal domain of TPX2 shown in typical kinase orientation, an upstream stretch of TPX2 binding at the N-terminal lobe of Aurora-A, and a downstream stretch binding between the two lobes, and a dotted line marking the approximate path of the linker connecting the two TPX2 stretches (disordered and not modelled);
(B) a view of the complex after a 180° rotation about the vertical axis in respect to view in panel A showing more clearly the two stretches of TPX2 that bind to Aurora-A;
(C) the upstream stretch of TPX2 (residues 7-21^{TPX}) that binds at a hydrophobic surface groove present in the N-terminal lobe of the kinase, wherein details of the extensive interactions are shown in the same orientation as in panel B;
(D) the downstream helical stretch of TPX2 (residues 30-43^{TPX}) that binds Aurora-A near helix αC and the activation segment, close to but not directly in contact with phospho-Thr288^{AUR}, wherein details of interactions being shown in the same orientation as in panel B and C.

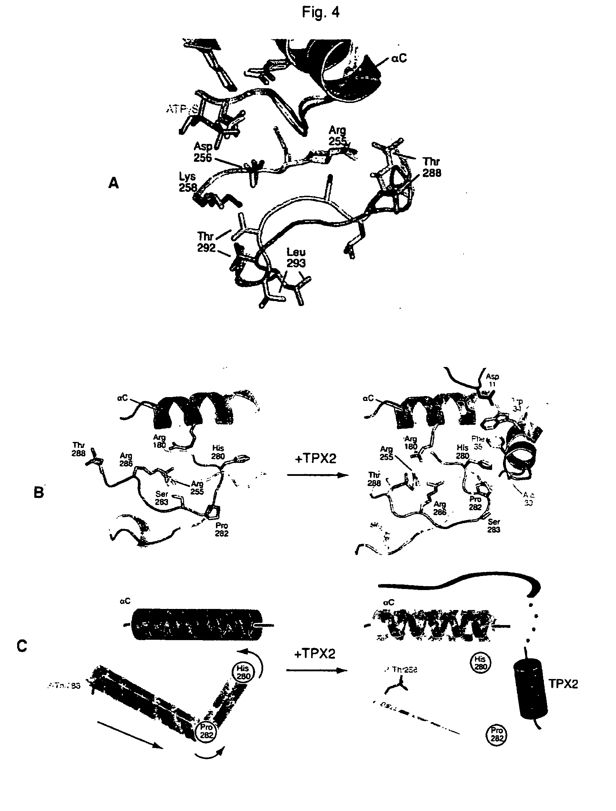

- (figure 4): ribbon style drawings of conformational states of phosphorylated Aurora-A in the presence and absence of TPX2 as
(A) an overlay showing that the structures of Aurora-A when bound to TPX2 and when unbound are closely superposable at the position of active site residues and of helix αC, but diverge at the activation segment between residues His280^{AUR} and Leu293^{AUR}, wherein Phospho-Thr288^{AUR} points inwards in the TPX2-bound structure and outwards in the kinase alone structure;
(B) an illustration of conformational changes upon TPX2 binding, according to which the activation segments of the kinase alone structure (left panel) and of the TPX2-bound structure (right panel) are shown in a view rotated by approximately 90° with respect to panels A-C, and TPX2 binding results in the reorganization of the activation segment, with a 10 Å movement of Thr288^{AUR};
(C) a schematic representation of the molecular mechanism of TPX2-mediated activation of Aurora-A, according to which the upstream stretch of TPX2 anchors the regulator to the N-terminal lobe of the kinase and the downstream stretch (helix) hooks the activation segment triggering a lever-arm like movement, where rotations at His280^{AUR} and Pro282^{AUR} pull on Thr288^{AUR}.

Using X-ray crystallography, crystal structures of phosphorylated human Aurora-A(ΔN) alone at 2.75 Å resolution and in complex with a minimal activating domain of TPX2 at 2.5 Å resolution have been determined and the specific site of TPX2-mediated activation of Aurora-A has been found. TPX2 binds at two sites on the kinase. One stretch of TPX2 (residues 8-19) binds at the N-terminal lobe of the kinase and another stretch (31-38) binds between the two lobes at the phosphorylated activation segment, positioning it for substrate binding. The TPX2-binding site located on the N-terminal lobe of the kinase consists of an extended surface groove formed by Aurora-specific residues. The groove consists of two pockets, one shaped by residues E170, L169, V206, R179, L178, V182, Y199 and the other shaped by residues Q127, W128, R126, L159 and F157. The two pockets are occupied by hydrophobic side chains of TPX2, Y8, Y10, A12 and P13 on one side, and F16, I17 and F19 on the other. From the structure and biochemical data it is concluded that binding of this TPX2 stretch is necessary but not sufficient for activation of Aurora-A, allowing the downstream TPX2 stretch to bind with enough affinity to achieve activation. Binding of a small-molecule inhibitor to the TPX2-recognition groove on the N-terminal lobe of Aurora-A *would* decrease the activity of this kinase by blocking its specific activation mechanism.

The Aurora-A kinase and the TPX2 protein described herein are intended to include any polypeptide which has the activity of the naturally occurring Aurora-A kinase and TPX2 protein, respectively. Aurora-A and TPX2 contemplated herein include all vertebrate and mammalian forms such as rat, mouse, pig, goat, horse, guinea pig, rabbit, monkey, orangutan and human. Such terms also include polypeptides that differ from naturally occurring forms of Aurora-A kinase and TPX2 protein by having amino acid deletions, substitutions, and additions, but which retain the activity of Aurora-A kinase and TPX2 protein, respectively. Particular Aurora-A sequences are shown in figure 2E and particular TPX2 sequences are shown in figure 2D. According to the present invention, the human sequences are preferred.

The crystal structures of the invention preferably contains at least 25%, more preferably at least 50%, more preferably at least 75%, more preferably at least 90%, more preferably at least 95%, more preferably at least 99%, and most preferably all of the coordinates listed in Table A and Table B, respectively.

The crystal of the phosphorylated human Aurora-A(ΔN) kinase of the invention preferably has the following unit cell dimensions in angstroms: a = 81.18 ± 5%, b = 81.18 ± 5%, c = 169.62 ± 5% and the space group P6₁22.

The crystal of the phosphorylated human Aurora-A(ΔN) kinase/TPX2(1-43) complex of the invention preferably has the following unit cell dimensions in angstroms: a = 59.63 ± 5%, b = 81.72 ± 5%, c = 70.38 ± 5% and an orthorhombic space group P2₁2₁2₁.

The three-dimensional structure of the phosphorylated human Aurora-A(ΔN) kinase and of the phosphorylated human Aurora-A(ΔN) kinase/TPX2(1-43) complex of this invention are defined by a set of structure coordinates as set forth in Table A and Table B, respectively. The term "structure coordinates" refers to Cartesian coordinates derived from mathematical equations related to the patterns obtained on diffraction of a monochromatic beam of X-rays by the atoms (scattering centers) of the protein or protein complex in crystal form. The diffraction data are used to calculate an electron density map of the repeating unit of the crystal. The electron density maps are then used to establish the positions of the individual atoms of the complex.

Those of skill in the art will understand that a set of structure coordinates for a kinase or a kinase/ligand complex or a fragment thereof, is a relative set of points that define a shape in three dimensions. Thus, it is possible that an entirely different set of coordinates could define a similar or identical shape. Moreover, slight variations in the individual coordinates will have little effect on overall shape.

The variations in coordinates discussed above may be generated because of mathematical manipulations of the structure coordinates. For example, the structure coordinates set forth in Table A could be manipulated by crystallographic permutations of the structure coordinates, fractionalization of the structure coordinates; integer additions or subtractions to sets of the structure coordinates, inversion of the structure coordinates or any combination of the above.

Alternatively, modifications in the crystal structure due to mutations, additions, substitutions, and/or deletions of amino acids, or other changes in any of the components that make up the crystal could also account for variations in structure coordinates. If such variations are within an acceptable standard error as compared to the original coordinates, the resulting three-dimensional shape is considered to be the same.

Various computational analyses are therefore necessary to determine whether a molecule or molecular complex or a portion thereof is sufficiently similar to all or parts of the kinase or the kinase/ligand complex described above as to be considered the same. Such analyses may be carried out in current software applications, such as the Molecular Similarity application of QUANTA (Molecular Simulations Inc., San Diego, CA) version 4.1, and as described in the accompanying User's Guide.

The Molecular Similarity application permits comparisons between different structures, different conformations of the same structure, and different parts of the same structure. The procedure used in Molecular Similarity to compare structures is divided into four steps: 1) load the structures to be compared; 2) define the atom equivalences in these structures; 3) perform a fitting operation; and 4) analyze the results.

Each structure is identified by a name. One structure is identified as the target (i. e., the fixed structure); all remaining structures are working structures (i. e., moving structures). Since atom equivalency within QUANTA is defined by user input, for the purpose of this invention we will define equivalent atoms as protein backbone atoms (N, C and O) for all conserved residues between the two structures being compared. We will also consider only rigid fitting operations.

When a rigid fitting method is used, the working structure is translated and rotated to obtain an optimum fit with the target structure. The fitting operation uses an algorithm that computes the optimum translation and rotation to be applied to the moving structure, such that the root mean square difference of the fit over the specified pairs of equivalent atom is an absolute minimum. This number, given in angstroms, is reported by QUANTA.

For the purpose of this invention, any molecule or molecular complex that has a root mean square deviation of conserved residue backbone atoms (N, C, O) of less than 1.5 A when superimposed on the relevant backbone atoms described by structure coordinates listed in Table A or Table B are considered identical. More preferably, the root mean square deviation is less than 1.0 A. In a preferred embodiment of the present invention, the molecule or molecular complex comprises at least a portion of the ligand binding site defined by structure coordinates of Aurora-A amino acids Q127, W128, R126, L159, F157, E170, L169, V206, Y199, H187, R179, L178, V182, Y199, L188, 1184, V252, K250, P282, H280 according to Table B, or a mutant or homologue of said molecule or molecular complex. More preferred are molecules or molecular complexes comprising all or any part of the ligand binding site defined by structure coordinates of Aurora-A amino acids Y199, L178, W128, H187, L188, I184 according to Table B, or a mutant or homologue of said molecule or molecular complex.

The term "complex" or "molecular complex" means Aurora-A or a mutant or homologue of Aurora-A in a covalent or non-covalent association with a chemical entity or compound.

For purposes of the present invention, by "at least a portion of" it is meant all or any part of the ligand binding site defined by these structure coordinates.

By "mutant or homologue" as used herein it is meant a molecule or molecular complex having a similar structure and/or sequences to Aurora-A or TPX2. By "similar structure" it is meant a mutant or homologue having a binding pocket or binding domain that have a root mean square deviation from the backbone atoms of said Aurora-A or TPX2 amino acids of not more than 1.5 Angstroms. By "similar sequence" it is meant a mutant or homologue having 30%, or more preferably 75%, identity with Aurora-A or TPX2 over an amino acid sequnce of at least 30, preferably at least 50, in particular at least 100 and especially at least 200 consecutive amino acid.

The term "root mean square deviation" means the square root of the arithmetic mean of the squares of the deviations from the mean. It is a way to express the deviation or variation from a trend or object. For purposes of this invention, the "root mean square deviation" defines the variation in the backbone of a protein or protein complex from the relevant portion of the backbone of the Aurora-A fragment of the complex as defined by the structure coordinates described herein.

Once the structure coordinates of a protein crystal have been determined they are useful in solving the structures of other crystals.

Thus, in accordance with the present invention, the structure coordinates of the kinase ot the kinase/ligand complex, and portions thereof is stored in a machine-readable storage medium. Such data may be used for a variety of purposes, such as drug discovery and x-ray crystallographic analysis of protein crystals.

Accordingly, in one embodiment of this invention is provided a machine-readable data storage medium comprising a data storage material encoded with the structure coordinates set forth in Table A and/or with the structure coordinates set forth in Table B.

For the first time, the present invention permits the use of structure-based or rational drug design techniques to design, select, and synthesize chemical entities, including inhibitory and stimulatory compounds that are capable of binding to Aurora-A, or any portion thereof.

One particularly useful drug design technique enabled by this invention is iterative drug design. Iterative drug design is a method for optimizing associations between a protein and a compound by determining and evaluating the three-dimensional structures of successive sets of protein/compound complexes.

Those of skill in the art will realize that association of natural ligands or substrates with the binding pockets of their corresponding kinases or enzymes is the basis of many biological mechanisms of action. The term "binding pocket" as used herein, refers to a region of a molecule or molecular complex, that, as a result of its shape, favorably associates with another chemical entity or compound. Similarly, many drugs exert their biological effects through association with the binding pockets of kinases and enzymes. Such associations may occur with all or any parts of the binding pockets. An understanding of such associations will help lead to the design of drugs having more favorable associations with their target kinase, and thus, improved biological effects. Therefore, this information is valuable in designing potential ligands or inhibitors of Aurora-A kinase.

The term "associating with" refers to a condition of proximity between chemical entities or compounds, or portions thereof. The association may be non-covalent--wherein the juxtaposition is energetically favored by hydrogen bonding or van der Waals or electrostatic interactions--or it may be covalent.

In iterative drug design, crystals of a series of protein/compound complexes are obtained and then the three-dimensional structures of each complex is solved. Such an approach provides insight into the association between the proteins and compounds of each complex. This is accomplished by selecting compounds with inhibitory activity, obtaining crystals of this new protein/compound complex, solving the three-dimensional structure of the complex, and comparing the associations between the new protein/compound complex and previously solved protein/compound complexes. By observing how changes in the compound affected the protein/compound associations, these associations may be optimized.

In some cases, iterative drug design is carried out by forming successive protein-compound complexes and then crystallizing each new complex. Alternatively, a pre-formed protein crystal is soaked in the presence of a compound, thereby forming a protein/compound complex and obviating the need to crystallize each individual protein/compound complex.

As used herein, the term "soaked" refers to a process in which the crystal is transferred to a solution containing the compound of interest.

The structure coordinates set forth in Table A and Table B can also be used to aid in obtaining structural information about another crystallized molecule or molecular complex. This may be achieved by any of a number of well-known techniques, including molecular replacement.

The structure coordinates set forth in Table A and Table B can also be used for determining at least a portion of the three-dimensional structure of molecules or molecular complexes which contain at least some structurally similar features to Aurora-A. In particular, structural information about another crystallized molecule or molecular complex may be obtained. This may be achieved by any of a number of well-known techniques, including molecular replacement.

Therefore, in another embodiment this invention provides a method of utilizing molecular replacement to obtain structural information about a crystallized molecular complex whose structure is unknown comprising the steps of:
a) generating an X-ray diffraction pattern from said crystallized molecular complex;
b) applying at least a portion of the structure coordinates set forth in Table A and Table B to the X-ray diffraction pattern to generate a three-dimensional electron density map of the molecular complex whose structure is unknown; and
c) using all or a portion of the structure coordinates set forth in Table A and Table B to generate homology models of Aurora-A or any other kinase ligand binding domain.

Preferably, the crystallized molecular complex is obtained by soaking a crystal of this invention in a solution.

By using molecular replacement, all or part of the structure coordinates of the Aurora-A ligand complex provided by this invention or molecular complex whose structure is unknown more quickly and efficiently than attempting to determine such information ab initio.

Molecular replacement provides an accurate estimation of the phases for an unknown structure. Phases are a factor in equations used to solve crystal structures that can not be determined directly. Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory estimate of the phases for the unknown structure.

Thus, this method involves generating a preliminary model of a molecule or molecular complex whose structure coordinates are unknown, by orienting and positioning the relevant portion of the Aurora-A ligand complex according to Table B within the unit cell of the crystal of the unknown molecule or molecular complex so as best to account for the observed X-ray diffraction pattern of the crystal of the molecule or molecular complex whose structure is unknown. Phases can then be calculated from this model and combined with the observed X-ray diffraction pattern amplitudes to generate an electron density map of the structure whose coordinates are unknown. This, in turn, can be subjected to any well-known model building and structure refinement techniques to provide a final, accurate structure of the unknown crystallized molecule or molecular complex [E. Lattman,"Use of the Rotation and Translation Functions", in Meth. Enzymol., 115, pp. 55-77 (1985); M. G. Rossmann, ed.,"The Molecular Replacement Method", Int. Sci. Rev. Set., No. 13, Gordon & Breach, New York (1972)].

The structure of any portion of any crystallized molecule or molecular complex, or mutant, or homologue that is sufficiently homologous to any portion of the Aurora-A ligand complex can be solved by this method.

The structure coordinates are also particularly useful to solve the structure of crystals of Aurora-A ligand co-complexed with a variety of chemical entities. This approach enables the determination of the optimal sites for interaction between chemical entities, including interaction of candidate Aurora-A inhibitors with the complex. For example, high resolution X-ray diffraction data collected from crystals exposed to different types of solvent allows the determination of where each type of solvent molecule resides. Small molecules that bind tightly to these sites can then be designed and synthesized and tested for their Aurora-A inhibition activity.

All of the complexes referred to above may be studied using well-known X-ray diffraction techniques and may be refined versus 1.5-3 A resolution X-ray data to an R value of about 0.20 or less using computer software, such as X-PLOR [Yale University, 1992, distributed by Molecular Simulations, Inc.; see, e. g., Blundell & Johnson, supra; Meth. Enzymol., vol. 114 & 115, H. W. Wyckoff et al., eds., Academic Press (1985)]. This information may thus be used to optimize Aurora-A agonists, partial agonists, antagonists, partial antagonists, and more importantly, to design new Aurora-A agonists/antagonists.

Accordingly, the present invention is also directed to a binding site in Aurora-A for an Aurora-A ligand in which a portion of Aurora-A ligand is in van der Walls contact or hydrogen bonding contact with at least one of the following residues: Y199, L178, W 128, L159, H187, L188, I184 of Aurora-A.

For purposes of this invention, by Aurora-A binding site it is also meant to include mutants or homologues thereof. in a preferred embodiment, the mutants or homologues have at least 25% identity, more preferably 50% identity, more preferably 75% identity, and most preferably 95% identity to residues Q127, W128, R126, L159, F157, E170, L169, V206, Y199, H187, R179, L178, V182, Y199, L188, I184, V252, K250, P282, H280 of Aurora-A binding sites.

The present invention is also directed to a machine-readable data storage medium, comprising a data storage material encoded with machine readable data, wherein the data is defined by the structure coordinates of an Aurora-A ligand complex according to Table B or a homologue of said complex, wherein said homologue comprises backbone atoms that have a root mean square deviation from the backbone atoms of the complex of not more than 3.0 Å preferably of not more that 2.0 Å.

The present invention also provides for computational methods using three-dimensional models of the Aurora-A kinase that are based on crystals of the kinase or the kinase ligand complex. Generally, the computational method of designing an Aurora-A ligand determines which amino acid or amino acids of Aurora-A interact with a chemical moiety (at lease one) of the ligand using a three-dimensional model of a crystallized protein comprising the Aurora-A kinase with a bound ligand and selecting a chemical modification (at least one) of the chemical moiety to produce a second chemical moiety with a structure that either decreases or increases an interaction between the interacting amino acid and the second chemical moiety compared to the interaction between the interacting amino acid and the corresponding chemical moiety on the natural ligand, i.e. TPX2 or a fragment thereof.

The computational methods of the present invention are for designing kinase synthetic ligands using such crystal and three dimensional structural information to generate synthetic ligands that modulate the conformational changes of the kinase. These computational methods are particularly useful in designing an agonist, partial agonist, antagonist or partial antagonist to the kinase, wherein the agonist, partial agonist, antagonist or partial antagonist has an extended moiety that prevents any one of a number of ligand-induced molecular events that alter the kinase's influence on one of its targets, such as preventing the normal coordination of the activation domain observed for a naturally occurring ligand or other ligands that mimic the naturally occurring ligand, such as an agonist. As described herein, synthetic ligands of the kinase will be useful in modulating kinase activity in a variety of medical conditions.

Aurora-A is known to comprise various segments as follows: 1) an amino-terminal ligand-binding site; and 2) an Aurora-A activation segement.

This modularity permits different segments and sites of each protein to separately accomplish different functions, although the segments and sites influence each other.

Exploiting the TPX2-binding site in the N-terminal lobe of Aurora-A has the advantage of blocking the activation of this oncogenic kinase and of blocking it specifically. An Aurora-A specific inhibitor would be an important cancer therapeutic agent in particular for breast and colon tumours.

The polypeptides referred to herein (e. g., Aurora-A, TPX2, and the like) may be produced by any well-known method, including synthetic methods, such as solid phase, liquid phase and combination solid phase/liquid phase syntheses; recombinant DNA methods, including cDNA cloning, optionally combined with site directed mutagenesis; and/or purification of the natural products, optionally combined with enzymatic cleavage methods to produce fragments of naturally occurring forms of Aurora-A and TPX2. The peptides can be expressed, crystallized, its three dimensional structure determined with a ligand bound (either using crystal data from the same kinase or a different kinase or a combination thereof), and computational methods used to design ligands to its ligand binding site, particularly ligands that contain an extension moiety that coordinates the activation segment of Aurora-A.

Typically Aurora-A and TPX2 as well as fragments thereof are purified to homogeneity for crystallization.

Purity of Aurora-A is measured with SDS-PAGE, mass spectrometry and hydrophobic HPLC. The purified Aurora-A for crystallization should be at least 97.5 % pure or 97.5%, preferably at least 99.0% pure or 99.0% pure, more preferably at least 99.5% pure or 99.5% pure.

Initially purification of the unliganded kinase can be obtained by conventional techniques, such as size exclusion chromatography, hydrophobic interaction chromatography (HPLC), ion exchange chromatography (HPLC), and heparin affinity chromatography.

To achieve higher purification for improved crystals of Aurora-A, it will be desirable to ligand shift purify the kinase using a column that separates the kinase according to charge, such as an ion exchange or hydrophobic interaction column, and then bind the eluted kinase with a ligand, especially an agonist or partial agonist. The ligand induces a change in the kinase's surface charge such that when rechromatographed on the same column, the kinase then elutes at the position of the liganded kinase are removed by the original column run with the unliganded kinase. Usually saturating concentrations of ligand are used in the column and the protein can be preincubated with the ligand prior to passing it over the column.

More recently developed methods involve engineering a "tag" such as with histidine placed on the end of the protein, such as on the amino terminus, and then using a nickle chelation column for purification, Janknecht R., Proc. Natl. Acad. Sci. USA Vol 88: 8972-8976 (1991) incorporated by reference.

Typically, purified Aurora-A is equilibrated at a saturating concentration of ligand at a temperature that preserves the integrity of the protein. Ligand equilibration can be stablished between 2 and 37 °C, although the kinase tends to be more stable in the 2-20 °C range.

Preferably crystals are made with the hanging and/or sittiing drop methods.

Regulated temperature control is desirable to improve crystal stability and quality. Temperatures between 4 and 25 °C are generally used and it is often preferable to test crystallization over a range of temperatures. It is preferable to use crystallization temperatures from 18 to 25 °C, more preferably 20 to 23 °C, and most preferably 22 °C.

The Aurora-A ligand of this invention is any peptide, peptide mimetic or nonpeptide, including small organic molecules, that is capable of acting as a ligand for Aurora-A. In a preferred embodiment, the Aurora-A ligand is an Aurora-A modulator. By" Aurora-A modulator "it is meant an agonist or activator, a partial agonist or partial activator, an antagonist or inhibitor, or a partial antagonist or partial inhibitor of the Aurora-A kinase.

Agonists or partial agonists induce changes in kinases that place them in an active conformation that allows them to influence one of its targets. There may be several different ligand-induced changes in the kinase's conformation.

Antagonists or partial antagonists bind to kinases, but fail to induce conformational changes that alter the kinase's target-influencing properties or physiologically telcram conformations. Binding of an antagonist or partial antagonist can also block the binding and therefore the actions of an agonist or partial agonist.

Partial agonists, or partial antagonists, bind to kinases and induce only part of the changes in the kinases that are induced by agonists or antagonists, respectively. The differences can be qualitative or quantitative. Thus, a partial agonist or partial antagonist may induce some of the conformation changes induced by agonists or antagonists, respectively, but not others, or it may only induce certain changes to a limited extent.

As described herein, the unliganded kinase is in a configuration that is either inactive, has some activity or has phosphorylating activity. Binding of agonist ligands induces conformational changes in the kinase such that the kinase becomes more active and/or is protected from deactivation, in particular from dephosphorylation.

According to a particular embodiment, the present invention relates to allosteric inhibitors of Aurora-A. The binding of such an allosteric inhibitor to Aurora-A results in a conformational change of the kinase, thereby decreasing the kinases's activity, preferably by blocking its activation mechanism. The preferred binding site of an allosteric inhibitor according to the present invention is the TPX2-recognition groove where TPX2 binds Aurora-A, as described herein.

Consequently, an extended chemical moiety (or more) from the ligand that stabilizes the binding or contact of the TPX2 binding site with the binding site of Aurora-A can be designed. Typically such chemical moieties will extend past and away from the molecular recognition domain on the ligand and usually past the buried binding cavity of the ligand.

Ligand binding by the kinase is a dynamic process, which regulates kinase function by inducing an altered conformation.

The three-dimensional structure of the liganded Aurora-A kinase can be used in the development of new Aurora-A synthetic ligands. In addition, Aurora-A is overall well suited to modern methods including three-dimensional structure elucidation and combinatorial chemistry such as those disclosed in EP 335 628, U. S. patent 5,463,564, which are incorporated herein by reference. Computer programs that use crystallography data when practicing the present invention enable the rational design of ligands to Aurora-A. Programs such as RASMOL can be used with the atomic coordinates from crystals generated by practicing the invention or used to practice the invention by generating three dimensional models and/or determining the structures involved in ligand binding. Computer programs such as INSIGHT and GRASP allow for further manipulation and the ability to introduce new structures. In addition, high throughput binding and bioactivity assays can be devised using purified recombinant protein and modern assays described herein and known in the art in order to refine the structure of a ligand and thereby the activity.

Generally the computational method of designing an Aurora-A synthetic ligand comprises two steps:
1) determining which amino acid or amino acids of Aurora-A interacts with a first chemical moiety (at least one) of the ligand using a three dimensional model of a crystallized protein comprising Aurora-A with a bound ligand; and
2) selecting chemical modifications (at least one) of the first chemical moiety to produce a second chemical moiety with a structure to either decrease or increase an interaction between the interacting amino acid and the second chemical moiety compared to the interaction between the interacting amino acid and the first chemical moiety.

Preferably the method is carried out wherein said three dimensional model is generated by comparing isomorphous ligand derivatives to produce improved phasing. Further preferred is wherein said method comprises determining a change in interaction between said interacting amino acid and said ligand after chemical modification of said first chemical moiety, especially wherein said three dimensional model is generated by comparing isomorphous ligand derivatives to produce improved phasing. Also preferred is wherein said selecting uses said first chemical moiety that interacts with at least one of the interacting amino acids Y199, L178, W128, L159, H187, L188, I184.

As shown herein, interacting amino acids form contacts with the ligand and the center of the atoms of the interacting amino acids are usually 2 to 4 angstroms away from the center of the atoms of the ligand. Generally these distances are determined by computer, however distances can be determined manually once the three dimensional model is made. See also Wagner et al., Nature 378 (6558): 670-697 (1995) for stereochemical figures of-three dimensional models. More commonly, the atoms of the ligand and the atoms of interacting amino acids are 3 to 4 angstroms apart. The invention can be practiced by repeating steps I and 2 to refine the fit of the ligand to the ligand bindiung site and to determine a better ligand, such as an agonist, partial agonist, antagonist or partial antagonist.

The three dimensional model of Aurora-A can be represented in two dimensions to determine which amino acids contact the ligand and to select a position on the ligand for chemical modification and changing the interaction with a particular amino acid compared to that before chemical modification. The chemical modification may be made using a computer, manually using a two dimensional representation of the three dimensional model or by chemically synthesizing the ligand. The ligand can also interact with distant amino acids after chemical modification of the ligand to create a new ligand. Distant amino acids are generally not in contact with the ligand before chemical modification. A chemical modification can change the structure of the ligand to make as new ligand that interacts with a distant amino acid usually at least 4.5 angstroms away from the ligand, preferably wherein said first chemical moiety is 6 to 12 angstroms away from a distant amino acid. Often distant amino acids will not line the surface of the binding cavity for the ligand, they are too far away from the ligand to be part of a pocket or binding cavity. The interaction between an amino acid of the ligand binding site and an atom of a ligand can be made by any force or attraction described in nature. Usually the interaction between the atom of the amino acid and the ligand will be the result of a hydrogen bonding interaction, charge interaction, hydrophobic interaction, van der Waals interaction or dipole interaction. In the case of the hydrophobic interaction it is recognized that this is not a per se interaction between the amino acid and ligand, but rather the usual result, in part, of the repulsion of water or other hydrophilic group from a hydrophobic surface. Reducing or enhancing the interaction of the ligand binding site and a ligand can be measured by calculating or testing binding energies, computationally or using thermodynamic or kinetic methods as known in the art.

Chemical modifications will often enhance or reduce interactions of an atom of an amino acid of the ligand binding site and an atom of a ligand. Steric hindrance will be a common means of changing the interaction of the ligand binding cavity with the activation segment.

The present invention also provides methods for identifying compounds that modulate kinase activity. Various methods or combinations thereof can be used to identify these compounds. For example, test compounds can be modeled that fit spatially into the Aurora-A ligand binding site as defined by structure coordinates according to Table B, or using a three-dimensional structural model of Aurora-A, mutant Aurora-A or Aurora-A homolog or portion thereof. Structure coordinates of the ligand binding site, in particular amino acids Q127, W128, R126, L159, F157, E170, L169, V206, Y199, H187, R179, L178. V182, Y199, L188, I184, V252, K250, P282, H280 can also be used to identify structural and chemical features. Identified structural or chemical features can then be employed to design or select compounds as potential Aurora-A modulators. By structural and chemical features it is meant to include, but is not limited to, van der Waals interactions, hydrogen bonding interactions, charge interaction, hydrophobic bonding interaction, hydrophobic interaction and dipole interaction. Alternatively, or in conjunction, the three-dimensional structural model or the ligand binding site can be employed to design or select compounds as potential Aurora-A modulators. Compounds identified as potential Aurora-A modulators can then be synthesized and screened in an assay characterized by binding of a test compound to the Aurora-A.

Examples of assays useful in screening of potential Aurora-A modulators include, but are not limited to, screening in silico, in vitro assays and high throughput assays, for instance, based on phosphorylation of histone H3 as described herein (Crosio, C., Fimia, G. M., Loury, R., Kimura, M., Okano, Y., Zhou, H., Sen, S., Allis, C. D., and Sassone-Corsi, P. (2002). Mitotic phosphorylation of Histone H3: spatio-temporal regulation by mammalian Aurora kinases. Mol. Cell Biol. 22, 874-885).

A preferred method of the invention can be described as a computational method of designing an kinase antagonist from an kinase agonist comprising:
1) determining a structure of a molecular recognition site of said agonist using a three dimensional model of a crystallized protein comprising an Aurora-A, and
2) selecting at least one chemical modification of said agonist that provides a ligand structure that extends beyond a binding site for said agonist and in the direction of at least one protein site important in Aurora-A biological function.

Another preferred method of the invention can be described as a computational method of designing a selective kinase modulator such as an kinase super agonist or antagonist comprising:
1) determining at least one interacting amino acid of an Aurora-A that interacts with at least one first chemical moiety of said ligand using a three dimensional model of a crystallized protein comprising Aurora-A with a bound ligand, and
2) selecting at least one chemical modification of said first chemical moiety to produce a second chemical moiety with a structure to reduce or enhance an interaction between said
interacting amino acid and said second chemical moiety compared to said interaction between said interacting amino acid and said first chemical moiety.

However, as will be understood by those of skill in the art upon this disclosure, other structure based design methods can be used. Various computational structure based design methods have been disclosed in the art.

For example, a number computer modeling systems are available in which the sequence of the Aurora-A and the Aurora-A structure (i. e., atomic coordinates of Aurora-A and/or the atomic coordinates of the active site, the bond and dihedral angles, and distances between atoms in the active site such as provided in Table A and Table B) can be input. This computer system then generates the structural details of the site in which a potential Aurora-A modulator binds so that complementary structural details of the potential modulators can be determined. Design in these modeling systems is generally based upon the compound being capable of physically and structurally associating with Aurora-A. In addition, the compound must be able to assume a conformation that allows it to associate with Aurora-A. Some modeling systems estimate the potential inhibitory or binding effect of a potential Aurora-A modulator prior to actual synthesis and testing.

Methods for screening chemical entities or fragments for their ability to associate with Aurora-A are also well known. Often these methods begin by visual inspection of the active site on the computer screen. Selected fragments or chemical entities are then positioned with the Aurora-A. Docking is accomplished using software such as QUANTA and SYBYL, following by energy minimization and molecular dynamics with standard molecular mechanic forcefields such as CHARMM and AMBER. Examples of computer programs which assist in the selection of chemical fragment or chemical entities useful in the present invention include, but are not limited to, GRID (Goodford, P. J. J. Med. Chem.1985 28: 849-857), AUTODOCK (Goodsell, D. S. and Olsen, A. J. Proteins, Structure, Functions, and Genetics 1990 8: 195-202), and DOCK (Kunts et al. J. Mol. Biol. 1982 161: 269-288).

Upon selection of preferred chemical entities or fragments, their relationship to each other and Aurora-A can be visualized and the entities or fragments can be assembled into a single potential modulator.

Programs useful in assembling the individual chemical entities include, but are not limited to CAVEAT (Bartlett et al. Molecular Recognition in Chemical and Biological Problems Special Publication, Royal Chem. Soc. 78,182-196 (1989)) and 3D Database systems (Martin, Y. C. J. Med. Chem. 1992 35: 2145-2154).

Alternatively, compounds may be designed de novo using either an empty active site or optionally including some portion of a known inhibitor. Methods of this type of design include, but are not limited to LUDI (Bohm H-J, J. Comp. Aid. Molec. Design 1992 6: 61-78) and LeapFrog (Tripos Associates, St. Louis. MO).

Examples of preferred ligands include the above-described indole and indene derivatives of formula (I), and optical isomers, physiologically acceptable salts and prodrugs thereof.

The physiologically acceptable salts in the present case can be acid addition or base addition salts.

For acid addition salts, inorganic acids, such as hydrochloric acid, sulphuric acid, nitric acid or phosphoric acid, or organic acids, in particular carboxylic acids, e.g. acetic acid, tartaric acid, lactic acid, citric acid, malic acid, mandelic acid, ascorbic acid, maleic acid, fumaric acid, gluconic acid or sulphonic acids, e.g. methanesulphonic acid, benzenesulphonic acid and toluenesulphonic acid, and the like are used.

The base addition salts include salts of the compounds of the formula (l) with inorganic bases, such as sodium hydroxide or potassium hydroxide, or with organic bases, such as mono-, di- or triethanolamine.

Prodrugs of the compounds of the formula I are, for example, physiologically easily hydrolysable esters such as alkyl, pivaloyloxymethyl, acetoxymethyl, phthalidyl, indenyl and methoxymethyl esters.

If the compounds according to the invention have asymmetric centres, racemates and optical isomers are included as mixtures or in pure form (enantiomers, diastereomers).

The term "alkyl" includes straight-chain or branched alkyl groups, such as CH₃, C₂H₅, n-propyl, CH(CH₃)₂, n-butyl, CH(CH₃)-C₂H₅, isobutyl, C(CH₃)₃, n-pentyl or n-hexyl, in particular CH₃, C₂H₅ or CH(CH₃)₂, preferably having - if not stated otherwise - 1 to 8, in particular 1 to 6 and particularly preferred 1 to 4 carbon atoms.

The term "alkylene" includes straight-chain or branched alkylene groups, such as methylene and ethylene, preferably having - if not stated otherwise - 1 to 8, in particular 1 to 6 and particularly preferrred 1 to 4 carbon atoms.

The term "alkenylene" includes straight-chain or branched, mono- or polyunsaturated alkylene groups, such as ethenylene, preferably having - if not stated otherwise - 2 to 8, in particular 2 to 6 and particularly preferably 2 to 4, carbon atoms.

The term "alkynylene" includes straight-chain or branched, mono- or polyunsaturated alkylene groups, such as ethynylene, preferably having - if not stated otherwise - 2 to 8, in particular 2 to 6 and particularly preferably 2 to 4, carbon atoms. "Aryl" is in particular a mono- or bicyclic aromatic radical, preferably having 5 to 14 carbon atoms and especially represents naphthyl, indenyl, and in particular phenyl.

"Heteroaryl" is in particular a mono- or bicyclic heteroaromatic radical preferably having 5 to 14 ring atoms and containing 1, 2 or 3 heteroatom(s) independently selected from the group consisting of O, N and S. According to a particular embodiment, bicyclic radicals contain a 5- or 6-membered heteroaromatic radical which is benzo-fused. Heteroaryl includes nitrogen-containing radicals, such as pyrrolyl, imidazolyl, pyrazolyl, pyridazinyl, pyrazinyl, indolyl, quinolinyl, especially pyridyl, pyrimidyl and isoquinolinyl; radicals which contain an oxygen atom or a sulphur atom, such as thienyl, benzothienyl, furanyl and especially benzofuranyl; radicals which contain two or more different heteroatoms, such as thiazolyl, isothiazolyl, thiadiazolyl, isoxazolyl and oxazolyl. Preferred aromatic heterocyclic radicals are, pyridyl and indolyl.

The term "arylene" is preferably naphthylene and in particular phenylene, such as 1,4-phenylene.

According to a preferred embodiment, one of residues R¹ and R², preferably residue R², is hydrogen while the other, preferably R¹, has a meaning different from hydrogen. In this context, alkylene radicals preferably contain a relatively short main chain connecting the indole or indene moiety with the amino, oxy or thio moiety (-NHR⁸, -OR⁸ or -SR⁸, respectively). Accordingly, the main chain of preferred alkylene radicals contain 1 to 3 carbon atoms. Said alkylene radicals may be branched. Linear alkylene radicals are preferred. Specific examples of alkylene radicals include methylene, eth-1,1-ylene, prop-1,1-ylene and prop-2,2-ylene, with methylene being preferred.

According to a particularly preferred embodiment, one of residues R¹ and R², preferably R², is hydrogen and the other, preferably R¹, represents alkylene-NHR⁸.

R⁸ preferably represents an acyl radical so as to form an amide, ester or thioester bond within residue R¹ and/or R². It is preferred that residue R⁸ represents an amino acid selected from the group consisting of aspartate (asp), glutamate (glu), and cysteine (cys), i.e., n and m are zero, or a peptide connected via aspartate, glutamate or cysteine, i.e., aa represents an amino acid radical, and at least one of n and m is an integer of 1 or higher, wherein the amino acid radicals can be the same or different. The aspartate, glutamate and cysteine radicals may be bonded to the alkylene radical via any of their functional groups. The aspartate and glutamate radicals are preferably bonded via any of its carboxy groups, the β-carboxy function of aspartate and the γ-carboxy function of glutamate being preferred. Cystein radicals are preferably bonded via their side chain thio functionality. Accordingly, the amino acid radicals aa are connected to the alkylene group via the remaining functional groups of the aspartate, glutamate or cysteine radicals. According to a preferred embodiment, the remaining functional groups are the α-amino group and the α-carboxy group, of which one or both can be bonded to the amino acid radicals aa. According to this embodiment, the connecting aspartate, glutamate or cystein radical is itself part of the peptide chain which is connected to the alkylene residue via the side chain of said aspartate, glutamate or cystein radical. A specific example of such a preferred residue R⁸ is a radical of the formula (II) 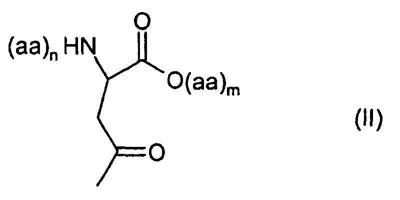 wherein
- aa: represents an amino acid radical;
- n: is zero or an integer of 1 to 10; and
- m: is zero or an integer of 1 to 10.

A specific example of such preferred residues R1 and/or R2 is thus the radical of formula (III) 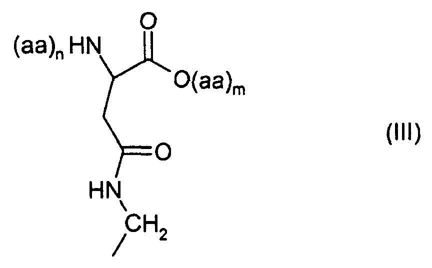 wherein
- aa: represents an amino acid radical;
- n: is zero or an integer of 1 to 10; and
- m: is zero or an integer of 1 to 10.

The amino acid radicals aa may be any amino acid. Preferably said amino acid radicals are selected to mimick - together with the connecting amino acid - a portion of TPX2 that binds to Aurora-A. Said portion of TPX2 is in particular a portion comprising the amino acid sequence from residues 7 to 21, preferably 8 to 19 (compare Fig. 3C) or a fragment thereof. Accordingly, suitable peptides may have the sequence Tyr-Xaa1-Tyr-Xaa2-Ala-Pro-Xaa3-Xaa4-Phe-Xaa5-Xaa6-Phe or a portion thereof, wherein Xaa1-6 may be any amino acid radical. Xaa1 preferably is a serine radical or a similar amino acid. Xaa2 preferably is an aspartate radical or a similar amino acid. Xaa3 preferably is a serine radical or a similar amino acid. Xaa4 preferably is an aspartate radical or a similar amino acid. Xaa5 preferably is an isoleucine radical or a similar amino acid. Xaa6 preferably is an asparagine radical or a similar amino acid. A particularly preferred portion is the sequence Tyr-Xaa1-Tyr-Xaa2-Ala-Pro-Xaa3-Xaa4-Phe. By similar amino acid is meant an amino acid that is considered to result in a conservative change of the peptide's structure when it replaces the amino acid to which it is similar. For instance, aspartate is similar to glutamate. The peptide preferably has a lenght of 3 to 15, more preferably of 4 to 10 and advantagously of 5 to 8 amino acid, the sum of n + m thus being 2 to 14, more preferably of 3 to 9 and advantagously of 4 to 7, respectively.

R³ preferably is a radical different from hydrogen and advantageously represents alkylene-R⁹, alkenylene-R⁹, or alkynylene-R⁹. In this context, the main chain connecting the indene or indole moiety with residue R⁹ preferably is relatively long. Accordingly, it is preferred that the main chain of the alkylene radical, alkenylene radical or alkynylene radical contains 3 to 8, e.g. 4, carbon atoms. Said alkylene radical, alkenylene radical and alkynylene radical may be branched. Linear radicals are preferred. Specific examples of said radicals include prop-1,3-ylene, but-1,4-ylene, pent-1,5-ylene and hex-1,6-ylene, with but-1,4-ylene being preferred.

R⁹ preferably represents aryl or heteroaryl. Specific examples of aryl and heteroaryl radicals include phenyl, naphthyl, indenyl, pyridyl, and indolyl, with pyridyl, e.g. pyrid-2-yl, being preferred.

A specific example of such a preferred residue R³ is a radical of the formula (IV)

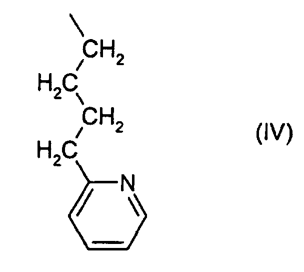

While residues R⁴, R⁵, R⁶ and R⁷ all may be hydrogen, it is preferred that at least one of said residues, especially R⁶, is different from hydrogen.

R⁵ and/or R⁶ preferably represent OR¹⁰, NHR¹⁰, SR¹⁰, or alkylene-R¹⁰. In this context, the preferred embodiments regarding the alkylene radical are those as described in connection with residues R¹ and R².

R¹⁰ preferably represents aryl or substituted aryl. In this context, aryl is preferably phenyl. Substituted aryl, in general, contains 1, 2 or 3 substituents which may be the same or different, mono-substitution being preferred. The substituents are preferably selected from the group consisting of hydroxy, -OPO₃H₂, -CH₂PO₃H₂, -CF₂PO₃H₂, -COOH, -CH(COOH)₂, -OPO₃(R¹¹)₂, -CH₂OPO₃(R¹¹)₂, -CF₂PO₃(R¹¹)₂, -COOR¹¹, and -CH(COOR¹¹)₂, wherein R¹¹ is a radical that is cleavable *in vivo,* converting the carboxylic acid esters, phosphate and phosphonate esters to carboxylates, phosphates or phosphonates, respectively. Suitable examples of R¹¹ residues are alkyl, CH₂OCO-alkyl, and C₂H₄-S-CO-alkyl. Especially preferred substituents are selected from the group consisting of -OPO₃H₂, -CH₂PO₃H₂, -CF₂PO₃H₂, -OPO₃(R¹¹)₂, -CH₂OPO₃(R¹¹)₂, and -CF₂PO₃(R¹¹)₂,. A preferred example of a R¹¹ residue is -CH₂OCO-alkyl, alkyl being linear or branched and having 1 to 6 carbon atoms, e.g. methyl.

A specific example of such preferred residues R5 and/or R6 is the radical of formula (V)

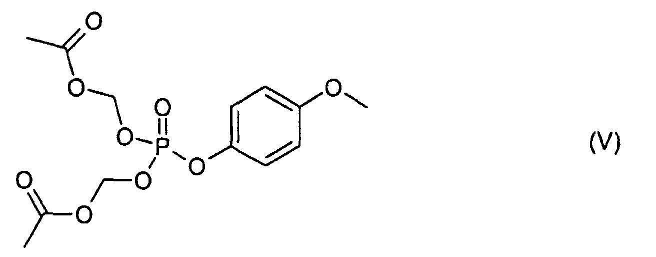

According to a specific embodiment, the present invention relates to indole derivatives of formula (Ia) 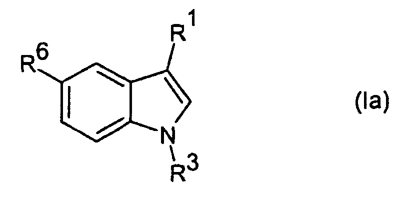 wherein
R¹, R³ and R⁶ are defined as above,
and optical isomers, physiologically acceptable salts and prodrugs thereof.

A preferred indole derivative of the present invention has the formula (1) 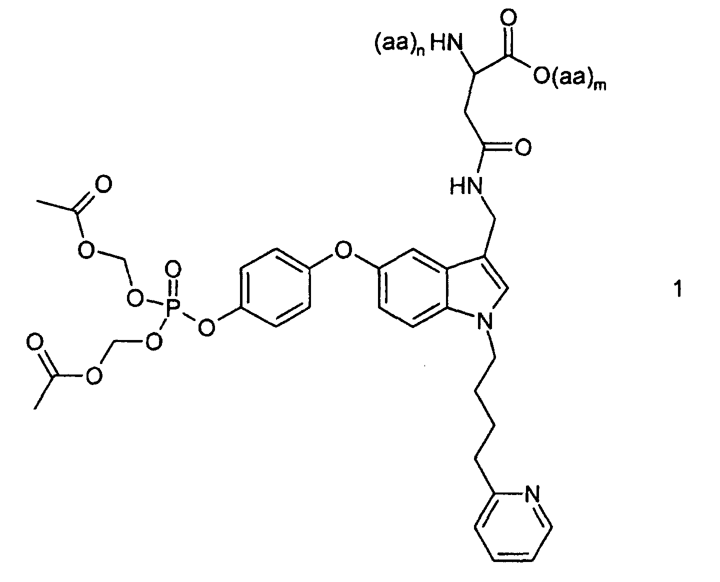 wherein
aa, n and m are defined as above.

The indene and indole derivatives of formula (I) can be prepared by methods well-known to those skilled in the art.

The present invention is further directed to a method for treating cancer, in particular breast and colon carcinomas, comprising administering an effective amount of an Aurora-A modulator, such as the indole and indene derivatives as described herein, preferably an antagonist or partial antagonist, identified by a computational process of the invention.

The compounds according to the invention are thus suitable for the treatment of disorders in which the interaction of Aurora-A with TPX2 is responsible for the formation or the progressive course of these disorders. In particular, the compounds according to the invention can be used for the treatment of cancer.

The compounds according to the invention can either be administered as individual therapeutic active compounds or as mixtures with other therapeutic active compounds: they can be administered as such, but in general they are administered in the form of pharmaceutical compositions, i.e. as mixtures of the active compounds with pharmaceutically acceptable excipients, in particular vehicles or diluents and/or additives. The compounds or compositions can be administered enterally, e.g. orally or rectally, or parenterally, e.g. subcutaneously, intravenously or intramuscularly.

The nature of the pharmaceutical composition and of the pharmaceutical carrier or diluent depends on the desired manner of administration. Oral compositions can be present, for example, as tablets or capsules and can contain customary excipients, such as binding agents (e.g. syrup, acacia, gelatin, sorbitol, tragacanth or polyvinylpyrrolidone), fillers (e.g. lactose, sugar, maize starch, calcium phosphate, sorbitol or glycine), lubricants (e.g. magnesium stearate, talc, polyethylene glycol or silica), disintegrating agents (e.g. starch) or wetting agents (e.g. sodium laurylsulphate). Oral liquid preparations can be present in the form of aqueous or oily suspensions, solutions, emulsions, syrups, elixirs or sprays etc. or can be present as dry powders for reconstitution with water or another suitable carrier. Liquid preparations of this type can contain customary additives, for example suspending agents, flavourings, diluents or emulsifiers. For parenteral administration, solutions or suspensions with customary pharmaceutical carriers can be employed.

The following examples are to illustrate the invention, but should not be interpreted as a limitation thereon.

### Examples

### Experimental Procedures

### Protein and Antibody Preparation

AuroraΔN was cloned in pET M11 (residues 122-403, wild-type and D274N) were expressed in CodonPlus RIL *E.coli* (Stratagene) and purified with TALON resin (Clontech) using manufacturer's instructions. AuroraΔN was treated with TEV protease to remove the His tag, passed through a TALON column to remove TEV and then purified to homogeneity by size exclusion chromatography. Human and *Xenopus* GST TPX2 fragments were expressed in BL21(DE3) *E.coli.* TPX2 proteins were purified using glutathione sepharose (Pharmacia). To form the AuroraΔN-TPX2 complex, cell lysate from *E.coli* expressing GST TPX2 was passed through a glutathione sepharose column, washed, and then purified AuroraΔN was passed through the same column. Column resin was incubated with TEV protease to elute the complex, which was further purified by size exclusion chromatography.

The polyclonal antibody against full-length TPX2 was produced in rabbits with bacterially expressed GST TPX2 (Wittmann et al., 2000) and affinity purified. The 1C1 monoclonal anti-*Xenopus* Aurora-A is described in: Giet R, Uzbekov R, Cubizolles F, Le Guellec K, Prigent C., J Biol Chem. (1999) 274, 15005-13. The polyclonal anti-human Aurora-A was obtained by injecting rabbits with bacterially expressed full-length human Aurora-A.

### HeLa cell extract and Xenopus egg extract preparation

HeLa cells (CCL2; ATCC, Manassas, VA) were grown in 10% fetal calf serum (FCS) and 2 mM L-glutamine in modified Eagle's medium at 37°C and 5% CO₂. Cells were arrested in S phase using a double thymidine (2 mM) block as described (Stein, 1994) and released from S phase by washing away the thymidine. After 10 h, cells were trypsinized, incubated for 10 min in ice-cold lysis buffer (25 mM Tris pH 7.6, 200 mM NaCl, 1% Triton, protease inhibitors), and centrifuged for 10 min at 15,000 g. *Xenopus* cytostatic factor arrested extracts (CSF extracts) were prepared as described (Murray, 1991).

### In vitro kinase assay

Full-length TPX2, TPX2(1-43) or TPX2(15-43) at 4 µM were incubated with full length Aurora-A or AuroraΔN in kinase buffer (20 mM Hepes pH 7.5, 200 mM KCI, 5 mM MgCl2, 0.5 mM EGTA, 1 mM DTT, 0.05 % Triton X-100, 50 µM ATP) containing γ³²P-ATP (Amersham) in the presence or absence of histone H3 (0.2 mg/ml, Roche), for 15 min at 25°C. For cleavage, GST TPX2(1-43) was incubated with TEV 16-18h at 4° C (Figure 2C) or by adding TEV after the kinase reaction and incubating 10 min at 30° C (Figure 1D). In Figure 2D, AuroraΔN (8µM) was incubated in kinase buffer containing histone H3 and γ³²P-ATP for 15 min at 25° C. After separation by SDS-PAGE, phosphorylated histone H3 and TPX2 proteins were detected by autoradiography.

### Phosphatase assay

Active human Aurora-A (2 µM) was incubated with TPX2, TPX2(1-43) or TPX2(15-43) (3 µM) for 10 min at 4°C. Samples were diluted in phosphatase buffer (50 mM Tris-HCl pH 7, 0.1 mM EDTA, 1 mM MnCl₂, 5 mM caffeine, 5 mM DTT, 0.025% Tween 20) and incubated in the absence or presence of PP1 (α isoform, NEB) for 0.5h at 30°C. Afterwards proteins were separated by SDS-PAGE and analyzed by Western blotting with anti-Aurora-A or anti-phospho-Aurora-A (Cell Signalling, 1:10000 dilution).

### lmmunoprecipitation

For coating beads with anti-GST, 6 µg of antibody were incubated with 20 µl of protein A-conjugated Dynabeads 280 (Dynal) in a total volume of 100 µl PBS-T (PBS, 0.1 % Triton X-100), for at least 1 h at 4°C. The beads were washed twice with PBS-T and twice with HeLa cells lysis buffer or CSF-XB (10 mM Hepes pH 7.7, 50 mM sucrose, 100 mM KCl, 2 mM MgCl₂, 0.1 mM CaCl₂ and 5 mM EGTA). 50 µl extract was incubated with GST, GST TPX2 1-43, GST TPX2 15-43, GST TPX2 *Xenopus* or GST TPX2(1-39) *Xenopus* proteins (final concentrations: 250 nM *Xenopus;* 1 µM HeLa) in the absence or presence of RanQ69L-GTP (16 µm) for 15 minutes at 20 °C and then added to the beads. After 1 h incubation on ice, the beads were washed twice with lysis buffer or CSF-XB, washed twice with PBS-T and boiled in SDS-PAGE sample buffer. The samples were then subjected to gel electrophoresis and analyzed by Western blotting with anti-GST, anti-*Xenopus* TPX2, anti-*Xenopus* Aurora-A or anti-human Aurora-A.

### In vitro pull-down assays

GST or GST TPX2(1-43) (240 µg) was bound to 100 µl glutathione sepharose (Pharmacia) in 1ml binding buffer (PBS, 0.05% Tween-20, 2 mM DTT) for 0.5 h. Beads were washed three times, divided into four equal quantities in 1 ml binding buffer and 35 µg full-length human Aurora-A, AuroraΔN or AuroraΔN(D274N) was added. After binding for 0.5 h at 4°C, the resin was washed four times, then incubated overnight in 50 µl binding buffer plus 2 µg TEV protease. 10 µl supernatant from each was analysed by SDS-PAGE. TEV cleavage was necessary because AuroraΔN and GST TPX 1-43 co-migrate by SDS-PAGE.

### Crystallographic methods

Needle-like crystals of AuroraΔN in complex with TPX2 1-43, approximately 10 µm in thickness, were grown at 18°C by vapour diffusion using 18% (w/v) PEG8000, 100 mM MES pH 6.5, 200 mM MgSO₄ as well buffer and hanging drops comprising a 1:1 mix of 20 mg/ml complex pre-mixed with 2 mM ATPγS and 0.2 mM MgSO₄, and 20% PEG8000, 100 mM MES pH 6.5, 200 mM MgSO₄. For cryo-protection, the drop buffer was supplemented with 17.5% (v/v) glycerol. Hexagonal prism crystals of the wild-type AuroraΔN kinase alone, approximately 50µm in all dimensions, were grown by vapour diffusion using 20% PEG300, 5% PEG8000, 100 mM Tris 8.5, 10% glycerol as well buffer and sitting drops comprising a 1:1 mix of well buffer and 9 mg/ml protein pre-incubated with 2 mM ATPγS and 2 mM MgSO₄. Diffraction data were collected at 100K and processed using the CCP4 suite of programs (CCP4, 1994). Structures were solved by molecular replacement using CNS (Brunger et al., 1998) and the coordinates of the cAMP-dependent protein kinase catalytic subunit as an initial model (Mashhoon et al., 2001). CNS was used for refinement and O (Jones et al., 1991) for model building. The statistics for data collection and refinement are shown in Table 1. Structure figures were prepared using PyMOL (DeLano, 2002). Coordinates and structure factors have been deposited in the Protein Data Bank with accession codes 1ol7 and 1ol5 for phosphoryated AuroraΔN alone and in complex with TPX2(1-43), respectively.

### Mapping of a minimal domain of TPX2 sufficient for binding and activating Aurora-A

A minimal domain of TPX2 sufficient to interact with Aurora-A was mapped. The Aurora-A binding domain of TPX2 resides within its N-terminal 150 residues. Inspection of a sequence alignment of TPX2 shows that only a third of this region is conserved across species (residues 1-43 in human TPX2, 1-39 in *Xenopus* TPX2). This fragment is able to co-immunoprecipitate Aurora-A from *Xenopus* egg extracts to the same extent as the full-length protein (Figure 1A, lanes 6 and 4 respectively). In the egg extracts, efficient binding of TPX2 to Aurora-A requires Ran locked into its GTP-bound form (RanQ69L), which releases TPX2 from a complex with importin α/β. *Xenopus* TPX2(1-39) binds Aurora-A in a RanGTP-independent manner (Figure 1A, lanes 5 and 6), consistent with it lacking the portion of TPX2 regulated by Ran. The corresponding fragment of human TPX2 (residues 1-43) is able to interact with Aurora-A using pull-down experiments in HeLa extract, while a shorter construct encompassing residues 15-43 is unable to do so (Figure 1B, lanes 2 and 3 respectively).

The minimal Aurora-A binding domain of human TPX2(1-43) is sufficient to stimulate the activity of the kinase *in vitro* (Figure 1C). Phosphorylation of histone H3, an Aurora-A substrate, is markedly increased by the addition of TPX2(1-43) (Figure 1C compare lane 1 with lane 3), but not by the shorter TPX2(15-43) (lane 4). Full-length TPX2 also increases the activity of the kinase, but appears less effective than the fragment. Full-length TPX2 may require additional factors such as microtubules for full activation. While microtubules can stimulate TPX2 activation of Aurora-A, no enhancement is detected for TPX2 1-43, which does not bind microtubules. Full-length TPX2 is itself a substrate for Aurora-A. The phosphorylation observed for GST-fused TPX2(1-43) (Figure 1C, lane 3) is however likely a non-specific artifact of the *in vitro* reaction because upon cleavage of the fusion protein phosphorylation signal is also detected on GST (lane 2). Furthermore, mass spectroscopy does not reveal Aurora-A phosphorylation sites in full-length TPX2.

The presence of TPX2(1-43) protects Aurora-A from deactivating dephosphorylation (Figure 1E). In the absence of TPX2, Aurora-A is completely dephosphorylated by phosphatase PP1 (lanes 1 and 2). In the presence of full-length TPX2, PP1 treatment dephosphorylates most sites of Aurora-A (lanes 3 and 4, upper panel) but prevents dephosphorylation of Thr288^{AUR} (lane 4, lower panel, arrow). An even stronger protection effect is observed upon addition of TPX2(1-43) (lane 6). In the presence of TPX2 15-43, Aurora-A is fully dephosphorylated as in the absence of TPX2 altogether (lanes 8 and 2), consistent with the shorter fragment being unable to bind Aurora-A (Figure 1B). Thus, residues 1-43 of human TPX2 are necessary and sufficient for Aurora-A binding, activation and protection from dephosphorylation.

### Overall structure of TPX2-bound Aurora-A

For crystallisation purposes, a minimal functional complex of Aurora-A and TPX2 was focused on. The N-terminus of Aurora-A is overall poorly conserved across species and is not required to bind TPX2. A fragment of Aurora-A lacking the N-terminus (residues 122-403, AuroraΔN) interacts with the active fragment of TPX2 (1-43) as efficiently as the full-length kinase (Figure 2A lanes 5 and 4). Both fragments are highly conserved (Figure 2D, 2E) and indeed *Xenopus* TPX2(1-39) can bind human AuroraΔN. AuroraΔN is phosphorylated when expressed in *E. coli,* as detected by a phospho-specific antibody (Figure 2B, lane 1). This appears to be due to Aurora-A autophosphorylation rather than to the activity of a bacterial kinase, since no phosphorylation can be detected upon expressing a mutant where an important catalytic residue, Asp274^{AUR}, has been mutated to Asn (Figure 2B, lane 2). The unphosphorylated mutant is catalytically inactive but retains TPX2-binding activity (Figure 2A, lane 6). AuroraΔN is activated by TPX2(1-43) (Figure 2C, lanes 2,3).

The complex between human phosphorylated AuroraΔN and TPX2(1-43) is active in histone H3 phosphorylation (Figure 2C, lane 7). The complex was crystallized in the presence of Mg²⁺ ions and the ATP analogue ATPγS. The structure was determined by molecular replacement and refined it using 2.5 A resolution data to a *R*free of 25.2% (see Table 1). The polypeptide model includes the catalytic core of the kinase (residues 123-387^{AUR}) and two segments of TPX2 (residues 7-21 ^{TPX} and 30-43^{TPX}). No ordered electron density is present for the eight intervening residues of TPX2 (22-29^{TPX}), which have therefore not been modelled.

The kinase catalytic core has an overall bilobate structure (Figure 3A,B). Briefly, the N-terminal lobe (residues 123-210) consists of a β-sheet and two α-helices, including the prominent helix αC whereas the C-terminal and larger lobe (residues 217-387) is mostly α-helical. The active site is situated at the interface between the lobes and includes the ATP-binding site, the catalytic base (Asp256^{AUR}) and the kinase activation segment (residues 274-299^{AUR}). In contrast to the structure of unphosphorylated Aurora-A, the activation segment is well ordered in the electron density and includes two phosphorylated threonine residues (Thr287^{AUR} and Thr288^{AUR}). Although the crystals were formed in the presence of ATP-γS, the electron density for the nucleotide reveals only the adenosine with two ordered phosphates and has been modelled as an ADP.

### Specific recognition of TPX2 at two sites on Aurora-A

TPX2 binds Aurora-A with two separate stretches recognized at two distinct sites on the kinase. The upstream stretch (residues 7-21^{TPX}) binds at the N-terminal lobe of Aurora-A (Figure 3A,B). The downstream stretch (residues 30-43^{TPX}) binds in a α-helical conformation between the N- and C-terminal lobes (Figure 3A,B). The two Aurora-A-binding motifs of TPX2 appear to be connected by a flexible linker (disordered in the structure) that is variable in length and sequence across species (Figure 2D). Additionally, the two stretches are connected by an intramolecular hydrogen bond between Asp11^{TPX} and Trp34^{TPX}.

The upstream stretch of TPX2 has a mostly extended conformation, with a kink in the middle induced at a proline residue (Pro13^{TPX}) (for details see Figure 3C). The conserved segment ⁸YSYDAPS¹⁴ (Figure 2D) is engaged in extensive main-chain and side-chain interactions with Aurora-A. In particular, Tyr8^{TPX} , Tyr10^{TPX} and Ala12^{TPX} tightly nestle into a hydrophobic groove between the β-sheet, helix αB and helix αC. An adjacent hydrophobic groove accommodates the side chains of TPX2 residues from Phe16^{TPX} to Phe19^{TPX}. The N-terminal residues of the Aurora-A catalytic core make key contributions to this interface, in particular with Arg126^{AUR} forming a cation-π interaction with Phe16^{TPX}.

The downstream helical stretch of TPX2 interacts with both helix αC and the activation segment of Aurora-A, bridging them (see Figure 3D for details). Most prominently, two conserved aromatic residues (Trp34^{TPX} and Phe35^{TPX}) interact with His187^{AUR} and His280^{AUR}, and Ala39^{TPX} additionally contacts the activation segment at Pro282^{AUR}. While the side of the TPX2 helix in contact with the kinase is lined by hydrophobic and conserved residues, the opposite side exposed to solvent comprises hydrophilic and variable residues. Residues 40-43^{TPX} at the end of the helix assume an extended conformation and are involved in contacts with a symmetry-related Aurora-A molecule within the crystal lattice.

TPX2 is phosphorylated in mitotic extracts and may well be regulated by phosphorylation. Even if TPX2 was phosphorylated in the first 43 residues, examination of the crystal structure suggests that the interaction of TPX2 with Aurora-A is unlikely to be regulated this way because all the conserved serine residues point towards solvent.

### Phosphorylated Aurora-A is in an active conformation when bound to TPX2

Comparison with other kinase structures reveals that phosphorylated TPX2-bound Aurora-A closely matches the conformation of kinases in the active conformation. Using the program DALI (Holm and Sander, 1993), it was found particularly similar to cAMP-dependent protein kinase (PDB code 1YDS, 1.3 Å r.m.s.d., 257 structurally equivalent residues). Structural comparison shows that all the conserved residues at the active site are correctly oriented for catalysis (Figure 4A). These include the positively-charged residue aligning the phosphates for catalysis (Lys162^{AUR}, equivalent to Lys72^{cAPK}), the negatively-charged residue coordinating the magnesium ion bound to the nucleotide (Asp274^{AUR}, equivalent to Asp184^{cAPK}), and the catalytic base (Asp256^{AUR}, equivalent to Asp166^{cAPK}) whose role is to transfer the γ-phosphate of ATP to the hydroxyl group of a substrate serine or threonine residue.

The phosphorylated activation segment of TPX2-bound Aurora-A is in a conformation typical of active Ser/Thr kinases. It is virtually superposable to that of active cAPK (Figure 4A), with an r.m.s.d. of 0.8 Å between the Cα atoms of residues 274-299^{AUR} and 184-208^{cAPK}. In cAPK, the phosphorylated Thr197^{cAPK} interacts with Arg165^{cAPK} and with a basic surface patch (His87^{cAPK} and Lys189^{cAPK}), influencing the preceding catalytic residue (Asp166^{cAPK}). In Aurora-A, the phosphoryl moiety of Thr288^{AUR} is at the same structural position and interacts with the corresponding Arg255^{AUR} and with a similar, though not identical, basic patch (Arg180^{AUR} and Arg286^{AUR}), linking the catalytic residue (Asp256^{AUR}) to helix αC and to the activation segment. Mutation of Thr288^{AUR} to Asp generates a protein more active than unphosphorylated wild-type Aurora-A, but much less active than the phosphorylated wild-type kinase. This mutation is thus a poor mimic of phosphorylation at Thr288^{AUR}, and although a Glu may potentially produce a stronger effect by more closely approximating the position of the phosphate, it would also be inadequate to balance the positive charges of the three surrounding arginines.

### The phosphorylated activation segment of Aurora-A is in an inactive conformation in the absence of TPX2

To address the contribution of TPX2 to the conformation at the active site of TPX2-bound Aurora-A, the structure of phosphorylated AuroraΔN has been determined in the absence of TPX2. The model includes residues 127-389^{AUR} and has been refined at 2.75 Å resolution to an Rfree of 29.6% (Table 1). The overall structure of phosphorylated Aurora-A is similar for the TPX2-bound and unbound forms. The two lobes of the kinase have the same relative orientation and helix αC does not change conformation upon binding the regulator (Figure 4A). Overall, more than 95% of the amino-acid residues superpose with an overall r.m.s.d. of 0.9 Å. However, the two structures differ significantly in the activation segment.

The phosphorylated activation segments of Aurora-A in the TPX2-bound state (Figure 4A) and unbound state (Figure 4A) diverge between residues His280^{AUR} and Leu293^{AUR} (rmsd of 5.4 Å). In the absence of TPX2, the phosphorylated Thr288^{AUR} is exposed to solvent rather than pointing towards Arg255^{AUR} as observed when TPX2 is bound (Figure 4A). The region of the activation segment around the phosphorylated Thr288^{AUR} (residues 286-291^{AUR}) is relatively disordered, with average B-factors of 85 Å² as compared with 53 Å² for the rest of the molecule. However, the tracing of the polypeptide chain is unambiguous and this fragment of the activation segment is overall less closely packed to the C-terminal lobe of the kinase. The segment becomes well ordered again at Thr292^{AUR} and Leu293^{AUR}. This part of the activation segment is in a different conformation with respect to the TPX2-bound form (Figure 4A), with Thr292^{AUR} being unable to hydrogen bond and thus influence the catalytic Asp256^{AUR} (Figure 4A). This conformation seems to be stabilized by the movement of Leu293^{AUR}, which inserts its side chain into a small apolar pocket. The presence of a Leu at this position is uncommon in other kinases, which typically have a Pro residue instead (cAPK, for example, Figures 2E). A further movement of residues 298-306 accommodates changes in the adjacent activation segment, and leads to the formation of a Glu302-His366 interaction. This interaction may be affected by the negative charge introduced in the S349D mutant of *Xenopus* Aurora-A explaining why this mutant is catalytically inactive.

### Structural basis for the activation of Aurora-A by TPX2

Comparison of phosphorylated Aurora-A in the unbound and TPX2-bound forms reveals the molecular basis for the enhancement of kinase activity. In the absence of TPX2, the crucial phosphothreonine does not bind to helix αC and the activation segment overlaps with the substrate-binding site rather than providing a substrate-binding platform. The phosphorylated Thr288^{AUR} has no direct connection with TPX2, the phosphate moiety being 14 Å from the nearest TPX2 atom. Nonetheless, TPX2 induces a 10 Å movement of the phosphoryl moiety required to achieve the active conformation (Figure 4B).

The helical stretch of TPX2 contacts the activation segment at His280^{AUR} and Pro282^{AUR}, which appear to be the pivot points of the conformational change (Figures 4B, 4C). In essence, a rotation about His280^{AUR} swings Pro282^{AUR} and Ser283^{AUR} towards the TPX2 helix, pulling downstream residues of the activation segment to pack more closely with the kinase core (Figure 4B). In this lever-arm-like mechanism, a small change at the pivot points (His280^{AUR}, Pro282^{AUR}) produces a large movement in the arm (P-Thr288^{AUR} Figure 4C). While in the unbound form the activation segment is rather mobile and has probably sufficient conformational freedom to adopt an active conformation in the presence of substrate (basal kinase activity, Figure 1C, lane 1), in the TPX2-bound form, the active conformation is ready for substrate binding and catalysis (increased kinase activity, Figure 1C, lanes 2 and 3). The C-terminal residues (40-43^{TPX}) protruding from a neighbouring TPX2 molecule in the crystals bind to the activation segment, albeit in the opposite main chain direction to a real substrate. Substrate binding alone does not fully activate Aurora-A in solution (Figure 1C), and it is therefore very unlikely that this substrate-like contact is responsible for the active conformation in the crystal structure. Substrate binding, however, might assist in inducing an active conformation, especially in the absence of TPX2.

In addition to enhancing the activity of Aurora-A, TPX2 also protects the kinase from dephosphorylation by phosphatases. The site for PP1 binding on Aurora-A is mapped to the C-terminus, a region that is distant from the TPX2 binding site and that is structurally unaffected upon binding of the regulator. Thus TPX2 is unlikely to protect Aurora-A from dephosphorylation by preventing phosphatase binding. Indeed, even in the presence of TPX2, the phosphatase is capable of removing the phosphates from most side chains with the exception of Thr288^{AUR} (Figure 1E). TPX2 prevents Thr288^{AUR} dephosphorylation by moving the phosphate moiety from a solvent-exposed position to a buried position, which is inaccessible to an incoming phosphatase. The other phosphorylated threonine in the kinase catalytic core, Thr287^{AUR}, is conspicuously exposed to solvent and may serve as a decoy to further mask Thr288^{AUR} (Figure 3B).

**Table 1.**

| **Summary of crystallographic analysis** | | | |
|---|---|---|---|
| | | Phospho-Aurora-A + TPX2 | Phospho-Aurora-A |
| **Crystals** | | | |
| Spacegroup | | *P*2₁2₁2₁ | *P*6₁22 |
| Lattice constants | *a* (Å) | 59.63 | 81.18 |
| | *b* (Å) | 81.72 | 81.18 |
| | *c* (Å) | 83.05 | 169.62 |

| **Data collection** | | | |
|---|---|---|---|
| X-ray source | | SLS XO6SA | ESRF ID14EH1 |
| Resolution range (Å) | | 40-2.5 | 70-2.75 |
| (Highest resolution shell) | | (2.64-2.5) | (2.9-2.75) |
| Unique reflections | | 14609 | 9235 |
| Completeness(%) | | 100 (100) | 100 (100) |
| Multiplicity | | 5.6 (5.7) | 10.1 (10.6) |
| Rmerge (%) | | 9.9 (24.8) | 11.9 (34.6) |
| I/σ(I) | | 5.1 (1.1) | 3.8(2.1) |

| **Refinement** | | | |
|---|---|---|---|
| Resolution range (Å) | | 40-2.5 | 20-2.75 |
| Number of residues | | 294 | 263 |
| Number of waters | | 144 | 9 |
| Rfactor (%) | | 19.4 | 25.7 |
| Rfree^{a} (%) | | 25.2 | 29.6 |

| **Ramachandran plot** | | | |
|---|---|---|---|
| Most favoured (%) | | 91.5 | 82.4 |
| Allowed (%) | | 8.1 | 16.3 |
| Generously allowed (%) | | 0.0 | 0.9 |
| Forbidden (%) | | 0.4^{b} | 0.4^{b} |

| | | | |
|---|---|---|---|
| ^{a} Free Rfactor was computed using 5% of the data assigned randomly (Brunger, 1992). | | | |
| ^{b} Ser226^{AUR} resides in a loop and the conformation is supported by excellent electron density. | | | |

**Table A**

| | | | | | | | | | | | |
|---|---|---|---|---|---|---|---|---|---|---|---|
| ATOM C | 1 | CB | GLN | A | 127 | 267.519 | -61.189 | 87.734 | 1.00 | 66.58 | A |
| ATOM C | 2 | CG | GLN | A | 127 | 266.971 | -61.391 | 86.330 | 1.00 | 76.29 | A |
| ATOM C | 3 | CD | GLN | A | 127 | 266.372 | -60.121 | 85.741 | 1.00 | 79.34 | A |
| ATOM O | 4 | OE1 | GLN | A | 127 | 265.589 | -60.183 | 84.781 | 1.00 | 82.34 | A |
| ATOM N | 5 | NE2 | GLN | A | 127 | 266.735 | -58.962 | 86.307 | 1.00 | 82.40 | A |
| ATOM C | 6 | C | GLN | A | 127 | 269.192 | -59.883 | 89.051 | 1.00 | 63.35 | A |
| ATOM O | 7 | O | GLN | A | 127 | 269.877 | -58.853 | 89.024 | 1.00 | 70.04 | A |
| ATOM N | 8 | N | GLN | A | 127 | 269.910 | -61.949 | 87.808 | 1.00 | 58.04 | A |
| ATOM C | 9 | CA | GLN | A | 127 | 269.002 | -60.755 | 87.810 | 1.00 | 67.74 | A |
| ATOM N | 10 | N | TRP | A | 128 | 268.566 | -60.307 | 90.137 | 1.00 | 61.21 | A |
| ATOM C | 11 | CA | TRP | A | 128 | 268.621 | -59.552 | 91.366 | 1.00 | 53.96 | A |
| ATOM C | 12 | CB | TRP | A | 128 | 267.315 | -59.733 | 92.133 | 1.00 | 50.38 | A |
| ATOM C | 13 | CG | TRP | A | 128 | 266.140 | -59.256 | 91.369 | 1.00 | 49.11 | A |
| ATOM C | 14 | CD2 | TRP | A | 128 | 265.908 | -57.928 | 90.897 | 1.00 | 50.34 | A |
| ATOM C | 15 | CE2 | TRP | A | 128 | 264.697 | -57.962 | 90.150 | 1.00 | 53.33 | A |
| ATOM C | 16 | CE3 | TRP | A | 128 | 266.611 | -56.713 | 91.017 | 1.00 | 53.44 | A |
| ATOM C | 17 | CD1 | TRP | A | 128 | 265.096 | -60.014 | 90.921 | 1.00 | 47.17 | A |
| ATOM N | 18 | NE1 | TRP | A | 128 | 264.228 | -59.249 | 90.191 | 1.00 | 49.20 | A |
| ATOM C | 19 | CZ2 | TRP | A | 128 | 264.160 | -56.816 | 89.514 | 1.00 | 56.77 | A |
| ATOM C | 20 | CZ3 | TRP | A | 128 | 266.094 | -55.571 | 90.392 | 1.00 | 60.23 | A |
| ATOM C | 21 | CH2 | TRP | A | 128 | 264.869 | -55.633 | 89.639 | 1.00 | 61.57 | A |
| ATOM C | 22 | C | TRP | A | 128 | 269.787 | -59.909 | 92.262 | 1.00 | 54.43 | A |
| ATOM O | 23 | O | TRP | A | 128 | 270.317 | -61.026 | 92.231 | 1.00 | 54.76 | A |
| ATOM N | 24 | N | ALA | A | 129 | 270.184 | -58.955 | 93.085 | 1.00 | 55.48 | A |
| ATOM C | 25 | CA | ALA | A | 129 | 271.283 | -59.206 | 94.006 | 1.00 | 60.01 | A |
| ATOM C | 26 | CB | ALA | A | 129 | 272.608 | -58.800 | 93.341 | 1.00 | 66.60 | A |
| ATOM C | 27 | C | ALA | A | 129 | 271.010 | -58.371 | 95.258 | 1.00 | 56.86 | A |
| ATOM O | 28 | O | ALA | A | 129 | 270.365 | -57.326 | 95.173 | 1.00 | 59.08 | A |
| ATOM N | 29 | N | LEU | A | 130 | 271.517 | -58.821 | 96.401 | 1.00 | 51.68 | A |
| ATOM C | 30 | CA | LEU | A | 130 | 271.314 | -58.126 | 97.665 | 1.00 | 53.06 | A |
| ATOM C | 31 | CB | LEU | A | 130 | 272.108 | -58.770 | 98.787 | 1.00 | 43.18 | A |
| ATOM C | 32 | CG | LEU | A | 130 | 272.080 | -58.110 | 100.164 | 1.00 | 36.68 | A |
| ATOM C | 33 | CD1 | LEU | A | 130 | 270.621 | -57.934 | 100.570 | 1.00 | 47.13 | A |
| ATOM C | 34 | CD2 | LEU | A | 130 | 272.839 | -58.975 | 101.186 | 1.00 | 38.64 | A |
| ATOM C | 35 | C | LEU | A | 130 | 271.750 | -56.702 | 97.591 | 1.00 | 55.17 | A |
| ATOM O | 36 | O | LEU | A | 130 | 271.223 | -55.834 | 98.305 | 1.00 | 61.19 | A |
| ATOM N | 37 | N | GLU | A | 131 | 272.686 | -56.469 | 96.690 | 1.00 | 58.94 | A |
| ATOM C | 38 | CA | GLU | A | 131 | 273.276 | -55.172 | 96.539 | 1.00 | 57.04 | A |
| ATOM C | 39 | CB | GLU | A | 131 | 274.689 | -55.400 | 96.030 | 1.00 | 63.45 | A |
| ATOM C | 40 | CG | GLU | A | 131 | 275.441 | -56.450 | 96.938 | 1.00 | 73.38 | A |
| ATOM C | 41 | CD | GLU | A | 131 | 275.249 | -57.909 | 96.486 | 1.00 | 79.54 | A |
| ATOM O | 42 | OE1 | GLU | A | 131 | 274.529 | -58.142 | 95.477 | 1.00 | 80.97 | A |
| ATOM O | 43 | OE2 | GLU | A | 131 | 275.838 | -58.820 | 97.135 | 1.00 | 88.53 | A |
| ATOM C | 44 | C | GLU | A | 131 | 272.458 | -54.235 | 95.682 | 1.00 | 55.07 | A |
| ATOM O | 45 | O | GLU | A | 131 | 272.851 | -53.119 | 95.439 | 1.00 | 51.83 | A |
| ATOM N | 46 | N | ASP | A | 132 | 271.280 | -54.704 | 95.299 | 1.00 | 53.60 | A |
| ATOM C | 47 | CA | ASP | A | 132 | 270.298 | -53.994 | 94.487 | 1.00 | 58.59 | A |
| ATOM C | 48 | CB | ASP | A | 132 | 269.514 | -54.987 | 93.656 | 1.00 | 69.09 | A |
| ATOM C | 49 | CG | ASP | A | 132 | 270.030 | -55.141 | 92.284 | 1.00 | 68.36 | A |
| ATOM O | 50 | OD1 | ASP | A | 132 | 269.776 | -54.250 | 91.447 | 1.00 | 73.44 | A |
| ATOM O | 51 | OD2 | ASP | A | 132 | 270.704 | -56.159 | 92.048 | 1.00 | 76.16 | A |
| ATOM C | 52 | C | ASP | A | 132 | 269.267 | -53.412 | 95.449 | 1.00 | 59.74 | A |
| ATOM O | 53 | O | ASP | A | 132 | 268.472 | -52.547 | 95.093 | 1.00 | 56.43 | A |
| ATOM N | 54 | N | PHE | A | 133 | 269.239 | -53.925 | 96.666 | 1.00 | 62.15 | A |
| ATOM C | 55 | CA | PHE | A | 133 | 268.239 | -53.448 | 97.598 | 1.00 | 62.91 | A |
| ATOM C | 56 | CB | PHE | A | 133 | 267.324 | -54.622 | 97.974 | 1.00 | 59.55 | A |
| ATOM C | 57 | CG | PHE | A | 133 | 266.737 | -55.337 | 96.775 | 1.00 | 61.26 | A |
| ATOM C | 58 | CD1 | PHE | A | 133 | 267.467 | -56.312 | 96.095 | 1.00 | 60.00 | A |
| ATOM C | 59 | CD2 | PHE | A | 133 | 265.442 | -55.059 | 96.346 | 1.00 | 60.03 | A |
| ATOM C | 60 | CE1 | PHE | A | 133 | 266.910 | -56.996 | 94.991 | 1.00 | 65.06 | A |
| ATOM C | 61 | CE2 | PHE | A | 133 | 264.883 | -55.719 | 95.264 | 1.00 | 60.82 | A |
| ATOM C | 62 | CZ | PHE | A | 133 | 265.607 | -56.697 | 94.585 | 1.00 | 61.32 | A |
| ATOM C | 63 | C | PHE | A | 133 | 268.740 | -52.733 | 98.847 | 1.00 | 61.07 | A |
| ATOM O | 64 | O | PHE | A | 133 | 269.866 | -52.972 | 99.318 | 1.00 | 66.24 | A |
| ATOM N | 65 | N | GLU | A | 134 | 267.878 | -51.829 | 99.340 | 1.00 | 59.55 | A |
| ATOM C | 66 | CA | GLU | A | 134 | 268.062 | -51.035 | 100.573 | 1.00 | 58.90 | A |
| ATOM C | 67 | CB | GLU | A | 134 | 267.589 | -49.593 | 100.353 | 1.00 | 54.77 | A |
| ATOM C | 68 | CG | GLU | A | 134 | 268.478 | -48.772 | 99.488 | 1.00 | 63.45 | A |
| ATOM C | 69 | CD | GLU | A | 134 | 268.205 | -47.282 | 99.556 | 1.00 | 61.84 | A |
| ATOM O | 70 | OE1 | GLU | A | 134 | 268.808 | -46.576 | 100.384 | 1.00 | 75.06 | A |
| ATOM O | 71 | OE2 | GLU | A | 134 | 267.388 | -46.803 | 98.768 | 1.00 | 73.27 | A |
| ATOM C | 72 | C | GLU | A | 134 | 267.139 | -51.698 | 101.627 | 1.00 | 53.85 | A |
| ATOM O | 73 | O | GLU | A | 134 | 265.909 | -51.607 | 101.526 | 1.00 | 60.03 | A |
| ATOM N | 74 | N | ILE | A | 135 | 267.713 | -52.362 | 102.621 | 1.00 | 52.32 | A |
| ATOM C | 75 | CA | ILE | A | 135 | 266.917 | -53.042 | 103.625 | 1.00 | 50.89 | A |
| ATOM C | 76 | CB | ILE | A | 135 | 267.750 | -54.131 | 104.343 | 1.00 | 48.23 | A |
| ATOM C | 77 | CG2 | ILE | A | 135 | 266.828 | -55.078 | 105.127 | 1.00 | 51.35 | A |
| ATOM C | 78 | CG1 | ILE | A | 135 | 268.552 | -54.940 | 103.317 | 1.00 | 48.00 | A |
| ATOM C | 79 | CD1 | ILE | A | 135 | 267.731 | -55.720 | 102.398 | 1.00 | 35.46 | A |
| ATOM C | 80 | C | ILE | A | 135 | 266.298 | -52.129 | 104.674 | 1.00 | 50.54 | A |
| ATOM O | 81 | O | ILE | A | 135 | 266.973 | -51.281 | 105.250 | 1.00 | 50.32 | A |
| ATOM N | 82 | N | GLY | A | 136 | 265.004 | -52.334 | 104.924 | 1.00 | 49.85 | A |
| ATOM C | 83 | CA | GLY | A | 136 | 264.286 | -51.534 | 105.903 | 1.00 | 44.78 | A |
| ATOM C | 84 | C | GLY | A | 136 | 264.165 | -52.255 | 107.228 | 1.00 | 44.85 | A |
| ATOM O | 85 | O | GLY | A | 136 | 265.040 | -53.071 | 107.574 | 1.00 | 45.10 | A |
| ATOM N | 86 | N | ARG | A | 137 | 263.071 | -51.996 | 107.940 | 1.00 | 40.54 | A |
| ATOM C | 87 | CA | ARG | A | 137 | 262.891 | -52.598 | 109.248 | 1.00 | 47.22 | A |
| ATOM C | 88 | CB | ARG | A | 137 | 261.911 | -51.766 | 110.065 | 1.00 | 46.15 | A |
| ATOM C | 89 | CG | ARG | A | 137 | 260.481 | -51.887 | 109.585 | 1.00 | 46.77 | A |
| ATOM C | 90 | CD | ARG | A | 137 | 259.521 | -51.284 | 110.589 | 1.00 | 47.76 | A |
| ATOM N | 91 | NE | ARG | A | 137 | 258.174 | -51.283 | 110.058 | 1.00 | 44.18 | A |
| ATOM C | 92 | CZ | ARG | A | 137 | 257.364 | -52.330 | 110.097 | 1.00 | 49.13 | A |
| ATOM N | 93 | NH1 | ARG | A | 137 | 257.779 | -53.468 | 110.655 | 1.00 | 50.41 | A |
| ATOM N | 94 | NH2 | ARG | A | 137 | 256.144 | -52.230 | 109.579 | 1.00 | 42.98 | A |
| ATOM C | 95 | C | ARG | A | 137 | 262.358 | -54.014 | 109.150 | 1.00 | 48.40 | A |
| ATOM O | 96 | O | ARG | A | 137 | 261.734 | -54.370 | 108.150 | 1.00 | 52.53 | A |
| ATOM N | 97 | N | PRO | A | 138 | 262.589 | -54.840 | 110.190 | 1.00 | 48.54 | A |
| ATOM C | 98 | CD | PRO | A | 138 | 263.443 | -54.581 | 111.363 | 1.00 | 46.06 | A |
| ATOM C | 99 | CA | PRO | A | 138 | 262.108 | -56.226 | 110.213 | 1.00 | 46.02 | A |
| ATOM C | 100 | CB | PRO | A | 138 | 262.685 | -56.765 | 111.524 | 1.00 | 49.55 | A |
| ATOM C | 101 | CG | PRO | A | 138 | 263.905 | -55.955 | 111.710 | 1.00 | 42.97 | A |
| ATOM C | 102 | C | PRO | A | 138 | 260.561 | -56.262 | 110.207 | 1.00 | 49.85 | A |
| ATOM O | 103 | O | PRO | A | 138 | 259.920 | -55.810 | 111.142 | 1.00 | 50.42 | A |
| ATOM N | 104 | N | LEU | A | 139 | 259.980 | -56.799 | 109.138 | 1.00 | 45.75 | A |
| ATOM C | 105 | CA | LEU | A | 139 | 258.545 | -56.896 | 109.005 | 1.00 | 39.58 | A |
| ATOM C | 106 | CB | LEU | A | 139 | 258.181 | -57.130 | 107.558 | 1.00 | 37.24 | A |
| ATOM C | 107 | CG | LEU | A | 139 | 258.109 | -55.885 | 106.664 | 1.00 | 42.68 | A |
| ATOM C | 108 | CD1 | LEU | A | 139 | 257.816 | -56.315 | 105.170 | 1.00 | 32.13 | A |
| ATOM C | 109 | CD2 | LEU | A | 139 | 256.990 | -54.961 | 107.170 | 1.00 | 43.77 | A |
| ATOM C | 110 | C | LEU | A | 139 | 257.968 | -58.002 | 109.880 | 1.00 | 40.81 | A |
| ATOM O | 111 | O | LEU | A | 139 | 256.907 | -57.861 | 110.480 | 1.00 | 45.92 | A |
| ATOM N | 112 | N | GLY | A | 140 | 258.675 | -59.111 | 109.970 | 1.00 | 45.15 | A |
| ATOM C | 113 | CA | GLY | A | 140 | 258.198 | -60.218 | 110.789 | 1.00 | 48.82 | A |
| ATOM C | 114 | C | GLY | A | 140 | 259.222 | -61.317 | 111.040 | 1.00 | 48.37 | A |
| ATOM O | 115 | O | GLY | A | 140 | 260.326 | -61.310 | 110.492 | 1.00 | 53.08 | A |
| ATOM N | 116 | N | LYS | A | 141 | 258.842 | -62.283 | 111.856 | 1.00 | 52.66 | A |
| ATOM C | 117 | CA | LYS | A | 141 | 259.740 | -63.376 | 112.192 | 1.00 | 56.79 | A |
| ATOM C | 118 | CB | LYS | A | 141 | 259.961 | -63.404 | 113.707 | 1.00 | 61.89 | A |
| ATOM C | 119 | CG | LYS | A | 141 | 260.862 | -64.513 | 114.210 | 1.00 | 68.25 | A |
| ATOM C | 120 | CD | LYS | A | 141 | 260.894 | -64.538 | 115.750 | 1.00 | 78.79 | A |
| ATOM C | 121 | CE | LYS | A | 141 | 261.863 | -65.624 | 116.242 | 1.00 | 83.42 | A |
| ATOM N | 122 | NZ | LYS | A | 141 | 261.987 | -65.651 | 117.745 | 1.00 | 90.84 | A |
| ATOM C | 123 | C | LYS | A | 141 | 259.219 | -64.728 | 111.704 | 1.00 | 57.82 | A |
| ATOM O | 124 | O | LYS | A | 141 | 258.150 | -65.199 | 112.084 | 1.00 | 57.17 | A |
| ATOM N | 125 | N | GLY | A | 142 | 259.990 | -65.342 | 110.823 | 1.00 | 63.79 | A |
| ATOM C | 126 | CA | GLY | A | 142 | 259.627 | -66.648 | 110.315 | 1.00 | 67.77 | A |
| ATOM C | 127 | C | GLY | A | 142 | 260.381 | -67.717 | 111.087 | 1.00 | 68.12 | A |
| ATOM O | 128 | O | GLY | A | 142 | 260.756 | -67.533 | 112.255 | 1.00 | 76.19 | A |
| ATOM N | 129 | N | LYS | A | 143 | 260.638 | -68.829 | 110.410 | 1.00 | 66.29 | A |
| ATOM C | 130 | CA | LYS | A | 143 | 261.359 | -69.941 | 111.016 | 1.00 | 62.91 | A |
| ATOM C | 131 | CB | LYS | A | 143 | 260.576 | -71.221 | 110.783 | 1.00 | 56.50 | A |
| ATOM C | 132 | CG | LYS | A | 143 | 260.990 | -72.331 | 111.694 | 1.00 | 59.59 | A |
| ATOM C | 133 | CD | LYS | A | 143 | 259.804 | -73.170 | 112.130 | 1.00 | 65.95 | A |
| ATOM C | 134 | CE | LYS | A | 143 | 260.182 | -74.141 | 113.303 | 1.00 | 69.37 | A |
| ATOM N | 135 | NZ | LYS | A | 143 | 259.069 | -75.109 | 113.596 | 1.00 | 70.95 | A |
| ATOM C | 136 | C | LYS | A | 143 | 262.773 | -70.081 | 110.500 | 1.00 | 62.52 | A |
| ATOM O | 137 | O | LYS | A | 143 | 263.725 | -70.302 | 111.243 | 1.00 | 61.60 | A |
| ATOM N | 138 | N | PHE | A | 144 | 262.909 | -69.898 | 109.200 | 1.00 | 68.60 | A |
| ATOM C | 139 | CA | PHE | A | 144 | 264.215 | -70.021 | 108.556 | 1.00 | 74.62 | A |
| ATOM C | 140 | CB | PHE | A | 144 | 264.109 | -70.886 | 107.296 | 1.00 | 69.32 | A |
| ATOM C | 141 | CG | PHE | A | 144 | 263.583 | -72.278 | 107.565 | 1.00 | 69.58 | A |
| ATOM C | 142 | CD1 | PHE | A | 144 | 262.211 | -72.526 | 107.690 | 1.00 | 66.23 | A |
| ATOM C | 143 | CD2 | PHE | A | 144 | 264.460 | -73.335 | 107.731 | 1.00 | 63.51 | A |
| ATOM C | 144 | CE1 | PHE | A | 144 | 261.738 | -73.816 | 107.978 | 1.00 | 73.24 | A |
| ATOM C | 145 | CE2 | PHE | A | 144 | 263.985 | -74.608 | 108.014 | 1.00 | 69.83 | A |
| ATOM C | 146 | CZ | PHE | A | 144 | 262.629 | -74.850 | 108.138 | 1.00 | 66.31 | A |
| ATOM C | 147 | C | PHE | A | 144 | 264.768 | -68.665 | 108.234 | 1.00 | 74.80 | A |
| ATOM O | 148 | O | PHE | A | 144 | 265.636 | -68.496 | 107.395 | 1.00 | 82.71 | A |
| ATOM N | 149 | N | GLY | A | 145 | 264.248 | -67.691 | 108.955 | 1.00 | 83.57 | A |
| ATOM C | 150 | CA | GLY | A | 145 | 264.676 | -66.320 | 108.782 | 1.00 | 77.10 | A |
| ATOM C | 151 | C | GLY | A | 145 | 263.544 | -65.296 | 108.827 | 1.00 | 75.14 | A |
| ATOM O | 152 | O | GLY | A | 145 | 262.338 | -65.557 | 108.521 | 1.00 | 74.64 | A |
| ATOM N | 153 | N | ASN | A | 146 | 263.966 | -64.097 | 109.195 | 1.00 | 67.46 | A |
| ATOM C | 154 | CA | ASN | A | 146 | 263.050 | -62.976 | 109.318 | 1.00 | 65.84 | A |
| ATOM C | 155 | CB | ASN | A | 146 | 263.674 | -61.895 | 110.208 | 1.00 | 68.52 | A |
| ATOM C | 156 | CG | ASN | A | 146 | 263.873 | -62.369 | 111.664 | 1.00 | 74.23 | A |
| ATOM O | 157 | OD1 | ASN | A | 146 | 264.045 | -63.577 | 111.946 | 1.00 | 76.10 | A |
| ATOM N | 158 | ND2 | ASN | A | 146 | 263.871 | -61.415 | 112.588 | 1.00 | 74.93 | A |
| ATOM C | 159 | C | ASN | A | 146 | 262.727 | -62.402 | 107.955 | 1.00 | 60.10 | A |
| ATOM O | 160 | O | ASN | A | 146 | 263.442 | -62.661 | 106.979 | 1.00 | 60.40 | A |
| ATOM N | 161 | N | VAL | A | 147 | 261.651 | -61.614 | 107.909 | 1.00 | 49.34 | A |
| ATOM C | 162 | CA | VAL | A | 147 | 261.208 | -60.938 | 106.697 | 1.00 | 34.29 | A |
| ATOM C | 163 | CB | VAL | A | 147 | 259.691 | -61.195 | 106.433 | 1.00 | 35.55 | A |
| ATOM C | 164 | CG1 | VAL | A | 147 | 259.202 | -60.303 | 105.293 | 1.00 | 22.76 | A |
| ATOM C | 165 | CG2 | VAL | A | 147 | 259.444 | -62.657 | 106.040 | 1.00 | 25.36 | A |
| ATOM C | 166 | C | VAL | A | 147 | 261.432 | -59.427 | 106.905 | 1.00 | 36.01 | A |
| ATOM O | 167 | O | VAL | A | 147 | 261.071 | -58.888 | 107.912 | 1.00 | 33.80 | A |
| ATOM N | 168 | N | TYR | A | 148 | 262.006 | -58.743 | 105.931 | 1.00 | 38.73 | A |
| ATOM C | 169 | CA | TYR | A | 148 | 262.298 | -57.313 | 106.040 | 1.00 | 38.16 | A |
| ATOM C | 170 | CB | TYR | A | 148 | 263.815 | -57.053 | 105.947 | 1.00 | 44.86 | A |
| ATOM C | 171 | CG | TYR | A | 148 | 264.625 | -57.783 | 106.981 | 1.00 | 51.34 | A |
| ATOM C | 172 | CD1 | TYR | A | 148 | 264.946 | -59.110 | 106.823 | 1.00 | 49.12 | A |
| ATOM C | 173 | CE1 | TYR | A | 148 | 265.660 | -59.799 | 107.804 | 1.00 | 57.49 | A |
| ATOM C | 174 | CD2 | TYR | A | 148 | 265.035 | -57.152 | 108.146 | 1.00 | 56.42 | A |
| ATOM C | 175 | CE2 | TYR | A | 148 | 265.743 | -57.839 | 109.136 | 1.00 | 62.05 | A |
| ATOM C | 176 | CZ | TYR | A | 148 | 266.051 | -59.159 | 108.958 | 1.00 | 59.22 | A |
| ATOM O | 177 | OH | TYR | A | 148 | 266.740 | -59.818 | 109.951 | 1.00 | 62.90 | A |
| ATOM C | 178 | C | TYR | A | 148 | 261.647 | -56.485 | 104.972 | 1.00 | 40.31 | A |
| ATOM O | 179 | O | TYR | A | 148 | 261.240 | -56.989 | 103.910 | 1.00 | 39.68 | A |
| ATOM N | 180 | N | LEU | A | 149 | 261.564 | -55.187 | 105.254 | 1.00 | 37.70 | A |
| ATOM C | 181 | CA | LEU | A | 149 | 260.992 | -54.236 | 104.287 | 1.00 | 36.17 | A |
| ATOM C | 182 | CB | LEU | A | 149 | 260.495 | -52.976 | 105.019 | 1.00 | 38.73 | A |
| ATOM C | 183 | CG | LEU | A | 149 | 259.781 | -51.920 | 104.186 | 1.00 | 39.39 | A |
| ATOM C | 184 | CD1 | LEU | A | 149 | 258.437 | -52.465 | 103.793 | 1.00 | 46.05 | A |
| ATOM C | 185 | CD2 | LEU | A | 149 | 259.655 | -50.660 | 104.957 | 1.00 | 40.68 | A |
| ATOM C | 186 | C | LEU | A | 149 | 262.212 | -53.920 | 103.405 | 1.00 | 35.83 | A |
| ATOM O | 187 | O | LEU | A | 149 | 263.340 | -54.217 | 103.822 | 1.00 | 37.60 | A |
| ATOM N | 188 | N | ALA | A | 150 | 262.029 | -53.321 | 102.228 | 1.00 | 37.02 | A |
| ATOM C | 189 | CA | ALA | A | 150 | 263.171 | -53.041 | 101.363 | 1.00 | 35.35 | A |
| ATOM C | 190 | CB | ALA | A | 150 | 263.889 | -54.345 | 100.965 | 1.00 | 48.80 | A |
| ATOM C | 191 | C | ALA | A | 150 | 262.746 | -52.334 | 100.123 | 1.00 | 44.17 | A |
| ATOM O | 192 | O | ALA | A | 150 | 261.665 | -52.604 | 99.566 | 1.00 | 41.00 | A |
| ATOM N | 193 | N | ARG | A | 151 | 263.619 | -51.441 | 99.666 | 1.00 | 47.00 | A |
| ATOM C | 194 | CA | ARG | A | 151 | 263.319 | -50.674 | 98.468 | 1.00 | 50.37 | A |
| ATOM C | 195 | CB | ARG | A | 151 | 263.377 | -49.171 | 98.793 | 1.00 | 53.91 | A |
| ATOM C | 196 | CG | ARG | A | 151 | 262.912 | -48.272 | 97.649 | 1.00 | 53.96 | A |
| ATOM C | 197 | CD | ARG | A | 151 | 262.884 | -46.817 | 98.014 | 1.00 | 57.39 | A |
| ATOM N | 198 | NE | ARG | A | 151 | 264.145 | -46.372 | 98.607 | 1.00 | 58.37 | A |
| ATOM C | 199 | CZ | ARG | A | 151 | 264.561 | -45.110 | 98.572 | 1.00 | 58.07 | A |
| ATOM N | 200 | NH1 | ARG | A | 151 | 263.814 | -44.175 | 97.969 | 1.00 | 56.20 | A |
| ATOM N | 201 | NH2 | ARG | A | 151 | 265.718 | -44.793 | 99.131 | 1.00 | 55.48 | A |
| ATOM C | 202 | C | ARG | A | 151 | 264.311 | -51.021 | 97.348 | 1.00 | 55.38 | A |
| ATOM O | 203 | O | ARG | A | 151 | 265.503 | -51.247 | 97.625 | 1.00 | 60.78 | A |
| ATOM N | 204 | N | GLU | A | 152 | 263.826 | -51.059 | 96.105 | 1.00 | 54.28 | A |
| ATOM C | 205 | CA | GLU | A | 152 | 264.664 | -51.366 | 94.972 | 1.00 | 54.46 | A |
| ATOM C | 206 | CB | GLU | A | 152 | 263.811 | -51.817 | 93.823 | 1.00 | 60.23 | A |
| ATOM C | 207 | CG | GLU | A | 152 | 264.520 | -52.780 | 92.888 | 1.00 | 67.69 | A |
| ATOM C | 208 | CD | GLU | A | 152 | 265.617 | -52.120 | 92.087 | 1.00 | 75.21 | A |
| ATOM O | 209 | OE1 | GLU | A | 152 | 266.773 | -52.020 | 92.593 | 1.00 | 78.70 | A |
| ATOM O | 210 | OE2 | GLU | A | 152 | 265.305 | -51.689 | 90.951 | 1.00 | 76.28 | A |
| ATOM C | 211 | C | GLU | A | 152 | 265.436 | -50.117 | 94.598 | 1.00 | 54.18 | A |
| ATOM O | 212 | O | GLU | A | 152 | 264.868 | -49.045 | 94.388 | 1.00 | 55.50 | A |
| ATOM N | 213 | N | LYS | A | 153 | 266.746 | -50.275 | 94.489 | 1.00 | 55.96 | A |
| ATOM C | 214 | CA | LYS | A | 153 | 267.583 | -49.115 | 94.231 | 1.00 | 57.21 | A |
| ATOM C | 215 | CB | LYS | A | 153 | 269.059 | -49.471 | 94.257 | 1.00 | 56.21 | A |
| ATOM C | 216 | CG | LYS | A | 153 | 269.661 | -49.042 | 95.586 | 1.00 | 49.03 | A |
| ATOM C | 217 | CD | LYS | A | 153 | 270.821 | -49.929 | 95.975 | 1.00 | 56.59 | A |
| ATOM C | 218 | CE | LYS | A | 153 | 271.022 | -49.981 | 97.450 | 1.00 | 62.23 | A |
| ATOM N | 219 | NZ | LYS | A | 153 | 272.289 | -50.704 | 97.804 | 1.00 | 63.82 | A |
| ATOM C | 220 | C | LYS | A | 153 | 267.300 | -48.233 | 93.056 | 1.00 | 60.69 | A |
| ATOM O | 221 | O | LYS | A | 153 | 267.418 | -47.029 | 93.188 | 1.00 | 68.16 | A |
| ATOM N | 222 | N | GLN | A | 154 | 266.890 | -48.704 | 91.909 | 1.00 | 60.83 | A |
| ATOM C | 223 | CA | GLN | A | 154 | 266.722 | -47.630 | 90.954 | 1.00 | 60.63 | A |
| ATOM C | 224 | CB | GLN | A | 154 | 267.313 | -48.015 | 89.612 | 1.00 | 65.34 | A |
| ATOM C | 225 | CG | GLN | A | 154 | 268.585 | -48.908 | 89.762 | 1.00 | 73.31 | A |
| ATOM C | 226 | CD | GLN | A | 154 | 268.842 | -49.643 | 88.514 | 1.00 | 76.62 | A |
| ATOM O | 227 | OE1 | GLN | A | 154 | 268.751 | -49.059 | 87.368 | 1.00 | 77.22 | A |
| ATOM N | 228 | NE2 | GLN | A | 154 | 269.150 | -50.963 | 88.658 | 1.00 | 79.75 | A |
| ATOM C | 229 | C | GLN | A | 154 | 265.288 | -47.279 | 90.868 | 1.00 | 58.41 | A |
| ATOM O | 230 | O | GLN | A | 154 | 264.929 | -46.135 | 90.760 | 1.00 | 66.80 | A |
| ATOM N | 231 | N | SER | A | 155 | 264.465 | -48.297 | 91.026 | 1.00 | 63.11 | A |
| ATOM C | 232 | CA | SER | A | 155 | 263.012 | -48.182 | 90.986 | 1.00 | 57.08 | A |
| ATOM C | 233 | CB | SER | A | 155 | 262.438 | -49.593 | 90.891 | 1.00 | 57.59 | A |
| ATOM O | 234 | OG | SER | A | 155 | 261.096 | -49.532 | 90.511 | 1.00 | 71.09 | A |
| ATOM C | 235 | C | SER | A | 155 | 262.393 | -47.454 | 92.192 | 1.00 | 54.17 | A |
| ATOM O | 236 | O | SER | A | 155 | 261.494 | -46.653 | 92.054 | 1.00 | 41.48 | A |
| ATOM N | 237 | N | LYS | A | 156 | 262.919 | -47.739 | 93.368 | 1.00 | 54.36 | A |
| ATOM C | 238 | CA | LYS | A | 156 | 262.415 | -47.187 | 94.614 | 1.00 | 65.54 | A |
| ATOM C | 239 | CB | LYS | A | 156 | 262.133 | -45.687 | 94.476 | 1.00 | 68.88 | A |
| ATOM C | 240 | CG | LYS | A | 156 | 263.375 | -44.809 | 94.527 | 1.00 | 68.98 | A |
| ATOM C | 241 | CD | LYS | A | 156 | 263.018 | -43.342 | 94.698 | 1.00 | 65.67 | A |
| ATOM C | 242 | CE | LYS | A | 156 | 262.170 | -42.850 | 93.529 | 1.00 | 62.20 | A |
| ATOM N | 243 | NZ | LYS | A | 156 | 261.774 | -41.416 | 93.679 | 1.00 | 56.13 | A |
| ATOM C | 244 | C | LYS | A | 156 | 261.138 | -47.958 | 95.009 | 1.00 | 64.77 | A |
| ATOM O | 245 | O | LYS | A | 156 | 260.317 | -47.485 | 95.803 | 1.00 | 68.30 | A |
| ATOM N | 246 | N | PHE | A | 157 | 261.012 | -49.159 | 94.446 | 1.00 | 62.48 | A |
| ATOM C | 247 | CA | PHE | A | 157 | 259.891 | -50.051 | 94.679 | 1.00 | 52.64 | A |
| ATOM C | 248 | CB | PHE | A | 157 | 259.878 | -51.110 | 93.580 | 1.00 | 54.51 | A |
| ATOM C | 249 | CG | PHE | A | 157 | 258.621 | -51.933 | 93.525 | 1.00 | 52.32 | A |
| ATOM C | 250 | CD1 | PHE | A | 157 | 257.665 | -51.694 | 92.549 | 1.00 | 54.81 | A |
| ATOM C | 251 | CD2 | PHE | A | 157 | 258.395 | -52.950 | 94.448 | 1.00 | 55.89 | A |
| ATOM C | 252 | CE1 | PHE | A | 157 | 256.499 | -52.451 | 92.487 | 1.00 | 53.63 | A |
| ATOM C | 253 | CE2 | PHE | A | 157 | 257.232 | -53.719 | 94.401 | 1.00 | 54.27 | A |
| ATOM C | 254 | CZ | PHE | A | 157 | 256.286 | -53.467 | 93.419 | 1.00 | 58.00 | A |
| ATOM C | 255 | C | PHE | A | 157 | 260.015 | -50.719 | 96.056 | 1.00 | 51.79 | A |
| ATOM O | 256 | O | PHE | A | 157 | 261.020 | -51.370 | 96.340 | 1.00 | 47.35 | A |
| ATOM N | 257 | N | ILE | A | 158 | 258.996 | -50.539 | 96.900 | 1.00 | 50.33 | A |
| ATOM C | 258 | CA | ILE | A | 158 | 258.997 | -51.137 | 98.212 | 1.00 | 48.14 | A |
| ATOM C | 259 | CB | ILE | A | 158 | 258.329 | -50.196 | 99.284 | 1.00 | 48.26 | A |
| ATOM C | 260 | CG2 | ILE | A | 158 | 257.863 | -50.988 | 100.478 | 1.00 | 42.57 | A |
| ATOM C | 261 | CG1 | ILE | A | 158 | 259.382 | -49.205 | 99.795 | 1.00 | 48.74 | A |
| ATOM C | 262 | CD1 | ILE | A | 158 | 258.914 | -48.326 | 100.849 | 1.00 | 60.25 | A |
| ATOM C | 263 | C | ILE | A | 158 | 258.354 | -52.530 | 98.176 | 1.00 | 52.33 | A |
| ATOM O | 264 | O | ILE | A | 158 | 257.269 | -52.732 | 97.635 | 1.00 | 50.55 | A |
| ATOM N | 265 | N | LEU | A | 159 | 259.034 | -53.472 | 98.823 | 1.00 | 52.37 | A |
| ATOM C | 266 | CA | LEU | A | 159 | 258.631 | -54.858 | 98.835 | 1.00 | 48.01 | A |
| ATOM C | 267 | CB | LEU | A | 159 | 259.322 | -55.530 | 97.663 | 1.00 | 49.17 | A |
| ATOM C | 268 | CG | LEU | A | 159 | 260.759 | -55.019 | 97.480 | 1.00 | 47.74 | A |
| ATOM C | 269 | CD1 | LEU | A | 159 | 261.690 | -55.880 | 98.307 | 1.00 | 46.92 | A |
| ATOM C | 270 | CD2 | LEU | A | 159 | 261.150 | -55.055 | 96.008 | 1.00 | 47.94 | A |
| ATOM C | 271 | C | LEU | A | 159 | 259.089 | -55.468 | 100.111 | 1.00 | 44.03 | A |
| ATOM O | 272 | O | LEU | A | 159 | 259.595 | -54.777 | 100.931 | 1.00 | 48.23 | A |
| ATOM N | 273 | N | ALA | A | 160 | 258.889 | -56.765 | 100.280 | 1.00 | 43.85 | A |
| ATOM C | 274 | CA | ALA | A | 160 | 259.347 | -57.465 | 101.474 | 1.00 | 37.28 | A |
| ATOM C | 275 | CB | ALA | A | 160 | 258.187 | -58.117 | 102.184 | 1.00 | 41.03 | A |
| ATOM C | 276 | C | ALA | A | 160 | 260.346 | -58.531 | 101.030 | 1.00 | 43.40 | A |
| ATOM O | 277 | O | ALA | A | 160 | 260.084 | -59.301 | 100.096 | 1.00 | 40.92 | A |
| ATOM N | 278 | N | LEU | A | 161 | 261.494 | -58.566 | 101.693 | 1.00 | 46.08 | A |
| ATOM C | 279 | CA | LEU | A | 161 | 262.564 | -59.520 | 101.378 | 1.00 | 44.64 | A |
| ATOM C | 280 | CB | LEU | A | 161 | 263.916 | -58.794 | 101.334 | 1.00 | 47.23 | A |
| ATOM C | 281 | CG | LEU | A | 161 | 264.865 | -59.050 | 100.188 | 1.00 | 47.49 | A |
| ATOM C | 282 | CD1 | LEU | A | 161 | 264.153 | -58.846 | 98.870 | 1.00 | 42.04 | A |
| ATOM C | 283 | CD2 | LEU | A | 161 | 266.031 | -58.125 | 100.309 | 1.00 | 46.85 | A |
| ATOM C | 284 | C | LEU | A | 161 | 262.607 | -60.592 | 102.441 | 1.00 | 45.60 | A |
| ATOM O | 285 | O | LEU | A | 161 | 263.013 | -60.338 | 103.570 | 1.00 | 56.35 | A |
| ATOM N | 286 | N | LYS | A | 162 | 262.194 | -61.801 | 102.078 | 1.00 | 46.81 | A |
| ATOM C | 287 | CA | LYS | A | 162 | 262.178 | -62.940 | 103.009 | 1.00 | 41.78 | A |
| ATOM C | 288 | CB | LYS | A | 162 | 261.104 | -63.922 | 102.585 | 1.00 | 37.22 | A |
| ATOM C | 289 | CG | LYS | A | 162 | 260.826 | -65.007 | 103.588 | 1.00 | 34.96 | A |
| ATOM C | 290 | CD | LYS | A | 162 | 259.600 | -65.804 | 103.219 | 1.00 | 30.75 | A |
| ATOM C | 291 | CE | LYS | A | 162 | 259.522 | -67.056 | 104.081 | 1.00 | 32.45 | A |
| ATOM N | 292 | NZ | LYS | A | 162 | 258.194 | -67.801 | 103.796 | 1.00 | 32.97 | A |
| ATOM C | 293 | C | LYS | A | 162 | 263.521 | -63.634 | 103.018 | 1.00 | 43.17 | A |
| ATOM O | 294 | O | LYS | A | 162 | 263.869 | -64.350 | 102.087 | 1.00 | 50.65 | A |
| ATOM N | 295 | N | VAL | A | 163 | 264.299 | -63.369 | 104.049 | 1.00 | 47.41 | A |
| ATOM C | 296 | CA | VAL | A | 163 | 265.625 | -63.965 | 104.187 | 1.00 | 50.56 | A |
| ATOM C | 297 | CB | VAL | A | 163 | 266.539 | -63.091 | 105.120 | 1.00 | 49.49 | A |
| ATOM C | 298 | CG1 | VAL | A | 163 | 267.951 | -63.664 | 105.180 | 1.00 | 43.15 | A |
| ATOM C | 299 | CG2 | VAL | A | 163 | 266.555 | -61.660 | 104.586 | 1.00 | 46.95 | A |
| ATOM C | 300 | C | VAL | A | 163 | 265.570 | -65.371 | 104.756 | 1.00 | 48.41 | A |
| ATOM O | 301 | O | VAL | A | 163 | 264.994 | -65.586 | 105.798 | 1.00 | 53.08 | A |
| ATOM N | 302 | N | LEU | A | 164 | 266.195 | -66.329 | 104.085 | 1.00 | 53.99 | A |
| ATOM C | 303 | CA | LEU | A | 164 | 266.261 | -67.732 | 104.569 | 1.00 | 55.36 | A |
| ATOM C | 304 | CB | LEU | A | 164 | 265.516 | -68.657 | 103.598 | 1.00 | 54.99 | A |
| ATOM C | 305 | CG | LEU | A | 164 | 264.173 | -68.154 | 103.039 | 1.00 | 57.06 | A |
| ATOM C | 306 | CD1 | LEU | A | 164 | 264.096 | -68.423 | 101.535 | 1.00 | 57.49 | A |
| ATOM C | 307 | CD2 | LEU | A | 164 | 263.093 | -68.851 | 103.764 | 1.00 | 55.47 | A |
| ATOM C | 308 | C | LEU | A | 164 | 267.715 | -68.188 | 104.665 | 1.00 | 52.23 | A |
| ATOM O | 309 | O | LEU | A | 164 | 268.488 | -67.990 | 103.745 | 1.00 | 50.98 | A |
| ATOM N | 310 | N | PHE | A | 165 | 268.081 | -68.800 | 105.774 | 1.00 | 52.15 | A |
| ATOM C | 311 | CA | PHE | A | 165 | 269.422 | -69.297 | 105.892 | 1.00 | 51.87 | A |
| ATOM C | 312 | CB | PHE | A | 165 | 269.859 | -69.322 | 107.352 | 1.00 | 54.79 | A |
| ATOM C | 313 | CG | PHE | A | 165 | 270.210 | -67.979 | 107.875 | 1.00 | 59.43 | A |
| ATOM C | 314 | CD1 | PHE | A | 165 | 269.232 | -67.171 | 108.426 | 1.00 | 61.03 | A |
| ATOM C | 315 | CD2 | PHE | A | 165 | 271.517 | -67.493 | 107.769 | 1.00 | 59.15 | A |
| ATOM C | 316 | CE1 | PHE | A | 165 | 269.542 | -65.896 | 108.856 | 1.00 | 59.31 | A |
| ATOM C | 317 | CE2 | PHE | A | 165 | 271.835 | -66.216 | 108.196 | 1.00 | 59.42 | A |
| ATOM C | 318 | CZ | PHE | A | 165 | 270.845 | -65.412 | 108.745 | 1.00 | 60.41 | A |
| ATOM C | 319 | C | PHE | A | 165 | 269.598 | -70.683 | 105.287 | 1.00 | 52.40 | A |
| ATOM O | 320 | O | PHE | A | 165 | 268.924 | -71.654 | 105.675 | 1.00 | 45.64 | A |
| ATOM N | 321 | N | LYS | A | 166 | 270.504 | -70.772 | 104.320 | 1.00 | 52.32 | A |
| ATOM C | 322 | CA | LYS | A | 166 | 270.786 | -72.050 | 103.696 | 1.00 | 53.94 | A |
| ATOM C | 323 | CB | LYS | A | 166 | 271.911 | -71.916 | 102.708 | 1.00 | 47.98 | A |
| ATOM C | 324 | CG | LYS | A | 166 | 271.540 | -71.081 | 101.498 | 1.00 | 41.92 | A |
| ATOM C | 325 | CD | LYS | A | 166 | 272.530 | -71.309 | 100.383 | 1.00 | 38.85 | A |
| ATOM C | 326 | CE | LYS | A | 166 | 272.490 | -70.201 | 99.345 | 1.00 | 31.90 | A |
| ATOM N | 327 | NZ | LYS | A | 166 | 273.477 | -70.401 | 98.252 | 1.00 | 43.47 | A |
| ATOM C | 328 | C | LYS | A | 166 | 271.159 | -73.067 | 104.740 | 1.00 | 51.94 | A |
| ATOM O | 329 | O | LYS | A | 166 | 270.625 | -74.147 | 104.743 | 1.00 | 62.20 | A |
| ATOM N | 330 | N | ALA | A | 167 | 272.031 | -72.699 | 105.659 | 1.00 | 51.64 | A |
| ATOM C | 331 | CA | ALA | A | 167 | 272.445 | -73.632 | 106.682 | 1.00 | 51.81 | A |
| ATOM C | 332 | CB | ALA | A | 167 | 273.293 | -72.935 | 107.670 | 1.00 | 53.31 | A |
| ATOM C | 333 | C | ALA | A | 167 | 271.234 | -74.240 | 107.369 | 1.00 | 53.85 | A |
| ATOM O | 334 | O | ALA | A | 167 | 271.105 | -75.457 | 107.454 | 1.00 | 57.18 | A |
| ATOM N | 335 | N | GLN | A | 168 | 270.318 | -73.403 | 107.833 | 1.00 | 59.28 | A |
| ATOM C | 336 | CA | GLN | A | 168 | 269.139 | -73.902 | 108.534 | 1.00 | 59.95 | A |
| ATOM C | 337 | CB | GLN | A | 168 | 268.348 | -72.736 | 109.131 | 1.00 | 66.12 | A |
| ATOM C | 338 | CG | GLN | A | 168 | 268.398 | -72.648 | 110.644 | 1.00 | 72.61 | A |
| ATOM C | 339 | CD | GLN | A | 168 | 267.038 | -72.270 | 111.248 | 1.00 | 77.64 | A |
| ATOM O | 340 | OE1 | GLN | A | 168 | 266.542 | -72.938 | 112.173 | 1.00 | 76.82 | A |
| ATOM N | 341 | NE2 | GLN | A | 168 | 266.432 | -71.196 | 110.727 | 1.00 | 72.17 | A |
| ATOM C | 342 | C | GLN | A | 168 | 268.221 | -74.718 | 107.635 | 1.00 | 55.73 | A |
| ATOM O | 343 | O | GLN | A | 168 | 267.646 | -75.736 | 108.043 | 1.00 | 53.22 | A |
| ATOM N | 344 | N | LEU | A | 169 | 268.076 | -74.262 | 106.404 | 1.00 | 47.44 | A |
| ATOM C | 345 | CA | LEU | A | 169 | 267.214 | -74.954 | 105.430 | 1.00 | 53.18 | A |
| ATOM C | 346 | CB | LEU | A | 169 | 267.236 | -74.247 | 104.084 | 1.00 | 46.05 | A |
| ATOM C | 347 | CG | LEU | A | 169 | 266.495 | -72.939 | 104.055 | 1.00 | 47.35 | A |
| ATOM C | 348 | CD1 | LEU | A | 169 | 266.858 | -72.184 | 102.803 | 1.00 | 45.64 | A |
| ATOM C | 349 | CD2 | LEU | A | 169 | 265.005 | -73.224 | 104.130 | 1.00 | 44.94 | A |
| ATOM C | 350 | C | LEU | A | 169 | 267.743 | -76.381 | 105.180 | 1.00 | 53.79 | A |
| ATOM O | 351 | O | LEU | A | 169 | 266.983 | -77.361 | 105.065 | 1.00 | 54.87 | A |
| ATOM N | 352 | N | GLU | A | 170 | 269.057 | -76.488 | 105.056 | 1.00 | 54.40 | A |
| ATOM C | 353 | CA | GLU | A | 170 | 269.692 | -77.771 | 104.839 | 1.00 | 58.30 | A |
| ATOM C | 354 | CB | GLU | A | 170 | 271.162 | -77.587 | 104.678 | 1.00 | 57.36 | A |
| ATOM C | 355 | CG | GLU | A | 170 | 271.585 | -77.158 | 103.319 | 1.00 | 66.04 | A |
| ATOM C | 356 | CD | GLU | A | 170 | 273.099 | -76.836 | 103.260 | 1.00 | 73.19 | A |
| ATOM O | 357 | OE1 | GLU | A | 170 | 273.912 | -77.755 | 103.560 | 1.00 | 76.86 | A |
| ATOM O | 358 | OE2 | GLU | A | 170 | 273.473 | -75.670 | 102.918 | 1.00 | 77.40 | A |
| ATOM C | 359 | C | GLU | A | 170 | 269.481 | -78.643 | 106.045 | 1.00 | 60.58 | A |
| ATOM O | 360 | O | GLU | A | 170 | 268.882 | -79.730 | 105.913 | 1.00 | 71.27 | A |
| ATOM N | 361 | N | LYS | A | 171 | 269.931 | -78.162 | 107.207 | 1.00 | 59.38 | A |
| ATOM C | 362 | CA | LYS | A | 171 | 269.817 | -78.942 | 108.423 | 1.00 | 59.74 | A |
| ATOM C | 363 | CB | LYS | A | 171 | 270.264 | -78.118 | 109.600 | 1.00 | 58.50 | A |
| ATOM C | 364 | C | LYS | A | 171 | 268.409 | -79.500 | 108.655 | 1.00 | 62.87 | A |
| ATOM O | 365 | O | LYS | A | 171 | 268.227 | -80.438 | 109.441 | 1.00 | 65.46 | A |
| ATOM N | 366 | N | ALA | A | 172 | 267.417 | -78.939 | 107.970 | 1.00 | 58.04 | A |
| ATOM C | 367 | CA | ALA | A | 172 | 266.044 | -79.373 | 108.153 | 1.00 | 55.58 | A |
| ATOM C | 368 | CB | ALA | A | 172 | 265.139 | -78.157 | 108.445 | 1.00 | 44.25 | A |
| ATOM C | 369 | C | ALA | A | 172 | 265.506 | -80.129 | 106.962 | 1.00 | 57.83 | A |
| ATOM O | 370 | O | ALA | A | 172 | 264.406 | -80.699 | 107.038 | 1.00 | 63.97 | A |
| ATOM N | 371 | N | GLY | A | 173 | 266.255 | -80.108 | 105.860 | 1.00 | 59.05 | A |
| ATOM C | 372 | CA | GLY | A | 173 | 265.825 | -80.806 | 104.659 | 1.00 | 58.57 | A |
| ATOM C | 373 | C | GLY | A | 173 | 264.519 | -80.290 | 104.096 | 1.00 | 56.33 | A |
| ATOM O | 374 | O | GLY | A | 173 | 263.636 | -81.045 | 103.722 | 1.00 | 57.22 | A |
| ATOM N | 375 | N | VAL | A | 174 | 264.392 | -78.983 | 104.043 | 1.00 | 52.27 | A |
| ATOM C | 376 | CA | VAL | A | 174 | 263.183 | -78.389 | 103.520 | 1.00 | 51.67 | A |
| ATOM C | 377 | CB | VAL | A | 174 | 262.555 | -77.420 | 104.523 | 1.00 | 44.57 | A |
| ATOM C | 378 | CG1 | VAL | A | 174 | 262.061 | -78.172 | 105.687 | 1.00 | 45.83 | A |
| ATOM C | 379 | CG2 | VAL | A | 174 | 263.557 | -76.374 | 104.919 | 1.00 | 43.22 | A |
| ATOM C | 380 | C | VAL | A | 174 | 263.518 | -77.628 | 102.230 | 1.00 | 55.27 | A |
| ATOM O | 381 | O | VAL | A | 174 | 262.825 | -76.679 | 101.851 | 1.00 | 62.81 | A |
| ATOM N | 382 | N | GLU | A | 175 | 264.577 | -78.044 | 101.548 | 1.00 | 50.98 | A |
| ATOM C | 383 | CA | GLU | A | 175 | 264.955 | -77.393 | 100.296 | 1.00 | 52.00 | A |
| ATOM C | 384 | CB | GLU | A | 175 | 266.251 | -77.994 | 99.721 | 1.00 | 47.79 | A |
| ATOM C | 385 | CG | GLU | A | 175 | 267.462 | -77.861 | 100.680 | 1.00 | 59.64 | A |
| ATOM C | 386 | CD | GLU | A | 175 | 267.730 | -79.101 | 101.479 | 1.00 | 59.83 | A |
| ATOM O | 387 | OE1 | GLU | A | 175 | 266.822 | -79.948 | 101.619 | 1.00 | 66.12 | A |
| ATOM O | 388 | OE2 | GLU | A | 175 | 268.853 | -79.219 | 101.990 | 1.00 | 70.43 | A |
| ATOM C | 389 | C | GLU | A | 175 | 263.852 | -77.526 | 99.266 | 1.00 | 51.17 | A |
| ATOM O | 390 | O | GLU | A | 175 | 263.637 | -76.642 | 98.463 | 1.00 | 57.75 | A |
| ATOM N | 391 | N | HIS | A | 176 | 263.166 | -78.661 | 99.266 | 1.00 | 60.20 | A |
| ATOM C | 392 | CA | HIS | A | 176 | 262.080 | -78.920 | 98.312 | 1.00 | 61.34 | A |
| ATOM C | 393 | CB | HIS | A | 176 | 261.735 | -80.414 | 98.276 | 1.00 | 62.47 | A |
| ATOM C | 394 | CG | HIS | A | 176 | 261.265 | -80.959 | 99.579 | 1.00 | 70.92 | A |
| ATOM C | 395 | CD2 | HIS | A | 176 | 261.875 | -81.020 | 100.783 | 1.00 | 68.70 | A |
| ATOM N | 396 | ND1 | HIS | A | 176 | 260.020 | -81.533 | 99.740 | 1.00 | 72.07 | A |
| ATOM C | 397 | CE1 | HIS | A | 176 | 259.886 | -81.925 | 100.994 | 1.00 | 75.43 | A |
| ATOM N | 398 | NE2 | HIS | A | 176 | 260.996 | -81.625 | 101.644 | 1.00 | 77.22 | A |
| ATOM C | 399 | C | HIS | A | 176 | 260.828 | -78.116 | 98.620 | 1.00 | 60.76 | A |
| ATOM O | 400 | O | HIS | A | 176 | 260.058 | -77.805 | 97.733 | 1.00 | 64.86 | A |
| ATOM N | 401 | N | GLN | A | 177 | 260.607 | -77.784 | 99.886 | 1.00 | 63.60 | A |
| ATOM C | 402 | CA | GLN | A | 177 | 259.436 | -76.993 | 100.239 | 1.00 | 59.32 | A |
| ATOM C | 403 | CB | GLN | A | 177 | 259.188 | -77.080 | 101.756 | 1.00 | 65.79 | A |
| ATOM C | 404 | CG | GLN | A | 177 | 258.573 | -78.484 | 102.090 | 1.00 | 78.25 | A |
| ATOM C | 405 | CD | GLN | A | 177 | 258.481 | -78.896 | 103.539 | 1.00 | 84.93 | A |
| ATOM O | 406 | OE1 | GLN | A | 177 | 257.670 | -79.822 | 103.897 | 1.00 | 89.62 | A |
| ATOM N | 407 | NE2 | GLN | A | 177 | 259.316 | -78.274 | 104.407 | 1.00 | 87.76 | A |
| ATOM C | 408 | C | GLN | A | 177 | 259.647 | -75.552 | 99.730 | 1.00 | 54.25 | A |
| ATOM O | 409 | O | GLN | A | 177 | 258.739 | -75.013 | 99.073 | 1.00 | 51.39 | A |
| ATOM N | 410 | N | LEU | A | 178 | 260.844 | -74.972 | 99.942 | 1.00 | 45.84 | A |
| ATOM C | 411 | CA | LEU | A | 178 | 261.123 | -73.611 | 99.474 | 1.00 | 49.49 | A |
| ATOM C | 412 | CB | LEU | A | 178 | 262.514 | -73.175 | 99.900 | 1.00 | 42.21 | A |
| ATOM C | 413 | CG | LEU | A | 178 | 263.032 | -71.858 | 99.325 | 1.00 | 49.26 | A |
| ATOM C | 414 | CD1 | LEU | A | 178 | 261.981 | -70.693 | 99.584 | 1.00 | 37.20 | A |
| ATOM C | 415 | CD2 | LEU | A | 178 | 264.421 | -71.557 | 99.950 | 1.00 | 46.43 | A |
| ATOM C | 416 | C | LEU | A | 178 | 261.030 | -73.631 | 97.939 | 1.00 | 46.87 | A |
| ATOM O | 417 | O | LEU | A | 178 | 260.678 | -72.659 | 97.280 | 1.00 | 52.87 | A |
| ATOM N | 418 | N | ARG | A | 179 | 261.314 | -74.778 | 97.360 | 1.00 | 52.38 | A |
| ATOM C | 419 | CA | ARG | A | 179 | 261.258 | -74.887 | 95.908 | 1.00 | 48.78 | A |
| ATOM C | 420 | CB | ARG | A | 179 | 261.877 | -76.207 | 95.443 | 1.00 | 55.74 | A |
| ATOM C | 421 | CG | ARG | A | 179 | 262.661 | -76.085 | 94.149 | 1.00 | 55.19 | A |
| ATOM C | 422 | CD | ARG | A | 179 | 263.898 | -77.002 | 94.178 | 1.00 | 71.41 | A |
| ATOM N | 423 | NE | ARG | A | 179 | 264.829 | -76.685 | 93.086 | 1.00 | 73.48 | A |
| ATOM C | 424 | CZ | ARG | A | 179 | 264.827 | -77.258 | 91.873 | 1.00 | 77.16 | A |
| ATOM N | 425 | NH1 | ARG | A | 179 | 263.941 | -78.208 | 91.565 | 1.00 | 70.26 | A |
| ATOM N | 426 | MH2 | ARG | A | 179 | 265.709 | -76.874 | 90.951 | 1.00 | 75.62 | A |
| ATOM C | 427 | C | ARG | A | 179 | 259.841 | -74.758 | 95.383 | 1.00 | 46.80 | A |
| ATOM O | 428 | O | ARG | A | 179 | 259.605 | -74.009 | 94.443 | 1.00 | 37.96 | A |
| ATOM N | 429 | N | ARG | A | 180 | 258.912 | -75.494 | 95.988 | 1.00 | 41.56 | A |
| ATOM C | 430 | CA | ARG | A | 180 | 257.504 | -75.451 | 95.630 | 1.00 | 46.45 | A |
| ATOM C | 431 | CB | ARG | A | 180 | 256.749 | -76.543 | 96.386 | 1.00 | 47.01 | A |
| ATOM C | 432 | CG | ARG | A | 180 | 256.416 | -77.762 | 95.546 | 1.00 | 51.52 | A |
| ATOM C | 433 | CD | ARG | A | 180 | 255.582 | -78.783 | 96.257 | 1.00 | 53.97 | A |
| ATOM N | 434 | NE | ARG | A | 180 | 256.374 | -79.863 | 96.824 | 1.00 | 69.57 | A |
| ATOM C | 435 | CZ | ARG | A | 180 | 256.902 | -79.849 | 98.046 | 1.00 | 76.20 | A |
| ATOM N | 436 | NH1 | ARG | A | 180 | 256.721 | -78.796 | 98.846 | 1.00 | 85.82 | A |
| ATOM N | 437 | NH2 | ARG | A | 180 | 257.614 | -80.893 | 98.475 | 1.00 | 82.98 | A |
| ATOM C | 438 | C | ARG | A | 180 | 256.882 | -74.100 | 95.969 | 1.00 | 48.19 | A |
| ATOM O | 439 | O | ARG | A | 180 | 256.088 | -73.556 | 95.189 | 1.00 | 55.26 | A |
| ATOM N | 440 | N | GLU | A | 181 | 257.235 | -73.560 | 97.130 | 1.00 | 49.77 | A |
| ATOM C | 441 | CA | GLU | A | 181 | 256.694 | -72.279 | 97.536 | 1.00 | 48.24 | A |
| ATOM C | 442 | CB | GLU | A | 181 | 257.367 | -71.820 | 98.841 | 1.00 | 51.28 | A |
| ATOM C | 443 | CG | GLU | A | 181 | 256.969 | -70.425 | 99.303 | 1.00 | 55.39 | A |
| ATOM C | 444 | CD | GLU | A | 181 | 257.553 | -70.042 | 100.652 | 1.00 | 57.20 | A |
| ATOM O | 445 | OE1 | GLU | A | 181 | 258.173 | -70.904 | 101.305 | 1.00 | 46.80 | A |
| ATOM O | 446 | OE2 | GLU | A | 181 | 257.362 | -68.871 | 101.058 | 1.00 | 56.40 | A |
| ATOM C | 447 | C | GLU | A | 181 | 256.958 | -71.259 | 96.430 | 1.00 | 49.63 | A |
| ATOM O | 448 | O | GLU | A | 181 | 256.044 | -70.571 | 95.930 | 1.00 | 44.79 | A |
| ATOM N | 449 | N | VAL | A | 182 | 258.227 | -71.191 | 96.037 | 1.00 | 46.11 | A |
| ATOM C | 450 | CA | VAL | A | 182 | 258.659 | -70.228 | 95.035 | 1.00 | 45.55 | A |
| ATOM C | 451 | CB | VAL | A | 182 | 260.183 | -70.266 | 94.833 | 1.00 | 40.91 | A |
| ATOM C | 452 | CG1 | VAL | A | 182 | 260.560 | -69.403 | 93.678 | 1.00 | 38.12 | A |
| ATOM C | 453 | CG2 | VAL | A | 182 | 260.875 | -69.730 | 96.059 | 1.00 | 51.08 | A |
| ATOM C | 454 | C | VAL | A | 182 | 257.954 | -70.443 | 93.695 | 1.00 | 46.82 | A |
| ATOM O | 455 | O | VAL | A | 182 | 257.459 | -69.483 | 93.056 | 1.00 | 40.29 | A |
| ATOM N | 456 | N | GLU | A | 183 | 257.936 | -71.690 | 93.237 | 1.00 | 48.08 | A |
| ATOM C | 457 | CA | GLU | A | 183 | 257.280 | -72.012 | 91.968 | 1.00 | 52.62 | A |
| ATOM C | 458 | CB | GLU | A | 183 | 257.493 | -73.478 | 91.608 | 1.00 | 56.60 | A |
| ATOM C | 459 | CG | GLU | A | 183 | 258.695 | -73.784 | 90.808 | 1.00 | 70.69 | A |
| ATOM C | 460 | CD | GLU | A | 183 | 258.923 | -75.283 | 90.746 | 1.00 | 77.34 | A |
| ATOM O | 461 | OE1 | GLU | A | 183 | 257.954 | -76.011 | 90.397 | 1.00 | 83.76 | A |
| ATOM O | 462 | OE2 | GLU | A | 183 | 260.068 | -75.727 | 91.052 | 1.00 | 82.45 | A |
| ATOM C | 463 | C | GLU | A | 183 | 255.800 | -71.754 | 91.912 | 1.00 | 49.85 | A |
| ATOM O | 464 | O | GLU | A | 183 | 255.313 | -71.138 | 90.947 | 1.00 | 46.36 | A |
| ATOM N | 465 | N | ILE | A | 184 | 255.098 | -72.317 | 92.898 | 1.00 | 46.74 | A |
| ATOM C | 466 | CA | ILE | A | 184 | 253.660 | -72.157 | 92.965 | 1.00 | 43.86 | A |
| ATOM C | 467 | CB | ILE | A | 184 | 253.067 | -72.962 | 94.115 | 1.00 | 40.26 | A |
| ATOM C | 468 | CG2 | ILE | A | 184 | 251.553 | -72.714 | 94.209 | 1.00 | 29.84 | A |
| ATOM C | 469 | CG1 | ILE | A | 184 | 253.313 | -74.446 | 93.860 | 1.00 | 36.80 | A |
| ATOM C | 470 | CD1 | ILE | A | 184 | 252.748 | -75.369 | 94.961 | 1.00 | 40.80 | A |
| ATOM C | 471 | C | ILE | A | 184 | 253.221 | -70.713 | 93.099 | 1.00 | 46.29 | A |
| ATOM O | 472 | O | ILE | A | 184 | 252.448 | -70.199 | 92.284 | 1.00 | 49.47 | A |
| ATOM N | 473 | N | GLN | A | 185 | 253.720 | -70.054 | 94.132 | 1.00 | 49.28 | A |
| ATOM C | 474 | CA | GLN | A | 185 | 253.328 | -68.666 | 94.388 | 1.00 | 52.47 | A |
| ATOM C | 475 | CB | GLN | A | 185 | 253.948 | -68.189 | 95.721 | 1.00 | 44.73 | A |
| ATOM C | 476 | CG | GLN | A | 185 | 253.150 | -67.168 | 96.526 | 1.00 | 48.30 | A |
| ATOM C | 477 | CD | GLN | A | 185 | 253.861 | -66.745 | 97.745 | 1.00 | 54.47 | A |
| ATOM O | 478 | OE1 | GLN | A | 185 | 254.700 | -67.516 | 98.331 | 1.00 | 61.67 | A |
| ATOM N | 479 | NE2 | GLN | A | 185 | 253.553 | -65.501 | 98.201 | 1.00 | 56.50 | A |
| ATOM C | 480 | C | GLN | A | 185 | 253.707 | -67.716 | 93.231 | 1.00 | 50.63 | A |
| ATOM O | 481 | O | GLN | A | 185 | 252.971 | -66.780 | 92.894 | 1.00 | 52.61 | A |
| ATOM N | 482 | N | SER | A | 186 | 254.843 | -67.972 | 92.610 | 1.00 | 47.27 | A |
| ATOM C | 483 | CA | SER | A | 186 | 255.315 | -67.114 | 91.541 | 1.00 | 52.03 | A |
| ATOM C | 484 | CB | SER | A | 186 | 256.692 | -67.586 | 91.048 | 1.00 | 51.09 | A |
| ATOM O | 485 | OG | SER | A | 186 | 256.609 | -68.877 | 90.496 | 1.00 | 54.11 | A |
| ATOM C | 486 | C | SER | A | 186 | 254.346 | -67.050 | 90.373 | 1.00 | 51.30 | A |
| ATOM O | 487 | O | SER | A | 186 | 254.116 | -65.999 | 89.812 | 1.00 | 53.71 | A |
| ATOM N | 488 | N | HIS | A | 187 | 253.774 | -68.184 | 90.001 | 1.00 | 55.86 | A |
| ATOM C | 489 | CA | HIS | A | 187 | 252.846 | -68.233 | 88.883 | 1.00 | 57.28 | A |
| ATOM C | 490 | CB | HIS | A | 187 | 252.945 | -69.605 | 88.190 | 1.00 | 68.44 | A |
| ATOM C | 491 | CG | HIS | A | 187 | 254.283 | -69.866 | 87.541 | 1.00 | 77.26 | A |
| ATOM C | 492 | CD2 | HIS | A | 187 | 255.147 | -69.041 | 86.892 | 1.00 | 78.77 | A |
| ATOM N | 493 | ND1 | HIS | A | 187 | 254.874 | -71.114 | 87.532 | 1.00 | 80.45 | A |
| ATOM C | 494 | CE1 | HIS | A | 187 | 256.041 | -71.048 | 86.911 | 1.00 | 81.91 | A |
| ATOM N | 495 | NE2 | HIS | A | 187 | 256.232 | -69.802 | 86.512 | 1.00 | 81.51 | A |
| ATOM C | 496 | C | HIS | A | 187 | 251.418 | -67.953 | 89.300 | 1.00 | 57.51 | A |
| ATOM O | 497 | O | HIS | A | 187 | 250.496 | -68.154 | 88.529 | 1.00 | 60.26 | A |
| ATOM N | 498 | N | LEU | A | 188 | 251.230 | -67.497 | 90.530 | 1.00 | 59.97 | A |
| ATOM C | 499 | CA | LEU | A | 188 | 249.900 | -67.189 | 91.028 | 1.00 | 56.67 | A |
| ATOM C | 500 | CB | LEU | A | 188 | 249.832 | -67.488 | 92.511 | 1.00 | 57.07 | A |
| ATOM C | 501 | CG | LEU | A | 188 | 248.970 | -68.663 | 92.952 | 1.00 | 55.73 | A |
| ATOM C | 502 | CD1 | LEU | A | 188 | 249.478 | -69.896 | 92.257 | 1.00 | 62.61 | A |
| ATOM C | 503 | CD2 | LEU | A | 188 | 249.027 | -68.827 | 94.465 | 1.00 | 53.98 | A |
| ATOM C | 504 | C | LEU | A | 188 | 249.598 | -65.728 | 90.777 | 1.00 | 57.85 | A |
| ATOM O | 505 | O | LEU | A | 188 | 250.503 | -64.881 | 90.799 | 1.00 | 64.49 | A |
| ATOM N | 506 | N | ARG | A | 189 | 248.331 | -65.440 | 90.507 | 1.00 | 59.25 | A |
| ATOM C | 507 | CA | ARG | A | 189 | 247.893 | -64.067 | 90.239 | 1.00 | 63.66 | A |
| ATOM C | 508 | CB | ARG | A | 189 | 247.927 | -63.796 | 88.734 | 1.00 | 64.78 | A |
| ATOM C | 509 | CG | ARG | A | 189 | 249.318 | -63.660 | 88.125 | 1.00 | 70.33 | A |
| ATOM C | 510 | CD | ARG | A | 189 | 249.903 | -62.285 | 88.356 | 1.00 | 72.14 | A |
| ATOM N | 511 | NE | ARG | A | 189 | 250.864 | -61.937 | 87.301 | 1.00 | 81.14 | A |
| ATOM C | 512 | CZ | ARG | A | 189 | 250.716 | -60.923 | 86.432 | 1.00 | 82.52 | A |
| ATOM N | 513 | NH1 | ARG | A | 189 | 249.636 | -60.138 | 86.478 | 1.00 | 79.99 | A |
| ATOM N | 514 | NH2 | ARG | A | 189 | 251.658 | -60.669 | 85.521 | 1.00 | 82.64 | A |
| ATOM C | 515 | C | ARG | A | 189 | 246.469 | -63.844 | 90.761 | 1.00 | 59.43 | A |
| ATOM O | 516 | O | ARG | A | 189 | 245.497 | -64.167 | 90.072 | 1.00 | 61.20 | A |
| ATOM N | 517 | N | HIS | A | 190 | 246.342 | -63.283 | 91.964 | 1.00 | 56.49 | A |
| ATOM C | 518 | CA | HIS | A | 190 | 245.023 | -63.075 | 92.565 | 1.00 | 48.34 | A |
| ATOM C | 519 | CB | HIS | A | 190 | 244.506 | -64.377 | 93.201 | 1.00 | 52.70 | A |
| ATOM C | 520 | CG | HIS | A | 190 | 243.036 | -64.372 | 93.489 | 1.00 | 49.06 | A |
| ATOM C | 521 | CD2 | HIS | A | 190 | 242.038 | -65.207 | 93.107 | 1.00 | 52.53 | A |
| ATOM N | 522 | ND1 | HIS | A | 190 | 242.449 | -63.432 | 94.302 | 1.00 | 51.06 | A |
| ATOM C | 523 | CE1 | HIS | A | 190 | 241.155 | -63.686 | 94.414 | 1.00 | 46.17 | A |
| ATOM N | 524 | NE2 | HIS | A | 190 | 240.878 | -64.757 | 93.697 | 1.00 | 43.53 | A |
| ATOM C | 525 | C | HIS | A | 190 | 245.116 | -61.998 | 93.611 | 1.00 | 47.54 | A |
| ATOM O | 526 | O | HIS | A | 190 | 246.077 | -61.946 | 94.377 | 1.00 | 44.41 | A |
| ATOM N | 527 | N | PRO | A | 191 | 244.119 | -61.102 | 93.645 | 1.00 | 49.93 | A |
| ATOM C | 528 | CD | PRO | A | 191 | 242.947 | -61.054 | 92.749 | 1.00 | 46.91 | A |
| ATOM C | 529 | CA | PRO | A | 191 | 244.070 | -59.992 | 94.607 | 1.00 | 49.33 | A |
| ATOM C | 530 | CB | PRO | A | 191 | 242.694 | -59.384 | 94.346 | 1.00 | 50.21 | A |
| ATOM C | 531 | CG | PRO | A | 191 | 242.518 | -59.644 | 92.887 | 1.00 | 48.82 | A |
| ATOM C | 532 | C | PRO | A | 191 | 244.239 | -60.419 | 96.072 | 1.00 | 46.55 | A |
| ATOM O | 533 | O | PRO | A | 191 | 244.892 | -59.719 | 96.848 | 1.00 | 42.94 | A |
| ATOM N | 534 | N | ASN | A | 192 | 243.647 | -61.555 | 96.434 | 1.00 | 42.75 | A |
| ATOM C | 535 | CA | ASN | A | 192 | 243.715 | -62.060 | 97.791 | 1.00 | 48.46 | A |
| ATOM C | 536 | CB | ASN | A | 192 | 242.378 | -62.678 | 98.192 | 1.00 | 45.61 | A |
| ATOM C | 537 | CG | ASN | A | 192 | 241.206 | -61.781 | 97.847 | 1.00 | 48.26 | A |
| ATOM O | 538 | OD1 | ASN | A | 192 | 240.715 | -61.767 | 96.724 | 1.00 | 55.77 | A |
| ATOM N | 539 | ND2 | ASN | A | 192 | 240.766 | -61.020 | 98.804 | 1.00 | 48.89 | A |
| ATOM C | 540 | C | ASN | A | 192 | 244.831 | -63.085 | 97.953 | 1.00 | 44.35 | A |
| ATOM O | 541 | O | ASN | A | 192 | 244.725 | -64.017 | 98.747 | 1.00 | 54.33 | A |
| ATOM N | 542 | N | ILE | A | | 245.909 | -62.901 | 97.204 | 1.00 | 42.87 | A |
| ATOM C | 543 | CA | ILE | A | 193 | 247.067 | -63.781 | 97.277 | 1.00 | 40.40 | A |
| ATOM C | 544 | CB | ILE | A | 193 | 247.054 | -64.847 | 96.138 | 1.00 | 40.53 | A |
| ATOM C | 545 | CG2 | ILE | A | 193 | 248.292 | -65.733 | 96.243 | 1.00 | 22.41 | A |
| ATOM C | 546 | CG1 | ILE | A | 193 | 245.800 | -65.732 | 96.274 | 1.00 | 36.30 | A |
| ATOM C | 547 | CD1 | ILE | A | 193 | 245.709 | -66.793 | 95.246 | 1.00 | 46.23 | A |
| ATOM C | 548 | C | ILE | A | 193 | 248.330 | -62.944 | 97.180 | 1.00 | 43.53 | A |
| ATOM O | 549 | O | ILE | A | 193 | 248.481 | -62.176 | 96.228 | 1.00 | 41.20 | A |
| ATOM N | 550 | N | LEU | A | 194 | 249.217 | -63.070 | 98.171 | 1.00 | 46.32 | A |
| ATOM C | 551 | CA | LEU | A | 194 | 250.449 | -62.307 | 98.168 | 1.00 | 48.93 | A |
| ATOM C | 552 | CB | LEU | A | 194 | 251.233 | -62.511 | 99.477 | 1.00 | 44.51 | A |
| ATOM C | 553 | CG | LEU | A | 194 | 252.485 | -61.630 | 99.579 | 1.00 | 41.08 | A |
| ATOM C | 554 | CD1 | LEU | A | 194 | 252.055 | -60.195 | 99.956 | 1.00 | 43.64 | A |
| ATOM C | 555 | CD2 | LEU | A | 194 | 253.437 | -62.183 | 100.655 | 1.00 | 41.05 | A |
| ATOM C | 556 | C | LEU | A | 194 | 251.290 | -62.773 | 96.988 | 1.00 | 49.00 | A |
| ATOM O | 557 | O | LEU | A | 194 | 251.553 | -63.975 | 96.826 | 1.00 | 53.77 | A |
| ATOM N | 558 | N | ARG | A | 195 | 251.712 | -61.823 | 96.162 | 1.00 | 43.08 | A |
| ATOM C | 559 | CA | ARG | A | 195 | 252.553 | -62.166 | 95.023 | 1.00 | 45.19 | A |
| ATOM C | 560 | CB | ARG | A | 195 | 252.509 | -61.043 | 93.969 | 1.00 | 43.15 | A |
| ATOM C | 561 | CG | ARG | A | 195 | 251.180 | -60.913 | 93.213 | 1.00 | 46.56 | A |
| ATOM C | 562 | CD | ARG | A | 195 | 251.400 | -60.798 | 91.730 | 1.00 | 59.57 | A |
| ATOM N | 563 | NE | ARG | A | 195 | 252.816 | -60.787 | 91.377 | 1.00 | 72.51 | A |
| ATOM C | 564 | CZ | ARG | A | 195 | 253.288 | -60.716 | 90.133 | 1.00 | 78.66 | A |
| ATOM N | 565 | NH1 | ARG | A | 195 | 252.446 | -60.646 | 89.119 | 1.00 | 80.03 | A |
| ATOM N | 566 | NH2 | ARG | A | 195 | 254.603 | -60.723 | 89.898 | 1.00 | 82.29 | A |
| ATOM C | 567 | C | ARG | A | 195 | 254.004 | -62.450 | 95.390 | 1.00 | 44.60 | A |
| ATOM O | 568 | O | ARG | A | 195 | 254.470 | -62.001 | 96.429 | 1.00 | 47.11 | A |
| ATOM N | 569 | N | LEU | A | 196 | 254.710 | -63.177 | 94.522 | 1.00 | 48.18 | A |
| ATOM C | 570 | CA | LEU | A | 196 | 256.116 | -63.465 | 94.735 | 1.00 | 48.30 | A |
| ATOM C | 571 | CB | LEU | A | 196 | 256.345 | -64.954 | 95.048 | 1.00 | 50.39 | A |
| ATOM C | 572 | CG | LEU | A | 196 | 257.768 | -65.343 | 95.383 | 1.00 | 41.45 | A |
| ATOM C | 573 | CD1 | LEU | A | 196 | 258.090 | -64.804 | 96.698 | 1.00 | 58.09 | A |
| ATOM C | 574 | CD2 | LEU | A | 196 | 257.886 | -66.788 | 95.411 | 1.00 | 44.21 | A |
| ATOM C | 575 | C | LEU | A | 196 | 256.799 | -63.091 | 93.432 | 1.00 | 50.04 | A |
| ATOM O | 576 | O | LEU | A | 196 | 256.887 | -63.926 | 92.532 | 1.00 | 56.48 | A |
| ATOM N | 577 | N | TYR | A | 197 | 257.301 | -61.854 | 93.359 | 1.00 | 52.17 | A |
| ATOM C | 578 | CA | TYR | A | 197 | 257.970 | -61.330 | 92.172 | 1.00 | 49.64 | A |
| ATOM C | 579 | CB | TYR | A | 197 | 258.419 | -59.908 | 92.460 | 1.00 | 44.72 | A |
| ATOM C | 580 | CG | TYR | A | 197 | 257.289 | -59.034 | 92.959 | 1.00 | 43.03 | A |
| ATOM C | 581 | CD1 | TYR | A | 197 | 257.445 | -58.246 | 94.117 | 1.00 | 44.46 | A |
| ATOM C | 582 | CE1 | TYR | A | 197 | 256.439 | -57.433 | 94.583 | 1.00 | 51.15 | A |
| ATOM C | 583 | CD2 | TYR | A | 197 | 256.085 | -58.978 | 92.278 | 1.00 | 41.30 | A |
| ATOM C | 584 | CE2 | TYR | A | 197 | 255.057 | -58.161 | 92.730 | 1.00 | 50.46 | A |
| ATOM C | 585 | CZ | TYR | A | 197 | 255.243 | -57.390 | 93.887 | 1.00 | 52.65 | A |
| ATOM O | 586 | OH | TYR | A | 197 | 254.234 | -56.573 | 94.338 | 1.00 | 63.16 | A |
| ATOM C | 587 | C | TYR | A | 197 | 259.142 | -62.186 | 91.684 | 1.00 | 51.16 | A |
| ATOM O | 588 | O | TYR | A | 197 | 259.129 | -62.683 | 90.572 | 1.00 | 58.21 | A |
| ATOM N | 589 | N | GLY | A | 198 | 260.141 | -62.378 | 92.529 | 1.00 | 53.24 | A |
| ATOM C | 590 | CA | GLY | A | 198 | 261.296 | -63.164 | 92.148 | 1.00 | 47.33 | A |
| ATOM C | 591 | C | GLY | A | 198 | 262.028 | -63.697 | 93.360 | 1.00 | 48.93 | A |
| ATOM O | 592 | O | GLY | A | 198 | 261.447 | -63.928 | 94.412 | 1.00 | 49.80 | A |
| ATOM N | 593 | N | TYR | A | 199 | 263.316 | -63.941 | 93.204 | 1.00 | 48.77 | A |
| ATOM C | 594 | CA | TYR | A | 199 | 264.134 | -64.436 | 94.315 | 1.00 | 50.98 | A |
| ATOM C | 595 | CB | TYR | A | 199 | 263.712 | -65.834 | 94.699 | 1.00 | 54.11 | A |
| ATOM C | 596 | CG | TYR | A | 199 | 264.323 | -66.921 | 93.853 | 1.00 | 56.57 | A |
| ATOM C | 597 | CD1 | TYR | A | 199 | 265.506 | -67.521 | 94.238 | 1.00 | 57.21 | A |
| ATOM C | 598 | CE1 | TYR | A | 199 | 266.088 | -68.526 | 93.471 | 1.00 | 58.18 | A |
| ATOM C | 599 | CD2 | TYR | A | 199 | 263.715 | -67.351 | 92.664 | 1.00 | 59.67 | A |
| ATOM C | 600 | CE2 | TYR | A | 199 | 264.286 | -68.349 | 91.899 | 1.00 | 59.17 | A |
| ATOM C | 601 | CZ | TYR | A | 199 | 265.479 | -68.933 | 92.308 | 1.00 | 59.36 | A |
| ATOM O | 602 | OH | TYR | A | 199 | 266.075 | -69.917 | 91.535 | 1.00 | 63.54 | A |
| ATOM C | 603 | C | TYR | A | 199 | 265.563 | -64.463 | 93.826 | 1.00 | 48.41 | A |
| ATOM O | 604 | O | TYR | A | 199 | 265.787 | -64.357 | 92.640 | 1.00 | 47.41 | A |
| ATOM N | 605 | N | PHE | A | 200 | 266.542 | -64.591 | 94.701 | 1.00 | 43.25 | A |
| ATOM C | 606 | CA | PHE | A | 200 | 267.921 | -64.582 | 94.221 | 1.00 | 40.67 | A |
| ATOM C | 607 | CB | PHE | A | 200 | 268.427 | -63.159 | 93.950 | 1.00 | 43.34 | A |
| ATOM C | 608 | CG | PHE | A | 200 | 268.272 | -62.218 | 95.134 | 1.00 | 48.79 | A |
| ATOM C | 609 | CD1 | PHE | A | 200 | 269.249 | -62.115 | 96.166 | 1.00 | 50.06 | A |
| ATOM C | 610 | CD2 | PHE | A | 200 | 267.178 | -61.322 | 95.162 | 1.00 | 52.88 | A |
| ATOM C | 611 | CE1 | PHE | A | 200 | 269.107 | -61.097 | 97.192 | 1.00 | 53.41 | A |
| ATOM C | 612 | CE2 | PHE | A | 200 | 267.048 | -60.331 | 96.166 | 1.00 | 49.85 | A |
| ATOM C | 613 | CZ | PHE | A | 200 | 267.993 | -60.201 | 97.160 | 1.00 | 49.27 | A |
| ATOM C | 614 | C | PHE | A | 200 | 268.645 | -65.113 | 95.325 | 1.00 | 47.09 | A |
| ATOM O | 615 | O | PHE | A | 200 | 268.037 | -65.599 | 96.262 | 1.00 | 47.13 | A |
| ATOM N | 616 | N | HIS | A | 201 | 269.953 | -65.125 | 95.219 | 1.00 | 56.46 | A |
| ATOM C | 617 | CA | HIS | A | 201 | 270.635 | -65.604 | 96.382 | 1.00 | 56.94 | A |
| ATOM C | 618 | CB | HIS | A | 201 | 270.242 | -67.052 | 96.679 | 1.00 | 62.89 | A |
| ATOM C | 619 | CG | HIS | A | 201 | 270.668 | -68.030 | 95.648 | 1.00 | 66.27 | A |
| ATOM C | 620 | CD2 | HIS | A | 201 | 270.131 | -68.454 | 94.469 | 1.00 | 68.86 | A |
| ATOM N | 621 | ND1 | HIS | A | 201 | 271.876 | -68.658 | 95.778 | 1.00 | 70.42 | A |
| ATOM C | 622 | CE1 | HIS | A | 201 | 272.086 | -69.425 | 94.727 | 1.00 | 63.77 | A |
| ATOM N | 623 | NE2 | HIS | A | 201 | 271.048 | -69.319 | 93.917 | 1.00 | 69.53 | A |
| ATOM C | 624 | C | HIS | A | 201 | 272.082 | -65.339 | 96.531 | 1.00 | 59.01 | A |
| ATOM O | 625 | O | HIS | A | 201 | 272.657 | -64.604 | 95.737 | 1.00 | 56.46 | A |
| ATOM N | 626 | N | ASP | A | 202 | 272.636 | -65.783 | 97.652 | 1.00 | 53.50 | A |
| ATOM C | 627 | CA | ASP | A | 202 | 274.052 | -65.546 | 97.850 | 1.00 | 58.12 | A |
| ATOM C | 628 | CB | ASP | A | 202 | 274.321 | -64.225 | 98.617 | 1.00 | 61.13 | A |
| ATOM C | 629 | CG | ASP | A | 202 | 273.888 | -64.255 | 100.090 | 1.00 | 65.37 | A |
| ATOM O | 630 | OD1 | ASP | A | 202 | 273.969 | -65.308 | 100.803 | 1.00 | 69.09 | A |
| ATOM O | 631 | OD2 | ASP | A | 202 | 273.489 | -63.181 | 100.569 | 1.00 | 73.25 | A |
| ATOM C | 632 | C | ASP | A | 202 | 274.782 | -66.745 | 98.522 | 1.00 | 59.73 | A |
| ATOM O | 633 | O | ASP | A | 202 | 274.357 | -67.899 | 98.426 | 1.00 | 61.93 | A |
| ATOM N | 634 | N | ALA | A | 203 | 275.910 | -66.473 | 99.163 | 1.00 | 59.60 | A |
| ATOM C | 635 | CA | ALA | A | 203 | 276.705 | -67.507 | 99.793 | 1.00 | 59.87 | A |
| ATOM C | 636 | CB | ALA | A | 203 | 278.079 | -66.933 | 100.191 | 1.00 | 57.31 | A |
| ATOM C | 637 | C | ALA | A | 203 | 276.068 | -68.138 | 100.990 | 1.00 | 60.08 | A |
| ATOM O | 638 | O | ALA | A | 203 | 276.287 | -69.294 | 101.229 | 1.00 | 62.79 | A |
| ATOM N | 639 | N | THR | A | 204 | 275.291 | -67.377 | 101.748 | 1.00 | 60.13 | A |
| ATOM C | 640 | CA | THR | A | 204 | 274.700 | -67.893 | 102.968 | 1.00 | 58.14 | A |
| ATOM C | 641 | CB | THR | A | 204 | 275.067 | -67.010 | 104.168 | 1.00 | 54.92 | A |
| ATOM O | 642 | OG1 | THR | A | 204 | 275.068 | -65.622 | 103.799 | 1.00 | 51.82 | A |
| ATOM C | 643 | CG2 | THR | A | 204 | 276.406 | -67.388 | 104.681 | 1.00 | 55.25 | A |
| ATOM C | 644 | C | THR | A | 204 | 273.203 | -68.070 | 102.993 | 1.00 | 61.74 | A |
| ATOM O | 645 | O | THR | A | 204 | 272.662 | -69.063 | 103.547 | 1.00 | 66.23 | A |
| ATOM N | 646 | N | ARG | A | 205 | 272.517 | -67.099 | 102.434 | 1.00 | 58.56 | A |
| ATOM C | 647 | CA | ARG | A | 205 | 271.091 | -67.217 | 102.444 | 1.00 | 58.96 | A |
| ATOM C | 648 | CB | ARG | A | 205 | 270.505 | -66.287 | 103.493 | 1.00 | 56.07 | A |
| ATOM C | 649 | CG | ARG | A | 205 | 270.986 | -64.877 | 103.382 | 1.00 | 59.74 | A |
| ATOM C | 650 | CD | ARG | A | 205 | 271.764 | -64.493 | 104.616 | 1.00 | 62.84 | A |
| ATOM N | 651 | NE | ARG | A | 205 | 272.739 | -63.462 | 104.285 | 1.00 | 73.59 | A |
| ATOM C | 652 | CZ | ARG | A | 205 | 273.407 | -62.747 | 105.185 | 1.00 | 79.55 | A |
| ATOM N | 653 | NH1 | ARG | A | 205 | 273.198 | -62.946 | 106.482 | 1.00 | 80.70 | A |
| ATOM N | 654 | NH2 | ARG | A | 205 | 274.297 | -61.845 | 104.792 | 1.00 | 87.21 | A |
| ATOM C | 655 | C | ARG | A | 205 | 270.418 | -67.012 | 101.120 | 1.00 | 56.37 | A |
| ATOM O | 656 | O | ARG | A | 205 | 271.034 | -66.604 | 100.116 | 1.00 | 64.50 | A |
| ATOM N | 657 | N | VAL | A | 206 | 269.150 | -67.373 | 101.124 | 1.00 | 54.49 | A |
| ATOM C | 658 | CA | VAL | A | 206 | 268.304 | -67.238 | 99.960 | 1.00 | 50.29 | A |
| ATOM C | 659 | CB | VAL | A | 206 | 267.536 | -68.522 | 99.670 | 1.00 | 47.01 | A |
| ATOM C | 660 | CG1 | VAL | A | 206 | 266.800 | -68.370 | 98.374 | 1.00 | 38.39 | A |
| ATOM C | 661 | CG2 | VAL | A | 206 | 268.488 | -69.696 | 99.620 | 1.00 | 42.93 | A |
| ATOM C | 662 | C | VAL | A | 206 | 267.306 | -66.124 | 100.321 | 1.00 | 50.23 | A |
| ATOM O | 663 | O | VAL | A | 206 | 266.870 | -65.982 | 101.487 | 1.00 | 49.82 | A |
| ATOM N | 664 | N | TYR | A | 207 | 266.958 | -65.330 | 99.320 | 1.00 | 47.64 | A |
| ATOM C | 665 | CA | TYR | A | 207 | 266.053 | -64.226 | 99.517 | 1.00 | 47.19 | A |
| ATOM C | 666 | CB | TYR | A | 207 | 266.748 | -62.879 | 99.204 | 1.00 | 44.49 | A |
| ATOM C | 667 | CG | TYR | A | 207 | 268.077 | -62.684 | 99.852 | 1.00 | 52.24 | A |
| ATOM C | 668 | CD1 | TYR | A | 207 | 269.198 | -63.362 | 99.385 | 1.00 | 51.97 | A |
| ATOM C | 669 | CE1 | TYR | A | 207 | 270.438 | -63.194 | 99.978 | 1.00 | 60.89 | A |
| ATOM C | 670 | CD2 | TYR | A | 207 | 268.227 | -61.820 | 100.938 | 1.00 | 55.49 | A |
| ATOM C | 671 | CE2 | TYR | A | 207 | 269.499 | -61.638 | 101.553 | 1.00 | 62.31 | A |
| ATOM C | 672 | CZ | TYR | A | 207 | 270.583 | -62.327 | 101.069 | 1.00 | 60.88 | A |
| ATOM O | 673 | OH | TYR | A | 207 | 271.783 | -62.141 | 101.704 | 1.00 | 68.24 | A |
| ATOM C | 674 | C | TYR | A | 207 | 264.837 | -64.374 | 98.618 | 1.00 | 42.31 | A |
| ATOM O | 675 | O | TYR | A | 207 | 264.940 | -64.704 | 97.438 | 1.00 | 43.03 | A |
| ATOM N | 676 | N | LEU | A | 208 | 263.671 | -64.078 | 99.171 | 1.00 | 47.18 | A |
| ATOM C | 677 | CA | LEU | A | 208 | 262.441 | -64.129 | 98.377 | 1.00 | 49.66 | A |
| ATOM C | 678 | CB | LEU | A | 208 | 261.411 | -65.084 | 99.005 | 1.00 | 50.68 | A |
| ATOM C | 679 | CG | LEU | A | 208 | 261.731 | -66.573 | 98.967 | 1.00 | 50.97 | A |
| ATOM C | 680 | CD1 | LEU | A | 208 | 260.525 | -67.349 | 99.471 | 1.00 | 51.61 | A |
| ATOM C | 681 | CD2 | LEU | A | 208 | 262.019 | -66.964 | 97.575 | 1.00 | 53.77 | A |
| ATOM C | 682 | C | LEU | A | 208 | 261.849 | -62.733 | 98.264 | 1.00 | 50.25 | A |
| ATOM O | 683 | O | LEU | A | 208 | 261.525 | -62.085 | 99.278 | 1.00 | 54.78 | A |
| ATOM N | 684 | N | ILE | A | 209 | 261.692 | -62.286 | 97.027 | 1.00 | 50.37 | A |
| ATOM C | 685 | CA | ILE | A | 209 | 261.154 | -60.971 | 96.752 | 1.00 | 49.74 | A |
| ATOM C | 686 | CB | ILE | A | 209 | 261.695 | -60.499 | 95.420 | 1.00 | 50.44 | A |
| ATOM C | 687 | CG2 | ILE | A | 209 | 261.168 | -59.096 | 95.082 | 1.00 | 50.97 | A |
| ATOM C | 688 | CG1 | ILE | A | 209 | 263.225 | -60.485 | 95.511 | 1.00 | 49.66 | A |
| ATOM C | 689 | CD1 | ILE | A | 209 | 263.919 | -60.607 | 94.181 | 1.00 | 51.66 | A |
| ATOM C | 690 | C | ILE | A | 209 | 259.624 | -61.075 | 96.749 | 1.00 | 51.17 | A |
| ATOM O | 691 | O | ILE | A | 209 | 259.048 | -61.543 | 95.755 | 1.00 | 54.45 | A |
| ATOM N | 692 | N | LEU | A | 210 | 258.981 | -60.655 | 97.854 | 1.00 | 47.03 | A |
| ATOM C | 693 | CA | LEU | A | 210 | 257.530 | -60.728 | 97.990 | 1.00 | 43.40 | A |
| ATOM C | 694 | CB | LEU | A | 210 | 257.164 | -61.286 | 99.362 | 1.00 | 43.91 | A |
| ATOM C | 695 | CG | LEU | A | 210 | 257.762 | -62.625 | 99.773 | 1.00 | 42.46 | A |
| ATOM C | 696 | CD1 | LEU | A | 210 | 257.695 | -62.764 | 101.264 | 1.00 | 44.44 | A |
| ATOM C | 697 | CD2 | LEU | A | 210 | 257.029 | -63.701 | 99.100 | 1.00 | 44.23 | A |
| ATOM C | 698 | C | LEU | A | 210 | 256.873 | -59.393 | 97.827 | 1.00 | 42.69 | A |
| ATOM O | 699 | O | LEU | A | 210 | 257.528 | -58.371 | 97.627 | 1.00 | 50.83 | A |
| ATOM N | 700 | N | GLU | A | 211 | 255.552 | -59.403 | 97.901 | 1.00 | 45.93 | A |
| ATOM C | 701 | CA | GLU | A | 211 | 254.757 | -58.173 | 97.813 | 1.00 | 41.90 | A |
| ATOM C | 702 | CB | GLU | A | 211 | 253.383 | -58.471 | 97.201 | 1.00 | 39-85 | A |
| ATOM C | 703 | CG | GLU | A | 211 | 252.402 | -57.354 | 97.290 | 1.00 | 41.17 | A |
| ATOM C | 704 | CD | GLU | A | 211 | 250.976 | -57.788 | 96.890 | 1.00 | 51.54 | A |
| ATOM O | 705 | OE1 | GLU | A | 211 | 250.012 | -56.980 | 97.025 | 1.00 | 57.54 | A |
| ATOM O | 706 | OE2 | GLU | A | 211 | 250.798 | -58.950 | 96.438 | 1.00 | 52.59 | A |
| ATOM C | 707 | C | GLU | A | 211 | 254.608 | -57.669 | 99.259 | 1.00 | 38.67 | A |
| ATOM O | 708 | O | GLU | A | 211 | 254.507 | -58.477 | 100.200 | 1.00 | 42.39 | A |
| ATOM N | 709 | N | TYR | A | 212 | 254.619 | -56.346 | 99.428 | 1.00 | 43.63 | A |
| ATOM C | 710 | CA | TYR | A | 212 | 254.500 | -55.725 | 100.757 | 1.00 | 47.96 | A |
| ATOM C | 711 | CB | TYR | A | 212 | 255.181 | -54.338 | 100.753 | 1.00 | 46.03 | A |
| ATOM C | 712 | CG | TYR | A | 212 | 255.029 | -53.534 | 102.025 | 1.00 | 44.95 | A |
| ATOM C | 713 | CD1 | TYR | A | 212 | 255.255 | -54.104 | 103.259 | 1.00 | 42.54 | A |
| ATOM C | 714 | CE1 | TYR | A | 212 | 255.084 | -53.363 | 104.429 | 1.00 | 48.54 | A |
| ATOM C | 715 | CD2 | TYR | A | 212 | 254.639 | -52.193 | 101.984 | 1.00 | 44.86 | A |
| ATOM C | 716 | CE2 | TYR | A | 212 | 254.473 | -51.424 | 103.164 | 1.00 | 50.04 | A |
| ATOM C | 717 | CZ | TYR | A | 212 | 254.693 | -52.019 | 104.372 | 1.00 | 48.45 | A |
| ATOM O | 718 | OH | TYR | A | 212 | 254.523 | -51.276 | 105.514 | 1.00 | 53.45 | A |
| ATOM C | 719 | C | TYR | A | 212 | 253.041 | -55.618 | 101.248 | 1.00 | 50.57 | A |
| ATOM O | 720 | O | TYR | A | 212 | 252.153 | -55.140 | 100.508 | 1.00 | 51.02 | A |
| ATOM N | 721 | N | ALA | A | 213 | 252.799 | -56.105 | 102.475 | 1.00 | 48.23 | A |
| ATOM C | 722 | CA | ALA | A | 213 | 251.459 | -56.040 | 103.078 | 1.00 | 51.96 | A |
| ATOM C | 723 | CB | ALA | A | 213 | 251.084 | -57.386 | 103.656 | 1.00 | 42.77 | A |
| ATOM C | 724 | C | ALA | A | 213 | 251.528 | -54.973 | 104.177 | 1.00 | 46.25 | A |
| ATOM O | 725 | O | ALA | A | 213 | 251.958 | -55.259 | 105.280 | 1.00 | 57.39 | A |
| ATOM N | 726 | N | PRO | A | 214 | 251.109 | -53.734 | 103.869 | 1.00 | 45.44 | A |
| ATOM C | 727 | CD | PRO | A | 214 | 250.592 | -53.315 | 102.549 | 1.00 | 45.81 | A |
| ATOM C | 728 | CA | PRO | A | 214 | 251.118 | -52.597 | 104.782 | 1.00 | 37.64 | A |
| ATOM C | 729 | CB | PRO | A | 214 | 250.400 | -51.512 | 103.991 | 1.00 | 44.41 | A |
| ATOM C | 730 | CG | PRO | A | 214 | 250.755 | -51.821 | 102.584 | 1.00 | 45.51 | A |
| ATOM C | 731 | C | PRO | A | 214 | 250.481 | -52.833 | 106.112 | 1.00 | 39.63 | A |
| ATOM O | 732 | O | PRO | A | 214 | 251.116 | -52.486 | 107.123 | 1.00 | 38.67 | A |
| ATOM N | 733 | N | LEU | A | 215 | 249.287 | -53.446 | 106.124 | 1.00 | 33.01 | A |
| ATOM C | 734 | CA | LEU | A | 215 | 248.570 | -53.617 | 107.371 | 1.00 | 35.54 | A |
| ATOM C | 735 | CB | LEU | A | 215 | 247.074 | -53.585 | 107.083 | 1.00 | 39.80 | A |
| ATOM C | 736 | CG | LEU | A | 215 | 246.571 | -52.332 | 106.331 | 1.00 | 39.22 | A |
| ATOM C | 737 | CD1 | LEU | A | 215 | 245.025 | -52.236 | 106.407 | 1.00 | 37.56 | A |
| ATOM C | 738 | CD2 | LEU | A | 215 | 247.156 | -51.166 | 106.898 | 1.00 | 36.39 | A |
| ATOM C | 739 | C | LEU | A | 215 | 248.923 | -54.805 | 108.248 | 1.00 | 43.21 | A |
| ATOM O | 740 | O | LEU | A | 215 | 248.208 | -55.121 | 109.220 | 1.00 | 39.03 | A |
| ATOM N | 741 | N | GLY | A | 216 | 250.022 | -55.466 | 107.906 | 1.00 | 42.62 | A |
| ATOM C | 742 | CA | GLY | A | 216 | 250.443 | -56.630 | 108.669 | 1.00 | 46.42 | A |
| ATOM C | 743 | C | GLY | A | 216 | 249.594 | -57.891 | 108.554 | 1.00 | 44.15 | A |
| ATOM O | 744 | O | GLY | A | 216 | 248.995 | -58.166 | 107.504 | 1.00 | 46.45 | A |
| ATOM N | 745 | N | THR | A | 217 | 249.523 | -58.640 | 109.656 | 1.00 | 39.98 | A |
| ATOM C | 746 | CA | THR | A | 217 | 248.774 | -59.889 | 109.669 | 1.00 | 43.21 | A |
| ATOM C | 747 | CB | THR | A | 217 | 249.583 | -61.040 | 110.286 | 1.00 | 43.67 | A |
| ATOM O | 748 | OG1 | THR | A | 217 | 249.791 | -60.782 | 111.678 | 1.00 | 51.00 | A |
| ATOM C | 749 | CG2 | THR | A | 217 | 250.905 | -61.201 | 109.605 | 1.00 | 41.04 | A |
| ATOM C | 750 | C | THR | A | 217 | 247.450 | -59.858 | 110.416 | 1.00 | 42.99 | A |
| ATOM O | 751 | O | THR | A | 217 | 247.185 | -58.962 | 111.227 | 1.00 | 41.59 | A |
| ATOM N | 752 | N | VAL | A | 218 | 246.646 | -60.881 | 110.166 | 1.00 | 41.73 | A |
| ATOM C | 753 | CA | VAL | A | 218 | 245.335 | -61.037 | 110.766 | 1.00 | 41.12 | A |
| ATOM C | 754 | CB | VAL | A | 218 | 244.564 | -62.091 | 109.970 | 1.00 | 41.84 | A |
| ATOM C | 755 | CG1 | VAL | A | 218 | 243.170 | -62.251 | 110.488 | 1.00 | 43.61 | A |
| ATOM C | 756 | CG2 | VAL | A | 218 | 244.544 | -61.670 | 108.463 | 1.00 | 33.71 | A |
| ATOM C | 757 | C | VAL | A | 218 | 245.610 | -61.486 | 112.201 | 1.00 | 40.94 | A |
| ATOM O | 758 | O | VAL | A | 218 | 244.789 | -61.326 | 113.078 | 1.00 | 51.00 | A |
| ATOM N | 759 | N | TYR | A | 219 | 246.801 | -62.026 | 112.429 | 1.00 | 45.88 | A |
| ATOM C | 760 | CA | TYR | A | 219 | 247.233 | -62.484 | 113.757 | 1.00 | 42.54 | A |
| ATOM C | 761 | CB | TYR | A | 219 | 248.607 | -63.148 | 113.673 | 1.00 | 45.91 | A |
| ATOM C | 762 | CG | TYR | A | 219 | 249.102 | -63.676 | 115.011 | 1.00 | 46.80 | A |
| ATOM C | 763 | CD1 | TYR | A | 219 | 248.755 | -64.925 | 115.464 | 1.00 | 45.83 | A |
| ATOM C | 764 | CE1 | TYR | A | 219 | 249.176 | -65.377 | 116.693 | 1.00 | 53.23 | A |
| ATOM C | 765 | CD2 | TYR | A | 219 | 249.884 | -62.892 | 115.825 | 1.00 | 46.38 | A |
| ATOM C | 766 | CE2 | TYR | A | 219 | 250.310 | -63.314 | 117.053 | 1.00 | 52.61 | A |
| ATOM C | 767 | CZ | TYR | A | 219 | 249.961 | -64.565 | 117.510 | 1.00 | 53.76 | A |
| ATOM O | 768 | OH | TYR | A | 219 | 250.361 | -64.985 | 118.789 | 1.00 | 54.10 | A |
| ATOM C | 769 | C | TYR | A | 219 | 247.323 | -61.287 | 114.702 | 1.00 | 44.93 | A |
| ATOM O | 770 | O | TYR | A | 219 | 246.745 | -61.322 | 115.825 | 1.00 | 40.09 | A |
| ATOM N | 771 | N | ARG | A | 220 | 248.031 | -60.243 | 114.240 | 1.00 | 37.14 | A |
| ATOM C | 772 | CA | ARG | A | 220 | 248.172 | -59.035 | 115.029 | 1.00 | 44.63 | A |
| ATOM C | 773 | CB | ARC | A | 220 | 249.213 | -58.086 | 114.396 | 1.00 | 42.95 | A |
| ATOM C | 774 | C | ARG | A | 220 | 246.828 | -58.318 | 115.225 | 1.00 | 43.95 | A |
| ATOM O | 775 | O | ARG | A | 220 | 246.563 | -57.773 | 116.314 | 1.00 | 44.55 | A |
| ATOM N | 776 | N | GLU | A | 221 | 245.973 | -58.369 | 114.198 | 1.00 | 44.85 | A |
| ATOM C | 777 | CA | GLU | A | 221 | 244.688 | -57.692 | 114.255 | 1.00 | 43.30 | A |
| ATOM C | 778 | CB | GLU | A | 221 | 244.031 | -57.678 | 112.884 | 1.00 | 46.86 | A |
| ATOM C | 779 | CG | GLU | A | 221 | 242.921 | -56.648 | 112.713 | 1.00 | 56.65 | A |
| ATOM C | 780 | CD | GLU | A | 221 | 243.442 | -55.193 | 112.758 | 1.00 | 63.09 | A |
| ATOM O | 781 | OE1 | GLU | A | 221 | 242.629 | -54.238 | 112.637 | 1.00 | 66.10 | A |
| ATOM O | 782 | OE2 | GLU | A | 221 | 244.670 | -55.005 | 112.919 | 1.00 | 66.07 | A |
| ATOM C | 783 | C | GLU | A | 221 | 243.806 | -58.412 | 115.243 | 1.00 | 46.56 | A |
| ATOM **O** | 784 | O | GLU | A | 221 | 242.959 | -57.802 | 115.900 | 1.00 | 44.66 | A |
| ATOM N | 785 | N | LEU | A | 222 | 244.028 | -59.719 | 115.362 | 1.00 | 46.74 | A |
| ATOM C | 786 | CA | LEU | A | 222 | 243.259 | -60.550 | 116.272 | 1.00 | 40.98 | A |
| ATOM C | 787 | CB | LEU | A | 222 | 243.444 | -62.013 | 115.880 | 1.00 | 42.79 | A |
| ATOM C | 788 | CG | LEU | A | 222 | 242.339 | -62.985 | 116.313 | 1.00 | 48.20 | A |
| ATOM C | 789 | CD1 | LEU | A | 222 | 240.982 | -62.509 | 115.826 | 1.00 | 42.35 | A |
| ATOM C | 790 | CD2 | LEU | A | 222 | 242.649 | -64.361 | 115.761 | 1.00 | 47.96 | A |
| ATOM C | 791 | C | LEU | A | 222 | 243.744 | -60.292 | 117.718 | 1.00 | 45.64 | A |
| ATOM O | 792 | O | LEU | A | 222 | 242.995 | -60.390 | 118.692 | 1.00 | 46.35 | A |
| ATOM N | 793 | N | GLN | A | 223 | 245.008 | -59.938 | 117.875 | 1.00 | 47.19 | A |
| ATOM C | 794 | CA | GLN | A | 223 | 245.539 | -59.670 | 119.203 | 1.00 | 48.16 | A |
| ATOM C | 795 | CB | GLN | A | 223 | 247.056 | -59.584 | 119.112 | 1.00 | 49.22 | A |
| ATOM C | 796 | CG | GLN | A | 223 | 247.819 | -60.926 | 119.032 | 1.00 | 56.96 | A |
| ATOM C | 797 | CD | GLN | A | 223 | 249.300 | -60.732 | 118.879 | 1.00 | 66.12 | A |
| ATOM O | 798 | OE1 | GLN | A | 223 | 249.807 | -59.935 | 117.981 | 1.00 | 68.93 | A |
| ATOM N | 799 | NE2 | GLN | A | 223 | 250.067 | -61.453 | 119.755 | 1.00 | 65.96 | A |
| ATOM C | 800 | C | GLN | A | 223 | 244.994 | -58.332 | 119.711 | 1.00 | 54.79 | A |
| ATOM O | 801 | O | GLN | A | 223 | 244.710 | -58.153 | 120.899 | 1.00 | 63.31 | A |
| ATOM N | 802 | N | LYS | A | 224 | 244.877 | -57.398 | 118.779 | 1.00 | 53.43 | A |
| ATOM C | 803 | CA | LYS | A | 224 | 244.412 | -56.042 | 119.021 | 1.00 | 52.16 | A |
| ATOM C | 804 | CB | LYS | A | 224 | 244.668 | -55.276 | 117.729 | 1.00 | 51.41 | A |
| ATOM C | 805 | CG | LYS | A | 224 | 244.740 | -53.805 | 117.796 | 1.00 | 58.82 | A |
| ATOM C | 806 | CD | LYS | A | 224 | 245.109 | -53.234 | 116.429 | 1.00 | 58.34 | A |
| ATOM C | 807 | CE | LYS | A | 224 | 244.105 | -52.175 | 115.957 | 1.00 | 57.11 | A |
| ATOM N | 808 | NZ | LYS | A | 224 | 243.509 | -52.549 | 114.632 | 1.00 | 62.05 | A |
| ATOM C | 809 | C | LYS | A | 224 | 242.909 | -55.999 | 119.415 | 1.00 | 46.25 | A |
| ATOM O | 810 | O | LYS | A | 224 | 242.551 | -55.374 | 120.392 | 1.00 | 53.53 | A |
| ATOM N | 811 | N | LEU | A | 225 | 242.054 | -56.685 | 118.658 | 1.00 | 40.03 | A |
| ATOM C | 812 | CA | LEU | A | 225 | 240.616 | -56.712 | 118.885 | 1.00 | 28.18 | A |
| ATOM C | 813 | CB | LEU | A | 225 | 239.918 | -56.682 | 117.544 | 1.00 | 32.03 | A |
| ATOM C | 814 | CG | LEU | A | 225 | 240.538 | -55.704 | 116.515 | 1.00 | 37.72 | A |
| ATOM C | 815 | CD1 | LEU | A | 225 | 239.725 | -55.736 | 115.208 | 1.00 | 28.64 | A |
| ATOM C | 816 | CD2 | LEU | A | 225 | 240.577 | -54.252 | 117.096 | 1.00 | 32.47 | A |
| ATOM C | 817 | C | LEU | A | 225 | 240.076 | -57.907 | 119.686 | 1.00 | 39.70 | A |
| ATOM O | 818 | O | LEU | A | 225 | 238.896 | -57.954 | 120.056 | 1.00 | 27.45 | A |
| ATOM N | 819 | N | SER | A | 226 | 240.939 | -58.877 | 119.958 | 1.00 | 33.23 | A |
| ATOM C | 820 | CA | SER | A | 226 | 240.528 | -60.051 | 120.695 | 1.00 | 38.91 | A |
| ATOM C | 821 | CB | SER | A | 226 | 239.864 | -59.653 | 122.015 | 1.00 | 42.93 | A |
| ATOM O | 822 | OG | SER | A | 226 | 240.536 | -58.570 | 122.642 | 1.00 | 59.01 | A |
| ATOM C | 823 | C | SER | A | 226 | 239.548 | -60.869 | 119.867 | 1.00 | 40.96 | A |
| ATOM O | 824 | O | SER | A | 226 | 239.777 | -62.029 | 119.592 | 1.00 | 45.01 | A |
| ATOM N | 825 | N | LYS | A | 227 | 238.433 | -60.270 | 119.484 | 1.00 | 43.12 | A |
| ATOM C | 826 | CA | LYS | A | 227 | 237.409 | -60.968 | 118.693 | 1.00 | 37.87 | A |
| ATOM C | 827 | CB | LYS | A | 227 | 236.160 | -61.246 | 119.551 | 1.00 | 41.34 | A |
| ATOM C | 828 | CG | LYS | A | 227 | 236.384 | -62.150 | 120.650 | 1.00 | 49.95 | A |
| ATOM C | 829 | CD | LYS | A | 227 | 235.126 | -62.365 | 121.469 | 1.00 | 59.51 | A |
| ATOM C | 830 | CE | LYS | A | 227 | 234.854 | -61.186 | 122.468 | 1.00 | 64.97 | A |
| ATOM N | 831 | NZ | LYS | A | 227 | 234.813 | -61.628 | 123.907 | 1.00 | 64.50 | A |
| ATOM C | 832 | C | LYS | A | 227 | 237.009 | -60.044 | 117.520 | 1.00 | 37.96 | A |
| ATOM O | 833 | O | LYS | A | 227 | 237.061 | -58.799 | 117.619 | 1.00 | 40.95 | A |
| ATOM N | 834 | N | PHE | A | 228 | 236.575 | -60.655 | 116.431 | 1.00 | 34.94 | A |
| ATOM C | 835 | CA | PHE | A | 228 | 236.175 | -59.917 | 115.262 | 1.00 | 34.74 | A |
| ATOM C | 836 | CB | PHE | A | 228 | 236.793 | -60.534 | 113.998 | 1.00 | 34.85 | A |
| ATOM C | 837 | CG | PHE | A | 228 | 238.248 | -60.293 | 113.849 | 1.00 | 43.10 | A |
| ATOM C | 838 | CD1 | PHE | A | 228 | 238.975 | -61.009 | 112.913 | 1.00 | 43.37 | A |
| ATOM C | 839 | CD2 | PHE | A | 228 | 238.899 | -59.395 | 114.656 | 1.00 | 38.38 | A |
| ATOM C | 840 | CE1 | PHE | A | 228 | 240.341 | -60.832 | 112.796 | 1.00 | 48.51 | A |
| ATOM C | 841 | CE2 | PHE | A | 228 | 240.266 | -59.207 | 114.554 | 1.00 | 47.20 | A |
| ATOM C | 842 | CZ | PHE | A | 228 | 240.999 | -59.919 | 113.631 | 1.00 | 48.16 | A |
| ATOM C | 843 | C | PHE | A | 228 | 234.654 | -60.063 | 115.208 | 1.00 | 40.38 | A |
| ATOM O | 844 | O | PHE | A | 228 | 234.102 | -61.082 | 115.664 | 1.00 | 36.38 | A |
| ATOM N | 845 | N | ASP | A | 229 | 233.963 | -59.053 | 114.687 | 1.00 | 40.55 | A |
| ATOM C | 846 | CA | ASP | A | 229 | 232.525 | -59.184 | 114.534 | 1.00 | 38.86 | A |
| ATOM C | 847 | CB | ASP | A | 229 | 231.823 | -57.832 | 114.440 | 1.00 | 49.26 | A |
| ATOM C | 848 | CG | ASP | A | 229 | 232.394 | -56.912 | 113.357 | 1.00 | 51.97 | A |
| ATOM O | 849 | OD1 | ASP | A | 229 | 232.396 | -57.278 | 112.179 | 1.00 | 54.02 | A |
| ATOM O | 850 | OD2 | ASP | A | 229 | 232.806 | -55.788 | 113.699 | 1.00 | 55.60 | A |
| ATOM C | 851 | C | ASP | A | 229 | 232.180 | -60.027 | 113.304 | 1.00 | 41.70 | A |
| ATOM O | 852 | O | ASP | A | 229 | 233.039 | -60.366 | 112.480 | 1.00 | 35.62 | A |
| ATOM N | 853 | N | GLU | A | 230 | 230.905 | -60.365 | 113.193 | 1.00 | 40.43 | A |
| ATOM C | 854 | CA | GLU | A | 230 | 230.434 | -61.213 | 112.114 | 1.00 | 43.61 | A |
| ATOM C | 855 | CB | GLU | A | 230 | 228.939 | -61.526 | 112.298 | 1.00 | 43.33 | A |
| ATOM C | 856 | CG | GLU | A | 230 | 228.630 | -62.147 | 113.653 | 1.00 | 43.90 | A |
| ATOM C | 857 | CD | GLU | A | 230 | 227.292 | -62.967 | 113.627 | 1.00 | 48.51 | A |
| ATOM O | 858 | OE1 | GLU | A | 230 | 226.230 | -62.374 | 113.308 | 1.00 | 37.93 | A |
| ATOM O | 859 | OE2 | GLU | A | 230 | 227.300 | -64.204 | 113.916 | 1.00 | 42.11 | A |
| ATOM C | 860 | C | GLU | A | 230 | 230.661 | -60.546 | 110.778 | 1.00 | 43.16 | A |
| ATOM O | 861 | O | GLU | A | 230 | 230.878 | -61.205 | 109.779 | 1.00 | 50.29 | A |
| ATOM N | 862 | N | GLN | A | 231 | 230.590 | -59.225 | 110.748 | 1.00 | 49.18 | A |
| ATOM C | 863 | CA | GLN | A | 231 | 230.742 | -58.501 | 109.489 | 1.00 | 49.70 | A |
| ATOM C | 864 | CB | GLN | A | 231 | 230.311 | -57.029 | 109.685 | 1.00 | 57.14 | A |
| ATOM C | 865 | CG | GLN | A | 231 | 229.958 | -56.236 | 108.412 | 1.00 | 62.54 | A |
| ATOM C | 866 | CD | GLN | A | 231 | 231.200 | -55.770 | 107.642 | 1.00 | 71.69 | A |
| ATOM O | 867 | OE1 | GLN | A | 231 | 232.065 | -55.042 | 108.191 | 1.00 | 72.68 | A |
| ATOM N | 868 | NE2 | GLN | A | 231 | 231.300 | -56.184 | 106.359 | 1.00 | 72.33 | A |
| ATOM C | 869 | C | GLN | A | 231 | 232.197 | -58.614 | 109.018 | 1.00 | 45.76 | A |
| ATOM O | 870 | O | GLN | A | 231 | 232.470 | -58.874 | 107.846 | 1.00 | 46.46 | A |
| ATOM N | 871 | N | ARG | A | 232 | 233.131 | -58.437 | 109.949 | 1.00 | 43.95 | A |
| ATOM C | 872 | CA | ARG | A | 232 | 234.560 | -58.503 | 109.661 | 1.00 | 38.33 | A |
| ATOM C | 873 | CB | ARG | A | 232 | 235.365 | -57.998 | 110.861 | 1.00 | 39.16 | A |
| ATOM C | 874 | CG | ARG | A | 232 | 236.867 | -58.073 | 110.703 | 1.00 | 31.46 | A |
| ATOM C | 875 | CD | ARG | A | 232 | 237.514 | -57.172 | 111.705 | 1.00 | 51.25 | A |
| ATOM N | 876 | NE | ARG | A | 232 | 238.871 | -56.815 | 111.334 | 1.00 | 58.24 | A |
| ATOM C | 877 | CZ | ARG | A | 232 | 239.275 | -55.581 | 111.111 | 1.00 | 65.45 | A |
| ATOM N | 878 | NH1 | ARG | A | 232 | 238.395 | -54.594 | 111.226 | 1.00 | 62.94 | A |
| ATOM N | 879 | NH2 | ARG | A | 232 | 240.551 | -55.369 | 110.780 | 1.00 | 72.17 | A |
| ATOM C | 880 | C | ARG | A | 232 | 234.950 | -59.946 | 109.369 | 1.00 | 42.02 | A |
| ATOM O | 881 | O | ARG | A | 232 | 235.757 | -60.183 | 108.517 | 1.00 | 45.04 | A |
| ATOM N | 882 | N | THR | A | 233 | 234.372 | -60.909 | 110.084 | 1.00 | 43.50 | A |
| ATOM C | 883 | CA | THR | A | 233 | 234.697 | -62.320 | 109.891 | 1.00 | 37.00 | A |
| ATOM C | 884 | CB | THR | A | 233 | 234.104 | -63.175 | 110.992 | 1.00 | 35.89 | A |
| ATOM O | 885 | OG1 | THR | A | 233 | 234.543 | -62.696 | 112.268 | 1.00 | 30.74 | A |
| ATOM C | 886 | CG2 | THR | A | 233 | 234.488 | -64.641 | 110.763 | 1.00 | 22.73 | A |
| ATOM C | 887 | C | THR | A | 233 | 234.149 | -62.834 | 108.555 | 1.00 | 45.73 | A |
| ATOM O | 888 | O | THR | A | 233 | 234.857 | -63.523 | 107.844 | 1.00 | 45.99 | A |
| ATOM N | 889 | N | ALA | A | 234 | 232.892 | -62.501 | 108.228 | 1.00 | 44.83 | A |
| ATOM C | 890 | CA | ALA | A | 234 | 232.256 | -62.897 | 106.960 | 1.00 | 41.77 | A |
| ATOM C | 891 | CB | ALA | A | 234 | 230.852 | -62.351 | 106.893 | 1.00 | 39.48 | A |
| ATOM C | 892 | C | ALA | A | 234 | 233.064 | -62.395 | 105.744 | 1.00 | 44.77 | A |
| ATOM O | 893 | O | ALA | A | 234 | 233.273 | -63.131 | 104.786 | 1.00 | 39.69 | A |
| ATOM N | 894 | N | THR | A | 235 | 233.543 | -61.155 | 105.799 | 1.00 | 43.07 | A |
| ATOM C | 895 | CA | THR | A | 235 | 234.328 | -60.579 | 104.722 | 1.00 | 44.31 | A |
| ATOM C | 896 | CB | THR | A | 235 | 234.557 | -59.058 | 104.926 | 1.00 | 44.88 | A |
| ATOM O | 897 | OG1 | THR | A | 235 | 233.306 | -58.373 | 104.984 | 1.00 | 49.29 | A |
| ATOM C | 898 | CG2 | THR | A | 235 | 235.343 | -58.498 | 103.807 | 1.00 | 37.93 | A |
| ATOM C | 899 | C | THR | A | 235 | 235.689 | -61.252 | 104.654 | 1.00 | 47.59 | A |
| ATOM O | 900 | O | THR | A | 235 | 236.169 | -61.504 | 103.551 | 1.00 | 49.69 | A |
| ATOM N | 901 | N | TYR | A | 236 | 236.310 | -61.521 | 105.813 | 1.00 | 47.35 | A |
| ATOM C | 902 | CA | TYR | A | 236 | 237.613 | -62.189 | 105.850 | 1.00 | 46.90 | A |
| ATOM C | 903 | CB | TYR | A | 236 | 238.206 | -62.221 | 107.255 | 1.00 | 42.67 | A |
| ATOM C | 904 | CG | TYR | A | 236 | 239.024 | -61.003 | 107.621 | 1.00 | 45.24 | A |
| ATOM C | 905 | CD1 | TYR | A | 236 | 239.154 | -59.930 | 106.767 | 1.00 | 40.32 | A |
| ATOM C | 906 | CE1 | TYR | A | 236 | 239.909 | -58.819 | 107.134 | 1.00 | 51.10 | A |
| ATOM C | 907 | CD2 | TYR | A | 236 | 239.667 | -60.929 | 108.847 | 1.00 | 46.47 | A |
| ATOM C | 908 | CE2 | TYR | A | 236 | 240.430 | -59.808 | 109.228 | 1.00 | 52.60 | A |
| ATOM C | 909 | CZ | TYR | A | 236 | 240.542 | -58.764 | 108.367 | 1.00 | 49.08 | A |
| ATOM O | 910 | OH | TYR | A | 236 | 241.265 | -57.653 | 108.719 | 1.00 | 58.26 | A |
| ATOM C | 911 | C | TYR | A | 236 | 237.496 | -63.604 | 105.334 | 1.00 | 47.98 | A |
| ATOM O | 912 | O | TYR | A | 236 | 238.469 | -64.148 | 104.832 | 1.00 | 58.71 | A |
| ATOM N | 913 | N | ILE | A | 237 | 236.296 | -64.177 | 105.416 | 1.00 | 50.41 | A |
| ATOM C | 914 | CA | ILE | A | 237 | 236.032 | -65.540 | 104.944 | 1.00 | 46.45 | A |
| ATOM C | 915 | CB | ILE | A | 237 | 234.816 | -66.156 | 105.635 | 1.00 | 45.13 | A |
| ATOM C | 916 | CG2 | ILE | A | 237 | 234.357 | -67.442 | 104.903 | 1.00 | 42.23 | A |
| ATOM C | 917 | CG1 | ILE | A | 237 | 235.157 | -66.440 | 107.101 | 1.00 | 42.93 | A |
| ATOM C | 918 | CD1 | ILE | A | 237 | 236.253 | -67.436 | 107.317 | 1.00 | 39.73 | A |
| ATOM C | 919 | C | ILE | A | 237 | 235.798 | -65.597 | 103.462 | 1.00 | 51.53 | A |
| ATOM O | 920 | O | ILE | A | 237 | 236.213 | -66.548 | 102.822 | 1.00 | 55.68 | A |
| ATOM N | 921 | N | THR | A | 238 | 235.148 | -64.571 | 102.914 | 1.00 | 51.45 | A |
| ATOM C | 922 | CA | THR | A | 238 | 234.858 | -64.526 | 101.471 | 1.00 | 44.65 | A |
| ATOM C | 923 | CB | THR | A | 238 | 233.929 | -63.346 | 101.106 | 1.00 | 44.82 | A |
| ATOM O | 924 | OG1 | THR | A | 238 | 232.653 | -63.512 | 101.719 | 1.00 | 43.65 | A |
| ATOM C | 925 | CG2 | THR | A | 238 | 233.701 | -63.294 | 99.660 | 1.00 | 44.50 | A |
| ATOM C | 926 | C | THR | A | 238 | 236.166 | -64.350 | 100.706 | 1.00 | 47.18 | A |
| ATOM O | 927 | O | THR | A | 238 | 236.467 | -65.090 | 99.782 | 1.00 | 48.37 | A |
| ATOM N | 928 | N | GLU | A | 239 | 236.956 | -63.367 | 101.116 | 1.00 | 49.98 | A |
| ATOM C | 929 | CA | GLU | A | 239 | 238.239 | -63.082 | 100.473 | 1.00 | 52.23 | A |
| ATOM C | 930 | CB | GLU | A | 239 | 238.937 | -61.932 | 101.203 | 1.00 | 54.22 | A |
| ATOM C | 931 | CG | GLU | A | 239 | 238.139 | -60.624 | 101.260 | 1.00 | 55.95 | A |
| ATOM C | 932 | CD | GLU | A | 239 | 238.811 | -59.573 | 102.137 | 1.00 | 58.38 | A |
| ATOM O | 933 | OE1 | GLU | A | 239 | 239.056 | -59.843 | 103.333 | 1.00 | 65.65 | A |
| ATOM O | 934 | OE2 | GLU | A | 239 | 239.099 | -58.468 | 101.634 | 1.00 | 60.73 | A |
| ATOM C | 935 | C | GLU | A | 239 | 239.109 | -64.330 | 100.496 | 1.00 | 50.02 | A |
| ATOM O | 936 | O | GLU | A | 239 | 239.851 | -64.616 | 99.561 | 1.00 | 55.72 | A |
| ATOM N | 937 | N | LEU | A | 240 | 238.978 | -65.104 | 101.557 | 1.00 | 49.14 | A |
| ATOM C | 938 | CA | LEU | A | 240 | 239.768 | -66.305 | 101.702 | 1.00 | 48.27 | A |
| ATOM C | 939 | CB | LEU | A | 240 | 239.776 | -66.717 | 103.173 | 1.00 | 46.82 | A |
| ATOM C | 940 | CG | LEU | A | 240 | 240.917 | -67.581 | 103.679 | 1.00 | 45.79 | A |
| ATOM C | 941 | CD1 | LEU | A | 240 | 242.225 | -67.030 | 103.195 | 1.00 | 50.28 | A |
| ATOM C | 942 | CD2 | LEU | A | 240 | 240.885 | -67.603 | 105.172 | 1.00 | 53.83 | A |
| ATOM C | 943 | C | LEU | A | 240 | 239.209 | -67.420 | 100.833 | 1.00 | 49.65 | A |
| ATOM O | 944 | O | LEU | A | 240 | 239.960 | -68.170 | 100.210 | 1.00 | 53.66 | A |
| ATOM N | 945 | N | ALA | A | 241 | 237.883 | -67.519 | 100.778 | 1.00 | 45.02 | A |
| ATOM C | 946 | CA | ALA | A | 241 | 237.222 | -68.549 | 99.965 | 1.00 | 50.04 | A |
| ATOM C | 947 | CB | ALA | A | 241 | 235.724 | -68.611 | 100.256 | 1.00 | 39.53 | A |
| ATOM C | 948 | C | ALA | A | 241 | 237.447 | -68.278 | 98.481 | 1.00 | 47.71 | A |
| ATOM O | 949 | O | ALA | A | 241 | 237.495 | -69.203 | 97.682 | 1.00 | 54.94 | A |
| ATOM N | 950 | N | ASN | A | 242 | 237.585 | -67.012 | 98.114 | 1.00 | 49.50 | A |
| ATOM C | 951 | CA | ASN | A | 242 | 237.839 | -66.637 | 96.729 | 1.00 | 51.78 | A |
| ATOM C | 952 | CB | ASN | A | 242 | 237.688 | -65.134 | 96.554 | 1.00 | 45.48 | A |
| ATOM C | 953 | CG | ASN | A | 242 | 236.231 | -64.680 | 96.592 | 1.00 | 51.39 | A |
| ATOM O | 954 | OD1 | ASN | A | 242 | 235.972 | -63.489 | 96.769 | 1.00 | 50.23 | A |
| ATOM N | 955 | ND2 | ASN | A | 242 | 235.276 | -65.620 | 96.412 | 1.00 | 43.58 | A |
| ATOM C | 956 | C | ASN | A | 242 | 239.241 | -67.046 | 96.330 | 1.00 | 50.85 | A |
| ATOM O | 957 | O | ASN | A | 242 | 239.419 | -67.736 | 95.323 | 1.00 | 53.87 | A |
| ATOM N | 958 | N | ALA | A | 243 | 240.230 | -66.625 | 97.119 | 1.00 | 48.27 | A |
| ATOM C | 959 | CA | ALA | A | 243 | 241.629 | -66.970 | 96.861 | 1.00 | 42.21 | A |
| ATOM C | 960 | CB | ALA | A | 243 | 242.534 | -66.345 | 97.913 | 1.00 | 45.13 | A |
| ATOM C | 961 | C | ALA | A | 243 | 241.816 | -68.473 | 96.880 | 1.00 | 43.95 | A |
| ATOM O | 962 | O | ALA | A | 243 | 242.498 | -69.016 | 96.026 | 1.00 | 42.43 | A |
| ATOM N | 963 | N | LEU | A | 244 | 241.219 | -69.152 | 97.858 | 1.00 | 40.59 | A |
| ATOM C | 964 | CA | LEU | A | 244 | 241.366 | -70.606 | 97.945 | 1.00 | 48.68 | A |
| ATOM C | 965 | CB | LEU | A | 244 | 240.689 | -71.141 | 99.204 | 1.00 | 42.37 | A |
| ATOM C | 966 | CG | LEU | A | 244 | 241.465 | -70.944 | 100.495 | 1.00 | 39.60 | A |
| ATOM C | 967 | CD1 | LEU | A | 244 | 240.751 | -71.763 | 101.602 | 1.00 | 37.99 | A |
| ATOM C | 968 | CD2 | LEU | A | 244 | 242.889 | -71.432 | 100.340 | 1.00 | 35.22 | A |
| ATOM C | 969 | C | LEU | A | 244 | 240.779 | -71.311 | 96.702 | 1.00 | 56.87 | A |
| ATOM O | 970 | O | LEU | A | 244 | 241.327 | -72.330 | 96.220 | 1.00 | 60.52 | A |
| ATOM N | 971 | N | SER | A | 245 | 239.662 | -70.777 | 96.200 | 1.00 | 61.51 | A |
| ATOM C | 972 | CA | SER | A | 245 | 239.010 | -71.317 | 95.018 | 1.00 | 61.35 | A |
| ATOM C | 973 | CB | SER | A | 245 | 237.785 | -70.501 | 94.692 | 1.00 | 64.64 | A |
| ATOM O | 974 | OG | SER | A | 245 | 237.339 | -70.779 | 93.390 | 1.00 | 71.14 | A |
| ATOM C | 975 | C | SER | A | 245 | 239.981 | -71.242 | 93.855 | 1.00 | 63.54 | A |
| ATOM O | 976 | O | SER | A | 245 | 240.179 | -72.233 | 93.134 | 1.00 | 63.37 | A |
| ATOM N | 977 | N | TYR | A | 246 | 240.581 | -70.063 | 93.679 | 1.00 | 58.29 | A |
| ATOM C | 978 | CA | TYR | A | 246 | 241.571 | -69.855 | 92.633 | 1.00 | 57.08 | A |
| ATOM C | 979 | CB | TYR | A | 246 | 242.123 | -68.455 | 92.719 | 1.00 | 51.38 | A |
| ATOM C | 980 | CG | TYR | A | 246 | 243.247 | -68.195 | 91.754 | 1.00 | 55.36 | A |
| ATOM C | 981 | CD1 | TYR | A | 246 | 242.994 | -67.652 | 90.492 | 1.00 | 50.94 | A |
| ATOM C | 982 | CE1 | TYR | A | 246 | 244.042 | -67.382 | 89.609 | 1.00 | 55.86 | A |
| ATOM C | 983 | CD2 | TYR | A | 246 | 244.584 | -68.473 | 92.111 | 1.00 | 54.39 | A |
| ATOM C | 984 | CE2 | TYR | A | 246 | 245.650 | -68.213 | 91.234 | 1.00 | 55.81 | A |
| ATOM C | 985 | CZ | TYR | A | 246 | 245.372 | -67.665 | 89.982 | 1.00 | 55.07 | A |
| ATOM O | 986 | OH | TYR | A | 246 | 246.409 | -67.409 | 89.112 | 1.00 | 55.05 | A |
| ATOM C | 987 | C | TYR | A | 246 | 242.732 | -70.852 | 92.733 | 1.00 | 57.54 | A |
| ATOM O | 988 | O | TYR | A | 246 | 243.234 | -71.322 | 91.718 | 1.00 | 64.40 | A |
| ATOM N | 989 | N | CYS | A | 247 | 243.150 | -71.180 | 93.951 | 1.00 | 56.76 | A |
| ATOM C | 990 | CA | CYS | A | 247 | 244.245 | -72.120 | 94.148 | 1.00 | 57.81 | A |
| ATOM C | 991 | CB | CYS | A | 247 | 244.779 | -72.066 | 95.599 | 1.00 | 52.55 | A |
| ATOM S | 992 | SG | CYS | A | 247 | 245.687 | -70.569 | 96.004 | 1.00 | 57.32 | A |
| ATOM C | 993 | C | CYS | A | 247 | 243.813 | -73.538 | 93.837 | 1.00 | 59.03 | A |
| ATOM O | 994 | O | CYS | A | 247 | 244.535 | -74.272 | 93.143 | 1.00 | 61.55 | A |
| ATOM N | 995 | N | HIS | A | 248 | 242.659 | -73.930 | 94.374 | 1.00 | 60.08 | A |
| ATOM C | 996 | CA | HIS | A | 248 | 242.151 | -75.291 | 94.156 | 1.00 | 58.63 | A |
| ATOM C | 997 | CB | HIS | A | 248 | 240.916 | -75.540 | 95.036 | 1.00 | 57.40 | A |
| ATOM C | 998 | CG | HIS | A | 248 | 241.217 | -75.606 | 96.496 | 1.00 | 54.60 | A |
| ATOM C | 999 | CD2 | HIS | A | 248 | 242.393 | -75.694 | 97.160 | 1.00 | 54.55 | A |
| ATOM N | 1000 | ND1 | HIS | A | 248 | 240.231 | -75.623 | 97.459 | 1.00 | 54.23 | A |
| ATOM C | 1001 | CE1 | HIS | A | 248 | 240.789 | -75.721 | 98.654 | 1.00 | 51.97 | A |
| ATOM N | 1002 | NE2 | HIS | A | 248 | 242.099 | -75.765 | 98.501 | 1.00 | 50.58 | A |
| ATOM C | 1003 | C | HIS | A | 248 | 241.806 | -75.535 | 92.668 | 1.00 | 58.67 | A |
| ATOM O | 1004 | O | HIS | A | 248 | 241.857 | -76.667 | 92.171 | 1.00 | 50.45 | A |
| ATOM N | 1005 | N | SER | A | 249 | 241.459 | -74.464 | 91.959 | 1.00 | 53.97 | A |
| ATOM C | 1006 | CA | SER | A | 249 | 241.133 | -74.589 | 90.550 | 1.00 | 58.32 | A |
| ATOM C | 1007 | CB | SER | A | 249 | 240.647 | -73.248 | 89.991 | 1.00 | 55.40 | A |
| ATOM O | 1008 | OG | SER | A | 249 | 241.757 | -72.480 | 89.526 | 1.00 | 54.94 | A |
| ATOM C | 1009 | C | SER | A | 249 | 242.407 | -75.020 | 89.798 | 1.00 | 58.15 | A |
| ATOM O | 1010 | O | SER | A | 249 | 242.326 | -75.573 | 88.707 | 1.00 | 70.94 | A |
| ATOM N | 1011 | N | LYS | A | 250 | 243.574 | -74.746 | 90.370 | 1.00 | 51.49 | A |
| ATOM C | 1012 | CA | LYS | A | 250 | 244.820 | -75.119 | 89.753 | 1.00 | 46.67 | A |
| ATOM C | 1013 | CB | LYS | A | 250 | 245.818 | -73.947 | 89.805 | 1.00 | 31.37 | A |
| ATOM C | 1014 | C | LYS | A | 250 | 245.364 | -76.343 | 90.494 | 1.00 | 50.71 | A |
| ATOM O | 1015 | O | LYS | A | 250 | 246.506 | -76.755 | 90.233 | 1.00 | 60.01 | A |
| ATOM N | 1016 | N | ARG | A | 251 | 244.553 | -76.931 | 91.389 | 1.00 | 52.78 | A |
| ATOM C | 1017 | CA | ARG | A | 251 | 244.965 | -78.113 | 92.167 | 1.00 | 55.47 | A |
| ATOM C | 1018 | CB | ARG | A | 251 | 245.383 | -79.258 | 91.244 | 1.00 | 58.86 | A |
| ATOM C | 1019 | CG | ARG | A | 251 | 244.211 | -79.957 | 90.536 | 1.00 | 62.01 | A |
| ATOM C | 1020 | CD | ARG | A | 251 | 244.126 | -81.392 | 90.989 | 1.00 | 65.71 | A |
| ATOM N | 1021 | NE | ARG | A | 251 | 242.807 | -81.741 | 91.512 | 1.00 | 68.34 | A |
| ATOM C | 1022 | CZ | ARG | A | 251 | 242.484 | -82.945 | 92.002 | 1.00 | 72.31 | A |
| ATOM N | 1023 | NH1 | ARG | A | 251 | 243.384 | -83.929 | 92.045 | 1.00 | 72.96 | A |
| ATOM N | 1024 | NH2 | ARG | A | 251 | 241.252 | -83.178 | 92.445 | 1.00 | 71.74 | A |
| ATOM C | 1025 | C | ARG | A | 251 | 246.099 | -77.840 | 93.137 | 1.00 | 53.93 | A |
| ATOM O | 1026 | O | ARG | A | 251 | 247.007 | -78.666 | 93.285 | 1.00 | 57.77 | A |
| ATOM N | 1027 | N | VAL | A | 252 | 246.043 | -76.686 | 93.799 | 1.00 | 56.22 | A |
| ATOM C | 1028 | CA | VAL | A | 252 | 247.072 | -76.314 | 94.774 | 1.00 | 53.20 | A |
| ATOM C | 1029 | CB | VAL | A | 252 | 247.721 | -74.966 | 94.401 | 1.00 | 50.06 | A |
| ATOM C | 1030 | CG1 | VAL | A | 252 | 248.682 | -74.549 | 95.463 | 1.00 | 49.54 | A |
| ATOM C | 1031 | CG2 | VAL | A | 252 | 248.418 | -75.083 | 93.080 | 1.00 | 48.46 | A |
| ATOM C | 1032 | C | VAL | A | 252 | 246.492 | -76.207 | 96.196 | 1.00 | 54.67 | A |
| ATOM O | 1033 | O | VAL | A | 252 | 245.536 | -75.465 | 96.426 | 1.00 | 56.55 | A |
| ATOM N | 1034 | N | ILE | A | 253 | 247.074 | -76.956 | 97.130 | 1.00 | 52.34 | A |
| ATOM C | 1035 | CA | ILE | A | 253 | 246.641 | -76.948 | 98.498 | 1.00 | 42.71 | A |
| ATOM C | 1036 | CB | ILE | A | 253 | 246.696 | -78.341 | 99.103 | 1.00 | 38.24 | A |
| ATOM C | 1037 | CG2 | ILE | A | 253 | 245.676 | -78.449 | 100.219 | 1.00 | 44.12 | A |
| ATOM C | 1038 | CG1 | ILE | A | 253 | 246.279 | -79.380 | 98.083 | 1.00 | 35.92 | A |
| ATOM C | 1039 | CD1 | ILE | A | 253 | 246.298 | -80.813 | 98.644 | 1.00 | 42.55 | A |
| ATOM C | 1040 | C | ILE | A | 253 | 247.600 | -76.096 | 99.279 | 1.00 | 48.08 | A |
| ATOM O | 1041 | O | ILE | A | 253 | 248.808 | -76.288 | 99.163 | 1.00 | 60.87 | A |
| ATOM N | 1042 | N | HIS | A | 254 | 247.084 | -75.165 | 100.086 | 1.00 | 50.60 | A |
| ATOM C | 1043 | CA | HIS | A | 254 | 247.955 | -74.319 | 100.906 | 1.00 | 45.11 | A |
| ATOM C | 1044 | CB | HIS | A | 254 | 247.187 | -73.086 | 101.374 | 1.00 | 43.92 | A |
| ATOM C | 1045 | CG | HIS | A | 254 | 248.071 | -72.014 | 101.916 | 1.00 | 44.85 | A |
| ATOM C | 1046 | CD2 | HIS | A | 254 | 248.486 | -70.854 | 101.360 | 1.00 | 39.20 | A |
| ATOM N | 1047 | MD1 | HIS | A | 254 | 248.670 | -72.089 | 103.158 | 1.00 | 39.59 | A |
| ATOM C | 1048 | CE1 | HIS | A | 254 | 249.414 | -71.014 | 103.339 | 1.00 | 37.61 | A |
| ATOM N | 1049 | NE2 | HIS | A | 254 | 249.318 | -70.250 | 102.265 | 1.00 | 38.54 | A |
| ATOM C | 1050 | C | HIS | A | 254 | 248.490 | -75.117 | 102.103 | 1.00 | 44.15 | A |
| ATOM O | 1051 | O | HIS | A | 254 | 249.681 | -75.123 | 102.372 | 1.00 | 48.22 | A |
| ATOM N | 1052 | N | ARG | A | 255 | 247.587 | -75.802 | 102.793 | 1.00 | 42.94 | A |
| ATOM C | 1053 | CA | ARG | A | 255 | 247.899 | -76.642 | 103.946 | 1.00 | 38.93 | A |
| ATOM C | 1054 | CB | ARG | A | 255 | 248.829 | -77.796 | 103.512 | 1.00 | 44.03 | A |
| ATOM C | 1055 | CG | ARG | A | 255 | 248.460 | -78.369 | 102.124 | 1.00 | 46.01 | A |
| ATOM C | 1056 | CD | ARG | A | 255 | 249.154 | -79.689 | 101.884 | 1.00 | 54.38 | A |
| ATOM N | 1057 | NE | ARG | A | 255 | 250.581 | -79.648 | 102.175 | 1.00 | 53.55 | A |
| ATOM C | 1058 | CZ | ARG | A | 255 | 251.402 | -80.673 | 102.012 | 1.00 | 53.89 | A |
| ATOM N | 1059 | NH1 | ARG | A | 255 | 250.938 | -81.808 | 101.562 | 1.00 | 52.04 | A |
| ATOM N | 1060 | NH2 | ARG | A | 255 | 252.679 | -80.560 | 102.313 | 1.00 | 55.38 | A |
| ATOM C | 1061 | C | ARG | A | 255 | 248.477 | -75.929 | 105.145 | 1.00 | 37.93 | A |
| ATOM O | 1062 | O | ARG | A | 255 | 248.891 | -76.588 | 106.101 | 1.00 | 37.60 | A |
| ATOM N | 1063 | N | ASP | A | 256 | 248.541 | -74.598 | 105.096 | 1.00 | 40.81 | A |
| ATOM C | 1064 | CA | ASP | A | 256 | 249.090 | -73.852 | 106.231 | 1.00 | 41.86 | A |
| ATOM C | 1065 | CB | ASP | A | 256 | 250.578 | -73.685 | 106.031 | 1.00 | 38.62 | A |
| ATOM C | 1066 | CG | ASP | A | 256 | 251.299 | -73.245 | 107.284 | 1.00 | 42.25 | A |
| ATOM O | 1067 | OD1 | ASP | A | 256 | 250.822 | -73.586 | 108.373 | 1.00 | 44.21 | A |
| ATOM O | 1068 | OD2 | ASP | A | 256 | 252.367 | -72.594 | 107.177 | 1.00 | 39.16 | A |
| ATOM C | 1069 | C | ASP | A | 256 | 248.415 | -72.491 | 106.390 | 1.00 | 45.37 | A |
| ATOM O | 1070 | O | ASP | A | 256 | 249.065 | -71.464 | 106.563 | 1.00 | 52.63 | A |
| ATOM N | 1071 | N | ILE | A | 257 | 247.092 | -72.503 | 106.340 | 1.00 | 49.04 | A |
| ATOM C | 1072 | CA | ILE | A | 257 | 246.307 | -71.289 | 106.456 | 1.00 | 56.39 | A |
| ATOM C | 1073 | CB | ILE | A | 257 | 244.939 | -71.461 | 105.776 | 1.00 | 52.32 | A |
| ATOM C | 1074 | CG2 | ILE | A | 257 | 244.163 | -70.205 | 105.893 | 1.00 | 55.85 | A |
| ATOM C | 1075 | CG1 | ILE | A | 257 | 245.143 | -71.785 | 104.305 | 1.00 | 60.94 | A |
| ATOM C | 1076 | CD1 | ILE | A | 257 | 243.873 | -72.064 | 103.557 | 1.00 | 73.36 | A |
| ATOM C | 1077 | C | ILE | A | 257 | 246.097 | -70.940 | 107.922 | 1.00 | 54.79 | A |
| ATOM O | 1078 | O | ILE | A | 257 | 245.502 | -71.720 | 108.659 | 1.00 | 61.06 | A |
| ATOM N | 1079 | N | LYS | A | 258 | 246.594 | -69.781 | 108.345 | 1.00 | 50.33 | A |
| ATOM C | 1080 | CA | LYS | A | 258 | 246.428 | -69.356 | 109.719 | 1.00 | 46.70 | A |
| ATOM C | 1081 | CB | LYS | A | 258 | 247.273 | -70.220 | 110.648 | 1.00 | 41.61 | A |
| ATOM C | 1082 | CG | LYS | A | 258 | 248.687 | -70.371 | 110.232 | 1.00 | 49.22 | A |
| ATOM C | 1083 | CD | LYS | A | 258 | 249.425 | -71.326 | 111.200 | 1.00 | 42.38 | A |
| ATOM C | 1084 | CE | LYS | A | 258 | 250.826 | -71.627 | 110.697 | 1.00 | 51.66 | A |
| ATOM N | 1085 | NZ | LYS | A | 258 | 251.488 | -72.718 | 111.456 | 1.00 | 40.89 | A |
| ATOM C | 1086 | C | LYS | A | 258 | 246.756 | -67.876 | 109.860 | 1.00 | 45.25 | A |
| ATOM O | 1087 | O | LYS | A | 258 | 247.504 | -67.337 | 109.042 | 1.00 | 41.75 | A |
| ATOM N | 1088 | N | PRO | A | 259 | 246.189 | -67.209 | 110.898 | 1.00 | 38.54 | A |
| ATOM C | 1089 | CD | PRO | A | 259 | 245.436 | -67.848 | 111.999 | 1.00 | 30.99 | A |
| ATOM C | 1090 | CA | PRO | A | 259 | 246.389 | -65.786 | 111.172 | 1.00 | 33.18 | A |
| ATOM C | 1091 | CB | PRO | A | 259 | 246.034 | -65.680 | 112.643 | 1.00 | 36.45 | A |
| ATOM C | 1092 | CG | PRO | A | 259 | 244.915 | -66.649 | 112.762 | 1.00 | 29.46 | A |
| ATOM C | 1093 | C | PRO | A | 259 | 247.783 | -65.250 | 110.858 | 1.00 | 39.75 | A |
| ATOM O | 1094 | O | PRO | A | 259 | 247.899 | -64.163 | 110.345 | 1.00 | 46.84 | A |
| ATOM N | 1095 | N | GLU | A | 260 | 248.836 | -65.999 | 111.177 | 1.00 | 43.95 | A |
| ATOM C | 1096 | CA | GLU | A | 260 | 250.227 | -65.591 | 110.916 | 1.00 | 44.86 | A |
| ATOM C | 1097 | CB | GLU | A | 260 | 251.224 | -66.603 | 111.505 | 1.00 | 50.78 | A |
| ATOM C | 1098 | CG | GLU | A | 260 | 251.039 | -66.922 | 112.948 | 1.00 | 65.67 | A |
| ATOM C | 1099 | CD | GLU | A | 260 | 249.918 | -67.897 | 113.213 | 1.00 | 69.34 | A |
| ATOM O | 1100 | OE1 | GLU | A | 260 | 248.727 | -67.499 | 113.338 | 1.00 | 69.34 | A |
| ATOM O | 1101 | OE2 | GLU | A | 260 | 250.264 | -69.089 | 113.299 | 1.00 | 80.21 | A |
| ATOM C | 1102 | C | GLU | A | 260 | 250.514 | -65.476 | 109.430 | 1.00 | 39.11 | A |
| ATOM O | 1103 | O | GLU | A | 260 | 251.197 | -64.554 | 109.027 | 1.00 | 44.76 | A |
| ATOM N | 1104 | N | ASN | A | 261 | 250.029 | -66.425 | 108.622 | 1.00 | 40.15 | A |
| ATOM C | 1105 | CA | ASN | A | 261 | 250.246 | -66.396 | 107.162 | 1.00 | 38.92 | A |
| ATOM C | 1106 | CB | ASN | A | 261 | 250.401 | -67.817 | 106.608 | 1.00 | 36.39 | A |
| ATOM C | 1107 | CG | ASN | A | 261 | 251.539 | -68.579 | 107.266 | 1.00 | 36.54 | A |
| ATOM O | 1108 | OD1 | ASN | A | 261 | 252.609 | -68.045 | 107.462 | 1.00 | 41.73 | A |
| ATOM N | 1109 | ND2 | ASN | A | 261 | 251.317 | -69.831 | 107.578 | 1.00 | 30.81 | A |
| ATOM C | 1110 | C | ASN | A | 261 | 249.151 | -65.638 | 106.361 | 1.00 | 38.95 | A |
| ATOM O | 1111 | O | ASN | A | 261 | 249.142 | -65.652 | 105.149 | 1.00 | 40.40 | A |
| ATOM N | 1112 | N | LEU | A | 262 | 248.245 | -64.953 | 107.046 | 1.00 | 42.51 | A |
| ATOM C | 1113 | CA | LEU | A | 262 | 247.197 | -64.169 | 106.386 | 1.00 | 42.29 | A |
| ATOM C | 1114 | CB | LEU | A | 262 | 245.800 | -64.450 | 106.967 | 1.00 | 36.57 | A |
| ATOM C | 1115 | CG | LEU | A | 262 | 245.209 | -65.819 | 106.657 | 1.00 | 37.41 | A |
| ATOM C | 1116 | CD1 | LEU | A | 262 | 243.773 | -65.817 | 107.071 | 1.00 | 33.79 | A |
| ATOM C | 1117 | CD2 | LEU | A | 262 | 245.324 | -66.152 | 105.193 | 1.00 | 39.29 | A |
| ATOM C | 1118 | C | LEU | A | 262 | 247.548 | -62.703 | 106.582 | 1.00 | 41.70 | A |
| ATOM O | 1119 | O | LEU | A | 262 | 247.543 | -62.205 | 107.717 | 1.00 | 44.29 | A |
| ATOM N | 1120 | N | LEU | A | 263 | 247.857 | -62.021 | 105.473 | 1.00 | 38.70 | A |
| ATOM C | 1121 | CA | LEU | A | 263 | 248.238 | -60.610 | 105.505 | 1.00 | 26.99 | A |
| ATOM C | 1122 | CB | LEU | A | 263 | 249.475 | -60.392 | 104.679 | 1.00 | 36.05 | A |
| ATOM C | 1123 | CG | LEU | A | 263 | 250.658 | -61.333 | 104.917 | 1.00 | 31.41 | A |
| ATOM C | 1124 | CD1 | LEU | A | 263 | 251.820 | -60.991 | 103.978 | 1.00 | 33.22 | A |
| ATOM C | 1125 | CD2 | LEU | A | 263 | 251.081 | -61.269 | 106.325 | 1.00 | 29.25 | A |
| ATOM C | 1126 | C | LEU | A | 263 | 247.144 | -59.677 | 105.013 | 1.00 | 35.80 | A |
| ATOM O | 1127 | O | LEU | A | 263 | 246.220 | -60.100 | 104.304 | 1.00 | 32.87 | A |
| ATOM N | 1128 | N | LEU | A | 264 | 247.245 | -58.408 | 105.410 | 1.00 | 31.98 | A |
| ATOM C | 1129 | CA | LEU | A | 264 | 246.246 | -57.428 | 105.041 | 1.00 | 33.35 | A |
| ATOM C | 1130 | CB | LEU | A | 264 | 245.703 | -56.729 | 106.303 | 1.00 | 31.72 | A |
| ATOM C | 1131 | CG | LEU | A | 264 | 244.971 | -57.619 | 107.303 | 1.00 | 28.92 | A |
| ATOM C | 1132 | CD1 | LEU | A | 264 | 244.728 | -56.820 | 108.620 | 1.00 | 24.69 | A |
| ATOM C | 1133 | CD2 | LEU | A | 264 | 243.644 | -58.095 | 106.660 | 1.00 | 26.84 | A |
| ATOM C | 1134 | C | LEU | A | 264 | 246.884 | -56.419 | 104.110 | 1.00 | 35.86 | A |
| ATOM O | 1135 | O | LEU | A | 264 | 247.974 | -55.944 | 104.368 | 1.00 | 40.12 | A |
| ATOM N | 1136 | N | GLY | A | 265 | 246.188 | -56.100 | 103.026 | 1.00 | 36.89 | A |
| ATOM C | 1137 | CA | GLY | A | 265 | 246.674 | -55.133 | 102.045 | 1.00 | 43.31 | A |
| ATOM C | 1138 | C | GLY | A | 265 | 246.399 | -53.673 | 102.351 | 1.00 | 43.89 | A |
| ATOM O | 1139 | O | GLY | A | 265 | 245.889 | -53.350 | 103.401 | 1.00 | 48.54 | A |
| ATOM N | 1140 | N | SER | A | 266 | 246.742 | -52.796 | 101.422 | 1.00 | 48.23 | A |
| ATOM C | 1141 | CA | SER | A | 266 | 246.567 | -51.364 | 101.591 | 1.00 | 49.09 | A |
| ATOM C | 1142 | CB | SER | A | 266 | 246.936 | -50.647 | 100.304 | 1.00 | 51.41 | A |
| ATOM O | 1143 | OG | SER | A | 266 | 246.057 | -51.012 | 99.237 | 1.00 | 64.31 | A |
| ATOM C | 1144 | C | SER | A | 266 | 245.138 | -51.019 | 101.970 | 1.00 | 51.39 | A |
| ATOM O | 1145 | O | SER | A | 266 | 244.915 | -50.247 | 102.883 | 1.00 | 54.83 | A |
| ATOM N | 1146 | N | ALA | A | 267 | 244.162 | -51.588 | 101.271 | 1.00 | 54.20 | A |
| ATOM C | 1147 | CA | ALA | A | 267 | 242.749 | -51.323 | 101.577 | 1.00 | 47.57 | A |
| ATOM C | 1148 | CB | ALA | A | 267 | 241.921 | -51.394 | 100.320 | 1.00 | 47.18 | A |
| ATOM C | 1149 | C | ALA | A | 267 | 242.203 | -52.330 | 102.595 | 1.00 | 48.50 | A |
| ATOM O | 1150 | O | ALA | A | 267 | 241.010 | -52.582 | 102.620 | 1.00 | 50.25 | A |
| ATOM N | 1151 | N | GLY | A | 268 | 243.084 | -52.913 | 103.410 | 1.00 | 45.91 | A |
| ATOM C | 1152 | CA | GLY | A | 268 | 242.665 | -53.860 | 104.420 | 1.00 | 38.28 | A |
| ATOM C | 1153 | C | GLY | A | 268 | 242.192 | -55.187 | 103.868 | 1.00 | 42.82 | A |
| ATOM O | 1154 | O | GLY | A | 268 | 241.652 | -56.003 | 104.639 | 1.00 | 43.08 | A |
| ATOM N | 1155 | N | GLU | A | 269 | 242.396 | -55.431 | 102.569 | 1.00 | 42.29 | A |
| ATOM C | 1156 | CA | GLU | A | 269 | 241.958 | -56.697 | 101.992 | 1.00 | 40.12 | A |
| ATOM C | 1157 | CB | GLU | A | 269 | 241.996 | -56.635 | 100.480 | 1.00 | 44.93 | A |
| ATOM C | 1158 | CG | GLU | A | 269 | 243.430 | -56.655 | 99.871 | 1.00 | 51.34 | A |
| ATOM C | 1159 | CD | GLU | A | 269 | 243.997 | -55.261 | 99.657 | 1.00 | 57.16 | A |
| ATOM O | 1160 | OE1 | GLU | A | 269 | 244.081 | -54.491 | 100.642 | 1.00 | 51.07 | A |
| ATOM O | 1161 | OE2 | GLU | A | 269 | 244.350 | -54.945 | 98.493 | 1.00 | 61.02 | A |
| ATOM C | 1162 | C | GLU | A | 269 | 242.872 | -57.847 | 102.472 | 1.00 | 44.10 | A |
| ATOM O | 1163 | O | GLU | A | 269 | 244.053 | -57.652 | 102.807 | 1.00 | 43.32 | A |
| ATOM N | 1164 | N | LEU | A | 270 | 242.331 | -59.059 | 102.481 | 1.00 | 43.44 | A |
| ATOM C | 1165 | CA | LEU | A | 270 | 243.076 | -60.228 | 102.927 | 1.00 | 44.15 | A |
| ATOM C | 1166 | CB | LEU | A | 270 | 242.102 | -61.333 | 103.323 | 1.00 | 50.71 | A |
| ATOM C | 1167 | CG | LEU | A | 270 | 242.624 | -62.428 | 104.232 | 1.00 | 53.26 | A |
| ATOM C | 1168 | CD1 | LEU | A | 270 | 242.304 | -62.033 | 105.661 | 1.00 | 60.78 | A |
| ATOM C | 1169 | CD2 | LEU | A | 270 | 241.999 | -63.736 | 103.906 | 1.00 | 50.44 | A |
| ATOM C | 1170 | C | LEU | A | 270 | 243.985 | -60.731 | 101.813 | 1.00 | 44.35 | A |
| ATOM O | 1171 | O | LEU | A | 270 | 243.728 | -60.526 | 100.635 | 1.00 | 49.43 | A |
| ATOM N | 1172 | N | LYS | A | 271 | 245.063 | -61.398 | 102.188 | 1.00 | 40.19 | A |
| ATOM C | 1173 | CA | LYS | A | 271 | 245.994 | -61.946 | 101.211 | 1.00 | 31.70 | A |
| ATOM C | 1174 | CB | LYS | A | 271 | 247.028 | -60.897 | 100.819 | 1.00 | 32.47 | A |
| ATOM C | 1175 | CG | LYS | A | 271 | 246.604 | -59.930 | 99.753 | 1.00 | 31.86 | A |
| ATOM C | 1176 | CD | LYS | A | 271 | 247.492 | -58.692 | 99.731 | 1.00 | 35.87 | A |
| ATOM C | 1177 | CE | LYS | A | 271 | 247.103 | -57.715 | 98.619 | 1.00 | 42.09 | A |
| ATOM N | 1178 | NZ | LYS | A | 271 | 247.551 | -58.225 | 97.303 | 1.00 | 58.37 | A |
| ATOM C | 1179 | C | LYS | A | 271 | 246.714 | -63.148 | 101.820 | 1.00 | 41.22 | A |
| ATOM O | 1180 | O | LYS | A | 271 | 247.433 | -63.023 | 102.807 | 1.00 | 49.74 | A |
| ATOM N | 1181 | N | ILE | A | 272 | 246.505 | -64.322 | 101.249 | 1.00 | 39.27 | A |
| ATOM C | 1182 | CA | ILE | A | 272 | 247.155 | -65.524 | 101.741 | 1.00 | 34.76 | A |
| ATOM C | 1183 | CB | ILE | A | 272 | 246.491 | -66.758 | 101.150 | 1.00 | 44.34 | A |
| ATOM C | 1184 | CG2 | ILE | A | 272 | 246.821 | -67.987 | 102.002 | 1.00 | 55.36 | A |
| ATOM C | 1185 | CG1 | ILE | A | 272 | 244.973 | -66.577 | 101.161 | 1.00 | 50.03 | A |
| ATOM C | 1186 | CD1 | ILE | A | 272 | 244.215 | -67.794 | 100.577 | 1.00 | 52.97 | A |
| ATOM C | 1187 | C | ILE | A | 272 | 248.617 | -65.530 | 101.319 | 1.00 | 41.49 | A |
| ATOM O | 1188 | O | ILE | A | 272 | 248.918 | -65.272 | 100.168 | 1.00 | 40.02 | A |
| ATOM N | 1189 | N | ALA | A | 273 | 249.524 | -65.832 | 102.243 | 1.00 | 39.46 | A |
| ATOM C | 1190 | CA | ALA | A | 273 | 250.936 | -65.861 | 101.931 | 1.00 | 38.71 | A |
| ATOM C | 1191 | CB | ALA | A | 273 | 251.588 | -64.617 | 102.514 | 1.00 | 40.33 | A |
| ATOM C | 1192 | C | ALA | A | 273 | 251.592 | -67.137 | 102.487 | 1.00 | 46.92 | A |
| ATOM O | 1193 | O | ALA | A | 273 | 250.910 | -68.063 | 102.901 | 1.00 | 49.16 | A |
| ATOM N | 1194 | N | ASP | A | 274 | 252.923 | -67.165 | 102.493 | 1.00 | 51.43 | A |
| ATOM C | 1195 | CA | ASP | A | 274 | 253.703 | -68.293 | 102.997 | 1.00 | 51.40 | A |
| ATOM C | 1196 | CB | ASP | A | 274 | 253.686 | -68.323 | 104.530 | 1.00 | 50.98 | A |
| ATOM C | 1197 | CG | ASP | A | 274 | 254.601 | -69.361 | 105.083 | 1.00 | 42.10 | A |
| ATOM O | 1198 | OD1 | ASP | A | 274 | 255.660 | -69.637 | 104.516 | 1.00 | 54.26 | A |
| ATOM O | 1199 | OD2 | ASP | A | 274 | 254.299 | -69.914 | 106.121 | 1.00 | 52.11 | A |
| ATOM C | 1200 | C | ASP | A | 274 | 253.224 | -69.618 | 102.433 | 1.00 | 51.38 | A |
| ATOM O | 1201 | O | ASP | A | 274 | 252.572 | -70.382 | 103.133 | 1.00 | 53.85 | A |
| ATOM N | 1202 | N | PHE | A | 275 | 253.540 | -69.863 | 101.159 | 1.00 | 53.60 | A |
| ATOM C | 1203 | CA | PHE | A | 275 | 253.173 | -71.097 | 100.476 | 1.00 | 46.97 | A |
| ATOM C | 1204 | CB | PHE | A | 275 | 252.975 | -70.871 | 98.973 | 1.00 | 42.90 | A |
| ATOM C | 1205 | CG | PHE | A | 275 | 251.733 | -70.169 | 98.636 | 1.00 | 45.54 | A |
| ATOM C | 1206 | CD1 | PHE | A | 275 | 251.527 | -68.830 | 99.048 | 1.00 | 45.75 | A |
| ATOM C | 1207 | CD2 | PHE | A | 275 | 250.737 | -70.843 | 97.917 | 1.00 | 41.77 | A |
| ATOM C | 1208 | CE1 | PHE | A | 275 | 250.305 | -68.152 | 98.737 | 1.00 | 48.01 | A |
| ATOM C | 1209 | CE2 | PHE | A | 275 | 249.503 | -70.195 | 97.591 | 1.00 | 46.76 | A |
| ATOM C | 1210 | CZ | PHE | A | 275 | 249.279 | -68.840 | 98.000 | 1.00 | 48.77 | A |
| ATOM C | 1211 | C | PHE | A | 275 | 254.270 | -72.149 | 100.678 | 1.00 | 43.41 | A |
| ATOM O | 1212 | O | PHE | A | 275 | 254.496 | -72.970 | 99.791 | 1.00 | 36.81 | A |
| ATOM N | 1213 | N | GLY | A | 276 | 254.938 | -72.109 | 101.843 | 1.00 | 42.93 | A |
| ATOM C | 1214 | CA | GLY | A | 276 | 255.969 | -73.079 | 102.214 | 1.00 | 37.83 | A |
| ATOM C | 1215 | C | GLY | A | 276 | 255.471 | -74.522 | 102.210 | 1.00 | 35.71 | A |
| ATOM O | 1216 | O | GLY | A | 276 | 256.197 | -75.412 | 101.834 | 1.00 | 45.98 | A |
| ATOM N | 1217 | N | TRP | A | 277 | 254.227 | -74.768 | 102.608 | 1.00 | 40.57 | A |
| ATOM C | 1218 | CA | TRP | A | 277 | 253.676 | -76.118 | 102.591 | 1.00 | 36.62 | A |
| ATOM C | 1219 | CB | TRP | A | 277 | 252.882 | -76.378 | 103.859 | 1.00 | 42.09 | A |
| ATOM C | 1220 | CG | TRP | A | 277 | 253.751 | -76.592 | 105.041 | 1.00 | 50.97 | A |
| ATOM C | 1221 | CD2 | TRP | A | 277 | 254.089 | -77.854 | 105.629 | 1.00 | 58.62 | A |
| ATOM C | 1222 | CE2 | TRP | A | 277 | 254.960 | -77.593 | 106.702 | 1.00 | 58.91 | A |
| ATOM C | 1223 | CE3 | TRP | A | 277 | 253.726 | -79.182 | 105.360 | 1.00 | 59.93 | A |
| ATOM C | 1224 | CD1 | TRP | A | 277 | 254.420 | -75.640 | 105.759 | 1.00 | 54.30 | A |
| ATOM N | 1225 | NE1 | TRP | A | 277 | 255.150 | -76.235 | 106.758 | 1.00 | 59.86 | A |
| ATOM C | 1226 | CZ2 | TRP | A | 277 | 255.482 | -78.610 | 107.501 | 1.00 | 61.19 | A |
| ATOM C | 1227 | CZ3 | TRP | A | 277 | 254.244 | -80.188 | 106.162 | 1.00 | 61.29 | A |
| ATOM C | 1228 | CH2 | TRP | A | 277 | 255.108 | -79.892 | 107.219 | 1.00 | 63.73 | A |
| ATOM C | 1229 | C | TRP | A | 277 | 252.777 | -76.364 | 101.408 | 1.00 | 35.74 | A |
| ATOM O | 1230 | O | TRP | A | 277 | 252.212 | -77.442 | 101.298 | 1.00 | 35.61 | A |
| ATOM N | 1231 | N | SER | A | 278 | 252.636 | -75.372 | 100.528 | 1.00 | 44.94 | A |
| ATOM C | 1232 | CA | SER | A | 278 | 251.779 | -75.474 | 99.336 | 1.00 | 46.77 | A |
| ATOM C | 1233 | CB | SER | A | 278 | 251.916 | -74.202 | 98.517 | 1.00 | 42.80 | A |
| ATOM O | 1234 | OG | SER | A | 278 | 250.880 | -74.139 | 97.565 | 1.00 | 54.88 | A |
| ATOM C | 1235 | C | SER | A | 278 | 252.200 | -76.703 | 98.464 | 1.00 | 50.47 | A |
| ATOM O | 1236 | O | SER | A | 278 | 253.367 | -77.084 | 98.378 | 1.00 | 54.17 | A |
| ATOM N | 1237 | N | VAL | A | 279 | 251.246 | -77.347 | 97.819 | 1.00 | 51.50 | A |
| ATOM C | 1238 | CA | VAL | A | 279 | 251.566 | -78.514 | 96.992 | 1.00 | 57.94 | A |
| ATOM C | 1239 | CB | VAL | A | 279 | 251.588 | -79.780 | 97.847 | 1.00 | 44.98 | A |
| ATOM C | 1240 | CG1 | VAL | A | 279 | 250.199 | -80.321 | 97.997 | 1.00 | 43.31 | A |
| ATOM C | 1241 | CG2 | VAL | A | 279 | 252.491 | -80.787 | 97.178 | 1.00 | 57.04 | A |
| ATOM C | 1242 | C | VAL | A | 279 | 250.626 | -78.680 | 95.765 | 1.00 | 59.15 | A |
| ATOM O | 1243 | 0 | VAL | A | 279 | 249.430 | -78.387 | 95.867 | 1.00 | 67.05 | A |
| ATOM N | 1244 | N | HIS | A | 280 | 251.204 | -79.094 | 94.615 | 1.00 | 63.81 | A |
| ATOM C | 1245 | CA | HIS | A | 280 | 250.486 | -79.252 | 93.335 | 1.00 | 67.43 | A |
| ATOM C | 1246 | CB | HIS | A | 280 | 251.439 | -79.066 | 92.122 | 1.00 | 66.79 | A |
| ATOM C | 1247 | CG | HIS | A | 280 | 250.875 | -78.185 | 91.056 | 1.00 | 72.05 | A |
| ATOM C | 1248 | CD2 | HIS | A | 280 | 250.729 | -78.371 | 89.721 | 1.00 | 75.11 | A |
| ATOM N | 1249 | ND1 | HIS | A | 280 | 250.404 | -76.921 | 91.326 | 1.00 | 73.34 | A |
| ATOM C | 1250 | CE1 | HIS | A | 280 | 249.997 | -76.358 | 90.201 | 1.00 | 80.03 | A |
| ATOM N | 1251 | NE2 | HIS | A | 280 | 250.185 | -77.217 | 89.213 | 1.00 | 83.14 | A |
| ATOM C | 1252 | C | HIS | A | 280 | 249.968 | -80.672 | 93.390 | 1.00 | 70.50 | A |
| ATOM O | 1253 | O | HIS | A | 280 | 250.581 | -81.589 | 92.745 | 1.00 | 82.51 | A |
| ATOM N | 1254 | N | ALA | A | 281 | 248.772 | -80.853 | 93.985 | 1.00 | 68.02 | A |
| ATOM C | 1255 | CA | ALA | A | 281 | 248.427 | -82.260 | 94.196 | 1.00 | 68.33 | A |
| ATOM C | 1256 | CB | ALA | A | 281 | 249.603 | -82.885 | 94.806 | 1.00 | 60.98 | A |
| ATOM C | 1257 | C | ALA | A | 281 | 247.205 | -82.754 | 95.016 | 1.00 | 68.64 | A |
| ATOM O | 1258 | O | ALA | A | 281 | 246.292 | -82.032 | 95.339 | 1.00 | 67.99 | A |
| ATOM N | 1259 | N | PRO | A | 282 | 247.225 | -84.034 | 95.373 | 1.00 | 70.43 | A |
| ATOM C | 1260 | CD | PRO | A | 282 | 246.861 | -84.236 | 93.943 | 1.00 | 64.78 | A |
| ATOM C | 1261 | CA | PRO | A | 282 | 246.609 | -85.171 | 96.058 | 1.00 | 67.06 | A |
| ATOM C | 1262 | CB | PRO | A | 282 | 246.654 | -86.295 | 95.053 | 1.00 | 67.65 | A |
| ATOM C | 1263 | CG | PRO | A | 282 | 246.201 | -85.826 | 93.934 | 1.00 | 63.34 | A |
| ATOM C | 1264 | C | PRO | A | 282 | 247.893 | -85.384 | 96.902 | 1.00 | 69.62 | A |
| ATOM O | 1265 | O | PRO | A | 282 | 248.995 | -85.628 | 96.346 | 1.00 | 80.00 | A |
| ATOM N | 1266 | N | SER | A | 283 | 247.878 | -85.322 | 98.204 | 1.00 | 65.02 | A |
| ATOM C | 1267 | CA | SER | A | 283 | 249.163 | -85.680 | 98.758 | 1.00 | 59.53 | A |
| ATOM C | 1268 | CB | SER | A | 283 | 250.127 | -84.498 | 98.799 | 1.00 | 55.46 | A |
| ATOM O | 1269 | OG | SER | A | 283 | 250.750 | -84.456 | 100.075 | 1.00 | 55.84 | A |
| ATOM C | 1270 | C | SER | A | 283 | 249.259 | -86.394 | 100.033 | 1.00 | 55.99 | A |
| ATOM O | 1271 | O | SER | A | 283 | 248.283 | -86.609 | 100.713 | 1.00 | 69.93 | A |
| ATOM N | 1272 | N | SER | A | 284 | 250.462 | -86.835 | 100.323 | 1.00 | 60.56 | A |
| ATOM C | 1273 | CA | SER | A | 284 | 250.699 | -87.467 | 101.588 | 1.00 | 63.49 | A |
| ATOM C | 1274 | CB | SER | A | 284 | 251.416 | -88.808 | 101.438 | 1.00 | 63.76 | A |
| ATOM O | 1275 | OG | SER | A | 284 | 252.588 | -88.697 | 100.633 | 1.00 | 77.24 | A |
| ATOM C | 1276 | C | SER | A | 284 | 251.609 | -86.432 | 102.280 | 1.00 | 63.54 | A |
| ATOM O | 1277 | O | SER | A | 284 | 251.934 | -85.336 | 101.744 | 1.00 | 57.98 | A |
| ATOM N | 1278 | N | ARG | A | 285 | 251.999 | -86.775 | 103.492 | 1.00 | 64.16 | A |
| ATOM C | 1279 | CA | ARG | A | 285 | 252.823 | -85.889 | 104.282 | 1.00 | 67.66 | A |
| ATOM C | 1280 | CB | ARG | A | 285 | 252.738 | -86.301 | 105.734 | 1.00 | 62.91 | A |
| ATOM C | 1281 | C | ARG | A | 285 | 254.278 | -85.923 | 103.806 | 1.00 | 68.61 | A |
| ATOM O | 1282 | O | ARG | A | 285 | 254.612 | -86.645 | 102.888 | 1.00 | 72.84 | A |
| ATOM N | 1283 | N | ARG | A | 286 | 255.135 | -85.146 | 104.466 | 1.00 | 71.24 | A |
| ATOM C | 1284 | CA | ARG | A | 286 | 256.561 | -85.059 | 104.163 | 1.00 | 75.06 | A |
| ATOM C | 1285 | CB | ARG | A | 286 | 256.943 | -83.563 | 104.008 | 1.00 | 73.44 | A |
| ATOM C | 1286 | CG | ARG | A | 286 | 255.881 | -82.698 | 103.382 | 1.00 | 71.75 | A |
| ATOM C | 1287 | CD | ARG | A | 286 | 255.139 | -83.449 | 102.361 | 1.00 | 72.37 | A |
| ATOM N | 1288 | NE | ARG | A | 286 | 255.481 | -82.999 | 101.030 | 1.00 | 72.92 | A |
| ATOM C | 1289 | CZ | ARG | A | 286 | 254.690 | -83.207 | 99.989 | 1.00 | 77.01 | A |
| ATOM N | 1290 | NH1 | ARG | A | 286 | 253.537 | -83.858 | 100.174 | 1.00 | 80.80 | A |
| ATOM N | 1291 | NH2 | ARG | A | 286 | 255.024 | -82.753 | 98.778 | 1.00 | 73.00 | A |
| ATOM C | 1292 | C | ARG | A | 286 | 257.251 | -85.649 | 105.339 | 1.00 | 79.18 | A |
| ATOM O | 1293 | O | ARG | A | 286 | 258.525 | -85.258 | 105.556 | 1.00 | 80.60 | A |
| ATOM N | 1294 | N | TPO | A | 287 | 256.458 | -86.471 | 106.097 | 1.00 | 82.59 | A |
| ATOM C | 1295 | CA | TPO | A | 287 | 256.848 | -87.091 | 107.376 | 1.00 | 81.36 | A |
| ATOM C | 1296 | CB | TPO | A | 287 | 258.340 | -87.583 | 107.506 | 1.00 | 85.77 | A |
| ATOM C | 1297 | CG2 | TPO | A | 287 | 258.788 | -88.436 | 108.791 | 1.00 | 78.62 | A |
| ATOM O | 1298 | OG1 | TPO | A | 287 | 259.242 | -87.965 | 106.406 | 1.00 | 96.25 | A |
| ATOM P | 1299 | P | TPO | A | 287 | 259.671 | -89.537 | 105.840 | 1.00 | 94.10 | A |
| ATOM O | 1300 | O1P | TPO | A | 287 | 261.195 | -89.889 | 106.372 | 1.00 | 105.80 | A |
| ATOM O | 1301 | O2P | TPO | A | 287 | 259.793 | -89.485 | 104.142 | 1.00 | 103.14 | A |
| ATOM O | 1302 | O3P | TPO | A | 287 | 258.773 | -90.552 | 106.786 | 1.00 | 103.19 | A |
| ATOM C | 1303 | C | TPO | A | 287 | 257.020 | -86.093 | 108.560 | 1.00 | 84.40 | A |
| ATOM O | 1304 | O | TPO | A | 287 | 256.708 | -86.453 | 109.760 | 1.00 | 82.15 | A |
| ATOM N | 1305 | N | TPO | A | 288 | 257.164 | -84.776 | 108.357 | 1.00 | 86.52 | A |
| ATOM C | 1306 | CA | TPO | A | 288 | 257.256 | -84.113 | 109.592 | 1.00 | 85.55 | A |
| ATOM C | 1307 | CB | TPO | A | 288 | 258.562 | -83.387 | 109.678 | 1.00 | 87.59 | A |
| ATOM C | 1308 | CG2 | TPO | A | 288 | 259.265 | -83.063 | 111.066 | 1.00 | 82.26 | A |
| ATOM O | 1309 | OG1 | TPO | A | 288 | 259.687 | -83.759 | 108.739 | 1.00 | 93.55 | A |
| ATOM P | 1310 | P | TPO | A | 288 | 261.048 | -84.822 | 108.978 | 1.00 | 87.77 | A |
| ATOM O | 1311 | O1P | TPO | A | 288 | 262.388 | -83.892 | 109.216 | 1.00 | 91.79 | A |
| ATOM O | 1312 | O2P | TPO | A | 288 | 261.352 | -85.656 | 107.542 | 1.00 | 93.35 | A |
| ATOM O | 1313 | O3P | TPO | A | 288 | 260.797 | -85.456 | 110.481 | 1.00 | 95.16 | A |
| ATOM C | 1314 | C | TPO | A | 288 | 256.287 | -82.985 | 109.892 | 1.00 | 89.28 | A |
| ATOM O | 1315 | O | TPO | A | 288 | 255.311 | -82.594 | 109.048 | 1.00 | 86.14 | A |
| ATOM N | 1316 | N | LEU | A | 289 | 256.536 | -82.411 | 111.055 | 1.00 | 87.01 | A |
| ATOM C | 1317 | CA | LEU | A | 289 | 255.511 | -81.523 | 111.435 | 1.00 | 88.25 | A |
| ATOM C | 1318 | CB | LEU | A | 289 | 254.346 | -82.345 | 111.883 | 1.00 | 78.91 | A |
| ATOM C | 1319 | C | LEU | A | 289 | 255.852 | -80.565 | 112.436 | 1.00 | 87.34 | A |
| ATOM O | 1320 | O | LEU | A | 289 | 256.026 | -80.894 | 113.634 | 1.00 | 90.27 | A |
| ATOM N | 1321 | N | CYS | A | 290 | 256.101 | -79.389 | 111.914 | 1.00 | 89.51 | A |
| ATOM C | 1322 | CA | CYS | A | 290 | 256.347 | -78.354 | 112.824 | 1.00 | 89.73 | A |
| ATOM C | 1323 | CB | CYS | A | 290 | 257.299 | -77.405 | 112.324 | 1.00 | 80.05 | A |
| ATOM C | 1324 | C | CYS | A | 290 | 254.931 | -77.891 | 112.490 | 1.00 | 90.22 | A |
| ATOM O | 1325 | O | CYS | A | 290 | 254.405 | -77.933 | 111.292 | 1.00 | 91.11 | A |
| ATOM N | 1326 | N | GLY | A | 291 | 254.262 | -77.496 | 113.541 | 1.00 | 90.88 | A |
| ATOM C | 1327 | CA | GLY | A | 291 | 252.946 | -77.026 | 113.294 | 1.00 | 84.36 | A |
| ATOM C | 1328 | C | GLY | A | 291 | 252.214 | -77.175 | 114.559 | 1.00 | 80.67 | A |
| ATOM O | 1329 | O | GLY | A | 291 | 252.395 | -78.093 | 115.381 | 1.00 | 72.19 | A |
| ATOM N | 1330 | N | THR | A | 292 | 251.411 | -76.152 | 114.668 | 1.00 | 72.87 | A |
| ATOM C | 1331 | CA | THR | A | 292 | 250.543 | -75.923 | 115.732 | 1.00 | 68.66 | A |
| ATOM C | 1332 | CB | THR | A | 292 | 250.229 | -74.450 | 115.788 | 1.00 | 64.26 | A |
| ATOM O | 1333 | OG1 | THR | A | 292 | 249.385 | -74.238 | 116.907 | 1.00 | 62.83 | A |
| ATOM C | 1334 | CG2 | THR | A | 292 | 249.511 | -73.967 | 114.478 | 1.00 | 66.76 | A |
| ATOM C | 1335 | C | THR | A | 292 | 249.401 | -76.698 | 115.093 | 1.00 | 69.67 | A |
| ATOM O | 1336 | O | THR | A | 292 | 248.736 | -76.230 | 114.162 | 1.00 | 81.32 | A |
| ATOM N | 1337 | N | LEU | A | 293 | 249.162 | -77.905 | 115.545 | 1.00 | 65.76 | A |
| ATOM C | 1338 | CA | LEU | A | 293 | 248.076 | -78.633 | 114.929 | 1.00 | 57.85 | A |
| ATOM C | 1339 | CB | LEU | A | 293 | 247.775 | -79.863 | 115.741 | 1.00 | 58.26 | A |
| ATOM C | 1340 | CG | LEU | A | 293 | 249.007 | -80.649 | 116.175 | 1.00 | 59.75 | A |
| ATOM C | 1341 | CD1 | LEU | A | 293 | 248.607 | -81.606 | 117.246 | 1.00 | 56.85 | A |
| ATOM C | 1342 | CD2 | LEU | A | 293 | 249.618 | -81.397 | 114.982 | 1.00 | 55.33 | A |
| ATOM C | 1343 | C | LEU | A | 293 | 246.812 | -77.819 | 114.737 | 1.00 | 54.37 | A |
| ATOM O | 1344 | O | LEU | A | 293 | 246.215 | -77.930 | 113.686 | 1.00 | 59.11 | A |
| ATOM N | 1345 | N | ASP | A | 294 | 246.467 | -76.947 | 115.687 | 1.00 | 45.72 | A |
| ATOM C | 1346 | CA | ASP | A | 294 | 245.241 | -76.166 | 115.622 | 1.00 | 46.04 | A |
| ATOM C | 1347 | CB | ASP | A | 294 | 245.471 | -74.762 | 116.195 | 1.00 | 47.18 | A |
| ATOM C | 1348 | CG | ASP | A | 294 | 245.754 | -74.782 | 117.713 | 1.00 | 53.87 | A |
| ATOM O | 1349 | OD1 | ASP | A | 294 | 244.938 | -75.399 | 116.452 | 1.00 | 53.07 | A |
| ATOM O | 1350 | OD2 | ASP | A | 294 | 246.772 | -74.166 | 118.153 | 1.00 | 44.18 | A |
| ATOM C | 1351 | C | ASP | A | 294 | 244.490 | -76.064 | 114.323 | 1.00 | 42.63 | A |
| ATOM O | 1352 | O | ASP | A | 294 | 243.322 | -76.434 | 114.275 | 1.00 | 40.46 | A |
| ATOM N | 1353 | N | TYR | A | 295 | 245.168 | -75.589 | 113.280 | 1.00 | 41.51 | A |
| ATOM C | 1354 | CA | TYR | A | 295 | 244.534 | -75.404 | 111.983 | 1.00 | 42.29 | A |
| ATOM C | 1355 | CB | TYR | A | 295 | 245.046 | -74.116 | 111.335 | 1.00 | 42.13 | A |
| ATOM C | 1356 | CG | TYR | A | 295 | 245.120 | -72.945 | 112.263 | 1.00 | 41.89 | A |
| ATOM C | 1357 | CD1 | TYR | A | 295 | 246.211 | -72.793 | 113.099 | 1.00 | 47.14 | A |
| ATOM C | 1358 | CE1 | TYR | A | 295 | 246.304 | -71.731 | 113.973 | 1.00 | 47.33 | A |
| ATOM C | 1359 | CD2 | TYR | A | 295 | 244.117 | -71.995 | 112.309 | 1.00 | 36.13 | A |
| ATOM C | 1360 | CE2 | TYR | A | 295 | 244.212 | -70.914 | 113.162 | 1.00 | 43.23 | A |
| ATOM C | 1361 | CZ | TYR | A | 295 | 245.302 | -70.785 | 113.996 | 1.00 | 47.19 | A |
| ATOM O | 1362 | OH | TYR | A | 295 | 245.414 | -69.690 | 114.838 | 1.00 | 57.32 | A |
| ATOM C | 1363 | C | TYR | A | 295 | 244.655 | -76.563 | 110.985 | 1.00 | 41.49 | A |
| ATOM O | 1364 | O | TYR | A | 295 | 244.224 | -76.467 | 109.822 | 1.00 | 43.26 | A |
| ATOM N | 1365 | N | LEU | A | 296 | 245.228 | -77.668 | 111.448 | 1.00 | 39.99 | A |
| ATOM C | 1366 | CA | LEU | A | 296 | 245.411 | -78.868 | 110.607 | 1.00 | 51.39 | A |
| ATOM C | 1367 | CB | LEU | A | 296 | 246.671 | -79.611 | 111.016 | 1.00 | 53.81 | A |
| ATOM C | 1368 | CG | LEU | A | 296 | 248.033 | -78.987 | 110.652 | 1.00 | 57.41 | A |
| ATOM C | 1369 | CD1 | LEU | A | 296 | 249.165 | -79.934 | 111.025 | 1.00 | 51.84 | A |
| ATOM C | 1370 | CD2 | LEU | A | 296 | 248.040 | -78.694 | 109.161 | 1.00 | 50.59 | A |
| ATOM C | 1371 | C | LEU | A | 296 | 244.205 | -79.803 | 110.651 | 1.00 | 48.90 | A |
| ATOM O | 1372 | 0 | LEU | A | 296 | 243.511 | -79.890 | 111.645 | 1.00 | 60.00 | A |
| ATOM N | 1373 | N | PRO | A | 297 | 243.914 | -80.480 | 109.550 | 1.00 | 49.64 | A |
| ATOM C | 1374 | CD | PRO | A | 297 | 244.428 | -80.152 | 108.220 | 1.00 | 48.93 | A |
| ATOM C | 1375 | CA | PRO | A | 297 | 242.782 | -81.390 | 109.447 | 1.00 | 53.31 | A |
| ATOM C | 1376 | CB | PRO | A | 297 | 242.450 | -81.327 | 107.951 | 1.00 | 50.04 | A |
| ATOM C | 1377 | CG | PRO | A | 297 | 243.782 | -81.194 | 107.367 | 1.00 | 48.93 | A |
| ATOM C | 1378 | C | PRO | A | 297 | 243.137 | -82.762 | 109.966 | 1.00 | 57.23 | A |
| ATOM O | 1379 | O | PRO | A | 297 | 244.303 | -83.109 | 110.051 | 1.00 | 56.18 | A |
| ATOM N | 1380 | N | PRO | A | 298 | 242.135 | -83.562 | 110.334 | 1.00 | 59.72 | A |
| ATOM C | 1381 | CD | PRO | A | 298 | 240.704 | -83.237 | 110.308 | 1.00 | 64.33 | A |
| ATOM C | 1382 | CA | PRO | A | 298 | 242.340 | -84.917 | 110.853 | 1.00 | 62.61 | A |
| ATOM C | 1383 | CB | PRO | A | 298 | 240.929 | -85.463 | 110.920 | 1.00 | 65.22 | A |
| ATOM C | 1384 | CG | PRO | A | 298 | 240.143 | -84.267 | 111.288 | 1.00 | 64.88 | A |
| ATOM C | 1385 | C | PRO | A | 298 | 243.245 | -85.779 | 109.963 | 1.00 | 64.63 | A |
| ATOM O | 1386 | O | PRO | A | 298 | 244.184 | -86.426 | 110.459 | 1.00 | 56.23 | A |
| ATOM N | 1387 | N | GLU | A | 299 | 242.975 | -85.781 | 108.656 | 1.00 | 60.03 | A |
| ATOM C | 1388 | CA | GLU | A | 299 | 243.778 | -86.574 | 107.711 | 1.00 | 58.56 | A |
| ATOM C | 1389 | CB | GLU | A | 299 | 243.303 | -86.339 | 106.265 | 1.00 | 57.01 | A |
| ATOM C | 1390 | CG | GLU | A | 299 | 243.028 | -84.864 | 105.914 | 1.00 | 52.94 | A |
| ATOM C | 1391 | CD | GLU | A | 299 | 241.596 | -84.474 | 106.109 | 1.00 | 48.80 | A |
| ATOM O | 1392 | OE1 | GLU | A | 299 | 241.003 | -84.897 | 107.126 | 1.00 | 47.74 | A |
| ATOM O | 1393 | OE2 | GLU | A | 299 | 241.075 | -83.733 | 105.248 | 1.00 | 48.97 | A |
| ATOM C | 1394 | C | GLU | A | 299 | 245.279 | -86.287 | 107.819 | 1.00 | 62.41 | A |
| ATOM O | 1395 | O | GLU | A | 299 | 246.094 | -87.208 | 108.002 | 1.00 | 64.05 | A |
| ATOM N | 1396 | N | MET | A | 300 | 245.648 | -85.014 | 107.709 | 1.00 | 63.79 | A |
| ATOM C | 1397 | CA | MET | A | 300 | 247.051 | -84.637 | 107.816 | 1.00 | 63.71 | A |
| ATOM C | 1398 | CB | MET | A | 300 | 247.225 | -83.145 | 107.597 | 1.00 | 67.63 | A |
| ATOM C | 1399 | CG | MET | A | 300 | 247.374 | -82.763 | 106.145 | 1.00 | 66.70 | A |
| ATOM S | 1400 | SD | MET | A | 300 | 248.052 | -81.127 | 106.017 | 1.00 | 71.20 | A |
| ATOM C | 1401 | CE | MET | A | 300 | 249.750 | -81.388 | 106.380 | 1.00 | 69.10 | A |
| ATOM C | 1402 | C | MET | A | 300 | 247.661 | -85.000 | 109.174 | 1.00 | 66.02 | A |
| ATOM O | 1403 | O | MET | A | 300 | 248.702 | -85.664 | 109.256 | 1.00 | 70.05 | A |
| ATOM N | 1404 | N | ILE | A | 301 | 247.025 | -84.550 | 110.244 | 1.00 | 67.53 | A |
| ATOM C | 1405 | CA | ILE | A | 301 | 247.504 | -84.829 | 111.596 | 1.00 | 68.63 | A |
| ATOM C | 1406 | CB | ILE | A | 301 | 246.490 | -84.278 | 112.606 | 1.00 | 67.77 | A |
| ATOM C | 1407 | CG2 | ILE | A | 301 | 246.782 | -84.750 | 113.995 | 1.00 | 61.97 | A |
| ATOM C | 1408 | CG1 | ILE | A | 301 | 246.505 | -82.750 | 112.495 | 1.00 | 67.98 | A |
| ATOM C | 1409 | CD1 | ILE | A | 301 | 245.392 | -82.041 | 113.240 | 1.00 | 76.63 | A |
| ATOM C | 1410 | C | ILE | A | 301 | 247.769 | -86.319 | 111.812 | 1.00 | 73.57 | A |
| ATOM O | 1411 | O | ILE | A | 301 | 248.662 | -86.676 | 112.588 | 1.00 | 78.37 | A |
| ATOM N | 1412 | N | GLU | A | 302 | 247.034 | -87.184 | 111.092 | 1.00 | 75.18 | A |
| ATOM C | 1413 | CA | GLU | A | 302 | 247.212 | -88.639 | 111.215 | 1.00 | 74.73 | A |
| ATOM C | 1414 | CB | GLU | A | 302 | 245.845 | -89.306 | 111.428 | 1.00 | 70.49 | A |
| ATOM C | 1415 | CG | GLU | A | 302 | 245.378 | -89.254 | 112.936 | 1.00 | 78.38 | A |
| ATOM C | 1416 | CD | GLU | A | 302 | 243.861 | -89.396 | 113.127 | 1.00 | 79.55 | A |
| ATOM O | 1417 | OE1 | GLU | A | 302 | 243.188 | -90.062 | 112.281 | 1.00 | 80.35 | A |
| ATOM O | 1418 | OE2 | GLU | A | 302 | 243.360 | -88.836 | 114.140 | 1.00 | 87.62 | A |
| ATOM C | 1419 | C | GLU | A | 302 | 248.026 | -89.290 | 110.065 | 1.00 | 75.98 | A |
| ATOM O | 1420 | O | GLU | A | 302 | 248.411 | -90.458 | 110.160 | 1.00 | 81.76 | A |
| ATOM N | 1421 | N | GLY | A | 303 | 248.339 | -88.531 | 109.006 | 1.00 | 78.40 | A |
| ATOM C | 1422 | CA | GLY | A | 303 | 249.160 | -89.053 | 107.912 | 1.00 | 72.91 | A |
| ATOM C | 1423 | C | GLY | A | 303 | 248.566 | -89.374 | 106.552 | 1.00 | 72.79 | A |
| ATOM O | 1424 | O | GLY | A | 303 | 249.237 | -89.368 | 105.513 | 1.00 | 76.85 | A |
| ATOM N | 1425 | N | ARG | A | 304 | 247.286 | -89.679 | 106.549 | 1.00 | 67.34 | A |
| ATOM C | 1426 | CA | ARG | A | 304 | 246.623 | -90.043 | 105.300 | 1.00 | 63.57 | A |
| ATOM C | 1427 | CB | ARG | A | 304 | 245.098 | -90.120 | 105.461 | 1.00 | 62.11 | A |
| ATOM C | 1428 | CG | ARG | A | 304 | 244.623 | -91.200 | 106.414 | 1.00 | 63.49 | A |
| ATOM C | 1429 | CD | ARG | A | 304 | 243.198 | -90.950 | 106.857 | 1.00 | 70.18 | A |
| ATOM N | 1430 | NE | ARG | A | 304 | 243.087 | -89.787 | 107.759 | 1.00 | 79.24 | A |
| ATOM C | 1431 | CZ | ARG | A | 304 | 242.896 | -89.857 | 109.080 | 1.00 | 78.33 | A |
| ATOM N | 1432 | NH1 | ARG | A | 304 | 242.788 | -91.031 | 109.665 | 1.00 | 78.68 | A |
| ATOM N | 1433 | NH2 | ARG | A | 304 | 242.819 | -88.757 | 109.821 | 1.00 | 83.87 | A |
| ATOM C | 1434 | C | ARG | A | 304 | 246.882 | -89.200 | 104.087 | 1.00 | 60.49 | A |
| ATOM O | 1435 | O | ARG | A | 304 | 247.550 | -88.195 | 104.122 | 1.00 | 65.51 | A |
| ATOM N | 1436 | N | MET | A | 305 | 246.278 | -89.661 | 103.013 | 1.00 | 61.50 | A |
| ATOM C | 1437 | CA | MET | A | 305 | 246.335 | -89.084 | 101.698 | 1.00 | 65.46 | A |
| ATOM C | 1438 | CB | MET | A | 305 | 246.041 | -90.197 | 100.663 | 1.00 | 63.39 | A |
| ATOM C | 1439 | C | MET | A | 305 | 245.233 | -88.042 | 101.699 | 1.00 | 69.37 | A |
| ATOM O | 1440 | O | MET | A | 305 | 244.036 | -88.349 | 101.691 | 1.00 | 78.96 | A |
| ATOM N | 1441 | N | HIS | A | 306 | 245.671 | -86.802 | 101.710 | 1.00 | 69.56 | A |
| ATOM C | 1442 | CA | HIS | A | 306 | 244.790 | -85.674 | 101.706 | 1.00 | 64.25 | A |
| ATOM C | 1443 | CB | HIS | A | 306 | 245.247 | -84.657 | 102.735 | 1.00 | 64.47 | A |
| ATOM C | 1444 | CG | HIS | A | 306 | 246.674 | -84.276 | 102.611 | 1.00 | 58.72 | A |
| ATOM C | 1445 | CD2 | HIS | A | 306 | 247.253 | -83.167 | 102.099 | 1.00 | 56.35 | A |
| ATOM N | 1446 | ND1 | HIS | A | 306 | 247.691 | -85.072 | 103.092 | 1.00 | 58.38 | A |
| ATOM C | 1447 | CE1 | HIS | A | 306 | 248.841 | -84.461 | 102.883 | 1.00 | 55.77 | A |
| ATOM N | 1448 | NE2 | HIS | A | 306 | 248.602 | -83.307 | 102.284 | 1.00 | 54.24 | A |
| ATOM C | 1449 | C | HIS | A | 306 | 244.657 | -85.001 | 100.363 | 1.00 | 64.13 | A |
| ATOM O | 1450 | O | HIS | A | 306 | 245.469 | -85.186 | 99.467 | 1.00 | 61.51 | A |
| ATOM N | 1451 | N | ASP | A | 307 | 243.667 | -84.122 | 100.305 | 1.00 | 66.29 | A |
| ATOM C | 1452 | CA | ASP | A | 307 | 243.316 | -83.353 | 99.117 | 1.00 | 67.53 | A |
| ATOM C | 1453 | CB | ASP | A | 307 | 241.988 | -83.886 | 98.618 | 1.00 | 76.36 | A |
| ATOM C | 1454 | CG | ASP | A | 307 | 241.022 | -84.130 | 99.789 | 1.00 | 82.91 | A |
| ATOM O | 1455 | OD1 | ASP | A | 307 | 240.938 | -83.232 | 100.665 | 1.00 | 87.78 | A |
| ATOM O | 1456 | OD2 | ASP | A | 307 | 240.376 | -85.208 | 99.863 | 1.00 | 84.54 | A |
| ATOM C | 1457 | C | ASP | A | 307 | 243.130 | -81.873 | 99.495 | 1.00 | 62.06 | A |
| ATOM O | 1458 | O | ASP | A | 307 | 243.430 | -81.435 | 100.603 | 1.00 | 62.07 | A |
| ATOM N | 1459 | N | GLU | A | 308 | 242.537 | -81.150 | 98.566 | 1.00 | 56.29 | A |
| ATOM C | 1460 | CA | GLU | A | 308 | 242.256 | -79.740 | 98.709 | 1.00 | 59.89 | A |
| ATOM C | 1461 | CB | GLU | A | 308 | 241.830 | -79.236 | 97.325 | 1.00 | 58.22 | A |
| ATOM C | 1462 | CG | GLU | A | 308 | 241.947 | -80.428 | 96.353 | 1.00 | 63.76 | A |
| ATOM C | 1463 | CD | GLU | A | 308 | 241.729 | -80.087 | 94.898 | 1.00 | 66.66 | A |
| ATOM O | 1464 | OE1 | GLU | A | 308 | 242.570 | -79.367 | 94.305 | 1.00 | 69.11 | A |
| ATOM O | 1465 | OE2 | GLU | A | 308 | 240.722 | -80.565 | 94.330 | 1.00 | 69.49 | A |
| ATOM C | 1466 | C | GLU | A | 308 | 241.201 | -79.444 | 99.802 | 1.00 | 56.96 | A |
| ATOM O | 1467 | O | GLU | A | 308 | 241.003 | -78.286 | 100.226 | 1.00 | 57.79 | A |
| ATOM N | 1468 | N | LYS | A | 309 | 240.558 | -80.495 | 100.296 | 1.00 | 54.61 | A |
| ATOM C | 1469 | CA | LYS | A | 309 | 239.526 | -80.332 | 101.317 | 1.00 | 54.70 | A |
| ATOM C | 1470 | CB | LYS | A | 309 | 238.697 | -81.615 | 101.449 | 1.00 | 46.40 | A |
| ATOM C | 1471 | CG | LYS | A | 309 | 237.740 | -81.859 | 100.300 | 1.00 | 53.44 | A |
| ATOM C | 1472 | CD | LYS | A | 309 | 236.674 | -80.778 | 100.253 | 1.00 | 58.61 | A |
| ATOM C | 1473 | CE | LYS | A | 309 | 235.600 | -81.071 | 99.214 | 1.00 | 60.43 | A |
| ATOM N | 1474 | NZ | LYS | A | 309 | 234.524 | -80.026 | 99.209 | 1.00 | 60.76 | A |
| ATOM C | 1475 | C | LYS | A | 309 | 240.111 | -79.967 | 102.668 | 1.00 | 54.64 | A |
| ATOM O | 1476 | O | LYS | A | 309 | 239.366 | -79.724 | 103.618 | 1.00 | 66.35 | A |
| ATOM N | 1477 | N | VAL | A | 310 | 241.439 | -79.941 | 102.770 | 1.00 | 57.91 | A |
| ATOM C | 1478 | CA | VAL | A | 310 | 242.086 | -79.593 | 104.039 | 1.00 | 53.66 | A |
| ATOM C | 1479 | CB | VAL | A | 310 | 243.573 | -79.974 | 104.058 | 1.00 | 52.18 | A |
| ATOM C | 1480 | CG1 | VAL | A | 310 | 243.749 | -81.375 | 103.528 | 1.00 | 54.98 | A |
| ATOM C | 1481 | CG2 | VAL | A | 310 | 244.347 | -79.010 | 103.252 | 1.00 | 55.72 | A |
| ATOM C | 1482 | C | VAL | A | 310 | 241.957 | -78.091 | 104.263 | 1.00 | 53.64 | A |
| ATOM O | 1483 | O | VAL | A | 310 | 241.771 | -77.638 | 105.404 | 1.00 | 47.54 | A |
| ATOM N | 1484 | N | ASP | A | 311 | 242.008 | -77.332 | 103.168 | 1.00 | 50.01 | A |
| ATOM C | 1485 | CA | ASP | A | 311 | 241.881 | -75.889 | 103.255 | 1.00 | 57.45 | A |
| ATOM C | 1486 | CB | ASP | A | 311 | 242.275 | -75.232 | 101.925 | 1.00 | 56.10 | A |
| ATOM C | 1487 | CG | ASP | A | 311 | 243.756 | -75.367 | 101.624 | 1.00 | 55.84 | A |
| ATOM O | 1488 | OD1 | ASP | A | 311 | 244.585 | -75.238 | 102.553 | 1.00 | 49.03 | A |
| ATOM O | 1489 | OD2 | ASP | A | 311 | 244.085 | -75.597 | 100.453 | 1.00 | 54.46 | A |
| ATOM C | 1490 | C | ASP | A | 311 | 240.454 | -75.467 | 103.667 | 1.00 | 59.07 | A |
| ATOM O | 1491 | O | ASP | A | 311 | 240.258 | -74.365 | 104.208 | 1.00 | 69.75 | A |
| ATOM N | 1492 | N | LEU | A | 312 | 239.471 | -76.343 | 103.445 | 1.00 | 56.37 | A |
| ATOM C | 1493 | CA | LEU | A | 312 | 238.117 | -76.031 | 103.815 | 1.00 | 45.08 | A |
| ATOM C | 1494 | CB | LEU | A | 312 | 237.135 | -76.975 | 103.126 | 1.00 | 46.93 | A |
| ATOM C | 1495 | CG | LEU | A | 312 | 236.719 | -76.481 | 101.746 | 1.00 | 45.65 | A |
| ATOM C | 1496 | CD1 | LEU | A | 312 | 236.262 | -77.698 | 100.966 | 1.00 | 57.08 | A |
| ATOM C | 1497 | CD2 | LEU | A | 312 | 235.607 | -75.431 | 101.878 | 1.00 | 42.14 | A |
| ATOM C | 1498 | C | LEU | A | 312 | 238.066 | -76.204 | 105.309 | 1.00 | 42.72 | A |
| ATOM O | 1499 | 0 | LEU | A | 312 | 237.357 | -75.451 | 105.986 | 1.00 | 41.50 | A |
| ATOM N | 1500 | N | TRP | A | 313 | 238.778 | -77.211 | 105.814 | 1.00 | 35.13 | A |
| ATOM C | 1501 | CA | TRP | A | 313 | 238.818 | -77.454 | 107.257 | 1.00 | 42.05 | A |
| ATOM C | 1502 | CB | TRP | A | 313 | 239.630 | -78.718 | 107.532 | 1.00 | 37.59 | A |
| ATOM C | 1503 | CG | TRP | A | 313 | 239.903 | -78.960 | 108.973 | 1.00 | 37.21 | A |
| ATOM C | 1504 | CD2 | TRP | A | 313 | 239.169 | -79.815 | 109.859 | 1.00 | 38.09 | A |
| ATOM C | 1505 | CE2 | TRP | A | 313 | 239.786 | -79.742 | 111.133 | 1.00 | 40.55 | A |
| ATOM C | 1506 | CE3 | TRP | A | 313 | 238.050 | -80.627 | 109.706 | 1.00 | 42.38 | A |
| ATOM C | 1507 | CD1 | TRP | A | 313 | 240.895 | -78.418 | 109.716 | 1.00 | 40.67 | A |
| ATOM N | 1508 | NE1 | TRP | A | 313 | 240.843 | -78.885 | 111.022 | 1.00 | 36.83 | A |
| ATOM C | 1509 | CZ2 | TRP | A | 313 | 239.325 | -80.470 | 112.237 | 1.00 | 41.12 | A |
| ATOM C | 1510 | CZ3 | TRP | A | 313 | 237.591 | -81.353 | 110.817 | 1.00 | 40.98 | A |
| ATOM C | 1511 | CH2 | TRP | A | 313 | 238.220 | -81.265 | 112.052 | 1.00 | 42.79 | A |
| ATOM C | 1512 | C | TRP | A | 313 | 239.448 | -76.259 | 108.009 | 1.00 | 45.03 | A |
| ATOM O | 1513 | O | TRP | A | 313 | 238.971 | -75.813 | 109.044 | 1.00 | 49.94 | A |
| ATOM N | 1514 | N | SER | A | 314 | 240.543 | -75.748 | 107.477 | 1.00 | 46.02 | A |
| ATOM C | 1515 | CA | SER | A | 314 | 241.220 | -74.624 | 108.087 | 1.00 | 41.93 | A |
| ATOM C | 1516 | CB | SER | A | 314 | 242.523 | -74.354 | 107.341 | 1.00 | 44.32 | A |
| ATOM O | 1517 | OG | SER | A | 314 | 243.333 | -75.505 | 107.387 | 1.00 | 38.93 | A |
| ATOM C | 1518 | C | SER | A | 314 | 240.338 | -73.392 | 108.062 | 1.00 | 39.99 | A |
| ATOM O | 1519 | O | SER | A | 314 | 240.328 | -72.596 | 109.011 | 1.00 | 45.37 | A |
| ATOM N | 1520 | N | LEU | A | 315 | 239.616 | -73.226 | 106.965 | 1.00 | 35.87 | A |
| ATOM C | 1521 | CA | LEU | A | 315 | 238.704 | -72.091 | 106.805 | 1.00 | 35.10 | A |
| ATOM C | 1522 | CB | LEU | A | 315 | 238.115 | -72.091 | 105.390 | 1.00 | 34.40 | A |
| ATOM C | 1523 | CG | LEU | A | 315 | 237.307 | -70.872 | 104.991 | 1.00 | 41.64 | A |
| ATOM C | 1524 | CD1 | LEU | A | 315 | 238.136 | -69.564 | 105.204 | 1.00 | 42.79 | A |
| ATOM C | 1525 | CD2 | LEU | A | 315 | 236.927 | -71.022 | 103.523 | 1.00 | 40.03 | A |
| ATOM C | 1526 | C | LEU | A | 315 | 237.569 | -72.154 | 107.851 | 1.00 | 38.86 | A |
| ATOM O | 1527 | O | LEU | A | 315 | 236.854 | -71.200 | 108.035 | 1.00 | 42.90 | A |
| ATOM N | 1528 | N | GLY | A | 316 | 237.421 | -73.301 | 108.515 | 1.00 | 40.90 | A |
| ATOM C | 1529 | CA | GLY | A | 316 | 236.420 | -73.475 | 109.540 | 1.00 | 29.38 | A |
| ATOM C | 1530 | C | GLY | A | 316 | 237.051 | -73.072 | 110.863 | 1.00 | 38.31 | A |
| ATOM O | 1531 | O | GLY | A | 316 | 236.444 | -72.317 | 111.644 | 1.00 | 44.99 | A |
| ATOM N | 1532 | N | VAL | A | 317 | 238.259 | -73.578 | 111.126 | 1.00 | 34.77 | A |
| ATOM C | 1533 | CA | VAL | A | 317 | 238.980 | -73.253 | 112.343 | 1.00 | 38.52 | A |
| ATOM C | 1534 | CB | VAL | A | 317 | 240.378 | -73.903 | 112.348 | 1.00 | 39.35 | A |
| ATOM C | 1535 | CG1 | VAL | A | 317 | 241.200 | -73.322 | 113.465 | 1.00 | 39.07 | A |
| ATOM C | 1536 | CG2 | VAL | A | 317 | 240.268 | -75.403 | 112.500 | 1.00 | 36.59 | A |
| ATOM C | 1537 | C | VAL | A | 317 | 239.145 | -71.743 | 112.382 | 1.00 | 39.43 | A |
| ATOM O | 1538 | O | VAL | A | 317 | 238.923 | -71.092 | 113.421 | 1.00 | 39.97 | A |
| ATOM N | 1539 | N | LEU | A | 318 | 239.517 | -71.195 | 111.226 | 1.00 | 35.94 | A |
| ATOM C | 1540 | CA | LEU | A | 318 | 239.728 | -69.769 | 111.107 | 1.00 | 35.49 | A |
| ATOM C | 1541 | CB | LEU | A | 318 | 240.217 | -69.443 | 109.701 | 1.00 | 34.72 | A |
| ATOM C | 1542 | CG | LEU | A | 318 | 241.505 | -68.678 | 109.594 | 1.00 | 35.85 | A |
| ATOM C | 1543 | CD1 | LEU | A | 318 | 242.492 | -69.129 | 110.631 | 1.00 | 35.87 | A |
| ATOM C | 1544 | CD2 | LEU | A | 318 | 242.040 | -68.906 | 108.254 | 1.00 | 39.24 | A |
| ATOM C | 1545 | C | LEU | A | 318 | 238.440 | -68.952 | 111.425 | 1.00 | 41.79 | A |
| ATOM O | 1546 | O | LEU | A | 318 | 238.499 | -67.943 | 112.191 | 1.00 | 36.26 | A |
| ATOM N | 1547 | N | CYS | A | 319 | 237.301 | -69.398 | 110.858 | 1.00 | 35.62 | A |
| ATOM C | 1548 | CA | CYS | A | 319 | 236.053 | -68.700 | 111.024 | 1.00 | 40.61 | A |
| ATOM C | 1549 | CB | CYS | A | 319 | 235.013 | -69.338 | 110.158 | 1.00 | 35.35 | A |
| ATOM S | 1550 | SG | CYS | A | 319 | 233.324 | -68.489 | 110.215 | 1.00 | 25.40 | A |
| ATOM C | 1551 | C | CYS | A | 319 | 235.640 | -68.734 | 112.482 | 1.00 | 35.51 | A |
| ATOM O | 1552 | O | CYS | A | 319 | 235.027 | -67.810 | 112.980 | 1.00 | 40.27 | A |
| ATOM N | 1553 | N | TYR | A | 320 | 236.009 | -69.795 | 113.182 | 1.00 | 38.46 | A |
| ATOM C | 1554 | CA | TYR | A | 320 | 235.683 | -69.942 | 114.604 | 1.00 | 39.25 | A |
| ATOM C | 1555 | CB | TYR | A | 320 | 235.867 | -71.406 | 115.016 | 1.00 | 37.24 | A |
| ATOM C | 1556 | CG | TYR | A | 320 | 235.634 | -71.683 | 116.479 | 1.00 | 38.04 | A |
| ATOM C | 1557 | CD1 | TYR | A | 320 | 236.573 | -71.333 | 117.438 | 1.00 | 34.91 | A |
| ATOM C | 1558 | CE1 | TYR | A | 320 | 236.380 | -71.640 | 118.800 | 1.00 | 31.79 | A |
| ATOM C | 1559 | CD2 | TYR | A | 320 | 234.484 | -72.331 | 116.892 | 1.00 | 39.29 | A |
| ATOM C | 1560 | CE2 | TYR | A | 320 | 234.248 | -72.628 | 118.233 | 1.00 | 35.49 | A |
| ATOM C | 1561 | CZ | TYR | A | 320 | 235.191 | -72.289 | 119.198 | 1.00 | 41.97 | A |
| ATOM O | 1562 | OH | TYR | A | 320 | 234.905 | -72.575 | 120.532 | 1.00 | 35.00 | A |
| ATOM C | 1563 | C | TYR | A | 320 | 236.613 | -69.038 | 115.435 | 1.00 | 43.31 | A |
| ATOM O | 1564 | O | TYR | A | 320 | 236.166 | -68.318 | 116.322 | 1.00 | 51.83 | A |
| ATOM N | 1565 | N | GLU | A | 321 | 237.910 | -69.073 | 115.144 | 1.00 | 42.04 | A |
| ATOM C | 1566 | CA | GLU | A | 321 | 238.860 | -68.249 | 115.871 | 1.00 | 43.24 | A |
| ATOM C | 1567 | CB | GLU | A | 321 | 240.273 | -68.552 | 115.419 | 1.00 | 36.62 | A |
| ATOM C | 1568 | CG | GLU | A | 321 | 241.311 | -67.733 | 116.144 | 1.00 | 43.20 | A |
| ATOM C | 1569 | CD | GLU | A | 321 | 242.689 | -68.087 | 115.740 | 1.00 | 47.61 | A |
| ATOM O | 1570 | OE1 | GLU | A | 321 | 242.845 | -69.101 | 115.003 | 1.00 | 49.19 | A |
| ATOM O | 1571 | OE2 | GLU | A | 321 | 243.606 | -67.350 | 116.171 | 1.00 | 55.54 | A |
| ATOM C | 1572 | C | GLU | A | 321 | 238.569 | -66.763 | 115.714 | 1.00 | 42.47 | A |
| ATOM O | 1573 | O | GLU | A | 321 | 238.794 | -65.970 | 116.656 | 1.00 | 48.77 | A |
| ATOM N | 1574 | N | PHE | A | 322 | 238.073 | -66.377 | 114.539 | 1.00 | 40.79 | A |
| ATOM C | 1575 | CA | PHE | A | 322 | 237.728 | -64.970 | 114.299 | 1.00 | 37.96 | A |
| ATOM C | 1576 | CB | PHE | A | 322 | 237.361 | -64.737 | 112.846 | 1.00 | 37.47 | A |
| ATOM C | 1577 | CG | PHE | A | 322 | 238.517 | -64.880 | 111.892 | 1.00 | 39.59 | A |
| ATOM C | 1578 | CD1 | PHE | A | 322 | 239.839 | -64.729 | 112.338 | 1.00 | 34.97 | A |
| ATOM C | 1579 | CD2 | PHE | A | 322 | 238.279 | -65.133 | 110.515 | 1.00 | 41.97 | A |
| ATOM C | 1580 | CE1 | PHE | A | 322 | 240.892 | -64.830 | 111.441 | 1.00 | 36.15 | A |
| ATOM C | 1581 | CE2 | PHE | A | 322 | 239.342 | -65.232 | 109.605 | 1.00 | 35.10 | A |
| ATOM C | 1582 | CZ | PHE | A | 322 | 240.649 | -65.081 | 110.070 | 1.00 | 38.47 | A |
| ATOM C | 1583 | C | PHE | A | 322 | 236.569 | -64.466 | 115.166 | 1.00 | 40.06 | A |
| ATOM O | 1584 | O | PHE | A | 322 | 236.542 | -63.305 | 115.532 | 1.00 | 37.07 | A |
| ATOM N | 1585 | N | LEU | A | 323 | 235.636 | -65.356 | 115.499 | 1.00 | 34.80 | A |
| ATOM C | 1586 | CA | LEU | A | 323 | 234.484 | -65.021 | 116.291 | 1.00 | 30.32 | A |
| ATOM C | 1587 | CB | LEU | A | 323 | 233.317 | -65.873 | 115.774 | 1.00 | 28.53 | A |
| ATOM C | 1588 | CG | LEU | A | 323 | 232.935 | -65.689 | 114.325 | 1.00 | 29.77 | A |
| ATOM C | 1589 | CD1 | LEU | A | 323 | 231.967 | -66.804 | 113.984 | 1.00 | 24.59 | A |
| ATOM C | 1590 | CD2 | LEU | A | 323 | 232.306 | -64.288 | 114.046 | 1.00 | 30.36 | A |
| ATOM C | 1591 | C | LEU | A | 323 | 234.661 | -65.226 | 117.824 | 1.00 | 40.81 | A |
| ATOM O | 1592 | O | LEU | A | 323 | 234.058 | -64.495 | 118.618 | 1.00 | 36.74 | A |
| ATOM N | 1593 | N | VAL | A | 324 | 235.435 | -66.247 | 118.216 | 1.00 | 38.37 | A |
| ATOM C | 1594 | CA | VAL | A | 324 | 235.629 | -66.590 | 119.612 | 1.00 | 35.74 | A |
| ATOM C | 1595 | CB | VAL | A | 324 | 235.648 | -68.102 | 119.816 | 1.00 | 37.17 | A |
| ATOM C | 1596 | CG1 | VAL | A | 324 | 235.879 | -68.419 | 121.294 | 1.00 | 34.96 | A |
| ATOM C | 1597 | CG2 | VAL | A | 324 | 234.352 | -68.697 | 119.303 | 1.00 | 26.08 | A |
| ATOM C | 1598 | C | VAL | A | 324 | 236.903 | -66.012 | 120.154 | 1.00 | 35.95 | A |
| ATOM O | 1599 | O | VAL | A | 324 | 236.947 | -65.631 | 121.301 | 1.00 | 44.11 | A |
| ATOM N | 1600 | N | GLY | A | 325 | 237.936 | -65.929 | 119.330 | 1.00 | 41.01 | A |
| ATOM C | 1601 | CA | GLY | A | 325 | 239.198 | -65.355 | 119.782 | 1.00 | 39.51 | A |
| ATOM C | 1602 | C | GLY | A | 325 | 240.283 | -66.402 | 119.960 | 1.00 | 44.55 | A |
| ATOM O | 1603 | O | GLY | A | 325 | 241.414 | -66.092 | 120.321 | 1.00 | 38.22 | A |
| ATOM N | 1604 | N | LYS | A | 326 | 239.929 | -67.663 | 119.722 | 1.00 | 45.05 | A |
| ATOM C | 1605 | CA | LYS | A | 326 | 240.881 | -68.764 | 119.861 | 1.00 | 45.42 | A |
| ATOM C | 1606 | CB | LYS | A | 326 | 240.994 | -69.180 | 121.325 | 1.00 | 52.62 | A |
| ATOM C | 1607 | CG | LYS | A | 326 | 239.699 | -69.600 | 121.927 | 1.00 | 55.78 | A |
| ATOM C | 1608 | CD | LYS | A | 326 | 239.856 | -69.912 | 123.415 | 1.00 | 60.70 | A |
| ATOM C | 1609 | CE | LYS | A | 326 | 238.486 | -70.233 | 124.058 | 1.00 | 66.17 | A |
| ATOM N | 1610 | NZ | LYS | A | 326 | 238.569 | -70.651 | 125.479 | 1.00 | 62.77 | A |
| ATOM C | 1611 | C | LYS | A | 326 | 240.419 | -69.937 | 119.005 | 1.00 | 46.11 | A |
| ATOM O | 1612 | O | LYS | A | 326 | 239.259 | -70.074 | 118.767 | 1.00 | 39.63 | A |
| ATOM N | 1613 | N | PRO | A | 327 | 241.336 | -70.820 | 118.568 | 1.00 | 53.36 | A |
| ATOM C | 1614 | CD | PRO | A | 327 | 242.773 | -70.850 | 118.901 | 1.00 | 43.78 | A |
| ATOM C | 1615 | CA | PRO | A | 327 | 240.980 | -71.968 | 117.726 | 1.00 | 41.74 | A |
| ATOM C | 1616 | CB | PRO | A | 327 | 242.331 | -72.631 | 117.446 | 1.00 | 38.86 | A |
| ATOM C | 1617 | CG | PRO | A | 327 | 243.349 | -71.561 | 117.738 | 1.00 | 42.00 | A |
| ATOM C | 1618 | C | PRO | A | 327 | 240.064 | -72.895 | 118.474 | 1.00 | 40.42 | A |
| ATOM O | 1619 | O | PRO | A | 327 | 240.166 | -73.021 | 119.700 | 1.00 | 46.66 | A |
| ATOM N | 1620 | N | PRO | A | 328 | 239.209 | -73.624 | 117.743 | 1.00 | 40.91 | A |
| ATOM C | 1621 | CD | PRO | A | 328 | 239.083 | -73.540 | 116.278 | 1.00 | 35.93 | A |
| ATOM C | 1622 | CA | PRO | A | 328 | 238.232 | -74.572 | 118.297 | 1.00 | 43.34 | A |
| ATOM C | 1623 | CB | PRO | A | 328 | 237.340 | -74.900 | 117.106 | 1.00 | 40.94 | A |
| ATOM C | 1624 | CG | PRO | A | 328 | 238.298 | -74.799 | 115.948 | 1.00 | 38.83 | A |
| ATOM C | 1625 | C | PRO | A | 328 | 238.785 | -75.820 | 118.933 | 1.00 | 44.48 | A |
| ATOM O | 1626 | O | PRO | A | 328 | 238.075 | -76.507 | 119.657 | 1.00 | 56.46 | A |
| ATOM N | 1627 | N | PHE | A | 329 | 240.053 | -76.118 | 118.674 | 1.00 | 50.25 | A |
| ATOM C | 1628 | CA | PHE | A | 329 | 240.691 | -77.334 | 119.223 | 1.00 | 45.46 | A |
| ATOM C | 1629 | CB | PHE | A | 329 | 241.119 | -78.268 | 118.089 | 1.00 | 42.34 | A |
| ATOM C | 1630 | CG | PHE | A | 329 | 240.026 | -78.559 | 117.139 | 1.00 | 42.28 | A |
| ATOM C | 1631 | CD1 | PHE | A | 329 | 238.895 | -79.252 | 117.575 | 1.00 | 39.79 | A |
| ATOM C | 1632 | CD2 | PHE | A | 329 | 240.097 | -78.138 | 115.808 | 1.00 | 39.80 | A |
| ATOM C | 1633 | CE1 | PHE | A | 329 | 237.824 | -79.529 | 116.665 | 1.00 | 42.28 | A |
| ATOM C | 1634 | CE2 | PHE | A | 329 | 239.034 | -78.408 | 114.902 | 1.00 | 45.87 | A |
| ATOM C | 1635 | CZ | PHE | A | 329 | 237.903 | -79.106 | 115.332 | 1.00 | 40.12 | A |
| ATOM C | 1636 | C | PHE | A | 329 | 241.904 | -76.978 | 120.064 | 1.00 | 45.73 | A |
| ATOM O | 1637 | O | PHE | A | 329 | 242.769 | -77.808 | 120.277 | 1.00 | 40.30 | A |
| ATOM N | 1638 | N | GLU | A | 330 | 241.958 | -75.741 | 120.543 | 1.00 | 44.00 | A |
| ATOM C | 1639 | CA | GLU | A | 330 | 243.070 | -75.285 | 121.345 | 1.00 | 50.19 | A |
| ATOM C | 1640 | CB | GLU | A | 330 | 242.855 | -73.844 | 121.787 | 1.00 | 54.34 | A |
| ATOM C | 1641 | CG | GLU | A | 330 | 244.140 | -73.087 | 122.035 | 1.00 | 65.74 | A |
| ATOM C | 1642 | CD | GLU | A | 330 | 243.932 | -71.802 | 122.845 | 1.00 | 74.00 | A |
| ATOM O | 1643 | OE1 | GLU | A | 330 | 244.822 | -70.906 | 122.794 | 1.00 | 80.13 | A |
| ATOM O | 1644 | OE2 | GLU | A | 330 | 242.884 | -71.701 | 123.543 | 1.00 | 77.27 | A |
| ATOM C | 1645 | C | GLU | A | 330 | 243.172 | -76.161 | 122.550 | 1.00 | 51.87 | A |
| ATOM O | 1646 | O | GLU | A | 330 | 242.158 | -76.668 | 123.029 | 1.00 | 54.61 | A |
| ATOM N | 1647 | N | ALA | A | 331 | 244.404 | -76.359 | 123.020 | 1.00 | 56.85 | A |
| ATOM C | 1648 | CA | ALA | A | 331 | 244.680 | -77.163 | 124.217 | 1.00 | 60.04 | A |
| ATOM C | 1649 | CB | ALA | A | 331 | 244.791 | -78.618 | 123.872 | 1.00 | 51.13 | A |
| ATOM C | 1650 | C | ALA | A | 331 | 245.992 | -76.676 | 124.795 | 1.00 | 60.84 | A |
| ATOM O | 1651 | O | ALA | A | 331 | 246.630 | -75.805 | 124.208 | 1.00 | 69.22 | A |
| ATOM N | 1652 | N | ASN | A | 332 | 246.378 | -77.197 | 125.962 | 1.00 | 64.86 | A |
| ATOM C | 1653 | CA | ASN | A | 332 | 247.637 | -76.790 | 126.560 | 1.00 | 61.08 | A |
| ATOM C | 1654 | CB | ASN | A | 332 | 247.514 | -76.742 | 128.100 | 1.00 | 60.00 | A |
| ATOM C | 1655 | CG | ASN | A | 332 | 246.822 | -75.435 | 128.611 | 1.00 | 67.42 | A |
| ATOM O | 1656 | OD1 | ASN | A | 332 | 247.429 | -74.359 | 128.654 | 1.00 | 68.56 | A |
| ATOM N | 1657 | ND2 | ASN | A | 332 | 245.547 | -75.544 | 129.000 | 1.00 | 72.60 | A |
| ATOM C | 1658 | C | ASN | A | 332 | 248.807 | -77.666 | 126.080 | 1.00 | 60.05 | A |
| ATOM O | 1659 | O | ASN | A | 332 | 249.966 | -77.257 | 126.234 | 1.00 | 65.40 | A |
| ATOM N | 1660 | N | THR | A | 333 | 248.512 | -78.823 | 125.460 | 1.00 | 53.02 | A |
| ATOM C | 1661 | CA | THR | A | 333 | 249.562 | -79.745 | 124.976 | 1.00 | 54.16 | A |
| ATOM C | 1662 | CB | THR | A | 333 | 249.652 | -81.103 | 125.782 | 1.00 | 55.23 | A |
| ATOM O | 1663 | OG1 | THR | A | 333 | 248.822 | -82.111 | 125.160 | 1.00 | 53.00 | A |
| ATOM C | 1664 | CG2 | THR | A | 333 | 249.296 | -80.907 | 127.225 | 1.00 | 55.86 | A |
| ATOM C | 1665 | C | THR | A | 333 | 249.400 | -80.153 | 123.497 | 1.00 | 59.22 | A |
| ATOM O | 1666 | O | THR | A | 333 | 248.379 | -79.845 | 122.897 | 1.00 | 59.44 | A |
| ATOM N | 1667 | N | TYR | A | 334 | 250.358 | -80.901 | 122.930 | 1.00 | 66.35 | A |
| ATOM C | 1668 | CA | TYR | A | 334 | 250.331 | -81.305 | 121.506 | 1.00 | 67.76 | A |
| ATOM C | 1669 | CB | TYR | A | 334 | 251.770 | -81.458 | 120.999 | 1.00 | 65.59 | A |
| ATOM C | 1670 | CG | TYR | A | 334 | 251.922 | -82.193 | 119.636 | 1.00 | 60.98 | A |
| ATOM C | 1671 | CD1 | TYR | A | 334 | 252.369 | -81.519 | 118.539 | 1.00 | 58.37 | A |
| ATOM C | 1672 | CE1 | TYR | A | 334 | 252.454 | -82.152 | 117.323 | 1.00 | 57.12 | A |
| ATOM C | 1673 | CD2 | TYR | A | 334 | 251.556 | -83.561 | 119.454 | 1.00 | 60.46 | A |
| ATOM C | 1674 | CE2 | TYR | A | 334 | 251.612 | -84.203 | 118.235 | 1.00 | 54.51 | A |
| ATOM C | 1675 | CZ | TYR | A | 334 | 252.081 | -83.483 | 117.170 | 1.00 | 61.53 | A |
| ATOM O | 1676 | OH | TYR | A | 334 | 252.259 | -84.054 | 115.932 | 1.00 | 64.63 | A |
| ATOM C | 1677 | C | TYR | A | 334 | 249.706 | -82.673 | 121.408 | 1.00 | 72.76 | A |
| ATOM O | 1678 | O | TYR | A | 334 | 249.561 | -83.257 | 120.347 | 1.00 | 80.80 | A |
| ATOM N | 1679 | N | GLN | A | 335 | 249.281 | -83.198 | 122.511 | 1.00 | 75.82 | A |
| ATOM C | 1680 | CA | GLN | A | 335 | 248.849 | -84.550 | 122.438 | 1.00 | 75.10 | A |
| ATOM C | 1681 | CB | GLN | A | 335 | 249.822 | -85.260 | 123.381 | 1.00 | 74.39 | A |
| ATOM C | 1682 | CG | GLN | A | 335 | 251.326 | -84.889 | 123.021 | 1.00 | 71.11 | A |
| ATOM C | 1683 | CD | GLN | A | 335 | 252.200 | -84.301 | 124.134 | 1.00 | 70.17 | A |
| ATOM O | 1684 | OE1 | GLN | A | 335 | 253.434 | -84.204 | 123.959 | 1.00 | 71.56 | A |
| ATOM N | 1685 | NE2 | GLN | A | 335 | 251.605 | -83.897 | 125.269 | 1.00 | 67.14 | A |
| ATOM C | 1686 | C | GLN | A | 335 | 247.415 | -84.426 | 122.926 | 1.00 | 72.12 | A |
| ATOM O | 1687 | O | GLN | A | 335 | 246.584 | -85.314 | 122.698 | 1.00 | 70.11 | A |
| ATOM N | 1688 | N | GLU | A | 336 | 247.129 | -83.272 | 123.527 | 1.00 | 67.17 | A |
| ATOM C | 1689 | CA | GLU | A | 336 | 245.815 | -82.981 | 124.050 | 1.00 | 66.23 | A |
| ATOM C | 1690 | CB | GLU | A | 336 | 245.911 | -81.929 | 125.131 | 1.00 | 54.23 | A |
| ATOM C | 1691 | C | GLU | A | 336 | 245.132 | -82.389 | 122.779 | 1.00 | 63.60 | A |
| ATOM O | 1692 | O | GLU | A | 336 | 243.998 | -82.726 | 122.478 | 1.00 | 68.92 | A |
| ATOM N | 1693 | N | THR | A | 337 | 245.807 | -81.474 | 122.060 | 1.00 | 63.51 | A |
| ATOM C | 1694 | CA | THR | A | 337 | 245.246 | -80.840 | 120.863 | 1.00 | 45.32 | A |
| ATOM C | 1695 | CB | THR | A | 337 | 246.221 | -79.805 | 120.292 | 1.00 | 48.60 | A |
| ATOM O | 1696 | OG1 | THR | A | 337 | 246.433 | -78.730 | 121.227 | 1.00 | 44.49 | A |
| ATOM C | 1697 | CG2 | THR | A | 337 | 245.691 | -79.262 | 118.948 | 1.00 | 33.80 | A |
| ATOM C | 1698 | C | THR | A | 337 | 245.031 | -81.916 | 119.825 | 1.00 | 55.74 | A |
| ATOM O | 1699 | O | THR | A | 337 | 244.036 | -81.911 | 119.094 | 1.00 | 53.43 | A |
| ATOM N | 1700 | N | TYR | A | 338 | 245.979 | -82.848 | 119.758 | 1.00 | 62.69 | A |
| ATOM C | 1701 | CA | TYR | A | 338 | 245.902 | -83.948 | 118.795 | 1.00 | 62.09 | A |
| ATOM C | 1702 | CB | TYR | A | 338 | 247.056 | -84.923 | 119.028 | 1.00 | 70.33 | A |
| ATOM C | 1703 | CG | TYR | A | 338 | 247.098 | -86.080 | 118.034 | 1.00 | 74.69 | A |
| ATOM C | 1704 | CD1 | TYR | A | 338 | 248.041 | -86.116 | 117.016 | 1.00 | 72.79 | A |
| ATOM C | 1705 | CE1 | TYR | A | 338 | 248.050 | -87.154 | 116.101 | 1.00 | 75.92 | A |
| ATOM C | 1706 | CD2 | TYR | A | 338 | 246.167 | -87.121 | 118.096 | 1.00 | 70.94 | A |
| ATOM C | 1707 | CE2 | TYR | A | 338 | 246.167 | -88.153 | 117.173 | 1.00 | 73.14 | A |
| ATOM C | 1708 | CZ | TYR | A | 338 | 247.102 | -88.168 | 116.186 | 1.00 | 73.24 | A |
| ATOM O | 1709 | OH | TYR | A | 338 | 247.091 | -89.196 | 115.273 | 1.00 | ?4.46 | A |
| ATOM C | 1710 | C | TYR | A | 338 | 244.586 | -84.698 | 118.971 | 1.00 | 62.03 | A |
| ATOM O | 1711 | O | TYR | A | 338 | 243.884 | -85.017 | 117.982 | 1.00 | 52.46 | A |
| ATOM N | 1712 | N | LYS | A | 339 | 244.267 | -84.991 | 120.242 | 1.00 | 58.53 | A |
| ATOM C | 1713 | CA | LYS | A | 339 | 243.048 | -85.733 | 120.58? | 1.00 | 51.87 | A |
| ATOM C | 1714 | CB | LYS | A | 339 | 243.020 | -86.042 | 122.063 | 1.00 | 52.84 | A |
| ATOM C | 1715 | C | LYS | A | 339 | 241.791 | -84.990 | 120.165 | 1.00 | 50.97 | A |
| ATOM O | 1716 | O | LYS | A | 339 | 240.975 | -85.531 | 119.412 | 1.00 | 50.87 | A |
| ATOM N | 1717 | N | ARG | A | 340 | 241.675 | -83.725 | 120.575 | 1.00 | 44.05 | A |
| ATOM C | 1718 | CA | ARG | A | 340 | 240.507 | -82.938 | 120.230 | 1.00 | 47.22 | A |
| ATOM C | 1719 | CB | ARG | A | 340 | 240.532 | -81.574 | 120.904 | 1.00 | 45.76 | A |
| ATOM C | 1720 | CG | ARG | A | 340 | 240.286 | -81.607 | 122.398 | 1.00 | 52.85 | A |
| ATOM C | 1721 | CD | ARG | A | 340 | 240.241 | -80.202 | 122.999 | 1.00 | 59.79 | A |
| ATOM N | 1722 | NE | ARG | A | 340 | 239.045 | -79.491 | 122.535 | 1.00 | 67.64 | A |
| ATOM C | 1723 | CZ | ARG | A | 340 | 238.843 | -78.186 | 122.672 | 1.00 | 65.86 | A |
| ATOM N | 1724 | NH1 | ARG | A | 340 | 239.763 | -77.446 | 123.254 | 1.00 | 67.71 | A |
| ATOM N | 1725 | NH2 | ARG | A | 340 | 237.722 | -77.631 | 122.240 | 1.00 | 68.60 | A |
| ATOM C | 1726 | C | ARG | A | 340 | 240.299 | -82.765 | 118.738 | 1.00 | 46.20 | A |
| ATOM O | 1727 | O | ARG | A | 340 | 239.144 | -82.785 | 118.274 | 1.00 | 48.74 | A |
| ATOM N | 1728 | N | ILE | A | 341 | 241.387 | -82.597 | 117.979 | 1.00 | 47.52 | A |
| ATOM C | 1729 | CA | ILE | A | 341 | 241.253 | -82.462 | 116.511 | 1.00 | 49.01 | A |
| ATOM C | 1730 | CB | ILE | A | 341 | 242.586 | -82.067 | 115.787 | 1.00 | 48.35 | A |
| ATOM C | 1731 | CG2 | ILE | A | 341 | 242.367 | -82.130 | 114.290 | 1.00 | 34.18 | A |
| ATOM C | 1732 | CG1 | ILE | A | 341 | 243.053 | -80.674 | 116.236 | 1.00 | 44.80 | A |
| ATOM C | 1733 | CD1 | ILE | A | 341 | 244.317 | -80.259 | 115.592 | 1.00 | 47.34 | A |
| ATOM C | 1734 | C | ILE | A | 341 | 240.783 | -83.752 | 115.851 | 1.00 | 53.55 | A |
| ATOM O | 1735 | O | ILE | A | 341 | 239.936 | -83.734 | 114.948 | 1.00 | 44.79 | A |
| ATOM N | 1736 | N | SER | A | 342 | 241.369 | -84.867 | 116.286 | 1.00 | 59.31 | A |
| ATOM C | 1737 | CA | SER | A | 342 | 241.014 | -86.175 | 115.743 | 1.00 | 65.95 | A |
| ATOM C | 1738 | CB | SER | A | 342 | 242.037 | -87.227 | 116.158 | 1.00 | 66.33 | A |
| ATOM O | 1739 | OG | SER | A | 342 | 241.613 | -88.516 | 115.745 | 1.00 | 63.35 | A |
| ATOM C | 1740 | C | SER | A | 342 | 239.628 | -86.604 | 116.202 | 1.00 | 65.99 | A |
| ATOM O | 1741 | O | SER | A | 342 | 238.941 | -87.327 | 115.475 | 1.00 | 61.18 | A |
| ATOM N | 1742 | N | ARG | A | 343 | 239.232 | -86.162 | 117.400 | 1.00 | 61.34 | A |
| ATOM C | 1743 | CA | ARG | A | 343 | 237.910 | -86.478 | 117.932 | 1.00 | 59.46 | A |
| ATOM C | 1744 | CB | ARG | A | 343 | 237.888 | -86.410 | 119.456 | 1.00 | 58.77 | A |
| ATOM C | 1745 | CG | ARG | A | 343 | 238.466 | -87.604 | 120.201 | 1.00 | 47.50 | A |
| ATOM C | 1746 | CD | ARG | A | 343 | 238.360 | -87.314 | 121.675 | 1.00 | 60.64 | A |
| ATOM N | 1747 | NE | ARG | A | 343 | 238.972 | -88.327 | 122.530 | 1.00 | 66.55 | A |
| ATOM C | 1748 | CZ | ARG | A | 343 | 238.930 | -88.278 | 123.865 | 1.00 | 74.36 | A |
| ATOM N | 1749 | NH1 | ARG | A | 343 | 238.309 | -87.268 | 124.510 | 1.00 | 73.84 | A |
| ATOM N | 1750 | NH2 | ARG | A | 343 | 239.502 | -89.248 | 124.573 | 1.00 | 82.35 | A |
| ATOM C | 1751 | C | ARG | A | 343 | 236.902 | -85.448 | 117.406 | 1.00 | 62.95 | A |
| ATOM O | 1752 | O | ARG | A | 343 | 235.682 | -85.706 | 117.372 | 1.00 | 61.25 | A |
| ATOM N | 1753 | N | VAL | A | 344 | 237.421 | -84.275 | 117.011 | 1.00 | 62.37 | A |
| ATOM C | 1754 | CA | VAL | A | 344 | 236.592 | -83.163 | 116.517 | 1.00 | 52.53 | A |
| ATOM C | 1755 | CB | VAL | A | 344 | 235.625 | -83.614 | 115.386 | 1.00 | 46.83 | A |
| ATOM C | 1756 | CG1 | VAL | A | 344 | 235.005 | -82.383 | 114.685 | 1.00 | 30.17 | A |
| ATOM C | 1757 | CG2 | VAL | A | 344 | 236.378 | -84.445 | 114.375 | 1.00 | 41.05 | A |
| ATOM C | 1758 | C | VAL | A | 344 | 235.794 | -82.735 | 117.737 | 1.00 | 56.35 | A |
| ATOM O | 1759 | O | VAL | A | 344 | 234.569 | -82.790 | 117.750 | 1.00 | 58.20 | A |
| ATOM N | 1760 | N | GLU | A | 345 | 236.521 | -82.345 | 118.775 | 1.00 | 52.70 | A |
| ATOM C | 1761 | CA | GLU | A | 345 | 235.945 | -81.929 | 120.037 | 1.00 | 55.50 | A |
| ATOM C | 1762 | CB | GLU | A | 345 | 236.726 | -82.610 | 121.170 | 1.00 | 59.39 | A |
| ATOM C | 1763 | CG | GLU | A | 345 | 236.049 | -82.656 | 122.512 | 1.00 | 70.94 | A |
| ATOM C | 1764 | CD | GLU | A | 345 | 236.741 | -83.623 | 123.456 | 1.00 | 80.37 | A |
| ATOM O | 1765 | OE1 | GLU | A | 345 | 236.904 | -84.815 | 123.076 | 1.00 | 83.68 | A |
| ATOM O | 1766 | OE2 | GLU | A | 345 | 237.114 | -83.190 | 124.572 | 1.00 | 84.85 | A |
| ATOM C | 1767 | C | GLU | A | 345 | 235.984 | -80.414 | 120.226 | 1.00 | 55.94 | A |
| ATOM O | 1768 | O | GLU | A | 345 | 237.012 | -79.866 | 120.647 | 1.00 | 52.34 | A |
| ATOM N | 1769 | N | PHE | A | 346 | 234.868 | -79.746 | 119.941 | 1.00 | 55.31 | A |
| ATOM C | 1770 | CA | PHE | A | 346 | 234.777 | -78.298 | 120.123 | 1.00 | 58.97 | A |
| ATOM C | 1771 | CB | PHE | A | 346 | 235.238 | -77.601 | 118.840 | 1.00 | 55.59 | A |
| ATOM C | 1772 | CG | PHE | A | 346 | 234.247 | -77.671 | 117.721 | 1.00 | 51.30 | A |
| ATOM C | 1773 | CD1 | PHE | A | 346 | 233.315 | -76.667 | 117.540 | 1.00 | 47.92 | A |
| ATOM C | 1774 | CD2 | PHE | A | 346 | 234.261 | -78.734 | 116.842 | 1.00 | 51.74 | A |
| ATOM C | 1775 | CE1 | PHE | A | 346 | 232.396 | -76.715 | 116.483 | 1.00 | 54.03 | A |
| ATOM C | 1776 | CE2 | PHE | A | 346 | 233.348 | -78.810 | 115.764 | 1.00 | 54.32 | A |
| ATOM C | 1777 | CZ | PHE | A | 346 | 232.409 | -77.803 | 115.575 | 1.00 | 51.48 | A |
| ATOM C | 1778 | C | PHE | A | 346 | 233.356 | -77.832 | 120.501 | 1.00 | 60.04 | A |
| ATOM O | 1779 | O | PHE | A | 346 | 232.368 | -78.494 | 120.173 | 1.00 | 69.92 | A |
| ATOM N | 1780 | N | THR | A | 347 | 233.261 | -76.697 | 121.184 | 1.00 | 56.17 | A |
| ATOM C | 1781 | CA | THR | A | 347 | 231.979 | -76.158 | 121.555 | 1.00 | 54.44 | A |
| ATOM C | 1782 | CB | THR | A | 347 | 231.732 | -76.329 | 123.013 | 1.00 | 47.09 | A |
| ATOM O | 1783 | OG1 | THR | A | 347 | 232.718 | -75.600 | 123.725 | 1.00 | 47.43 | A |
| ATOM C | 1784 | CG2 | THR | A | 347 | 231.799 | -77.783 | 123.402 | 1.00 | 48.24 | A |
| ATOM C | 1785 | C | THR | A | 347 | 231.885 | -74.683 | 121.223 | 1.00 | 56.33 | A |
| ATOM O | 1786 | O | THR | A | 347 | 232.902 | -73.985 | 121.113 | 1.00 | 62.80 | A |
| ATOM N | 1787 | N | PHE | A | 348 | 230.646 | -74.235 | 120.999 | 1.00 | 63.69 | A |
| ATOM C | 1788 | CA | PHE | A | 348 | 230.389 | -72.815 | 120.723 | 1.00 | 65.89 | A |
| ATOM C | 1789 | CB | PHE | A | 348 | 229.306 | -72.558 | 119.708 | 1.00 | 57.72 | A |
| ATOM C | 1790 | CG | PHE | A | 348 | 229.584 | -73.148 | 118.381 | 1.00 | 55.81 | A |
| ATOM C | 1791 | CD1 | PHE | A | 348 | 229.017 | -74.364 | 118.020 | 1.00 | 51.99 | A |
| ATOM C | 1792 | CD2 | PHE | A | 348 | 230.337 | -72.462 | 117.450 | 1.00 | 53.67 | A |
| ATOM C | 1793 | CE1 | PHE | A | 348 | 229.171 | -74.857 | 116.780 | 1.00 | 46.52 | A |
| ATOM C | 1794 | CE2 | PHE | A | 348 | 230.492 | -72.966 | 116.190 | 1.00 | 50.86 | A |
| ATOM C | 1795 | CZ | PHE | A | 348 | 229.903 | -74.166 | 115.860 | 1.00 | 51.38 | A |
| ATOM C | 1796 | C | PHE | A | 348 | 229.966 | -72.048 | 121.961 | 1.00 | 65.81 | A |
| ATOM O | 1797 | O | PHE | A | 348 | 229.223 | -72.532 | 122.808 | 1.00 | 73.51 | A |
| ATOM N | 1798 | N | PRO | A | 349 | 230.578 | -70.898 | 122.150 | 1.00 | 59.06 | A |
| ATOM C | 1799 | CD | PRO | A | 349 | 232.018 | -71.026 | 121.861 | 1.00 | 61.27 | A |
| ATOM C | 1800 | CA | PRO | A | 349 | 230.381 | -69.933 | 123.199 | 1.00 | 56.66 | A |
| ATOM C | 1801 | CB | PRO | A | 349 | 231.466 | -68.944 | 122.870 | 1.00 | 58.77 | A |
| ATOM C | 1802 | CG | PRO | A | 349 | 232.627 | -69.875 | 122.485 | 1.00 | 55.14 | A |
| ATOM C | 1803 | C | PRO | A | 349 | 228.929 | -69.409 | 122.948 | 1.00 | 57.97 | A |
| ATOM O | 1804 | O | PRO | A | 349 | 228.312 | -69.642 | 121.916 | 1.00 | 59.47 | A |
| ATOM N | 1805 | N | ASP | A | 350 | 228.344 | -68.735 | 123.909 | 1.00 | 59.19 | A |
| ATOM C | 1806 | CA | ASP | A | 350 | 226.961 | -68.282 | 123.714 | 1.00 | 51.98 | A |
| ATOM C | 1807 | CB | ASP | A | 350 | 226.338 | -67.819 | 125.044 | 1.00 | 65.27 | A |
| ATOM C | 1808 | CG | ASP | A | 350 | 226.140 | -68.958 | 126.020 | 1.00 | 72.68 | A |
| ATOM O | 1809 | OD1 | ASP | A | 350 | 225.764 | -68.668 | 127.188 | 1.00 | 77.91 | A |
| ATOM O | 1810 | OD2 | ASP | A | 350 | 226.359 | -70.131 | 125.607 | 1.00 | 78.14 | A |
| ATOM C | 1811 | C | ASP | A | 350 | 226.787 | -67.197 | 122.665 | 1.00 | 54.69 | A |
| ATOM O | 1812 | O | ASP | A | 350 | 225.753 | -67.202 | 121.947 | 1.00 | 40.28 | A |
| ATOM N | 1813 | N | PHE | A | 351 | 227.786 | -66.308 | 122.539 | 1.00 | 47.45 | A |
| ATOM C | 1814 | CA | PHE | A | 351 | 227.682 | -65.217 | 121.584 | 1.00 | 51.83 | A |
| ATOM C | 1815 | CB | PHE | A | 351 | 228.693 | -64.106 | 121.893 | 1.00 | 48.14 | A |
| ATOM C | 1816 | CG | PHE | A | 351 | 230.108 | -64.580 | 122.004 | 1.00 | 51.21 | A |
| ATOM C | 1817 | CD1 | PHE | A | 351 | 230.589 | -65.111 | 123.207 | 1.00 | 49.73 | A |
| ATOM C | 1818 | CD2 | PHE | A | 351 | 230.979 | -64.468 | 120.915 | 1.00 | 46.79 | A |
| ATOM C | 1819 | CE1 | PHE | A | 351 | 231.906 | -65.506 | 123.312 | 1.00 | 48.51 | A |
| ATOM C | 1820 | CE2 | PHE | A | 351 | 232.294 | -64.864 | 121.026 | 1.00 | 47.18 | A |
| ATOM C | 1821 | CZ | PHE | A | 351 | 232.758 | -65.379 | 122.215 | 1.00 | 45.61 | A |
| ATOM C | 1822 | C | PHE | A | 351 | 227.797 | -65.610 | 120.125 | 1.00 | 52.56 | A |
| ATOM O | 1823 | O | PHE | A | 351 | 227.604 | -64.771 | 119.250 | 1.00 | 57.29 | A |
| ATOM N | 1824 | N | VAL | A | 352 | 228.115 | -66.874 | 119.863 | 1.00 | 54.95 | A |
| ATOM C | 1825 | CA | VAL | A | 352 | 228.263 | -67.341 | 118.495 | 1.00 | 48.00 | A |
| ATOM C | 1826 | CB | VAL | A | 352 | 229.145 | -68.621 | 118.432 | 1.00 | 43.19 | A |
| ATOM C | 1827 | CG1 | VAL | A | 352 | 229.300 | -69.094 | 117.035 | 1.00 | 33.61 | A |
| ATOM C | 1828 | CG2 | VAL | A | 352 | 230.509 | -68.325 | 118.972 | 1.00 | 39.08 | A |
| ATOM C | 1829 | C | VAL | A | 352 | 226.875 | -67.603 | 117.916 | 1.00 | 48.25 | A |
| ATOM O | 1830 | O | VAL | A | 352 | 226.211 | -68.567 | 118.255 | 1.00 | 51.46 | A |
| ATOM N | 1831 | N | THR | A | 353 | 226.446 | -66.748 | 117.004 | 1.00 | 47.51 | A |
| ATOM C | 1832 | CA | THR | A | 353 | 225.122 | -66.886 | 116.413 | 1.00 | 53.02 | A |
| ATOM C | 1833 | CB | THR | A | 353 | 224.844 | -65.817 | 115.383 | 1.00 | 47.94 | A |
| ATOM O | 1834 | OG1 | THR | A | 353 | 225.671 | -66.052 | 114.246 | 1.00 | 54.22 | A |
| ATOM C | 1835 | CG2 | THR | A | 353 | 225.130 | -64.437 | 115.950 | 1.00 | 47.89 | A |
| ATOM C | 1836 | C | THR | A | 353 | 224.908 | -68.227 | 115.753 | 1.00 | 52.60 | A |
| ATOM O | 1837 | O | THR | A | 353 | 225.851 | -68.974 | 115.552 | 1.00 | 52.94 | A |
| ATOM N | 1838 | N | GLU | A | 354 | 223.654 | -68.513 | 115.404 | 1.00 | 55.25 | A |
| ATOM C | 1839 | CA | GLU | A | 354 | 223.259 | -69.786 | 114.819 | 1.00 | 56.40 | A |
| ATOM C | 1840 | CB | GLU | A | 354 | 221.736 | -69.929 | 114.829 | 1.00 | 53.45 | A |
| ATOM C | 1841 | C | GLU | A | 354 | 223.762 | -69.866 | 113.416 | 1.00 | 56.12 | A |
| ATOM O | 1842 | O | GLU | A | 354 | 224.078 | -70.954 | 112.927 | 1.00 | 62.85 | A |
| ATOM N | 1843 | N | GLY | A | 355 | 223.831 | -68.718 | 112.749 | 1.00 | 61.99 | A |
| ATOM C | 1844 | CA | GLY | A | 355 | 224.317 | -68.699 | 111.373 | 1.00 | 52.27 | A |
| ATOM C | 1845 | C | GLY | A | 355 | 225.787 | -69.093 | 111.250 | 1.00 | 52.90 | A |
| ATOM O | 1846 | O | GLY | A | 355 | 226.166 | -69.822 | 110.327 | 1.00 | 47.53 | A |
| ATOM N | 1847 | N | ALA | A | 356 | 226.611 | -68.602 | 112.182 | 1.00 | 47.86 | A |
| ATOM C | 1848 | CA | ALA | A | 356 | 228.022 | -68.912 | 112.199 | 1.00 | 45.96 | A |
| ATOM C | 1849 | CB | ALA | A | 356 | 228.721 | -67.995 | 113.123 | 1.00 | 42.05 | A |
| ATOM C | 1850 | C | ALA | A | 356 | 228.221 | -70.359 | 112.627 | 1.00 | 47.72 | A |
| ATOM O | 1851 | O | ALA | A | 356 | 229.011 | -71.078 | 112.025 | 1.00 | 46.93 | A |
| ATOM N | 1852 | N | ARG | A | 357 | 227.486 | -70.784 | 113.651 | 1.00 | 49.03 | A |
| ATOM C | 1853 | CA | ARG | A | 357 | 227.558 | -72.153 | 114.146 | 1.00 | 56.76 | A |
| ATOM C | 1854 | CB | ARG | A | 357 | 226.549 | -72.374 | 115.280 | 1.00 | 55.71 | A |
| ATOM C | 1855 | CG | ARG | A | 357 | 226.876 | -71.606 | 116.583 | 1.00 | 57.21 | A |
| ATOM C | 1856 | CD | ARG | A | 357 | 226.018 | -72.108 | 117.752 | 1.00 | 41.40 | A |
| ATOM N | 1857 | NE | ARG | A | 357 | 226.260 | -71.310 | 118.928 | 1.00 | 43.14 | A |
| ATOM C | 1858 | CZ | ARG | A | 357 | 225.979 | -71.698 | 120.148 | 1.00 | 39.58 | A |
| ATOM N | 1859 | NH1 | ARG | A | 357 | 225.456 | -72.893 | 120.340 | 1.00 | 42.66 | A |
| ATOM N | 1860 | NH2 | ARG | A | 357 | 226.191 | -70.869 | 121.163 | 1.00 | 51.41 | A |
| ATOM C | 1861 | C | ARG | A | 357 | 227.299 | -73.181 | 113.043 | 1.00 | 58.61 | A |
| ATOM O | 1862 | O | ARG | A | 357 | 227.922 | -74.260 | 112.996 | 1.00 | 62.70 | A |
| ATOM N | 1863 | N | ASP | A | 358 | 226.383 | -72.845 | 112.150 | 1.00 | 56.80 | A |
| ATOM C | 1864 | CA | ASP | A | 358 | 226.053 | -73.738 | 111.055 | 1.00 | 56.49 | A |
| ATOM C | 1865 | CB | ASP | A | 358 | 224.792 | -73.245 | 110.363 | 1.00 | 59.12 | A |
| ATOM C | 1866 | CG | ASP | A | 358 | 224.380 | -74.141 | 109.209 | 1.00 | 65.41 | A |
| ATOM O | 1867 | OD1 | ASP | A | 358 | 224.036 | -75.319 | 109.461 | 1.00 | 60.89 | A |
| ATOM O | 1868 | OD2 | ASP | A | 358 | 224.403 | -73.669 | 108.049 | 1.00 | 68.71 | A |
| ATOM C | 1869 | C | ASP | A | 358 | 227.178 | -73.837 | 110.017 | 1.00 | 56.31 | A |
| ATOM O | 1870 | O | ASP | A | 358 | 227.523 | -74.915 | 109.545 | 1.00 | 56.62 | A |
| ATOM N | 1871 | N | LEU | A | 359 | 227.735 | -72.694 | 109.640 | 1.00 | 57.58 | A |
| ATOM C | 1872 | CA | LEU | A | 359 | 228.812 | -72.663 | 108.658 | 1.00 | 55.45 | A |
| ATOM C | 1873 | CB | LEU | A | 359 | 229.158 | -71.227 | 108.302 | 1.00 | 53.49 | A |
| ATOM C | 1874 | CG | LEU | A | 359 | 230.326 | -71.029 | 107.344 | 1.00 | 49.10 | A |
| ATOM C | 1875 | CD1 | LEU | A | 359 | 230.077 | -71.756 | 106.082 | 1.00 | 42.56 | A |
| ATOM C | 1876 | CD2 | LEU | A | 359 | 230.525 | -69.538 | 107.083 | 1.00 | 51.15 | A |
| ATOM C | 1877 | C | LEU | A | 359 | 230.048 | -73.366 | 109.200 | 1.00 | 57.66 | A |
| ATOM O | 1878 | O | LEU | A | 359 | 230.680 | -74.142 | 108.481 | 1.00 | 65.55 | A |
| ATOM N | 1879 | N | ILE | A | 360 | 230.370 | -73.121 | 110.470 | 1.00 | 49.19 | A |
| ATOM C | 1880 | CA | ILE | A | 360 | 231.535 | -73.740 | 111.089 | 1.00 | 43.07 | A |
| ATOM C | 1881 | CB | ILE | A | 360 | 231.819 | -73.111 | 112.485 | 1.00 | 35.85 | A |
| ATOM C | 1882 | CG2 | ILE | A | 360 | 232.892 | -73.858 | 113.220 | 1.00 | 37.90 | A |
| ATOM C | 1883 | CG1 | ILE | A | 360 | 232.254 | -71.652 | 112.301 | 1.00 | 32.36 | A |
| ATOM C | 1884 | CD1 | ILE | A | 360 | 232.250 | -70.839 | 113.653 | 1.00 | 27.28 | A |
| ATOM C | 1885 | C | ILE | A | 360 | 231.368 | -75.240 | 111.229 | 1.00 | 40.84 | A |
| ATOM O | 1886 | O | ILE | A | 360 | 232.330 | -75.980 | 111.042 | 1.00 | 44.75 | A |
| ATOM N | 1887 | N | SER | A | 361 | 230.158 | -75.670 | 111.591 | 1.00 | 46.60 | A |
| ATOM C | 1888 | CA | SER | A | 361 | 229.855 | -77.089 | 111.780 | 1.00 | 52.65 | A |
| ATOM C | 1889 | CB | SER | A | 361 | 228.546 | -77.265 | 112.526 | 1.00 | 49.78 | A |
| ATOM O | 1890 | OG | SER | A | 361 | 228.742 | -77.062 | 113.925 | 1.00 | 60.97 | A |
| ATOM C | 1891 | C | SER | A | 361 | 229.827 | -77.906 | 110.497 | 1.00 | 53.49 | A |
| ATOM O | 1892 | O | SER | A | 361 | 229.885 | -79.159 | 110.538 | 1.00 | 53.09 | A |
| ATOM N | 1893 | N | ARG | A | 362 | 229.799 | -77.198 | 109.368 | 1.00 | 54.39 | A |
| ATOM C | 1894 | CA | ARG | A | 362 | 229.796 | -77.841 | 108.066 | 1.00 | 52.12 | A |
| ATOM C | 1895 | CB | ARG | A | 362 | 229.033 | -76.978 | 107.065 | 1.00 | 59.93 | A |
| ATOM C | 1896 | CG | ARG | A | 362 | 227.535 | -76.862 | 107.327 | 1.00 | 61.75 | A |
| ATOM C | 1897 | CD | ARG | A | 362 | 226.887 | -75.820 | 106.401 | 1.00 | 69.51 | A |
| ATOM N | 1898 | NE | ARG | A | 362 | 225.432 | -75.784 | 106.547 | 1.00 | 76.19 | A |
| ATOM C | 1899 | CZ | ARG | A | 362 | 224.625 | -76.795 | 106.216 | 1.00 | 80.23 | A |
| ATOM N | 1900 | NH1 | ARG | A | 362 | 225.128 | -77.919 | 105.716 | 1.00 | 81.25 | A |
| ATOM N | 1901 | NH2 | ARG | A | 362 | 223.313 | -76.697 | 106.397 | 1.00 | 77.76 | A |
| ATOM C | 1902 | C | ARG | A | 362 | 231.211 | -78.066 | 107.537 | 1.00 | 49.26 | A |
| ATOM O | 1903 | O | ARG | A | 362 | 231.460 | -79.036 | 106.822 | 1.00 | 49.03 | A |
| ATOM N | 1904 | N | LEU | A | 363 | 232.120 | -77.162 | 107.891 | 1.00 | 49.05 | A |
| ATOM C | 1905 | CA | LEU | A | 363 | 233.493 | -77.234 | 107.438 | 1.00 | 46.81 | A |
| ATOM C | 1906 | CB | LEU | A | 363 | 234.134 | -75.850 | 107.508 | 1.00 | 42.60 | A |
| ATOM C | 1907 | CG | LEU | A | 363 | 233.457 | -74.729 | 106.714 | 1.00 | 44.53 | A |
| ATOM C | 1908 | CD1 | LEU | A | 363 | 234.050 | -73.356 | 107.133 | 1.00 | 43.18 | A |
| ATOM C | 1909 | CD2 | LEU | A | 363 | 233.602 | -74.961 | 105.258 | 1.00 | 42.47 | A |
| ATOM C | 1910 | C | LEU | A | 363 | 234.270 | -78.228 | 108.280 | 1.00 | 49.03 | A |
| ATOM O | 1911 | O | LEU | A | 363 | 235.049 | -79.020 | 107.751 | 1.00 | 57.90 | A |
| ATOM N | 1912 | N | LEU | A | 364 | 234.062 | -78.186 | 109.589 | 1.00 | 47.92 | A |
| ATOM C | 1913 | CA | LEU | A | 364 | 234.774 | -79.071 | 110.488 | 1.00 | 49.66 | A |
| ATOM C | 1914 | CB | LEU | A | 364 | 234.792 | -78.462 | 111.909 | 1.00 | 46.24 | A |
| ATOM C | 1915 | CG | LEU | A | 364 | 235.511 | -77.115 | 112.043 | 1.00 | 47.59 | A |
| ATOM C | 1916 | CD1 | LEU | A | 364 | 235.230 | -76.547 | 113.413 | 1.00 | 44.59 | A |
| ATOM C | 1917 | CD2 | LEU | A | 364 | 237.029 | -77.318 | 111.836 | 1.00 | 45.40 | A |
| ATOM C | 1918 | C | LEU | A | 364 | 234.233 | -80.493 | 110.526 | 1.00 | 50.53 | A |
| ATOM O | 1919 | O | LEU | A | 364 | 233.804 | -80.963 | 111.575 | 1.00 | 56.94 | A |
| ATOM N | 1920 | N | LYS | A | 365 | 234.267 | -81.178 | 109.385 | 1.00 | 61.00 | A |
| ATOM C | 1921 | CA | LYS | A | 365 | 233.796 | -82.564 | 109.263 | 1.00 | 64.52 | A |
| ATOM C | 1922 | CB | LYS | A | 365 | 233.059 | -82.728 | 107.925 | 1.00 | 60.48 | A |
| ATOM C | 1923 | CG | LYS | A | 365 | 231.753 | -81.921 | 107.829 | 1.00 | 53.27 | A |
| ATOM C | 1924 | CD | LYS | A | 365 | 230.702 | -82.412 | 108.828 | 1.00 | 61.58 | A |
| ATOM C | 1925 | CE | LYS | A | 365 | 229.295 | -81.895 | 108.447 | 1.00 | 59.92 | A |
| ATOM N | 1926 | NZ | LYS | A | 365 | 228.297 | -82.223 | 109.508 | 1.00 | 75.42 | A |
| ATOM C | 1927 | C | LYS | A | 365 | 235.026 | -83.500 | 109.392 | 1.00 | 66.56 | A |
| ATOM O | 1928 | O | LYS | A | 365 | 236.146 | -83.084 | 109.123 | 1.00 | 71.71 | A |
| ATOM N | 1929 | N | HIS | A | 366 | 234.844 | -84.748 | 109.813 | 1.00 | 68.20 | A |
| ATOM C | 1930 | CA | HIS | A | 366 | 236.011 | -85.628 | 110.015 | 1.00 | 67.59 | A |
| ATOM C | 1931 | CB | HIS | A | 366 | 235.781 | -86.664 | 111.109 | 1.00 | 66.12 | A |
| ATOM C | 1932 | CG | HIS | A | 366 | 236.913 | -87.628 | 111.242 | 1.00 | 68.47 | A |
| ATOM C | 1933 | CD2 | HIS | A | 366 | 237.810 | -87.749 | 112.245 | 1.00 | 66.88 | A |
| ATOM N | 1934 | ND1 | HIS | A | 366 | 237.332 | -88.487 | 110.238 | 1.00 | 66.01 | A |
| ATOM C | 1935 | CE1 | HIS | A | 366 | 238.454 | -89.075 | 110.623 | 1.00 | 70.20 | A |
| ATOM N | 1936 | NE2 | HIS | A | 366 | 238.761 | -88.643 | 111.832 | 1.00 | 64.97 | A |
| ATOM C | 1937 | C | HIS | A | 366 | 236.184 | -86.385 | 108.739 | 1.00 | 68.25 | A |
| ATOM O | 1938 | O | HIS | A | 366 | 237.098 | -87.249 | 108.572 | 1.00 | 74.87 | A |
| ATOM N | 1939 | N | ASN | A | 367 | 235.262 | -86.111 | 107.848 | 1.00 | 65.45 | A |
| ATOM C | 1940 | CA | ASN | A | 367 | 235.379 | -86.795 | 106.656 | 1.00 | 62.81 | A |
| ATOM C | 1941 | CB | ASN | A | 367 | 234.189 | -87.664 | 106.498 | 1.00 | 64.61 | A |
| ATOM C | 1942 | CG | ASN | A | 367 | 234.335 | -88.548 | 105.333 | 1.00 | 69.77 | A |
| ATOM O | 1943 | OD1 | ASN | A | 367 | 234.339 | -88.071 | 104.175 | 1.00 | 65.35 | A |
| ATOM N | 1944 | ND2 | ASN | A | 367 | 234.518 | -89.855 | 105.596 | 1.00 | 69.27 | A |
| ATOM C | 1945 | C | ASN | A | 367 | 235.504 | -85.791 | 105.596 | 1.00 | 60.17 | A |
| ATOM O | 1946 | O | ASN | A | 367 | 234.685 | -84.923 | 105.475 | 1.00 | 67.65 | A |
| ATOM N | 1947 | N | PRO | A | 368 | 236.650 | -85.797 | 104.923 | 1.00 | 60.54 | A |
| ATOM C | 1948 | CD | PRO | A | 368 | 237.882 | -86.364 | 105.504 | 1.00 | 55.35 | A |
| ATOM C | 1949 | CA | PRO | A | 368 | 236.981 | -84.895 | 103.820 | 1.00 | 55.82 | A |
| ATOM C | 1950 | CB | PRO | A | 368 | 238.199 | -85.537 | 103.245 | 1.00 | 50.93 | A |
| ATOM C | 1951 | CG | PRO | A | 368 | 238.938 | -85.936 | 104.468 | 1.00 | 54.85 | A |
| ATOM C | 1952 | C | PRO | A | 368 | 235.870 | -84.741 | 102.783 | 1.00 | 59.90 | A |
| ATOM O | 1953 | O | PRO | A | 368 | 235.706 | -83.658 | 102.240 | 1.00 | 61.00 | A |
| ATOM N | 1954 | N | SER | A | 369 | 235.093 | -85.805 | 102.557 | 1.00 | 69.67 | A |
| ATOM C | 1955 | CA | SER | A | 369 | 234.000 | -85.800 | 101.588 | 1.00 | 72.88 | A |
| ATOM C | 1956 | CB | SER | A | 369 | 233.537 | -87.237 | 101.347 | 1.00 | 75.22 | A |
| ATOM O | 1957 | OG | SER | A | 369 | 234.654 | -88.115 | 101.256 | 1.00 | 85.92 | A |
| ATOM C | 1958 | C | SER | A | 369 | 232.810 | -84.951 | 102.046 | 1.00 | 71.84 | A |
| ATOM O | 1959 | O | SER | A | 369 | 232.217 | -84.231 | 101.229 | 1.00 | 75.53 | A |
| ATOM N | 1960 | N | GLN | A | 370 | 232.465 | -85.039 | 103.333 | 1.00 | 67.85 | A |
| ATOM C | 1961 | CA | GLN | A | 370 | 231.339 | -84.296 | 103.865 | 1.00 | 62.69 | A |
| ATOM C | 1962 | CB | GLN | A | 370 | 231.067 | -84.743 | 105.296 | 1.00 | 64.15 | A |
| ATOM C | 1963 | CG | GLN | A | 370 | 230.629 | -86.190 | 105.390 | 1.00 | 65.40 | A |
| ATOM C | 1964 | CD | GLN | A | 370 | 230.923 | -86.768 | 106.745 | 1.00 | 69.41 | A |
| ATOM O | 1965 | OE1 | GLN | A | 370 | 230.904 | -86.051 | 107.752 | 1.00 | 75.34 | A |
| ATOM N | 1966 | NE2 | GLN | A | 370 | 231.189 | -88.073 | 106.792 | 1.00 | 63.71 | A |
| ATOM C | 1967 | C | GLN | A | 370 | 231.551 | -82.778 | 103.799 | 1.00 | 63.34 | A |
| ATOM O | 1968 | O | GLN | A | 370 | 230.582 | -82.028 | 103.608 | 1.00 | 65.90 | A |
| ATOM N | 1969 | N | ARG | A | 371 | 232.802 | -82.324 | 103.934 | 1.00 | 56.18 | A |
| ATOM C | 1970 | CA | ARG | A | 371 | 233.085 | -80.905 | 103.889 | 1.00 | 54.33 | A |
| ATOM C | 1971 | CB | ARG | A | 371 | 234.581 | -80.691 | 103.961 | 1.00 | 48.64 | A |
| ATOM C | 1972 | CG | ARG | A | 371 | 235.109 | -80.785 | 105.354 | 1.00 | 46.61 | A |
| ATOM C | 1973 | CD | ARG | A | 371 | 236.618 | -80.922 | 105.403 | 1.00 | 43.30 | A |
| ATOM N | 1974 | NE | ARG | A | 371 | 236.988 | -81.971 | 106.350 | 1.00 | 42.51 | A |
| ATOM C | 1975 | CZ | ARG | A | 371 | 238.208 | -82.476 | 106.436 | 1.00 | 42.45 | A |
| ATOM N | 1976 | NH1 | ARG | A | 371 | 239.165 | -81.997 | 105.635 | 1.00 | 36.24 | A |
| ATOM N | 1977 | NH2 | ARG | A | 371 | 238.449 | -83.494 | 107.266 | 1.00 | 33.74 | A |
| ATOM C | 1978 | C | ARG | A | 371 | 232.496 | -80.275 | 102.651 | 1.00 | 56.25 | A |
| ATOM O | 1979 | O | ARG | A | 371 | 232.578 | -80.836 | 101.587 | 1.00 | 64.77 | A |
| ATOM N | 1980 | N | PRO | A | 372 | 231.885 | -79.089 | 102.783 | 1.00 | 60.04 | A |
| ATOM C | 1981 | CD | PRO | A | 372 | 231.813 | -78.305 | 104.029 | 1.00 | 57.31 | A |
| ATOM C | 1982 | CA | PRO | A | 372 | 231.260 | -78.365 | 101.659 | 1.00 | 57.19 | A |
| ATOM C | 1983 | CB | PRO | A | 372 | 230.522 | -77.231 | 102.348 | 1.00 | 55.89 | A |
| ATOM C | 1984 | CG | PRO | A | 372 | 231.448 | -76.927 | 103.522 | 1.00 | 60.48 | A |
| ATOM C | 1985 | C | PRO | A | 372 | 232.227 | -77.848 | 100.593 | 1.00 | 59.74 | A |
| ATOM O | 1986 | O | PRO | A | 372 | 233.424 | -77.896 | 100.753 | 1.00 | 60.73 | A |
| ATOM N | 1987 | N | MET | A | 373 | 231.685 | -77.356 | 99.497 | 1.00 | 66.48 | A |
| ATOM C | 1988 | CA | MET | A | 373 | 232.482 | -76.838 | 98.412 | 1.00 | 72.53 | A |
| ATOM C | 1989 | CB | MET | A | 373 | 231.763 | -77.051 | 97.082 | 1.00 | 78.23 | A |
| ATOM C | 1990 | CG | MET | A | 373 | 232.541 | -77.969 | 96.165 | 1.00 | 91.60 | A |
| ATOM S | 1991 | SD | MET | A | 373 | 231.857 | -78.033 | 94.463 | 1.00 | 92.28 | A |
| ATOM C | 1992 | CE | MET | A | 373 | 230.337 | -78.924 | 94.769 | 1.00 | 97.23 | A |
| ATOM C | 1993 | C | MET | A | 373 | 232.631 | -75.339 | 98.682 | 1.00 | 69.48 | A |
| ATOM O | 1994 | O | MET | A | 373 | 231.861 | -74.740 | 99.424 | 1.00 | 71.05 | A |
| ATOM N | 1995 | N | LEU | A | 374 | 233.618 | -74.722 | 98.060 | 1.00 | 66.97 | A |
| ATOM C | 1996 | CA | LEU | A | 374 | 233.847 | -73.303 | 98.261 | 1.00 | 65.90 | A |
| ATOM C | 1997 | CB | LEU | A | 374 | 235.155 | -72.857 | 97.562 | 1.00 | 58.73 | A |
| ATOM C | 1998 | CG | LEU | A | 374 | 236.446 | -73.311 | 98.258 | 1.00 | 51.64 | A |
| ATOM C | 1999 | CD1 | LEU | A | 374 | 237.625 | -73.104 | 97.321 | 1.00 | 47.33 | A |
| ATOM C | 2000 | CD2 | LEU | A | 374 | 236.629 | -72.531 | 99.548 | 1.00 | 52.27 | A |
| ATOM C | 2001 | C | LEU | A | 374 | 232.660 | -72.469 | 97.798 | 1.00 | 67.13 | A |
| ATOM O | 2002 | O | LEU | A | 374 | 232.500 | -71.320 | 98.219 | 1.00 | 75.20 | A |
| ATOM N | 2003 | N | ARG | A | 375 | 231.814 | -73.025 | 96.939 | 1.00 | 67.09 | A |
| ATOM C | 2004 | CA | ARG | A | 375 | 230.654 | -72.254 | 96.482 | 1.00 | 63.14 | A |
| ATOM C | 2005 | CB | ARG | A | 375 | 230.122 | -72.780 | 95.162 | 1.00 | 60.43 | A |
| ATOM C | 2006 | C | ARG | A | 375 | 229.561 | -72.275 | 97.541 | 1.00 | 63.36 | A |
| ATOM O | 2007 | O | ARG | A | 375 | 228.882 | -71.261 | 97.734 | 1.00 | 64.17 | A |
| ATOM N | 2008 | N | GLU | A | 376 | 229.413 | -73.397 | 98.245 | 1.00 | 56.91 | A |
| ATOM C | 2009 | CA | GLU | A | 376 | 228.404 | -73.501 | 99.306 | 1.00 | 64.22 | A |
| ATOM C | 2010 | CB | GLU | A | 376 | 228.314 | -74.947 | 99.815 | 1.00 | 67.11 | A |
| ATOM C | 2011 | CG | GLU | A | 376 | 227.797 | -75.921 | 98.783 | 1.00 | 81.78 | A |
| ATOM C | 2012 | CD | GLU | A | 376 | 228.016 | -77.372 | 99.187 | 1.00 | 87.25 | A |
| ATOM O | 2013 | OE1 | GLU | A | 376 | 229.137 | -77.887 | 98.974 | 1.00 | 95.99 | A |
| ATOM O | 2014 | OE2 | GLU | A | 376 | 227.073 | -78.001 | 99.732 | 1.00 | 98.67 | A |
| ATOM C | 2015 | C | GLU | A | 376 | 228.791 | -72.562 | 100.461 | 1.00 | 61.59 | A |
| ATOM O | 2016 | O | GLU | A | 376 | 227.950 | -72.145 | 101.275 | 1.00 | 63.73 | A |
| ATOM N | 2017 | N | VAL | A | 377 | 230.078 | -72.229 | 100.505 | 1.00 | 60.90 | A |
| ATOM C | 2018 | CA | VAL | A | 377 | 230.605 | -71.361 | 101.539 | 1.00 | 60.11 | A |
| ATOM C | 2019 | CB | VAL | A | 377 | 232.149 | -71.606 | 101.780 | 1.00 | 57.13 | A |
| ATOM C | 2020 | CG1 | VAL | A | 377 | 232.701 | -70.591 | 102.788 | 1.00 | 52.18 | A |
| ATOM C | 2021 | CG2 | VAL | A | 377 | 232.364 | -72.987 | 102.341 | 1.00 | 51.22 | A |
| ATOM C | 2022 | C | VAL | A | 377 | 230.354 | -69.928 | 101.140 | 1.00 | 59.92 | A |
| ATOM O | 2023 | O | VAL | A | 377 | 229.915 | -69.114 | 101.952 | 1.00 | 69.33 | A |
| ATOM N | 2024 | N | LEU | A | 378 | 230.639 | -69.625 | 99.884 | 1.00 | 54.97 | A |
| ATOM C | 2025 | CA | LEU | A | 378 | 230.466 | -68.269 | 99.384 | 1.00 | 57.61 | A |
| ATOM C | 2026 | CB | LEU | A | 378 | 231.178 | -68.090 | 98.026 | 1.00 | 55.37 | A |
| ATOM C | 2027 | CG | LEU | A | 378 | 232.564 | -67.442 | 97.937 | 1.00 | 56.77 | A |
| ATOM C | 2028 | CD1 | LEU | A | 378 | 232.986 | -66.902 | 99.309 | 1.00 | 59.47 | A |
| ATOM C | 2029 | CD2 | LEU | A | 378 | 233.517 | -68.445 | 97.421 | 1.00 | 52.34 | A |
| ATOM C | 2030 | C | LEU | A | 378 | 228.995 | -67.925 | 99.248 | 1.00 | 53.77 | A |
| ATOM O | 2031 | O | LEU | A | 378 | 228.626 | -66.775 | 99.062 | 1.00 | 56.35 | A |
| ATOM N | 2032 | N | GLU | A | 379 | 228.144 | -68.930 | 99.367 | 1.00 | 58.84 | A |
| ATOM C | 2033 | CA | GLU | A | 379 | 226.708 | -68.714 | 99.236 | 1.00 | 63.13 | A |
| ATOM C | 2034 | CB | GLU | A | 379 | 226.101 | -69.720 | 98.274 | 1.00 | 65.30 | A |
| ATOM C | 2035 | CG | GLU | A | 379 | 226.567 | -69.569 | 96.831 | 1.00 | 70.53 | A |
| ATOM C | 2036 | CD | GLU | A | 379 | 225.831 | -70.533 | 95.895 | 1.00 | 76.21 | A |
| ATOM O | 2037 | OE1 | GLU | A | 379 | 225.546 | -71.692 | 96.329 | 1.00 | 70.09 | A |
| ATOM O | 2038 | OE2 | GLU | A | 379 | 225.547 | -70.124 | 94.737 | 1.00 | 76.09 | A |
| ATOM C | 2039 | C | GLU | A | 379 | 225.991 | -68.820 | 100.555 | 1.00 | 60.26 | A |
| ATOM O | 2040 | O | GLU | A | 379 | 224.963 | -68.191 | 100.746 | 1.00 | 70.86 | A |
| ATOM N | 2041 | N | HIS | A | 380 | 226.535 | -69.606 | 101.468 | 1.00 | 54.34 | A |
| ATOM C | 2042 | CA | HIS | A | 380 | 225.921 | -69.775 | 102.771 | 1.00 | 54.61 | A |
| ATOM C | 2043 | CB | HIS | A | 380 | 226.975 | -70.234 | 103.793 | 1.00 | 48.39 | A |
| ATOM C | 2044 | CG | HIS | A | 380 | 226.402 | -70.617 | 105.121 | 1.00 | 45.24 | A |
| ATOM C | 2045 | CD2 | HIS | A | 380 | 226.402 | -71.793 | 105.781 | 1.00 | 42.14 | A |
| ATOM N | 2046 | ND1 | HIS | A | 380 | 225.711 | -69.731 | 105.921 | 1.00 | 51.46 | A |
| ATOM C | 2047 | CE1 | HIS | A | 380 | 225.309 | -70.349 | 107.018 | 1.00 | 50.88 | A |
| ATOM N | 2048 | NE2 | HIS | A | 380 | 225.715 | -71.604 | 106.957 | 1.00 | 47.24 | A |
| ATOM C | 2049 | C | HIS | A | 380 | 225.231 | -68.502 | 103.261 | 1.00 | 55.95 | A |
| ATOM O | 2050 | O | HIS | A | 380 | 225.807 | -67.409 | 103.206 | 1.00 | 60.83 | A |
| ATOM N | 2051 | N | PRO | A | 381 | 223.970 | -68.630 | 103.727 | 1.00 | 55.60 | A |
| ATOM C | 2052 | CD | PRO | A | 381 | 223.229 | -69.910 | 103.809 | 1.00 | 51.69 | A |
| ATOM C | 2053 | CA | PRO | A | 381 | 223.147 | -67.522 | 104.248 | 1.00 | 53.48 | A |
| ATOM C | 2054 | CB | PRO | A | 381 | 221.997 | -68.257 | 104.936 | 1.00 | 49.53 | A |
| ATOM C | 2055 | CG | PRO | A | 381 | 221.820 | -69.442 | 104.035 | 1.00 | 49.24 | A |
| ATOM C | 2056 | C | PRO | A | 381 | 223.875 | -66.545 | 105.212 | 1.00 | 56.98 | A |
| ATOM O | 2057 | O | PRO | A | 381 | 223.758 | -65.320 | 105.074 | 1.00 | 59.03 | A |
| ATOM N | 2058 | N | TRP | A | 382 | 224.635 | -67.091 | 106.164 | 1.00 | 54.77 | A |
| ATOM C | 2059 | CA | TRP | A | 382 | 225.340 | -66.282 | 107.146 | 1.00 | 55.06 | A |
| ATOM C | 2060 | CB | TRP | A | 382 | 225.971 | -67.165 | 108.240 | 1.00 | 54.64 | A |
| ATOM C | 2061 | CG | TRP | A | 382 | 226.691 | -66.368 | 109.261 | 1.00 | 55.47 | A |
| ATOM C | 2062 | CD2 | TRP | A | 382 | 228.111 | -66.195 | 109.372 | 1.00 | 58.05 | A |
| ATOM C | 2063 | CE2 | TRP | A | 382 | 228.344 | -65.342 | 110.476 | 1.00 | 60.80 | A |
| ATOM C | 2064 | CE3 | TRP | A | 382 | 229.210 | -66.680 | 108.649 | 1.00 | 60.95 | A |
| ATOM C | 2065 | CD1 | TRP | A | 382 | 226.137 | -65.634 | 110.266 | 1.00 | 58.89 | A |
| ATOM N | 2066 | NE1 | TRP | A | 382 | 227.123 | -65.011 | 111.004 | 1.00 | 59.37 | A |
| ATOM C | 2067 | CZ2 | TRP | A | 382 | 229.632 | -64.965 | 110.871 | 1.00 | 64.01 | A |
| ATOM C | 2068 | CZ3 | TRP | A | 382 | 230.491 | -66.302 | 109.042 | 1.00 | 60.80 | A |
| ATOM C | 2069 | CH2 | TRP | A | 382 | 230.688 | -65.458 | 110.140 | 1.00 | 65.05 | A |
| ATOM C | 2070 | C | TRP | A | 382 | 226.397 | -65.476 | 106.455 | 1.00 | 50.36 | A |
| ATOM O | 2071 | O | TRP | A | 382 | 226.579 | -64.300 | 106.749 | 1.00 | 56.46 | A |
| ATOM N | 2072 | N | ILE | A | 383 | 227.094 | -66.115 | 105.527 | 1.00 | 50.17 | A |
| ATOM C | 2073 | CA | ILE | A | 383 | 228.163 | -65.454 | 104.757 | 1.00 | 51.21 | A |
| ATOM C | 2074 | CB | ILE | A | 383 | 228.914 | -66.460 | 103.856 | 1.00 | 42.86 | A |
| ATOM C | 2075 | CG2 | ILE | A | 383 | 229.642 | -65.709 | 102.766 | 1.00 | 42.15 | A |
| ATOM C | 2076 | CG1 | ILE | A | 383 | 229.846 | -67.329 | 104.708 | 1.00 | 40.04 | A |
| ATOM C | 2077 | CD1 | ILE | A | 383 | 230.943 | -66.520 | 105.429 | 1.00 | 40.46 | A |
| ATOM C | 2078 | C | ILE | A | 383 | 227.659 | -64.305 | 103.873 | 1.00 | 52.36 | A |
| ATOM O | 2079 | O | ILE | A | 383 | 228.247 | -63.229 | 103.858 | 1.00 | 51.23 | A |
| ATOM N | 2080 | N | THR | A | 384 | 226.553 | -64.533 | 103.166 | 1.00 | 54.23 | A |
| ATOM C | 2081 | CA | THR | A | 384 | 225.978 | -63.535 | 102.275 | 1.00 | 58.09 | A |
| ATOM C | 2082 | CB | THR | A | 384 | 225.046 | -64.197 | 101.265 | 1.00 | 57.25 | A |
| ATOM O | 2083 | OG1 | THR | A | 384 | 224.320 | -65.235 | 101.920 | 1.00 | 65.44 | A |
| ATOM C | 2084 | CG2 | THR | A | 384 | 225.809 | -64.804 | 100.133 | 1.00 | 59.94 | A |
| ATOM C | 2085 | C | THR | A | 384 | 225.221 | -62.436 | 102.988 | 1.00 | 57.62 | A |
| ATOM O | 2086 | O | THR | A | 384 | 224.929 | -61.395 | 102.420 | 1.00 | 61.64 | A |
| ATOM N | 2087 | N | ALA | A | 385 | 224.911 | -62.655 | 104.249 | 1.00 | 60.34 | A |
| ATOM C | 2088 | CA | ALA | A | 385 | 224.195 | -61.658 | 105.030 | 1.00 | 57.14 | A |
| ATOM C | 2089 | CB | ALA | A | 385 | 223.197 | -62.376 | 105.944 | 1.00 | 61.04 | A |
| ATOM C | 2090 | C | ALA | A | 385 | 225.112 | -60.735 | 105.867 | 1.00 | 57.68 | A |
| ATOM O | 2091 | O | ALA | A | 385 | 224.644 | -59.765 | 106.467 | 1.00 | 56.76 | A |
| ATOM N | 2092 | N | ASN | A | 386 | 226.405 | -61.038 | 105.919 | 1.00 | 57.22 | A |
| ATOM C | 2093 | CA | ASN | A | 386 | 227.312 | -60.217 | 106.705 | 1.00 | 59.50 | A |
| ATOM C | 2094 | CB | ASN | A | 386 | 227.751 | -60.993 | 107.939 | 1.00 | 58.18 | A |
| ATOM C | 2095 | CG | ASN | A | 386 | 226.583 | -61.313 | 108.874 | 1.00 | 60.78 | A |
| ATOM O | 2096 | OD1 | ASN | A | 386 | 226.026 | -60.419 | 109.536 | 1.00 | 55.54 | A |
| ATOM N | 2097 | ND2 | ASN | A | 386 | 226.201 | -62.591 | 108.923 | 1.00 | 55.70 | A |
| ATOM C | 2098 | C | ASN | A | 386 | 228.540 | -59.733 | 105.965 | 1.00 | 55.04 | A |
| ATOM O | 2099 | O | ASN | A | 386 | 229.128 | -58.723 | 106.336 | 1.00 | 58.67 | A |
| ATOM N | 2100 | N | SER | A | 387 | 228.903 | -60.444 | 104.906 | 1.00 | 58.95 | A |
| ATOM C | 2101 | CA | SER | A | 387 | 230.095 | -60.110 | 104.132 | 1.00 | 63.83 | A |
| ATOM C | 2102 | CB | SER | A | 387 | 230.472 | -61.278 | 103.220 | 1.00 | 55.03 | A |
| ATOM O | 2103 | OG | SER | A | 387 | 231.719 | -61.061 | 102.599 | 1.00 | 57.80 | A |
| ATOM C | 2104 | C | SER | A | 387 | 229.865 | -58.867 | 103.278 | 1.00 | 64.86 | A |
| ATOM O | 2105 | O | SER | A | 387 | 228.745 | -58.607 | 102.826 | 1.00 | 68.20 | A |
| ATOM N | 2106 | N | SER | A | 388 | 230.916 | -58.091 | 103.063 | 1.00 | 67.77 | A |
| ATOM C | 2107 | CA | SER | A | 388 | 230.786 | -56.912 | 102.251 | 1.00 | 71.39 | A |
| ATOM C | 2108 | CB | SER | A | 388 | 231.430 | -55.722 | 102.969 | 1.00 | 71.24 | A |
| ATOM O | 2109 | OG | SER | A | 388 | 232.815 | -55.916 | 103.141 | 1.00 | 79.50 | A |
| ATOM C | 2110 | C | SER | A | 388 | 231.403 | -57.150 | 100.872 | 1.00 | 71.24 | A |
| ATOM O | 2111 | O | SER | A | 388 | 231.024 | -56.501 | 99.909 | 1.00 | 76.22 | A |
| ATOM N | 2112 | N | LYS | A | 389 | 232.336 | -58.091 | 100.767 | 1.00 | 70.18 | A |
| ATOM C | 2113 | CA | LYS | A | 389 | 232.951 | -58.347 | 99.475 | 1.00 | 67.46 | A |
| ATOM C | 2114 | CB | LYS | A | 389 | 234.459 | -58.686 | 99.667 | 1.00 | 49.89 | A |
| ATOM C | 2115 | C | LYS | A | 389 | 232.216 | -59.459 | 98.688 | 1.00 | 68.81 | A |
| ATOM O | 2116 | O | LYS | A | 389 | 231.441 | -60.236 | 99.337 | 1.00 | 76.61 | A |
| ATOM O | 2117 | OXT | LYS | A | 389 | 232.453 | -59.564 | 97.435 | 1.00 | 76.72 | A |
| ATOM P | 2118 | PB | ADP | S | 531 | 257.416 | -68.553 | 107.649 | 1.00 | 34.84 | S |
| ATOM O | 2119 | O1B | ADP | S | 531 | 258.545 | -67.776 | 107.191 | 1.00 | 50.81 | S |
| ATOM O | 2120 | O2B | ADP | S | 531 | 257.209 | -69.880 | 106.879 | 1.00 | 48.35 | S |
| ATOM O | 2121 | O3B | ADP | S | 531 | 257.422 | -68.756 | 109.226 | 1.00 | 53.79 | S |
| ATOM P | 2122 | PA | ADP | S | 531 | 256.077 | -66.204 | 106.616 | 1.00 | 35.25 | S |
| ATOM O | 2123 | O1A | ADP | S | 531 | 256.842 | -66.123 | 105.373 | 1.00 | 33.66 | S |
| ATOM O | 2124 | O2A | ADP | S | 531 | 254.551 | -65.860 | 106.461 | 1.00 | 54.13 | S |
| ATOM O | 2125 | O3A | ADP | S | 531 | 256.162 | -67.643 | 107.261 | 1.00 | 62.78 | S |
| ATOM O | 2126 | O5* | ADP | S | 531 | 256.892 | -65.243 | 107.657 | 1.00 | 48.32 | S |
| ATOM C | 2127 | C5* | ADP | S | 531 | 256.442 | -65.218 | 109.085 | 1.00 | 61.70 | S |
| ATOM C | 2128 | C4* | ADP | S | 531 | 255.856 | -63.898 | 109.556 | 1.00 | 46.54 | S |
| ATOM O | 2129 | O4* | ADP | S | 531 | 256.542 | -62.868 | 108.818 | 1.00 | 44.98 | S |
| ATOM C | 2130 | C3* | ADP | S | 531 | 254.372 | -63.620 | 109.292 | 1.00 | 37.55 | S |
| ATOM O | 2131 | O3* | ADP | S | 531 | 253.658 | -64.161 | 110.347 | 1.00 | 46.23 | S |
| ATOM C | 2132 | C2* | ADP | S | 531 | 254.337 | -62.080 | 109.181 | 1.00 | 46.36 | S |
| ATOM O | 2133 | O2* | ADP | S | 531 | 254.148 | -61.399 | 110.423 | 1.00 | 42.59 | S |
| ATOM C | 2134 | C1* | ADP | S | 531 | 255.710 | -61.716 | 108.597 | 1.00 | 43.16 | S |
| ATOM N | 2135 | N9 | ADP | S | 531 | 255.666 | -61.436 | 107.162 | 1.00 | 48.23 | S |
| ATOM C | 2136 | C8 | ADP | S | 531 | 255.946 | -62.302 | 106.136 | 1.00 | 47.43 | S |
| ATOM N | 2137 | N7 | ADP | S | 531 | 255.811 | -61.734 | 104.897 | 1.00 | 42.83 | S |
| ATOM C | 2138 | C5 | ADP | S | 531 | 255.418 | -60.464 | 105.177 | 1.00 | 40.36 | S |
| ATOM C | 2139 | C6 | ADP | S | 531 | 255.122 | -59.337 | 104.279 | 1.00 | 44.75 | S |
| ATOM N | 2140 | N6 | ADP | S | 531 | 255.151 | -59.400 | 102.949 | 1.00 | 22.67 | S |
| ATOM N | 2141 | N1 | ADP | S | 531 | 254.762 | -58.153 | 104.964 | 1.00 | 40.38 | S |
| ATOM C | 2142 | C2 | ADP | S | 531 | 254.725 | -58.057 | 106.364 | 1.00 | 50.55 | S |
| ATOM N | 2143 | N3 | ADP | S | 531 | 254.992 | -59.070 | 107.188 | 1.00 | 51.07 | S |
| ATOM C | 2144 | C4 | ADP | S | 531 | 255.351 | -60.245 | 106.574 | 1.00 | 46.49 | S |
| ATOM MG | 2145 | MG | MG2 | X | 1 | 254.502 | -68.175 | 108.413 | 1.00 | 47.20 | X |
| ATOM MG | 2146 | MG | MG2 | X | 2 | 255.864 | -71.389 | 106.282 | 1.00 | 52.14 | X |
| ATOM O | 2147 | OH2 | WAT | W | 1 | 264.531 | -71.881 | 94.078 | 1.00 | 38.88 | W |
| ATOM O | 2148 | OH2 | WAT | W | 2 | 242.403 | -78.272 | 113.237 | 1.00 | 54.89 | W |
| ATOM O | 2149 | OH2 | WAT | W | 3 | 232.705 | -62.634 | 117.460 | 1.00 | 37.08 | W |
| ATOM O | 2150 | OH2 | WAT | W | 4 | 251.977 | -73.020 | 102.685 | 1.00 | 62.00 | W |
| ATOM O | 2151 | OH2 | WAT | W | 5 | 275.163 | -72.604 | 97.774 | 1.00 | 53.95 | W |
| ATOM O | 2152 | OH2 | WAT | W | 6 | 232.526 | -85.909 | 111.573 | 1.00 | 35.05 | W |
| ATOM O | 2153 | OH2 | WAT | W | 7 | 259.170 | -71.102 | 103.608 | 1.00 | 40.42 | W |
| ATOM O | 2154 | OH2 | WAT | W | 8 | 249.904 | -55.205 | 99.315 | 1.00 | 26.87 | W |
| ATOM O | 2155 | OH2 | WAT | W | 9 | 229.701 | -63.236 | 117.265 | 1.00 | 25.50 | W |

**Table B**

| | | | | | | | | | | | |
|---|---|---|---|---|---|---|---|---|---|---|---|
| ATOM C | 1 | CB | SER | A | 123 | 174.078 | 193.853 | 20.627 | 1.00 | 33.78 | A |
| ATOM O | 2 | OG | SER | A | 123 | 173.358 | 193.080 | 21.584 | 1.00 | 34.86 | A |
| ATOM C | 3 | C | SER | A | 123 | 173.331 | 195.751 | 21.954 | 1.00 | 32.28 | A |
| ATOM O | 4 | O | SER | A | 123 | 174.318 | 196.187 | 22.580 | 1.00 | 32.38 | A |
| ATOM N | 5 | N | SER | A | 123 | 174.192 | 196.109 | 19.556 | 1.00 | 32.09 | A |
| ATOM C | 6 | CA | SER | A | 123 | 173.449 | 195.246 | 20.532 | 1.00 | 33.80 | A |
| ATOM N | 7 | N | LYS | A | 124 | 172.107 | 195.682 | 22.463 | 1.00 | 30.56 | A |
| ATOM C | 8 | CA | LYS | A | 124 | 171.860 | 196.059 | 23.849 | 1.00 | 31.07 | A |
| ATOM C | 9 | CB | LYS | A | 124 | 170.483 | 196.727 | 24.005 | 1.00 | 31.64 | A |
| ATOM C | 10 | CG | LYS | A | 124 | 170.231 | 197.847 | 23.021 | 1.00 | 33.77 | A |
| ATOM C | 11 | CD | LYS | A | 124 | 168.734 | 198.157 | 22.847 | 1.00 | 35.14 | A |
| ATOM C | 12 | CE | LYS | A | 124 | 168.571 | 198.989 | 21.582 | 1.00 | 38.52 | A |
| ATOM N | 13 | NZ | LYS | A | 124 | 167.170 | 199.182 | 21.123 | 1.00 | 40.74 | A |
| ATOM C | 14 | C | LYS | A | 124 | 171.929 | 194.785 | 24.714 | 1.00 | 30.29 | A |
| ATOM O | 15 | O | LYS | A | 124 | 171.724 | 194.845 | 25.913 | 1.00 | 30.99 | A |
| ATOM N | 16 | N | LYS | A | 125 | 172.256 | 193.646 | 24.102 | 1.00 | 30.71 | A |
| ATOM C | 17 | CA | LYS | A | 125 | 172.352 | 192.375 | 24.835 | 1.00 | 29.50 | A |
| ATOM C | 18 | CB | LYS | A | 125 | 171.942 | 191.210 | 23.951 | 1.00 | 32.42 | A |
| ATOM C | 19 | CG | LYS | A | 125 | 170.496 | 191.147 | 23.609 | 1.00 | 35.74 | A |
| ATOM C | 20 | CD | LYS | A | 125 | 170.221 | 189.915 | 22.777 | 1.00 | 38.96 | A |
| ATOM C | 21 | CE | LYS | A | 125 | 168.757 | 189.905 | 22.351 | 1.00 | 44.54 | A |
| ATOM N | 22 | NZ | LYS | A | 125 | 168.305 | 188.576 | 21.807 | 1.00 | 44.21 | A |
| ATOM C | 23 | C | LYS | A | 125 | 173.728 | 192.027 | 25.381 | 1.00 | 26.48 | A |
| ATOM O | 24 | O | LYS | A | 125 | 174.769 | 192.438 | 24.859 | 1.00 | 24.13 | A |
| ATOM N | 25 | N | ARG | A | 126 | 173.713 | 191.215 | 26.421 | 1.00 | 24.15 | A |
| ATOM C | 26 | CA | ARG | A | 126 | 174.952 | 190.756 | 27.020 | 1.00 | 22.33 | A |
| ATOM C | 27 | CB | ARG | A | 126 | 174.636 | 189.835 | 28.191 | 1.00 | 21.13 | A |
| ATOM C | 28 | CG | ARG | A | 126 | 175.841 | 189.331 | 28.897 | 1.00 | 18.73 | A |
| ATOM C | 29 | CD | ARG | A | 126 | 175.395 | 188.263 | 29.847 | 1.00 | 19.74 | A |
| ATOM N | 30 | NE | ARG | A | 126 | 176.552 | 187.628 | 30.420 | 1.00 | 16.73 | A |
| ATOM C | 31 | CZ | ARG | A | 126 | 176.503 | 186.741 | 31.394 | 1.00 | 17.59 | A |
| ATOM N | 32 | NH1 | ARG | A | 126 | 175.331 | 186.391 | 31.902 | 1.00 | 16.71 | A |
| ATOM N | 33 | NH2 | ARG | A | 126 | 177.633 | 186.214 | 31.854 | 1.00 | 18.45 | A |
| ATOM C | 34 | C | ARG | A | 126 | 175.752 | 189.995 | 25.943 | 1.00 | 22.44 | A |
| ATOM O | 35 | O | ARG | A | 126 | 175.252 | 189.077 | 25.296 | 1.00 | 20.29 | A |
| ATOM N | 36 | N | GLN | A | 127 | 176.997 | 190.406 | 25.763 | 1.00 | 21.10 | A |
| ATOM C | 37 | CA | GLN | A | 127 | 177.904 | 189.815 | 24.787 | 1.00 | 19.12 | A |
| ATOM C | 38 | CB | GLN | A | 127 | 178.707 | 190.934 | 24.102 | 1.00 | 18.99 | A |
| ATOM C | 39 | CG | GLN | A | 127 | 177.842 | 191.925 | 23.355 | 1.00 | 15.64 | A |
| ATOM C | 40 | CD | GLN | A | 127 | 177.154 | 191.297 | 22.162 | 1.00 | 17.04 | A |
| ATOM O | 41 | OE1 | GLN | A | 127 | 177.748 | 191.151 | 21.093 | 1.00 | 21.45 | A |
| ATOM N | 42 | NE2 | GLN | A | 127 | 175.902 | 190.912 | 22.339 | 1.00 | 20.66 | A |
| ATOM C | 43 | C | GLN | A | 127 | 178.861 | 188.861 | 25.496 | 1.00 | 17.70 | A |
| ATOM O | 44 | O | GLN | A | 127 | 179.049 | 188.941 | 26.715 | 1.00 | 13.46 | A |
| ATOM N | 45 | N | TRP | A | 128 | 179.460 | 187.956 | 24.735 | 1.00 | 16.62 | A |
| ATOM C | 46 | CA | TRP | A | 128 | 180.411 | 187.014 | 25.303 | 1.00 | 14.25 | A |
| ATOM C | 47 | CB | TRP | A | 128 | 180.890 | 186.029 | 24.237 | 1.00 | 13.22 | A |
| ATOM C | 48 | CG | TRP | A | 128 | 179.858 | 185.060 | 23.779 | 1.00 | 9.34 | A |
| ATOM C | 49 | CD2 | TRP | A | 128 | 179.264 | 184.022 | 24.557 | 1.00 | 13.44 | A |
| ATOM C | 50 | CE2 | TRP | A | 128 | 178.396 | 183.304 | 23.704 | 1.00 | 11.84 | A |
| ATOM C | 51 | CE3 | TRP | A | 128 | 179.389 | 183.618 | 25.899 | 1.00 | 15.05 | A |
| ATOM C | 52 | CD1 | TRP | A | 128 | 179.338 | 184.945 | 22.529 | 1.00 | 11.79 | A |
| ATOM N | 53 | NE1 | TRP | A | 128 | 178.460 | 183.893 | 22.469 | 1.00 | 12.24 | A |
| ATOM C | 54 | CZ2 | TRP | A | 128 | 177.648 | 182.204 | 24.146 | 1.00 | 10.99 | A |
| ATOM C | 55 | CZ3 | TRP | A | 128 | 178.646 | 182.523 | 26.336 | 1.00 | 15.81 | A |
| ATOM C | 56 | CH2 | TRP | A | 128 | 177.789 | 181.828 | 25.457 | 1.00 | 13.35 | A |
| ATOM C | 57 | C | TRP | A | 128 | 181.611 | 187.777 | 25.855 | 1.00 | 16.17 | A |
| ATOM O | 58 | O | TRP | A | 128 | 181.885 | 188.903 | 25.457 | 1.00 | 17.48 | A |
| ATOM N | 59 | N | ALA | A | 129 | 182.306 | 187.146 | 26.790 | 1.00 | 17.52 | A |
| ATOM C | 60 | CA | ALA | A | 129 | 183.501 | 187.695 | 27.415 | 1.00 | 17.26 | A |
| ATOM C | 61 | CB | ALA | A | 129 | 183.129 | 188.595 | 28.576 | 1.00 | 8.15 | A |
| ATOM C | 62 | C | ALA | A | 129 | 184.303 | 186.478 | 27.906 | 1.00 | 17.06 | A |
| ATOM O | 63 | O | ALA | A | 129 | 183.733 | 185.426 | 28.198 | 1.00 | 17.77 | A |
| ATOM N | 64 | N | LEU | A | 130 | 185.618 | 186.620 | 27.979 | 1.00 | 16.79 | A |
| ATOM C | 65 | CA | LEU | A | 130 | 186.479 | 185.540 | 28.425 | 1.00 | 19.53 | A |
| ATOM C | 66 | CB | LEU | A | 130 | 187.943 | 186.006 | 28.392 | 1.00 | 19.86 | A |
| ATOM C | 67 | CG | LEU | A | 130 | 189.050 | 184.967 | 28.619 | 1.00 | 22.85 | A |
| ATOM C | 68 | CD1 | LEU | A | 130 | 188.897 | 183.839 | 27.604 | 1.00 | 17.77 | A |
| ATOM C | 69 | CD2 | LEU | A | 130 | 190.429 | 185.631 | 28.492 | 1.00 | 16.04 | A |
| ATOM C | 70 | C | LEU | A | 130 | 186.090 | 185.081 | 29.834 | 1.00 | 21.19 | A |
| ATOM O | 71 | O | LEU | A | 130 | 186.080 | 183.885 | 30.121 | 1.00 | 24.25 | A |
| ATOM N | 72 | N | GLU | A | 131 | 185.756 | 186.032 | 30.705 | 1.00 | 23.78 | A |
| ATOM C | 73 | CA | GLU | A | 131 | 185.349 | 185.729 | 32.082 | 1.00 | 25.83 | A |
| ATOM C | 74 | CB | GLU | A | 131 | 184.952 | 187.016 | 32.834 | 1.00 | 30.42 | A |
| ATOM C | 75 | CG | GLU | A | 131 | 186.005 | 188.110 | 32.945 | 1.00 | 42.06 | A |
| ATOM C | 76 | CD | GLU | A | 131 | 186.135 | 188.977 | 31.686 | 1.00 | 46.89 | A |
| ATOM O | 77 | OE1 | GLU | A | 131 | 185.319 | 188.822 | 30.747 | 1.00 | 51.89 | A |
| ATOM O | 78 | OE2 | GLU | A | 131 | 187.058 | 189.821 | 31.640 | 1.00 | 50.51 | A |
| ATOM C | 79 | C | GLU | A | 131 | 184.146 | 184.751 | 32.145 | 1.00 | 25.30 | A |
| ATOM O | 80 | O | GLU | A | 131 | 183.790 | 184.281 | 33.227 | 1.00 | 21.67 | A |
| ATOM N | 81 | N | ASP | A | 132 | 183.503 | 184.473 | 31.008 | 1.00 | 22.51 | A |
| ATOM C | 82 | CA | ASP | A | 132 | 182.364 | 183.565 | 30.989 | 1.00 | 22.54 | A |
| ATOM C | 83 | CB | ASP | A | 132 | 181.458 | 183.825 | 29.782 | 1.00 | 24.79 | A |
| ATOM C | 84 | CG | ASP | A | 132 | 180.631 | 185.120 | 29.908 | 1.00 | 28.99 | A |
| ATOM O | 85 | OD1 | ASP | A | 132 | 180.136 | 185.417 | 31.025 | 1.00 | 29.67 | A |
| ATOM O | 86 | OD2 | ASP | A | 132 | 180.450 | 185.824 | 28.873 | 1.00 | 26.45 | A |
| ATOM C | 87 | C | ASP | A | 132 | 182.752 | 182.087 | 30.966 | 1.00 | 21.99 | A |
| ATOM O | 88 | O | ASP | A | 132 | 181.925 | 181.226 | 31.267 | 1.00 | 22.12 | A |
| ATOM N | 89 | N | PHE | A | 133 | 183.998 | 181.781 | 30.625 | 1.00 | 23.02 | A |
| ATOM C | 90 | CA | PHE | A | 133 | 184.411 | 180.378 | 30.554 | 1.00 | 22.89 | A |
| ATOM C | 91 | CB | PHE | A | 133 | 184.812 | 179.998 | 29.111 | 1.00 | 18.64 | A |
| ATOM C | 92 | CG | PHE | A | 133 | 183.901 | 180.565 | 28.039 | 1.00 | 17.41 | A |
| ATOM C | 93 | CD1 | PHE | A | 133 | 184.042 | 181.884 | 27.616 | 1.00 | 15.80 | A |
| ATOM C | 94 | CD2 | PHE | A | 133 | 182.904 | 179.783 | 27.456 | 1.00 | 16.46 | A |
| ATOM C | 95 | CE1 | PHE | A | 133 | 183.215 | 182.414 | 26.637 | 1.00 | 14.66 | A |
| ATOM C | 96 | CE2 | PHE | A | 133 | 182.063 | 180.305 | 26.466 | 1.00 | 15.10 | A |
| ATOM C | 97 | CZ | PHE | A | 133 | 182.224 | 181.626 | 26.060 | 1.00 | 18.42 | A |
| ATOM C | 98 | C | PHE | A | 133 | 185.557 | 179.984 | 31.484 | 1.00 | 22.91 | A |
| ATOM O | 99 | O | PHE | A | 133 | 186.466 | 180.766 | 31.755 | 1.00 | 24.66 | A |
| ATOM N | 100 | N | GLU | A | 134 | 185.484 | 178.767 | 32.005 | 1.00 | 22.49 | A |
| ATOM C | 101 | CA | GLU | A | 134 | 186.566 | 178.248 | 32.814 | 1.00 | 21.74 | A |
| ATOM C | 102 | CB | GLU | A | 134 | 186.054 | 177.264 | 33.870 | 1.00 | 23.93 | A |
| ATOM C | 103 | CG | GLU | A | 134 | 185.401 | 177.912 | 35.088 | 1.00 | 29.62 | A |
| ATOM C | 104 | CD | GLU | A | 134 | 184.751 | 176.887 | 36.020 | 1.00 | 37.53 | A |
| ATOM O | 105 | OE1 | GLU | A | 134 | 185.460 | 175.983 | 36.533 | 1.00 | 38.84 | A |
| ATOM O | 106 | OE2 | GLU | A | 134 | 183.517 | 176.974 | 36.236 | 1.00 | 43.58 | A |
| ATOM C | 107 | C | GLU | A | 134 | 187.313 | 177.528 | 31.695 | 1.00 | 21.49 | A |
| ATOM O | 108 | O | GLU | A | 134 | 186.702 | 176.784 | 30.922 | 1.00 | 23.43 | A |
| ATOM N | 109 | N | ILE | A | 135 | 188.608 | 177.794 | 31.564 | 1.00 | 19.74 | A |
| ATOM C | 110 | CA | ILE | A | 135 | 189.425 | 177.197 | 30.524 | 1.00 | 19.08 | A |
| ATOM C | 111 | CB | ILE | A | 135 | 190.554 | 178.149 | 30.072 | 1.00 | 22.58 | A |
| ATOM C | 112 | CG2 | ILE | A | 135 | 191.285 | 177.543 | 28.866 | 1.00 | 19.44 | A |
| ATOM C | 113 | CG1 | ILE | A | 135 | 189.990 | 179.541 | 29.762 | 1.00 | 20.52 | A |
| ATOM C | 114 | CD1 | ILE | A | 135 | 188.876 | 179.535 | 28.771 | 1.00 | 21.85 | A |
| ATOM C | 115 | C | ILE | A | 135 | 190.099 | 175.941 | 31.044 | 1.00 | 20.74 | A |
| ATOM O | 116 | O | ILE | A | 135 | 190.667 | 175.944 | 32.140 | 1.00 | 19.34 | A |
| ATOM N | 117 | N | GLY | A | 136 | 190.058 | 174.876 | 30.245 | 1.00 | 19.98 | A |
| ATOM C | 118 | CA | GLY | A | 136 | 190.688 | 173.629 | 30.637 | 1.00 | 19.38 | A |
| ATOM C | 119 | C | GLY | A | 136 | 191.995 | 173.397 | 29.906 | 1.00 | 18.61 | A |
| ATOM O | 120 | O | GLY | A | 136 | 192.742 | 174.324 | 29.668 | 1.00 | 20.61 | A |
| ATOM N | 121 | N | ARG | A | 137 | 192.267 | 172.156 | 29.533 | 1.00 | 22.50 | A |
| ATOM C | 122 | CA | ARG | A | 137 | 193.500 | 171.827 | 28.833 | 1.00 | 22.75 | A |
| ATOM C | 123 | CB | ARG | A | 137 | 193.771 | 170.325 | 28.920 | 1.00 | 22.49 | A |
| ATOM C | 124 | CG | ARG | A | 137 | 192.820 | 169.474 | 28.047 | 1.00 | 20.82 | A |
| ATOM C | 125 | CD | ARG | A | 137 | 193.107 | 168.016 | 28.252 | 1.00 | 15.70 | A |
| ATOM N | 126 | NE | ARG | A | 137 | 192.212 | 167.104 | 27.554 | 1.00 | 14.70 | A |
| ATOM C | 127 | CZ | ARG | A | 137 | 192.299 | 166.784 | 26.266 | 1.00 | 14.90 | A |
| ATOM N | 128 | NH1 | ARG | A | 137 | 193.237 | 167.305 | 25.493 | 1.00 | 13.75 | A |
| ATOM N | 129 | NH2 | ARG | A | 137 | 191.462 | 165.900 | 25.758 | 1.00 | 13.41 | A |
| ATOM C | 130 | C | ARG | A | 137 | 193.396 | 172.183 | 27.357 | 1.00 | 23.53 | A |
| ATOM O | 131 | O | ARG | A | 137 | 192.316 | 172.425 | 26.840 | 1.00 | 24.10 | A |
| ATOM N | 132 | N | PRO | A | 138 | 194.542 | 172.250 | 26.671 | 1.00 | 22.98 | A |
| ATOM C | 133 | CD | PRO | A | 138 | 195.850 | 172.477 | 27.313 | 1.00 | 19.97 | A |
| ATOM C | 134 | CA | PRO | A | 138 | 194.613 | 172.552 | 25.237 | 1.00 | 22.67 | A |
| ATOM C | 135 | CB | PRO | A | 138 | 196.116 | 172.791 | 25.007 | 1.00 | 21.39 | A |
| ATOM C | 136 | CG | PRO | A | 138 | 196.570 | 173.354 | 26.295 | 1.00 | 19.67 | A |
| ATOM C | 137 | C | PRO | A | 138 | 194.135 | 171.275 | 24.497 | 1.00 | 23.48 | A |
| ATOM O | 138 | O | PRO | A | 138 | 194.528 | 170.147 | 24.876 | 1.00 | 21.67 | A |
| ATOM N | 139 | N | LEU | A | 139 | 193.297 | 171.435 | 23.471 | 1.00 | 19.70 | A |
| ATOM C | 140 | CA | LEU | A | 139 | 192.809 | 170.281 | 22.716 | 1.00 | 19.33 | A |
| ATOM C | 141 | CB | LEU | A | 139 | 191.340 | 170.454 | 22.318 | 1.00 | 19.32 | A |
| ATOM C | 142 | CG | LEU | A | 139 | 190.349 | 170.509 | 23.473 | 1.00 | 18.46 | A |
| ATOM C | 143 | CD1 | LEU | A | 139 | 188.966 | 170.866 | 22.964 | 1.00 | 16.26 | A |
| ATOM C | 144 | CD2 | LEU | A | 139 | 190.342 | 169.178 | 24.163 | 1.00 | 15.61 | A |
| ATOM C | 145 | C | LEU | A | 139 | 193.636 | 170.125 | 21.454 | 1.00 | 18.97 | A |
| ATOM O | 146 | O | LEU | A | 139 | 193.714 | 169.045 | 20.883 | 1.00 | 20.68 | A |
| ATOM N | 147 | N | GLY | A | 140 | 194.256 | 171.215 | 21.026 | 1.00 | 19.57 | A |
| ATOM C | 148 | CA | GLY | A | 140 | 195.056 | 171.181 | 19.825 | 1.00 | 16.45 | A |
| ATOM C | 149 | C | GLY | A | 140 | 195.821 | 172.461 | 19.630 | 1.00 | 18.41 | A |
| ATOM O | 150 | O | GLY | A | 140 | 195.560 | 173.469 | 20.280 | 1.00 | 17.66 | A |
| ATOM N | 151 | N | LYS | A | 141 | 196.778 | 172.413 | 18.713 | 1.00 | 23.54 | A |
| ATOM C | 152 | CA | LYS | A | 141 | 197.635 | 173.547 | 18.399 | 1.00 | 25.64 | A |
| ATOM C | 153 | CB | LYS | A | 141 | 199.103 | 173.104 | 18.473 | 1.00 | 29.50 | A |
| ATOM C | 154 | CG | LYS | A | 141 | 200.101 | 174.192 | 18.080 | 1.00 | 37.58 | A |
| ATOM C | 155 | CD | LYS | A | 141 | 201.550 | 173.744 | 18.187 | 1.00 | 42.29 | A |
| ATOM C | 156 | CE | LYS | A | 141 | 202.483 | 174.918 | 17.891 | 1.00 | 46.12 | A |
| ATOM N | 157 | NZ | LYS | A | 141 | 203.914 | 174.481 | 17.890 | 1.00 | 49.84 | A |
| ATOM C | 158 | C | LYS | A | 141 | 197.313 | 174.071 | 16.995 | 1.00 | 26.94 | A |
| ATOM O | 159 | O | LYS | A | 141 | 197.578 | 173.409 | 15.986 | 1.00 | 26.47 | A |
| ATOM N | 160 | N | GLY | A | 142 | 196.724 | 175.256 | 16.938 | 1.00 | 26.51 | A |
| ATOM C | 161 | CA | GLY | A | 142 | 196.392 | 175.839 | 15.656 | 1.00 | 26.65 | A |
| ATOM C | 162 | C | GLY | A | 142 | 197.515 | 176.753 | 15.234 | 1.00 | 26.71 | A |
| ATOM O | 163 | O | GLY | A | 142 | 198.384 | 177.086 | 16.052 | 1.00 | 26.93 | A |
| ATOM N | 164 | N | LYS | A | 143 | 197.501 | 177.158 | 13.969 | 1.00 | 24.67 | A |
| ATOM C | 165 | CA | LYS | A | 143 | 198.529 | 178.037 | 13.452 | 1.00 | 22.25 | A |
| ATOM C | 166 | CB | LYS | A | 143 | 198.362 | 178.220 | 11.925 | 1.00 | 25.89 | A |
| ATOM C | 167 | CG | LYS | A | 143 | 199.535 | 178.982 | 11.307 | 1.00 | 30.09 | A |
| ATOM C | 168 | CD | LYS | A | 143 | 199.516 | 179.060 | 9.792 | 1.00 | 35.88 | A |
| ATOM C | 169 | CE | LYS | A | 143 | 200.619 | 180.037 | 9.330 | 1.00 | 41.66 | A |
| ATOM N | 170 | NZ | LYS | A | 143 | 200.871 | 180.206 | 7.857 | 1.00 | 42.97 | A |
| ATOM C | 171 | C | LYS | A | 143 | 198.554 | 179.404 | 14.160 | 1.00 | 22.22 | A |
| ATOM O | 172 | O | LYS | A | 143 | 199.641 | 179.921 | 14.425 | 1.00 | 22.44 | A |
| ATOM N | 173 | N | PHE | A | 144 | 197.394 | 179.986 | 14.477 | 1.00 | 20.35 | A |
| ATOM C | 174 | CA | PHE | A | 144 | 197.370 | 181.311 | 15.130 | 1.00 | 21.42 | A |
| ATOM C | 175 | CB | PHE | A | 144 | 196.429 | 182.258 | 14.362 | 1.00 | 17.68 | A |
| ATOM C | 176 | CG | PHE | A | 144 | 196.798 | 182.429 | 12.896 | 1.00 | 22.16 | A |
| ATOM C | 177 | CD1 | PHE | A | 144 | 196.541 | 181.421 | 11.959 | 1.00 | 20.33 | A |
| ATOM C | 178 | CD2 | PHE | A | 144 | 197.460 | 183.574 | 12.464 | 1.00 | 23.47 | A |
| ATOM C | 179 | CE1 | PHE | A | 144 | 196.944 | 181.564 | 10.624 | 1.00 | 20.91 | A |
| ATOM C | 180 | CE2 | PHE | A | 144 | 197.864 | 183.716 | 11.128 | 1.00 | 21.10 | A |
| ATOM C | 181 | CZ | PHE | A | 144 | 197.606 | 182.708 | 10.213 | 1.00 | 18.55 | A |
| ATOM C | 182 | C | PHE | A | 144 | 197.001 | 181.263 | 16.626 | 1.00 | 20.47 | A |
| ATOM O | 183 | O | PHE | A | 144 | 196.668 | 182.283 | 17.246 | 1.00 | 20.98 | A |
| ATOM N | 184 | N | GLY | A | 145 | 197.094 | 180.072 | 17.208 | 1.00 | 18.58 | A |
| ATOM C | 185 | CA | GLY | A | 145 | 196.786 | 179.915 | 18.616 | 1.00 | 19.23 | A |
| ATOM C | 186 | C | GLY | A | 145 | 196.229 | 178.538 | 18.922 | 1.00 | 20.99 | A |
| ATOM O | 187 | O | GLY | A | 145 | 195.911 | 177.756 | 18.016 | 1.00 | 21.28 | A |
| ATOM N | 188 | N | ASN | A | 146 | 196.100 | 178.233 | 20.204 | 1.00 | 20.66 | A |
| ATOM C | 189 | CA | ASN | A | 146 | 195.574 | 176.938 | 20.598 | 1.00 | 21.26 | A |
| ATOM C | 190 | CB | ASN | A | 146 | 196.140 | 176.544 | 21.964 | 1.00 | 24.59 | A |
| ATOM C | 191 | CG | ASN | A | 146 | 197.638 | 176.352 | 21.939 | 1.00 | 27.16 | A |
| ATOM O | 192 | OD1 | ASN | A | 146 | 198.205 | 175.853 | 20.956 | 1.00 | 32.36 | A |
| ATOM N | 193 | ND2 | ASN | A | 146 | 198.292 | 176.737 | 23.015 | 1.00 | 27.51 | A |
| ATOM C | 194 | C | ASN | A | 146 | 194.056 | 176.878 | 20.683 | 1.00 | 20.53 | A |
| ATOM O | 195 | O | ASN | A | 146 | 193.370 | 177.902 | 20.676 | 1.00 | 20.90 | A |
| ATOM N | 196 | N | VAL | A | 147 | 193.540 | 175.658 | 20.740 | 1.00 | 18.03 | A |
| ATOM C | 197 | CA | VAL | A | 147 | 192.121 | 175.430 | 20.922 | 1.00 | 14.64 | A |
| ATOM C | 198 | CB | VAL | A | 147 | 191.528 | 174.515 | 19.799 | 1.00 | 13.96 | A |
| ATOM C | 199 | CG1 | VAL | A | 147 | 190.053 | 174.217 | 20.092 | 1.00 | 10.05 | A |
| ATOM C | 200 | CG2 | VAL | A | 147 | 191.669 | 175.213 | 18.439 | 1.00 | 8.41 | A |
| ATOM C | 201 | C | VAL | A | 147 | 192.142 | 174.746 | 22.301 | 1.00 | 16.21 | A |
| ATOM O | 202 | O | VAL | A | 147 | 192.897 | 173.783 | 22.523 | 1.00 | 14.13 | A |
| ATOM N | 203 | N | TYR | A | 148 | 191.375 | 175.299 | 23.242 | 1.00 | 14.89 | A |
| ATOM C | 204 | CA | TYR | A | 148 | 191.315 | 174.786 | 24.614 | 1.00 | 14.47 | A |
| ATOM C | 205 | CB | TYR | A | 148 | 191.593 | 175.891 | 25.642 | 1.00 | 12.68 | A |
| ATOM C | 206 | CG | TYR | A | 148 | 192.910 | 176.619 | 25.491 | 1.00 | 18.66 | A |
| ATOM C | 207 | CD1 | TYR | A | 148 | 193.031 | 177.690 | 24.605 | 1.00 | 16.46 | A |
| ATOM C | 208 | CE1 | TYR | A | 148 | 194.243 | 178.347 | 24.434 | 1.00 | 18.77 | A |
| ATOM C | 209 | CD2 | TYR | A | 148 | 194.047 | 176.222 | 26.217 | 1.00 | 15.67 | A |
| ATOM C | 210 | CE2 | TYR | A | 148 | 195.269 | 176.880 | 26.050 | 1.00 | 20.14 | A |
| ATOM C | 211 | CZ | TYR | A | 148 | 195.355 | 177.938 | 25.153 | 1.00 | 18.86 | A |
| ATOM O | 212 | OH | TYR | A | 148 | 196.555 | 178.569 | 24.934 | 1.00 | 23.63 | A |
| ATOM C | 213 | C | TYR | A | 148 | 189.943 | 174.234 | 24.936 | 1.00 | 13.13 | A |
| ATOM O | 214 | O | TYR | A | 148 | 188.942 | 174.674 | 24.386 | 1.00 | 14.07 | A |
| ATOM N | 215 | N | LEU | A | 149 | 189.887 | 173.265 | 25.831 | 1.00 | 11.62 | A |
| ATOM C | 216 | CA | LEU | A | 149 | 188.600 | 172.753 | 26.223 | 1.00 | 11.86 | A |
| ATOM C | 217 | CB | LEU | A | 149 | 188.787 | 171.466 | 27.003 | 1.00 | 10.50 | A |
| ATOM C | 218 | CG | LEU | A | 149 | 187.547 | 170.675 | 27.388 | 1.00 | 12.61 | A |
| ATOM C | 219 | CD1 | LEU | A | 149 | 186.695 | 170.364 | 26.182 | 1.00 | 11.20 | A |
| ATOM C | 220 | CD2 | LEU | A | 149 | 188.001 | 169.410 | 28.051 | 1.00 | 11.24 | A |
| ATOM C | 221 | C | LEU | A | 149 | 188.071 | 173.911 | 27.101 | 1.00 | 13.55 | A |
| ATOM O | 222 | O | LEU | A | 149 | 188.857 | 174.728 | 27.565 | 1.00 | 12.48 | A |
| ATOM N | 223 | N | ALA | A | 150 | 186.763 | 174.008 | 27.316 | 1.00 | 14.24 | A |
| ATOM C | 224 | CA | ALA | A | 150 | 186.227 | 175.102 | 28.120 | 1.00 | 15.20 | A |
| ATOM C | 225 | CB | ALA | A | 150 | 186.230 | 176.406 | 27.318 | 1.00 | 15.60 | A |
| ATOM C | 226 | C | ALA | A | 150 | 184.817 | 174.800 | 28.612 | 1.00 | 17.25 | A |
| ATOM O | 227 | O | ALA | A | 150 | 184.122 | 173.929 | 28.083 | 1.00 | 18.30 | A |
| ATOM N | 228 | N | ARG | A | 151 | 184.398 | 175.532 | 29.629 | 1.00 | 17.29 | A |
| ATOM C | 229 | CA | ARG | A | 151 | 183.091 | 175.334 | 30.215 | 1.00 | 19.70 | A |
| ATOM C | 230 | CB | ARG | A | 151 | 183.245 | 174.479 | 31.492 | 1.00 | 20.02 | A |
| ATOM C | 231 | CG | ARG | A | 151 | 181.977 | 174.102 | 32.258 | 1.00 | 26.58 | A |
| ATOM C | 232 | CD | ARG | A | 151 | 182.321 | 173.557 | 33.686 | 1.00 | 30.64 | A |
| ATOM N | 233 | NE | ARG | A | 151 | 183.235 | 172.405 | 33.673 | 1.00 | 32.15 | A |
| ATOM C | 234 | CZ | ARG | A | 151 | 182.900 | 171.151 | 33.348 | 1.00 | 33.18 | A |
| ATOM N | 235 | NH1 | ARG | A | 151 | 181.644 | 170.851 | 33.004 | 1.00 | 33.09 | A |
| ATOM N | 236 | NH2 | ARG | A | 151 | 183.831 | 170.190 | 33.362 | 1.00 | 31.48 | A |
| ATOM C | 237 | C | ARG | A | 151 | 182.516 | 176.707 | 30.518 | 1.00 | 20.61 | A |
| ATOM O | 238 | O | ARG | A | 151 | 183.158 | 177.560 | 31.163 | 1.00 | 19.62 | A |
| ATOM N | 239 | N | GLU | A | 152 | 181.315 | 176.939 | 30.006 | 1.00 | 21.84 | A |
| ATOM C | 240 | CA | GLU | A | 152 | 180.633 | 178.196 | 30.264 | 1.00 | 21.85 | A |
| ATOM C | 241 | CB | GLU | A | 152 | 179.401 | 178.298 | 29.377 | 1.00 | 26.06 | A |
| ATOM C | 242 | CG | GLU | A | 152 | 178.766 | 179.690 | 29.289 | 1.00 | 28.31 | A |
| ATOM C | 243 | CD | GLU | A | 152 | 177.996 | 180.078 | 30.543 | 1.00 | 32.60 | A |
| ATOM O | 244 | OE1 | GLU | A | 152 | 178.576 | 180.804 | 31.391 | 1.00 | 33.50 | A |
| ATOM O | 245 | OE2 | GLU | A | 152 | 176.821 | 179.650 | 30.679 | 1.00 | 29.24 | A |
| ATOM C | 246 | C | GLU | A | 152 | 180.252 | 178.091 | 31.742 | 1.00 | 21.10 | A |
| ATOM O | 247 | O | GLU | A | 152 | 179.661 | 177.102 | 32.172 | 1.00 | 19.62 | A |
| ATOM N | 248 | N | LYS | A | 153 | 180.613 | 179.114 | 32.504 | 1.00 | 21.06 | A |
| ATOM C | 249 | CA | LYS | A | 153 | 180.366 | 179.156 | 33.933 | 1.00 | 20.90 | A |
| ATOM C | 250 | CB | LYS | A | 153 | 180.996 | 180.417 | 34.513 | 1.00 | 20.37 | A |
| ATOM C | 251 | CG | LYS | A | 153 | 182.501 | 180.458 | 34.440 | 1.00 | 22.17 | A |
| ATOM C | 252 | CD | LYS | A | 153 | 183.063 | 181.720 | 35.091 | 1.00 | 21.22 | A |
| ATOM C | 253 | CE | LYS | A | 153 | 184.568 | 181.658 | 35.002 | 1.00 | 22.77 | A |
| ATOM N | 254 | NZ | LYS | A | 153 | 185.178 | 182.877 | 35.572 | 1.00 | 24.11 | A |
| ATOM C | 255 | C | LYS | A | 153 | 178.927 | 179.048 | 34.421 | 1.00 | 21.26 | A |
| ATOM O | 256 | O | LYS | A | 153 | 178.649 | 178.362 | 35.390 | 1.00 | 25.58 | A |
| ATOM N | 257 | N | GLN | A | 154 | 177.982 | 179.704 | 33.786 | 1.00 | 23.03 | A |
| ATOM C | 258 | CA | GLN | A | 154 | 176.639 | 179.579 | 34.330 | 1.00 | 23.96 | A |
| ATOM C | 259 | CB | GLN | A | 154 | 175.744 | 180.686 | 33.800 | 1.00 | 23.79 | A |
| ATOM C | 260 | CG | GLN | A | 154 | 176.125 | 182.053 | 34.288 | 1.00 | 24.55 | A |
| ATOM C | 261 | CD | GLN | A | 154 | 175.243 | 183.133 | 33.709 | 1.00 | 24.55 | A |
| ATOM O | 262 | OE1 | GLN | A | 154 | 175.674 | 184.283 | 33.617 | 1.00 | 23.87 | A |
| ATOM N | 263 | NE2 | GLN | A | 154 | 173.998 | 182.779 | 33.309 | 1.00 | 22.50 | A |
| ATOM C | 264 | C | GLN | A | 154 | 175.951 | 178.229 | 34.086 | 1.00 | 24.10 | A |
| ATOM O | 265 | O | GLN | A | 154 | 175.345 | 177.675 | 34.996 | 1.00 | 25.34 | A |
| ATOM N | 266 | N | SER | A | 155 | 176.018 | 177.700 | 32.871 | 1.00 | 22.36 | A |
| ATOM C | 267 | CA | SER | A | 155 | 175.351 | 176.441 | 32.585 | 1.00 | 21.24 | A |
| ATOM C | 268 | CB | SER | A | 155 | 174.790 | 176.517 | 31.180 | 1.00 | 20.38 | A |
| ATOM O | 269 | OG | SER | A | 155 | 175.875 | 176.607 | 30.282 | 1.00 | 22.24 | A |
| ATOM C | 270 | C | SER | A | 155 | 176.234 | 175.182 | 32.691 | 1.00 | 23.66 | A |
| ATOM O | 271 | O | SER | A | 155 | 175.725 | 174.048 | 32.677 | 1.00 | 20.04 | A |
| ATOM N | 272 | N | LYS | A | 156 | 177.549 | 175.406 | 32.789 | 1.00 | 23.61 | A |
| ATOM C | 273 | CA | LYS | A | 156 | 178.572 | 174.358 | 32.849 | 1.00 | 24.47 | A |
| ATOM C | 274 | CB | LYS | A | 156 | 178.350 | 173.467 | 34.065 | 1.00 | 25.24 | A |
| ATOM C | 275 | CG | LYS | A | 156 | 178.523 | 174.254 | 35.368 | 1.00 | 32.64 | A |
| ATOM C | 276 | CD | LYS | A | 156 | 178.309 | 173.358 | 36.552 | 1.00 | 35.73 | A |
| ATOM C | 277 | CE | LYS | A | 156 | 178.506 | 174.079 | 37.870 | 1.00 | 41.50 | A |
| ATOM N | 278 | NZ | LYS | A | 156 | 178.457 | 173.059 | 38.978 | 1.00 | 42.75 | A |
| ATOM C | 279 | C | LYS | A | 156 | 178.634 | 173.543 | 31.548 | 1.00 | 22.03 | A |
| ATOM O | 280 | O | LYS | A | 156 | 179.131 | 172.417 | 31.509 | 1.00 | 22.31 | A |
| ATOM N | 281 | N | PHE | A | 157 | 178.152 | 174.152 | 30.472 | 1.00 | 20.94 | A |
| ATOM C | 282 | CA | PHE | A | 157 | 178.180 | 173.530 | 29.162 | 1.00 | 19.88 | A |
| ATOM C | 283 | CB | PHE | A | 157 | 177.370 | 174.367 | 28.174 | 1.00 | 21.87 | A |
| ATOM C | 284 | CG | PHE | A | 157 | 177.209 | 173.735 | 26.840 | 1.00 | 22.62 | A |
| ATOM C | 285 | CD1 | PHE | A | 157 | 176.501 | 172.546 | 26.704 | 1.00 | 26.84 | A |
| ATOM C | 286 | CD2 | PHE | A | 157 | 177.745 | 174.336 | 25.707 | 1.00 | 24.18 | A |
| ATOM C | 287 | CE1 | PHE | A | 157 | 176.326 | 171.957 | 25.448 | 1.00 | 27.13 | A |
| ATOM C | 288 | CE2 | PHE | A | 157 | 177.576 | 173.763 | 24.446 | 1.00 | 24.84 | A |
| ATOM C | 289 | CZ | PHE | A | 157 | 176.868 | 172.574 | 24.316 | 1.00 | 26.19 | A |
| ATOM C | 290 | C | PHE | A | 157 | 179.645 | 173.447 | 28.708 | 1.00 | 20.86 | A |
| ATOM O | 291 | O | PHE | A | 157 | 180.394 | 174.451 | 28.739 | 1.00 | 16.68 | A |
| ATOM N | 292 | N | ILE | A | 158 | 180.056 | 172.243 | 28.316 | 1.00 | 18.62 | A |
| ATOM C | 293 | CA | ILE | A | 158 | 181.412 | 171.997 | 27.862 | 1.00 | 17.65 | A |
| ATOM C | 294 | CB | ILE | A | 158 | 181.818 | 170.521 | 28.156 | 1.00 | 21.22 | A |
| ATOM C | 295 | CG2 | ILE | A | 158 | 183.120 | 170.183 | 27.472 | 1.00 | 20.54 | A |
| ATOM C | 296 | CG1 | ILE | A | 158 | 181.998 | 170.339 | 29.675 | 1.00 | 25.44 | A |
| ATOM C | 297 | CD1 | ILE | A | 158 | 181.751 | 168.898 | 30.193 | 1.00 | 32.22 | A |
| ATOM C | 298 | C | ILE | A | 158 | 181.502 | 172.306 | 26.363 | 1.00 | 19.32 | A |
| ATOM O | 299 | O | ILE | A | 158 | 180.622 | 171.940 | 25.588 | 1.00 | 21.25 | A |
| ATOM N | 300 | N | LEU | A | 159 | 182.544 | 173.021 | 25.958 | 1.00 | 19.68 | A |
| ATOM C | 301 | CA | LEU | A | 159 | 182.729 | 173.365 | 24.552 | 1.00 | 18.03 | A |
| ATOM C | 302 | CB | LEU | A | 159 | 181.915 | 174.625 | 24.214 | 1.00 | 18.70 | A |
| ATOM C | 303 | CG | LEU | A | 159 | 181.855 | 175.726 | 25.275 | 1.00 | 18.81 | A |
| ATOM C | 304 | CD1 | LEU | A | 159 | 183.137 | 176.522 | 25.197 | 1.00 | 25.89 | A |
| ATOM C | 305 | CD2 | LEU | A | 159 | 180.667 | 176.644 | 25.065 | 1.00 | 15.33 | A |
| ATOM C | 306 | C | LEU | A | 159 | 184.218 | 173.553 | 24.263 | 1.00 | 16.64 | A |
| ATOM O | 307 | O | LEU | A | 159 | 185.054 | 173.211 | 25.099 | 1.00 | 10.64 | A |
| ATOM N | 308 | N | ALA | A | 160 | 184.562 | 174.069 | 23.084 | 1.00 | 14.09 | A |
| ATOM C | 309 | CA | ALA | A | 160 | 185.981 | 174.271 | 22.742 | 1.00 | 14.90 | A |
| ATOM C | 310 | CB | ALA | A | 160 | 186.371 | 173.394 | 21.574 | 1.00 | 11.94 | A |
| ATOM C | 311 | C | ALA | A | 160 | 186.230 | 175.736 | 22.420 | 1.00 | 15.35 | A |
| ATOM O | 312 | O | ALA | A | 160 | 185.419 | 176.377 | 21.777 | 1.00 | 16.22 | A |
| ATOM N | 313 | N | LEU | A | 161 | 187.348 | 176.277 | 22.876 | 1.00 | 17.51 | A |
| ATOM C | 314 | CA | LEU | A | 161 | 187.656 | 177.685 | 22.636 | 1.00 | 17.81 | A |
| ATOM C | 315 | CB | LEU | A | 161 | 187.959 | 178.379 | 23.958 | 1.00 | 19.09 | A |
| ATOM C | 316 | CG | LEU | A | 161 | 187.661 | 179.882 | 24.003 | 1.00 | 24.28 | A |
| ATOM C | 317 | CD1 | LEU | A | 161 | 186.190 | 180.085 | 23.715 | 1.00 | 19.74 | A |
| ATOM C | 318 | CD2 | LEU | A | 161 | 188.020 | 180.482 | 25.396 | 1.00 | 26.64 | A |
| ATOM C | 319 | C | LEU | A | 161 | 188.857 | 177.829 | 21.697 | 1.00 | 18.94 | A |
| ATOM O | 320 | O | LEU | A | 161 | 189.984 | 177.506 | 22.070 | 1.00 | 15.15 | A |
| ATOM N | 321 | N | LYS | A | 162 | 188.605 | 178.302 | 20.477 | 1.00 | 15.44 | A |
| ATOM C | 322 | CA | LYS | A | 162 | 189.671 | 178.489 | 19.506 | 1.00 | 16.27 | A |
| ATOM C | 323 | CB | LYS | A | 162 | 189.142 | 178.254 | 18.093 | 1.00 | 15.06 | A |
| ATOM C | 324 | CG | LYS | A | 162 | 190.184 | 178.338 | 17.036 | 1.00 | 13.12 | A |
| ATOM C | 325 | CD | LYS | A | 162 | 189.615 | 177.968 | 15.682 | 1.00 | 15.23 | A |
| ATOM C | 326 | CE | LYS | A | 162 | 190.694 | 178.025 | 14.590 | 1.00 | 15.94 | A |
| ATOM N | 327 | NZ | LYS | A | 162 | 190.118 | 177.625 | 13.262 | 1.00 | 21.75 | A |
| ATOM C | 328 | C | LYS | A | 162 | 190.220 | 179.909 | 19.626 | 1.00 | 16.04 | A |
| ATOM O | 329 | O | LYS | A | 162 | 189.486 | 180.863 | 19.427 | 1.00 | 17.16 | A |
| ATOM N | 330 | N | VAL | A | 163 | 191.501 | 180.044 | 19.955 | 1.00 | 15.21 | A |
| ATOM C | 331 | CA | VAL | A | 163 | 192.110 | 181.357 | 20.107 | 1.00 | 16.50 | A |
| ATOM C | 332 | CB | VAL | A | 163 | 193.047 | 181.400 | 21.336 | 1.00 | 17.79 | A |
| ATOM C | 333 | CG1 | VAL | A | 163 | 193.563 | 182.816 | 21.546 | 1.00 | 15.20 | A |
| ATOM C | 334 | CG2 | VAL | A | 163 | 192.316 | 180.897 | 22.578 | 1.00 | 14.92 | A |
| ATOM C | 335 | C | VAL | A | 163 | 192.938 | 181.758 | 18.888 | 1.00 | 17.82 | A |
| ATOM O | 336 | O | VAL | A | 163 | 193.616 | 180.933 | 18.287 | 1.00 | 20.09 | A |
| ATOM N | 337 | N | LEU | A | 164 | 192.888 | 183.028 | 18.522 | 1.00 | 18.13 | A |
| ATOM C | 338 | CA | LEU | A | 164 | 193.670 | 183.509 | 17.387 | 1.00 | 15.87 | A |
| ATOM C | 339 | CB | LEU | A | 164 | 192.747 | 183.802 | 16.203 | 1.00 | 18.86 | A |
| ATOM C | 340 | CG | LEU | A | 164 | 191.903 | 182.603 | 15.752 | 1.00 | 20.75 | A |
| ATOM C | 341 | CD1 | LEU | A | 164 | 190.429 | 182.980 | 15.575 | 1.00 | 23.26 | A |
| ATOM C | 342 | CD2 | LEU | A | 164 | 192.481 | 182.084 | 14.477 | 1.00 | 19.09 | A |
| ATOM C | 343 | C | LEU | A | 164 | 194.352 | 184.789 | 17.835 | 1.00 | 16.04 | A |
| ATOM O | 344 | O | LEU | A | 164 | 193.687 | 185.709 | 18.295 | 1.00 | 13.41 | A |
| ATOM N | 345 | N | PHE | A | 165 | 195.670 | 184.858 | 17.738 | 1.00 | 15.52 | A |
| ATOM C | 346 | CA | PHE | A | 165 | 196.340 | 186.082 | 18.141 | 1.00 | 18.33 | A |
| ATOM C | 347 | CB | PHE | A | 165 | 197.804 | 185.823 | 18.497 | 1.00 | 19.71 | A |
| ATOM C | 348 | CG | PHE | A | 165 | 197.987 | 185.214 | 19.850 | 1.00 | 26.23 | A |
| ATOM C | 349 | CD1 | PHE | A | 165 | 197.826 | 183.840 | 20.033 | 1.00 | 27.06 | A |
| ATOM C | 350 | CD2 | PHE | A | 165 | 198.222 | 186.025 | 20.961 | 1.00 | 28.47 | A |
| ATOM C | 351 | CE1 | PHE | A | 165 | 197.899 | 183.281 | 21.298 | 1.00 | 32.07 | A |
| ATOM C | 352 | CE2 | PHE | A | 165 | 198.299 | 185.479 | 22.239 | 1.00 | 31.56 | A |
| ATOM C | 353 | CZ | PHE | A | 165 | 198.133 | 184.104 | 22.411 | 1.00 | 35.02 | A |
| ATOM C | 354 | C | PHE | A | 165 | 196.228 | 187.062 | 16.989 | 1.00 | 19.49 | A |
| ATOM O | 355 | O | PHE | A | 165 | 196.610 | 186.742 | 15.857 | 1.00 | 21.60 | A |
| ATOM N | 356 | N | LYS | A | 166 | 195.674 | 188.242 | 17.277 | 1.00 | 18.17 | A |
| ATOM C | 357 | CA | LYS | A | 166 | 195.458 | 189.299 | 16.282 | 1.00 | 17.63 | A |
| ATOM C | 358 | CB | LYS | A | 166 | 194.786 | 190.520 | 16.931 | 1.00 | 16.68 | A |
| ATOM C | 359 | CG | LYS | A | 166 | 193.319 | 190.346 | 17.376 | 1.00 | 15.54 | A |
| ATOM C | 360 | CD | LYS | A | 166 | 192.703 | 191.672 | 17.826 | 1.00 | 10.39 | A |
| ATOM C | 361 | CE | LYS | A | 166 | 191.259 | 191.452 | 18.272 | 1.00 | 12.43 | A |
| ATOM N | 362 | NZ | LYS | A | 166 | 190.505 | 192.644 | 18.792 | 1.00 | 7.46 | A |
| ATOM C | 363 | C | LYS | A | 166 | 196.696 | 189.792 | 15.536 | 1.00 | 19.44 | A |
| ATOM O | 364 | O | LYS | A | 166 | 196.615 | 190.103 | 14.343 | 1.00 | 19.53 | A |
| ATOM N | 365 | N | ALA | A | 167 | 197.828 | 189.885 | 16.237 | 1.00 | 19.10 | A |
| ATOM C | 366 | CA | ALA | A | 167 | 199.068 | 190.369 | 15.628 | 1.00 | 18.82 | A |
| ATOM C | 367 | CB | ALA | A | 167 | 200.140 | 190.591 | 16.710 | 1.00 | 11.48 | A |
| ATOM C | 368 | C | ALA | A | 167 | 199.551 | 189.377 | 14.571 | 1.00 | 18.71 | A |
| ATOM O | 369 | O | ALA | A | 167 | 200.085 | 189.762 | 13.534 | 1.00 | 19.61 | A |
| ATOM N | 370 | N | GLN | A | 168 | 199.351 | 188.092 | 14.840 | 1.00 | 18.71 | A |
| ATOM C | 371 | CA | GLN | A | 168 | 199.734 | 187.039 | 13.907 | 1.00 | 18.86 | A |
| ATOM C | 372 | CB | GLN | A | 168 | 199.584 | 185.666 | 14.561 | 1.00 | 21.65 | A |
| ATOM C | 373 | CG | GLN | A | 168 | 200.584 | 184.664 | 14.014 | 1.00 | 25.64 | A |
| ATOM C | 374 | CD | GLN | A | 168 | 200.544 | 183.310 | 14.690 | 1.00 | 29.19 | A |
| ATOM O | 375 | OE1 | GLN | A | 168 | 200.269 | 183.208 | 15.900 | 1.00 | 27.17 | A |
| ATOM N | 376 | NE2 | GLN | A | 168 | 200.846 | 182.249 | 13.920 | 1.00 | 28.74 | A |
| ATOM C | 377 | C | GLN | A | 168 | 198.824 | 187.137 | 12.688 | 1.00 | 17.82 | A |
| ATOM O | 378 | O | GLN | A | 168 | 199.273 | 187.079 | 11.560 | 1.00 | 20.56 | A |
| ATOM N | 379 | N | LEU | A | 169 | 197.530 | 187.289 | 12.931 | 1.00 | 19.46 | A |
| ATOM C | 380 | CA | LEU | A | 169 | 196.553 | 187.404 | 11.848 | 1.00 | 19.86 | A |
| ATOM C | 381 | CB | LEU | A | 169 | 195.146 | 187.572 | 12.393 | 1.00 | 20.65 | A |
| ATOM C | 382 | CG | LEU | A | 169 | 194.514 | 186.370 | 13.052 | 1.00 | 24.58 | A |
| ATOM C | 383 | CD1 | LEU | A | 169 | 193.168 | 186.837 | 13.566 | 1.00 | 23.53 | A |
| ATOM C | 384 | CD2 | LEU | A | 169 | 194.396 | 185.189 | 12.069 | 1.00 | 20.67 | A |
| ATOM C | 385 | C | LEU | A | 169 | 196.802 | 188.604 | 10.961 | 1.00 | 21.12 | A |
| ATOM O | 386 | O | LEU | A | 169 | 196.533 | 188.564 | 9.761 | 1.00 | 17.39 | A |
| ATOM N | 387 | N | GLU | A | 170 | 197.261 | 189.697 | 11.564 | 1.00 | 22.51 | A |
| ATOM C | 388 | CA | GLU | A | 170 | 197.520 | 190.915 | 10.807 | 1.00 | 23.02 | A |
| ATOM C | 389 | CB | GLU | A | 170 | 197.637 | 192.102 | 11.769 | 1.00 | 22.68 | A |
| ATOM C | 390 | CG | GLU | A | 170 | 196.267 | 192.607 | 12.232 | 1.00 | 27.99 | A |
| ATOM C | 391 | CD | GLU | A | 170 | 196.294 | 193.330 | 13.579 | 1.00 | 31.84 | A |
| ATOM O | 392 | OE1 | GLU | A | 170 | 197.334 | 193.955 | 13.920 | 1.00 | 33.33 | A |
| ATOM O | 393 | OE2 | GLU | A | 170 | 195.256 | 193.278 | 14.285 | 1.00 | 31.22 | A |
| ATOM C | 394 | C | GLU | A | 170 | 198.762 | 190.753 | 9.949 | 1.00 | 21.03 | A |
| ATOM O | 395 | O | GLU | A | 170 | 198.739 | 191.068 | 8.757 | 1.00 | 23.09 | A |
| ATOM N | 396 | N | LYS | A | 171 | 199.826 | 190.220 | 10.535 | 1.00 | 17.25 | A |
| ATOM C | 397 | CA | LYS | A | 171 | 201.059 | 190.014 | 9.790 | 1.00 | 19.63 | A |
| ATOM C | 398 | CB | LYS | A | 171 | 202.139 | 189.442 | 10.706 | 1.00 | 21.18 | A |
| ATOM C | 399 | CG | LYS | A | 171 | 203.525 | 189.395 | 10.073 | 1.00 | 25.15 | A |
| ATOM C | 400 | CD | LYS | A | 171 | 204.574 | 188.888 | 11.054 | 1.00 | 28.24 | A |
| ATOM C | 401 | CE | LYS | A | 171 | 205.952 | 188.807 | 10.395 | 1.00 | 32.60 | A |
| ATOM N | 402 | NZ | LYS | A | 171 | 207.003 | 188.297 | 11.331 | 1.00 | 33.23 | A |
| ATOM C | 403 | C | LYS | A | 171 | 200.838 | 189.074 | 8.609 | 1.00 | 19.42 | A |
| ATOM O | 404 | O | LYS | A | 171 | 201.451 | 189.235 | 7.551 | 1.00 | 21.92 | A |
| ATOM N | 405 | N | ALA | A | 172 | 199.957 | 188.095 | 8.782 | 1.00 | 17.92 | A |
| ATOM C | 406 | CA | ALA | A | 172 | 199.666 | 187.126 | 7.720 | 1.00 | 18.61 | A |
| ATOM C | 407 | CB | ALA | A | 172 | 199.168 | 185.826 | 8.341 | 1.00 | 13.39 | A |
| ATOM C | 408 | C | ALA | A | 172 | 198.666 | 187.627 | 6.679 | 1.00 | 16.48 | A |
| ATOM O | 409 | O | ALA | A | 172 | 198.600 | 187.082 | 5.577 | 1.00 | 18.45 | A |
| ATOM N | 410 | N | GLY | A | 173 | 197.890 | 188.651 | 7.053 | 1.00 | 16.48 | A |
| ATOM C | 411 | CA | GLY | A | 173 | 196.876 | 189.241 | 6.178 | 1.00 | 17.37 | A |
| ATOM C | 412 | C | GLY | A | 173 | 195.719 | 188.297 | 5.904 | 1.00 | 18.48 | A |
| ATOM O | 413 | O | GLY | A | 173 | 195.158 | 188.264 | 4.789 | 1.00 | 17.50 | A |
| ATOM N | 414 | N | VAL | A | 174 | 195.344 | 187.536 | 6.921 | 1.00 | 16.39 | A |
| ATOM C | 415 | CA | VAL | A | 174 | 194.274 | 186.573 | 6.770 | 1.00 | 18.80 | A |
| ATOM C | 416 | CB | VAL | A | 174 | 194.778 | 185.171 | 7.226 | 1.00 | 17.75 | A |
| ATOM C | 417 | CG1 | VAL | A | 174 | 195.976 | 184.770 | 6.371 | 1.00 | 17.03 | A |
| ATOM C | 418 | CG2 | VAL | A | 174 | 195.166 | 185.207 | 8.684 | 1.00 | 12.69 | A |
| ATOM C | 419 | C | VAL | A | 174 | 193.015 | 186.982 | 7.535 | 1.00 | 19.69 | A |
| ATOM O | 420 | O | VAL | A | 174 | 192.152 | 186.152 | 7.828 | 1.00 | 22.14 | A |
| ATOM N | 421 | N | GLU | A | 175 | 192.900 | 188.282 | 7.801 | 1.00 | 24.29 | A |
| ATOM C | 422 | CA | GLU | A | 175 | 191.751 | 188.834 | 8.521 | 1.00 | 25.76 | A |
| ATOM C | 423 | CB | GLU | A | 175 | 191.902 | 190.351 | 8.799 | 1.00 | 29.22 | A |
| ATOM C | 424 | CG | GLU | A | 175 | 193.333 | 190.938 | 8.903 | 1.00 | 38.41 | A |
| ATOM C | 425 | CD | GLU | A | 175 | 193.998 | 191.112 | 7.548 | 1.00 | 39.97 | A |
| ATOM O | 426 | OE1 | GLU | A | 175 | 193.310 | 190.986 | 6.511 | 1.00 | 47.49 | A |
| ATOM O | 427 | OE2 | GLU | A | 175 | 195.206 | 191.382 | 7.507 | 1.00 | 46.22 | A |
| ATOM C | 428 | C | GLU | A | 175 | 190.452 | 188.611 | 7.752 | 1.00 | 25.95 | A |
| ATOM O | 429 | O | GLU | A | 175 | 189.423 | 188.313 | 8.337 | 1.00 | 25.42 | A |
| ATOM N | 430 | N | HIS | A | 176 | 190.483 | 188.768 | 6.438 | 1.00 | 26.45 | A |
| ATOM C | 431 | CA | HIS | A | 176 | 189.254 | 188.539 | 5.708 | 1.00 | 24.68 | A |
| ATOM C | 432 | CB | HIS | A | 176 | 189.316 | 189.074 | 4.278 | 1.00 | 23.09 | A |
| ATOM C | 433 | CG | HIS | A | 176 | 187.983 | 189.014 | 3.596 | 1.00 | 32.41 | A |
| ATOM C | 434 | CD2 | HIS | A | 176 | 187.570 | 188.363 | 2.477 | 1.00 | 34.18 | A |
| ATOM N | 435 | ND1 | HIS | A | 176 | 186.845 | 189.576 | 4.148 | 1.00 | 32.75 | A |
| ATOM C | 436 | CE1 | HIS | A | 176 | 185.796 | 189.266 | 3.406 | 1.00 | 33.74 | A |
| ATOM N | 437 | NE2 | HIS | A | 176 | 186.207 | 188.529 | 2.386 | 1.00 | 34.72 | A |
| ATOM C | 438 | C | HIS | A | 176 | 188.848 | 187.062 | 5.703 | 1.00 | 23.32 | A |
| ATOM O | 439 | O | HIS | A | 176 | 187.661 | 186.755 | 5.693 | 1.00 | 22.64 | A |
| ATOM N | 440 | N | GLN | A | 177 | 189.803 | 186.137 | 5.744 | 1.00 | 21.78 | A |
| ATOM C | 441 | CA | GLN | A | 177 | 189.432 | 184.721 | 5.774 | 1.00 | 19.85 | A |
| ATOM C | 442 | CB | GLN | A | 177 | 190.628 | 183.818 | 5.421 | 1.00 | 21.23 | A |
| ATOM C | 443 | CG | GLN | A | 177 | 191.173 | 183.994 | 3.977 | 1.00 | 23.08 | A |
| ATOM C | 444 | CD | GLN | A | 177 | 192.113 | 185.179 | 3.856 | 1.00 | 26.14 | A |
| ATOM O | 445 | OE1 | GLN | A | 177 | 191.901 | 186.204 | 4.512 | 1.00 | 27.02 | A |
| ATOM N | 446 | NE2 | GLN | A | 177 | 193.161 | 185.056 | 3.012 | 1.00 | 25.09 | A |
| ATOM C | 447 | C | GLN | A | 177 | 188.859 | 184.317 | 7.129 | 1.00 | 18.66 | A |
| ATOM O | 448 | O | GLN | A | 177 | 188.057 | 183.390 | 7.195 | 1.00 | 18.16 | A |
| ATOM N | 449 | N | LEU | A | 178 | 189.267 | 185.001 | 8.202 | 1.00 | 17.80 | A |
| ATOM C | 450 | CA | LEU | A | 178 | 188.742 | 184.695 | 9.532 | 1.00 | 17.25 | A |
| ATOM C | 451 | CB | LEU | A | 178 | 189.473 | 185.495 | 10.616 | 1.00 | 14.48 | A |
| ATOM C | 452 | CG | LEU | A | 178 | 188.948 | 185.297 | 12.041 | 1.00 | 14.78 | A |
| ATOM C | 453 | CD1 | LEU | A | 178 | 188.822 | 183.792 | 12.391 | 1.00 | 13.65 | A |
| ATOM C | 454 | CD2 | LEU | A | 178 | 189.904 | 185.964 | 13.002 | 1.00 | 15.52 | A |
| ATOM C | 455 | C | LEU | A | 178 | 187.257 | 185.050 | 9.569 | 1.00 | 18.82 | A |
| ATOM O | 456 | O | LEU | A | 178 | 186.436 | 184.346 | 10.148 | 1.00 | 18.56 | A |
| ATOM N | 457 | N | ARG | A | 179 | 186.929 | 186.171 | 8.950 | 1.00 | 17.14 | A |
| ATOM C | 458 | CA | ARG | A | 179 | 185.570 | 186.637 | 8.893 | 1.00 | 20.36 | A |
| ATOM C | 459 | CB | ARG | A | 179 | 185.547 | 188.004 | 8.238 | 1.00 | 24.60 | A |
| ATOM C | 460 | CG | ARG | A | 179 | 184.191 | 188.581 | 8.190 | 1.00 | 30.51 | A |
| ATOM C | 461 | CD | ARG | A | 179 | 184.079 | 189.355 | 6.937 | 1.00 | 34.92 | A |
| ATOM N | 462 | NE | ARG | A | 179 | 183.602 | 190.700 | 7.192 | 1.00 | 44.42 | A |
| ATOM C | 463 | CZ | ARG | A | 179 | 184.338 | 191.679 | 7.721 | 1.00 | 50.64 | A |
| ATOM N | 464 | NH1 | ARG | A | 179 | 185.609 | 191.475 | 8.060 | 1.00 | 53.62 | A |
| ATOM N | 465 | NH2 | ARG | A | 179 | 183.800 | 192.881 | 7.910 | 1.00 | 54.27 | A |
| ATOM C | 466 | C | ARG | A | 179 | 184.677 | 185.663 | 8.112 | 1.00 | 20.68 | A |
| ATOM O | 467 | O | ARG | A | 179 | 183.516 | 185.428 | 8.490 | 1.00 | 20.49 | A |
| ATOM N | 468 | N | ARG | A | 180 | 185.211 | 185.102 | 7.027 | 1.00 | 16.87 | A |
| ATOM C | 469 | CA | ARG | A | 180 | 184.457 | 184.139 | 6.240 | 1.00 | 16.27 | A |
| ATOM C | 470 | CB | ARG | A | 180 | 185.145 | 183.851 | 4.880 | 1.00 | 15.64 | A |
| ATOM C | 471 | CG | ARG | A | 180 | 185.036 | 184.997 | 3.869 | 1.00 | 19.03 | A |
| ATOM C | 472 | CD | ARG | A | 180 | 185.243 | 184.593 | 2.394 | 1.00 | 19.17 | A |
| ATOM N | 473 | NE | ARG | A | 180 | 186.536 | 183.973 | 2.158 | 1.00 | 22.25 | A |
| ATOM C | 474 | CZ | ARG | A | 180 | 187.223 | 184.003 | 1.011 | 1.00 | 22.42 | A |
| ATOM N | 475 | NH1 | ARG | A | 180 | 186.778 | 184.633 | -0.067 | 1.00 | 16.67 | A |
| ATOM N | 476 | NH2 | ARG | A | 180 | 188.390 | 183.384 | 0.949 | 1.00 | 23.03 | A |
| ATOM C | 477 | C | ARG | A | 180 | 184.277 | 182.837 | 7.037 | 1.00 | 16.14 | A |
| ATOM O | 478 | O | ARG | A | 180 | 183.225 | 182.207 | 6.950 | 1.00 | 17.78 | A |
| ATOM N | 479 | N | GLU | A | 181 | 185.294 | 182.439 | 7.812 | 1.00 | 15.13 | A |
| ATOM C | 480 | CA | GLU | A | 181 | 185.211 | 181.222 | 8.627 | 1.00 | 15.82 | A |
| ATOM C | 481 | CB | GLU | A | 181 | 186.512 | 180.969 | 9.431 | 1.00 | 17.60 | A |
| ATOM C | 482 | CG | GLU | A | 181 | 186.517 | 179.610 | 10.164 | 1.00 | 22.11 | A |
| ATOM C | 483 | CD | GLU | A | 181 | 187.779 | 179.293 | 11.023 | 1.00 | 29.50 | A |
| ATOM O | 484 | OE1 | GLU | A | 181 | 188.877 | 179.898 | 10.811 | 1.00 | 23.91 | A |
| ATOM O | 485 | OE2 | GLU | A | 181 | 187.654 | 178.393 | 11.914 | 1.00 | 30.52 | A |
| ATOM C | 486 | C | GLU | A | 181 | 184.059 | 181.374 | 9.615 | 1.00 | 17.24 | A |
| ATOM O | 487 | O | GLU | A | 181 | 183.169 | 180.514 | 9.711 | 1.00 | 17.81 | A |
| ATOM N | 488 | N | VAL | A | 182 | 184.075 | 182.486 | 10.336 | 1.00 | 13.24 | A |
| ATOM C | 489 | CA | VAL | A | 182 | 183.056 | 182.746 | 11.323 | 1.00 | 16.10 | A |
| ATOM C | 490 | CB | VAL | A | 182 | 183.417 | 183.989 | 12.155 | 1.00 | 17.20 | A |
| ATOM C | 491 | CG1 | VAL | A | 182 | 182.271 | 184.362 | 13.059 | 1.00 | 11.33 | A |
| ATOM C | 492 | CG2 | VAL | A | 182 | 184.675 | 183.709 | 12.948 | 1.00 | 15.06 | A |
| ATOM C | 493 | C | VAL | A | 182 | 181.650 | 182.907 | 10.756 | 1.00 | 16.14 | A |
| ATOM O | 494 | O | VAL | A | 182 | 180.718 | 182.216 | 11.186 | 1.00 | 14.19 | A |
| ATOM N | 495 | N | GLU | A | 183 | 181.491 | 183.805 | 9.788 | 1.00 | 16.25 | A |
| ATOM C | 496 | CA | GLU | A | 183 | 180.162 | 184.021 | 9.212 | 1.00 | 17.30 | A |
| ATOM C | 497 | CB | GLU | A | 183 | 180.190 | 185.187 | 8.214 | 1.00 | 16.51 | A |
| ATOM C | 498 | CG | GLU | A | 183 | 180.630 | 186.499 | 8.842 | 1.00 | 21.63 | A |
| ATOM C | 499 | CD | GLU | A | 183 | 180.566 | 187.674 | 7.874 | 1.00 | 24.47 | A |
| ATOM O | 500 | OE1 | GLU | A | 183 | 180.841 | 187.477 | 6.673 | 1.00 | 27.21 | A |
| ATOM O | 501 | OE2 | GLU | A | 183 | 180.255 | 188.801 | 8.315 | 1.00 | 29.43 | A |
| ATOM C | 502 | C | GLU | A | 183 | 179.581 | 182.764 | 8.555 | 1.00 | 14.99 | A |
| ATOM O | 503 | O | GLU | A | 183 | 178.405 | 182.460 | 8.753 | 1.00 | 16.33 | A |
| ATOM N | 504 | N | ILE | A | 184 | 180.396 | 182.019 | 7.810 | 1.00 | 12.00 | A |
| ATOM C | 505 | CA | ILE | A | 184 | 179.908 | 180.808 | 7.159 | 1.00 | 10.45 | A |
| ATOM C | 506 | CB | ILE | A | 184 | 180.904 | 180.302 | 6.094 | 1.00 | 9.02 | A |
| ATOM C | 507 | CG2 | ILE | A | 184 | 180.595 | 178.854 | 5.712 | 1.00 | 2.93 | A |
| ATOM C | 508 | CG1 | ILE | A | 184 | 180.862 | 181.248 | 4.893 | 1.00 | 4.36 | A |
| ATOM C | 509 | CD1 | ILE | A | 184 | 182.025 | 181.076 | 3.939 | 1.00 | 5.66 | A |
| ATOM C | 510 | C | ILE | A | 184 | 179.633 | 179.675 | 8.149 | 1.00 | 12.74 | A |
| ATOM O | 511 | O | ILE | A | 184 | 178.552 | 179.077 | 8.135 | 1.00 | 13.03 | A |
| ATOM N | 512 | N | GLN | A | 185 | 180.585 | 179.380 | 9.026 | 1.00 | 14.41 | A |
| ATOM C | 513 | CA | GLN | A | 185 | 180.365 | 178.279 | 9.960 | 1.00 | 15.94 | A |
| ATOM C | 514 | CB | GLN | A | 185 | 181.663 | 177.933 | 10.682 | 1.00 | 15.68 | A |
| ATOM C | 515 | CG | GLN | A | 185 | 181.684 | 176.556 | 11.281 | 1.00 | 15.22 | A |
| ATOM C | 516 | CD | GLN | A | 185 | 182.955 | 176.333 | 12.074 | 1.00 | 16.62 | A |
| ATOM O | 517 | OE1 | GLN | A | 185 | 183.844 | 177.184 | 12.072 | 1.00 | 19.84 | A |
| ATOM N | 518 | NE2 | GLN | A | 185 | 183.048 | 175.198 | 12.760 | 1.00 | 15.50 | A |
| ATOM C | 519 | C | GLN | A | 185 | 179.257 | 178.551 | 10.977 | 1.00 | 14.85 | A |
| ATOM O | 520 | O | GLN | A | 185 | 178.507 | 177.637 | 11.339 | 1.00 | 11.63 | A |
| ATOM N | 521 | N | SER | A | 186 | 179.134 | 179.798 | 11.424 | 1.00 | 14.21 | A |
| ATOM C | 522 | CA | SER | A | 186 | 178.102 | 180.102 | 12.421 | 1.00 | 15.30 | A |
| ATOM C | 523 | CB | SER | A | 186 | 178.265 | 181.527 | 13.028 | 1.00 | 15.24 | A |
| ATOM O | 524 | OG | SER | A | 186 | 178.146 | 182.584 | 12.086 | 1.00 | 16.11 | A |
| ATOM C | 525 | C | SER | A | 186 | 176.686 | 179.916 | 11.898 | 1.00 | 15.48 | A |
| ATOM O | 526 | O | SER | A | 186 | 175.787 | 179.612 | 12.672 | 1.00 | 20.12 | A |
| ATOM N | 527 | N | HIS | A | 187 | 176.483 | 180.048 | 10.592 | 1.00 | 15.79 | A |
| ATOM C | 528 | CA | HIS | A | 187 | 175.153 | 179.908 | 10.018 | 1.00 | 15.76 | A |
| ATOM C | 529 | CB | HIS | A | 187 | 174.964 | 180.942 | 8.914 | 1.00 | 14.22 | A |
| ATOM C | 530 | CG | HIS | A | 187 | 174.857 | 182.348 | 9.414 | 1.00 | 14.55 | A |
| ATOM C | 531 | CD2 | HIS | A | 187 | 173.787 | 183.070 | 9.816 | 1.00 | 12.55 | A |
| ATOM N | 532 | ND1 | HIS | A | 187 | 175.944 | 183.189 | 9.516 | 1.00 | 13.11 | A |
| ATOM C | 533 | CE1 | HIS | A | 187 | 175.548 | 184.369 | 9.951 | 1.00 | 10.14 | A |
| ATOM N | 534 | NE2 | HIS | A | 187 | 174.243 | 184.324 | 10.140 | 1.00 | 15.06 | A |
| ATOM C | 535 | C | HIS | A | 187 | 174.838 | 178.515 | 9.469 | 1.00 | 17.67 | A |
| ATOM O | 536 | O | HIS | A | 187 | 173.762 | 178.272 | 8.931 | 1.00 | 17.40 | A |
| ATOM N | 537 | N | LEU | A | 188 | 175.777 | 177.595 | 9.616 | 1.00 | 18.89 | A |
| ATOM C | 538 | CA | LEU | A | 188 | 175.608 | 176.243 | 9.118 | 1.00 | 16.21 | A |
| ATOM C | 539 | CB | LEU | A | 188 | 176.972 | 175.721 | 8.652 | 1.00 | 15.40 | A |
| ATOM C | 540 | CG | LEU | A | 188 | 177.339 | 175.433 | 7.185 | 1.00 | 14.79 | A |
| ATOM C | 541 | CD1 | LEU | A | 188 | 176.514 | 176.200 | 6.155 | 1.00 | 14.64 | A |
| ATOM C | 542 | CD2 | LEU | A | 188 | 178.805 | 175.738 | 7.024 | 1.00 | 11.29 | A |
| ATOM C | 543 | C | LEU | A | 188 | 175.047 | 175.408 | 10.266 | 1.00 | 17.59 | A |
| ATOM O | 544 | O | LEU | A | 188 | 175.382 | 175.630 | 11.423 | 1.00 | 19.21 | A |
| ATOM N | 545 | N | ARG | A | 189 | 174.174 | 174.458 | 9.955 | 1.00 | 19.36 | A |
| ATOM C | 546 | CA | ARG | A | 189 | 173.579 | 173.603 | 10.988 | 1.00 | 21.36 | A |
| ATOM C | 547 | CB | ARG | A | 189 | 172.190 | 174.086 | 11.398 | 1.00 | 22.58 | A |
| ATOM C | 548 | CG | ARG | A | 189 | 172.131 | 175.420 | 12.110 | 1.00 | 32.57 | A |
| ATOM C | 549 | CD | ARG | A | 189 | 172.277 | 175.274 | 13.621 | 1.00 | 34.83 | A |
| ATOM N | 550 | NE | ARG | A | 189 | 172.168 | 176.568 | 14.298 | 1.00 | 40.56 | A |
| ATOM C | 551 | CZ | ARG | A | 189 | 172.993 | 177.598 | 14.091 | 1.00 | 43.35 | A |
| ATOM N | 552 | NH1 | ARG | A | 189 | 173.993 | 177.481 | 13.215 | 1.00 | 42.31 | A |
| ATOM N | 553 | NH2 | ARG | A | 189 | 172.827 | 178.740 | 14.770 | 1.00 | 42.32 | A |
| ATOM C | 554 | C | ARG | A | 189 | 173.423 | 172.219 | 10.415 | 1.00 | 19.45 | A |
| ATOM O | 555 | O | ARG | A | 189 | 172.582 | 171.996 | 9.558 | 1.00 | 19.37 | A |
| ATOM N | 556 | N | HIS | A | 190 | 174.218 | 171.283 | 10.900 | 1.00 | 16.48 | A |
| ATOM C | 557 | CA | HIS | A | 190 | 174.148 | 169.936 | 10.388 | 1.00 | 16.32 | A |
| ATOM C | 558 | CB | HIS | A | 190 | 174.865 | 169.868 | 9.042 | 1.00 | 13.06 | A |
| ATOM C | 559 | CG | HIS | A | 190 | 174.848 | 168.517 | 8.403 | 1.00 | 8.96 | A |
| ATOM C | 560 | CD2 | HIS | A | 190 | 175.640 | 167.430 | 8.584 | 1.00 | 9.19 | A |
| ATOM N | 561 | ND1 | HIS | A | 190 | 173.942 | 168.169 | 7.422 | 1.00 | 9.60 | A |
| ATOM C | 562 | CE1 | HIS | A | 190 | 174.177 | 166.929 | 7.026 | 1.00 | 8.25 | A |
| ATOM N | 563 | NE2 | HIS | A | 190 | 175.202 | 166.456 | 7.717 | 1.00 | 10.48 | A |
| ATOM C | 564 | C | HIS | A | 190 | 174.857 | 169.069 | 11.409 | 1.00 | 18.77 | A |
| ATOM O | 565 | O | HIS | A | 190 | 175.747 | 169.538 | 12.110 | 1.00 | 19.58 | A |
| ATOM N | 566 | N | PRO | A | 191 | 174.437 | 167.797 | 11.526 | 1.00 | 19.81 | A |
| ATOM C | 567 | CD | PRO | A | 191 | 173.236 | 167.329 | 10.808 | 1.00 | 18.67 | A |
| ATOM C | 568 | CA | PRO | A | 191 | 174.946 | 166.749 | 12.430 | 1.00 | 18.82 | A |
| ATOM C | 569 | CB | PRO | A | 191 | 174.058 | 165.545 | 12.108 | 1.00 | 15.98 | A |
| ATOM C | 570 | CG | PRO | A | 191 | 172.813 | 166.147 | 11.615 | 1.00 | 19.49 | A |
| ATOM C | 571 | C | PRO | A | 191 | 176.422 | 166.383 | 12.239 | 1.00 | 15.63 | A |
| ATOM O | 572 | O | PRO | A | 191 | 177.074 | 165.908 | 13.167 | 1.00 | 16.18 | A |
| ATOM N | 573 | N | ASN | A | 192 | 176.930 | 166.564 | 11.029 | 1.00 | 13.38 | A |
| ATOM C | 574 | CA | ASN | A | 192 | 178.306 | 166.216 | 10.740 | 1.00 | 13.06 | A |
| ATOM C | 575 | CB | ASN | A | 192 | 178.367 | 165.242 | 9.568 | 1.00 | 16.94 | A |
| ATOM C | 576 | CG | ASN | A | 192 | 177.621 | 163.963 | 9.853 | 1.00 | 21.40 | A |
| ATOM O | 577 | OD1 | ASN | A | 192 | 176.478 | 163.790 | 9.431 | 1.00 | 24.81 | A |
| ATOM N | 578 | ND2 | ASN | A | 192 | 178.255 | 163.062 | 10.596 | 1.00 | 19.25 | A |
| ATOM C | 579 | C | ASN | A | 192 | 179.189 | 167.407 | 10.467 | 1.00 | 13.65 | A |
| ATOM O | 580 | O | ASN | A | 192 | 180.258 | 167.276 | 9.869 | 1.00 | 15.58 | A |
| ATOM N | 581 | N | ILE | A | 193 | 178.725 | 168.584 | 10.865 | 1.00 | 13.34 | A |
| ATOM C | 582 | CA | ILE | A | 193 | 179.529 | 169.789 | 10.734 | 1.00 | 12.06 | A |
| ATOM C | 583 | CB | ILE | A | 193 | 178.886 | 170.843 | 9.814 | 1.00 | 11.35 | A |
| ATOM C | 584 | CG2 | ILE | A | 193 | 179.688 | 172.131 | 9.882 | 1.00 | 5.81 | A |
| ATOM C | 585 | CG1 | ILE | A | 193 | 178.807 | 170.317 | 8.381 | 1.00 | 6.17 | A |
| ATOM C | 586 | CD1 | ILE | A | 193 | 178.045 | 171.205 | 7.453 | 1.00 | 8.69 | A |
| ATOM C | 587 | C | ILE | A | 193 | 179.651 | 170.377 | 12.134 | 1.00 | 12.85 | A |
| ATOM O | 588 | O | ILE | A | 193 | 178.663 | 170.540 | 12.840 | 1.00 | 14.90 | A |
| ATOM N | 589 | N | LEU | A | 194 | 180.874 | 170.678 | 12.530 | 1.00 | 13.66 | A |
| ATOM C | 590 | CA | LEU | A | 194 | 181.123 | 171.253 | 13.839 | 1.00 | 14.76 | A |
| ATOM C | 591 | CB | LEU | A | 194 | 182.623 | 171.519 | 14.036 | 1.00 | 16.68 | A |
| ATOM C | 592 | CG | LEU | A | 194 | 183.117 | 171.537 | 15.486 | 1.00 | 15.84 | A |
| ATOM C | 593 | CD1 | LEU | A | 194 | 183.039 | 170.099 | 16.000 | 1.00 | 14.02 | A |
| ATOM C | 594 | CD2 | LEU | A | 194 | 184.545 | 172.073 | 15.588 | 1.00 | 14.49 | A |
| ATOM C | 595 | C | LEU | A | 194 | 180.381 | 172.578 | 13.964 | 1.00 | 16.81 | A |
| ATOM O | 596 | O | LEU | A | 194 | 180.450 | 173.433 | 13.070 | 1.00 | 18.34 | A |
| ATOM N | 597 | N | ARG | A | 195 | 179.683 | 172.727 | 15.085 | 1.00 | 15.08 | A |
| ATOM C | 598 | CA | ARG | A | 195 | 178.924 | 173.924 | 15.390 | 1.00 | 16.45 | A |
| ATOM C | 599 | CB | ARG | A | 195 | 177.878 | 173.634 | 16.474 | 1.00 | 19.44 | A |
| ATOM C | 600 | CG | ARG | A | 195 | 176.596 | 172.975 | 15.987 | 1.00 | 25.93 | A |
| ATOM C | 601 | CD | ARG | A | 195 | 175.746 | 173.967 | 15.232 | 1.00 | 29.82 | A |
| ATOM N | 602 | NE | ARG | A | 195 | 174.811 | 174.694 | 16.091 | 1.00 | 36.41 | A |
| ATOM C | 603 | CZ | ARG | A | 195 | 174.859 | 176.008 | 16.302 | 1.00 | 39.91 | A |
| ATOM N | 604 | NH1 | ARG | A | 195 | 175.804 | 176.729 | 15.718 | 1.00 | 44.61 | A |
| ATOM N | 605 | NH2 | ARG | A | 195 | 173.958 | 176.608 | 17.075 | 1.00 | 40.14 | A |
| ATOM C | 606 | C | ARG | A | 195 | 179.787 | 175.113 | 15.870 | 1.00 | 16.17 | A |
| ATOM O | 607 | O | ARG | A | 195 | 180.714 | 174.950 | 16.656 | 1.00 | 13.03 | A |
| ATOM N | 608 | N | LEU | A | 196 | 179.471 | 176.293 | 15.341 | 1.00 | 17.85 | A |
| ATOM C | 609 | CA | LEU | A | 196 | 180.114 | 177.536 | 15.749 | 1.00 | 16.50 | A |
| ATOM C | 610 | CB | LEU | A | 196 | 180.623 | 178.380 | 14.572 | 1.00 | 13.17 | A |
| ATOM C | 611 | CG | LEU | A | 196 | 181.348 | 179.669 | 14.959 | 1.00 | 15.31 | A |
| ATOM C | 612 | CD1 | LEU | A | 196 | 182.467 | 179.277 | 15.910 | 1.00 | 13.98 | A |
| ATOM C | 613 | CD2 | LEU | A | 196 | 181.922 | 180.404 | 13.749 | 1.00 | 12.29 | A |
| ATOM C | 614 | C | LEU | A | 196 | 178.975 | 178.281 | 16.466 | 1.00 | 15.90 | A |
| ATOM O | 615 | O | LEU | A | 196 | 178.037 | 178.767 | 15.839 | 1.00 | 15.06 | A |
| ATOM N | 616 | N | TYR | A | 197 | 179.062 | 178.337 | 17.791 | 1.00 | 18.19 | A |
| ATOM C | 617 | CA | TYR | A | 197 | 178.049 | 178.974 | 18.610 | 1.00 | 18.38 | A |
| ATOM C | 618 | CB | TYR | A | 197 | 178.154 | 178.464 | 20.033 | 1.00 | 15.79 | A |
| ATOM C | 619 | CG | TYR | A | 197 | 177.908 | 176.980 | 20.126 | 1.00 | 17.64 | A |
| ATOM C | 620 | CD1 | TYR | A | 197 | 178.955 | 176.080 | 20.280 | 1.00 | 15.26 | A |
| ATOM C | 621 | CE1 | TYR | A | 197 | 178.712 | 174.718 | 20.364 | 1.00 | 20.64 | A |
| ATOM C | 622 | CD2 | TYR | A | 197 | 176.607 | 176.469 | 20.054 | 1.00 | 21.32 | A |
| ATOM C | 623 | CE2 | TYR | A | 197 | 176.360 | 175.104 | 20.138 | 1.00 | 20.23 | A |
| ATOM C | 624 | CZ | TYR | A | 197 | 177.418 | 174.242 | 20.293 | 1.00 | 20.94 | A |
| ATOM O | 625 | OH | TYR | A | 197 | 177.185 | 172.900 | 20.387 | 1.00 | 26.42 | A |
| ATOM C | 626 | C | TYR | A | 197 | 178.174 | 180.481 | 18.561 | 1.00 | 19.88 | A |
| ATOM O | 627 | O | TYR | A | 197 | 177.180 | 181.193 | 18.454 | 1.00 | 19.95 | A |
| ATOM N | 628 | N | GLY | A | 198 | 179.394 | 180.985 | 18.611 | 1.00 | 20.35 | A |
| ATOM C | 629 | CA | GLY | A | 198 | 179.542 | 182.425 | 18.562 | 1.00 | 20.00 | A |
| ATOM C | 630 | C | GLY | A | 198 | 180.984 | 182.838 | 18.642 | 1.00 | 17.90 | A |
| ATOM O | 631 | O | GLY | A | 198 | 181.874 | 182.005 | 18.677 | 1.00 | 19.17 | A |
| ATOM N | 632 | N | TYR | A | 199 | 181.222 | 184.134 | 18.686 | 1.00 | 17.72 | A |
| ATOM C | 633 | CA | TYR | A | 199 | 182.589 | 184.593 | 18.748 | 1.00 | 19.93 | A |
| ATOM C | 634 | CB | TYR | A | 199 | 183.128 | 184.776 | 17.325 | 1.00 | 19.83 | A |
| ATOM C | 635 | CG | TYR | A | 199 | 182.894 | 186.166 | 16.788 | 1.00 | 21.41 | A |
| ATOM C | 636 | CD1 | TYR | A | 199 | 183.892 | 187.135 | 16.885 | 1.00 | 21.65 | A |
| ATOM C | 637 | CE1 | TYR | A | 199 | 183.677 | 188.430 | 16.486 | 1.00 | 22.65 | A |
| ATOM C | 638 | CD2 | TYR | A | 199 | 181.660 | 186.538 | 16.266 | 1.00 | 18.59 | A |
| ATOM C | 639 | CE2 | TYR | A | 199 | 181.426 | 187.841 | 15.862 | 1.00 | 22.04 | A |
| ATOM C | 640 | CZ | TYR | A | 199 | 182.439 | 188.787 | 15.972 | 1.00 | 23.54 | A |
| ATOM O | 641 | OH | TYR | A | 199 | 182.223 | 190.089 | 15.557 | 1.00 | 24.04 | A |
| ATOM C | 642 | C | TYR | A | 199 | 182.693 | 185.910 | 19.508 | 1.00 | 19.03 | A |
| ATOM O | 643 | O | TYR | A | 199 | 181.703 | 186.618 | 19.699 | 1.00 | 19.48 | A |
| ATOM N | 644 | N | PHE | A | 200 | 183.910 | 186.238 | 19.920 | 1.00 | 18.13 | A |
| ATOM C | 645 | CA | PHE | A | 200 | 184.177 | 187.478 | 20.627 | 1.00 | 17.58 | A |
| ATOM C | 646 | CB | PHE | A | 200 | 183.677 | 187.397 | 22.091 | 1.00 | 12.35 | A |
| ATOM C | 647 | CG | PHE | A | 200 | 184.369 | 186.344 | 22.955 | 1.00 | 13.81 | A |
| ATOM C | 648 | CD1 | PHE | A | 200 | 185.449 | 186.685 | 23.779 | 1.00 | 14.44 | A |
| ATOM C | 649 | CD2 | PHE | A | 200 | 183.898 | 185.033 | 22.998 | 1.00 | 12.16 | A |
| ATOM C | 650 | CE1 | PHE | A | 200 | 186.043 | 185.731 | 24.636 | 1.00 | 13.44 | A |
| ATOM C | 651 | CE2 | PHE | A | 200 | 184.474 | 184.075 | 23.845 | 1.00 | 13.42 | A |
| ATOM C | 652 | CZ | PHE | A | 200 | 185.552 | 184.426 | 24.669 | 1.00 | 13.28 | A |
| ATOM C | 653 | C | PHE | A | 200 | 185.675 | 187.756 | 20.549 | 1.00 | 18.86 | A |
| ATOM O | 654 | O | PHE | A | 200 | 186.482 | 186.840 | 20.451 | 1.00 | 24.13 | A |
| ATOM N | 655 | N | HIS | A | 201 | 186.056 | 189.019 | 20.573 | 1.00 | 19.06 | A |
| ATOM C | 656 | CA | HIS | A | 201 | 187.467 | 189.335 | 20.512 | 1.00 | 22.05 | A |
| ATOM C | 657 | CB | HIS | A | 201 | 187.820 | 189.966 | 19.159 | 1.00 | 20.51 | A |
| ATOM C | 658 | CG | HIS | A | 201 | 187.161 | 191.286 | 18.903 | 1.00 | 18.99 | A |
| ATOM C | 659 | CD2 | HIS | A | 201 | 185.922 | 191.593 | 18.450 | 1.00 | 19.83 | A |
| ATOM N | 660 | ND1 | HIS | A | 201 | 187.848 | 192.478 | 18.965 | 1.00 | 21.28 | A |
| ATOM C | 661 | CE1 | HIS | A | 201 | 187.071 | 193.459 | 18.544 | 1.00 | 19.32 | A |
| ATOM N | 662 | NE2 | HIS | A | 201 | 185.896 | 192.948 | 18.223 | 1.00 | 20.12 | A |
| ATOM C | 663 | C | HIS | A | 201 | 187.840 | 190.279 | 21.629 | 1.00 | 22.96 | A |
| ATOM O | 664 | O | HIS | A | 201 | 186.977 | 190.871 | 22.253 | 1.00 | 23.67 | A |
| ATOM N | 665 | N | ASP | A | 202 | 189.126 | 190.395 | 21.913 | 1.00 | 23.03 | A |
| ATOM C | 666 | CA | ASP | A | 202 | 189.535 | 191.331 | 22.936 | 1.00 | 24.25 | A |
| ATOM C | 667 | CB | ASP | A | 202 | 189.928 | 190.621 | 24.235 | 1.00 | 25.39 | A |
| ATOM C | 668 | CG | ASP | A | 202 | 191.175 | 189.780 | 24.097 | 1.00 | 28.38 | A |
| ATOM O | 669 | OD1 | ASP | A | 202 | 191.836 | 189.826 | 23.031 | 1.00 | 34.60 | A |
| ATOM O | 670 | OD2 | ASP | A | 202 | 191.495 | 189.066 | 25.067 | 1.00 | 24.71 | A |
| ATOM C | 671 | C | ASP | A | 202 | 190.696 | 192.148 | 22.398 | 1.00 | 25.07 | A |
| ATOM O | 672 | O | ASP | A | 202 | 190.896 | 192.235 | 21.193 | 1.00 | 24.05 | A |
| ATOM N | 673 | N | ALA | A | 203 | 191.480 | 192.733 | 23.287 | 1.00 | 26.38 | A |
| ATOM C | 674 | CA | ALA | A | 203 | 192.595 | 193.564 | 22.856 | 1.00 | 25.39 | A |
| ATOM C | 675 | CB | ALA | A | 203 | 193.217 | 194.257 | 24.082 | 1.00 | 23.30 | A |
| ATOM C | 676 | C | ALA | A | 203 | 193.678 | 192.847 | 22.039 | 1.00 | 25.31 | A |
| ATOM O | 677 | O | ALA | A | 203 | 194.184 | 193.405 | 21.070 | 1.00 | 24.76 | A |
| ATOM N | 678 | N | THR | A | 204 | 194.038 | 191.623 | 22.409 | 1.00 | 24.17 | A |
| ATOM C | 679 | CA | THR | A | 204 | 195.084 | 190.922 | 21.682 | 1.00 | 24.93 | A |
| ATOM C | 680 | CB | THR | A | 204 | 196.202 | 190.467 | 22.631 | 1.00 | 28.17 | A |
| ATOM O | 681 | OG1 | THR | A | 204 | 195.667 | 189.534 | 23.582 | 1.00 | 29.58 | A |
| ATOM C | 682 | CG2 | THR | A | 204 | 196.805 | 191.672 | 23.364 | 1.00 | 26.38 | A |
| ATOM C | 683 | C | THR | A | 204 | 194.638 | 189.711 | 20.869 | 1.00 | 26.55 | A |
| ATOM O | 684 | O | THR | A | 204 | 195.360 | 189.270 | 19.968 | 1.00 | 25.70 | A |
| ATOM N | 685 | N | ARG | A | 205 | 193.469 | 189.149 | 21.166 | 1.00 | 26.89 | A |
| ATOM C | 686 | CA | ARG | A | 205 | 193.039 | 188.002 | 20.382 | 1.00 | 26.09 | A |
| ATOM C | 687 | CB | ARG | A | 205 | 193.558 | 186.710 | 21.026 | 1.00 | 29.89 | A |
| ATOM C | 688 | CG | ARG | A | 205 | 193.403 | 186.595 | 22.508 | 1.00 | 35.65 | A |
| ATOM C | 689 | CD | ARG | A | 205 | 194.768 | 186.465 | 23.174 | 1.00 | 41.67 | A |
| ATOM N | 690 | NE | ARG | A | 205 | 194.650 | 186.517 | 24.630 | 1.00 | 50.08 | A |
| ATOM C | 691 | CZ | ARG | A | 205 | 195.657 | 186.756 | 25.467 | 1.00 | 53.61 | A |
| ATOM N | 692 | NH1 | ARG | A | 205 | 196.884 | 186.972 | 24.997 | 1.00 | 54.63 | A |
| ATOM N | 693 | NH2 | ARG | A | 205 | 195.429 | 186.782 | 26.781 | 1.00 | 57.41 | A |
| ATOM C | 694 | C | ARG | A | 205 | 191.561 | 187.869 | 20.036 | 1.00 | 23.76 | A |
| ATOM O | 695 | O | ARG | A | 205 | 190.728 | 188.654 | 20.478 | 1.00 | 26.35 | A |
| ATOM N | 696 | N | VAL | A | 206 | 191.253 | 186.892 | 19.187 | 1.00 | 21.67 | A |
| ATOM C | 697 | CA | VAL | A | 206 | 189.883 | 186.606 | 18.776 | 1.00 | 17.07 | A |
| ATOM C | 698 | CB | VAL | A | 206 | 189.704 | 186.683 | 17.247 | 1.00 | 16.64 | A |
| ATOM C | 699 | CG1 | VAL | A | 206 | 188.235 | 186.422 | 16.888 | 1.00 | 11.75 | A |
| ATOM C | 700 | CG2 | VAL | A | 206 | 190.136 | 188.055 | 16.741 | 1.00 | 15.29 | A |
| ATOM C | 701 | C | VAL | A | 206 | 189.541 | 185.179 | 19.225 | 1.00 | 16.35 | A |
| ATOM O | 702 | O | VAL | A | 206 | 190.380 | 184.277 | 19.146 | 1.00 | 15.34 | A |
| ATOM N | 703 | N | TYR | A | 207 | 188.308 | 184.987 | 19.688 | 1.00 | 15.70 | A |
| ATOM C | 704 | CA | TYR | A | 207 | 187.866 | 183.692 | 20.185 | 1.00 | 14.78 | A |
| ATOM C | 705 | CB | TYR | A | 207 | 187.555 | 183.754 | 21.686 | 1.00 | 14.30 | A |
| ATOM C | 706 | CG | TYR | A | 207 | 188.599 | 184.415 | 22.538 | 1.00 | 15.34 | A |
| ATOM C | 707 | CD1 | TYR | A | 207 | 188.725 | 185.809 | 22.565 | 1.00 | 15.56 | A |
| ATOM C | 708 | CE1 | TYR | A | 207 | 189.669 | 186.433 | 23.389 | 1.00 | 14.72 | A |
| ATOM C | 709 | CD2 | TYR | A | 207 | 189.445 | 183.655 | 23.347 | 1.00 | 13.39 | A |
| ATOM C | 710 | CE2 | TYR | A | 207 | 190.393 | 184.263 | 24.174 | 1.00 | 15.70 | A |
| ATOM C | 711 | CZ | TYR | A | 207 | 190.497 | 185.652 | 24.192 | 1.00 | 16.96 | A |
| ATOM O | 712 | OH | TYR | A | 207 | 191.413 | 186.252 | 25.028 | 1.00 | 17.44 | A |
| ATOM C | 713 | C | TYR | A | 207 | 186.624 | 183.132 | 19.506 | 1.00 | 14.64 | A |
| ATOM O | 714 | O | TYR | A | 207 | 185.604 | 183.807 | 19.390 | 1.00 | 14.86 | A |
| ATOM N | 715 | N | LEU | A | 208 | 186.716 | 181.882 | 19.082 | 1.00 | 12.98 | A |
| ATOM C | 716 | CA | LEU | A | 208 | 185.597 | 181.206 | 18.475 | 1.00 | 11.35 | A |
| ATOM C | 717 | CB | LEU | A | 208 | 186.025 | 180.515 | 17.181 | 1.00 | 12.38 | A |
| ATOM C | 718 | CG | LEU | A | 208 | 186.790 | 181.296 | 16.101 | 1.00 | 18.53 | A |
| ATOM C | 719 | CD1 | LEU | A | 208 | 186.415 | 180.695 | 14.759 | 1.00 | 13.01 | A |
| ATOM C | 720 | CD2 | LEU | A | 208 | 186.466 | 182.794 | 16.118 | 1.00 | 17.18 | A |
| ATOM C | 721 | C | LEU | A | 208 | 185.092 | 180.162 | 19.473 | 1.00 | 11.42 | A |
| ATOM O | 722 | O | LEU | A | 208 | 185.875 | 179.354 | 19.978 | 1.00 | 9.69 | A |
| ATOM N | 723 | N | ILE | A | 209 | 183.789 | 180.194 | 19.753 | 1.00 | 11.10 | A |
| ATOM C | 724 | CA | ILE | A | 209 | 183.146 | 179.268 | 20.670 | 1.00 | 10.69 | A |
| ATOM C | 725 | CB | ILE | A | 209 | 181.968 | 179.962 | 21.389 | 1.00 | 13.97 | A |
| ATOM C | 726 | CG2 | ILE | A | 209 | 181.316 | 178.980 | 22.422 | 1.00 | 10.28 | A |
| ATOM C | 727 | CG1 | ILE | A | 209 | 182.489 | 181.303 | 21.979 | 1.00 | 14.77 | A |
| ATOM C | 728 | CD1 | ILE | A | 209 | 181.466 | 182.209 | 22.621 | 1.00 | 10.27 | A |
| ATOM C | 729 | C | ILE | A | 209 | 182.659 | 178.124 | 19.785 | 1.00 | 13.02 | A |
| ATOM O | 730 | O | ILE | A | 209 | 181.689 | 178.238 | 19.040 | 1.00 | 13.94 | A |
| ATOM N | 731 | N | LEU | A | 210 | 183.373 | 177.019 | 19.856 | 1.00 | 12.66 | A |
| ATOM C | 732 | CA | LEU | A | 210 | 183.065 | 175.867 | 19.044 | 1.00 | 13.21 | A |
| ATOM C | 733 | CB | LEU | A | 210 | 184.348 | 175.383 | 18.375 | 1.00 | 13.93 | A |
| ATOM C | 734 | CG | LEU | A | 210 | 185.046 | 176.317 | 17.381 | 1.00 | 16.57 | A |
| ATOM C | 735 | CD1 | LEU | A | 210 | 186.507 | 175.915 | 17.264 | 1.00 | 14.44 | A |
| ATOM C | 736 | CD2 | LEU | A | 210 | 184.351 | 176.245 | 16.022 | 1.00 | 12.75 | A |
| ATOM C | 737 | C | LEU | A | 210 | 182.464 | 174.731 | 19.841 | 1.00 | 10.33 | A |
| ATOM O | 738 | O | LEU | A | 210 | 182.591 | 174.664 | 21.053 | 1.00 | 14.25 | A |
| ATOM N | 739 | N | GLU | A | 211 | 181.784 | 173.843 | 19.139 | 1.00 | 11.52 | A |
| ATOM C | 740 | CA | GLU | A | 211 | 181.218 | 172.646 | 19.739 | 1.00 | 12.06 | A |
| ATOM C | 741 | CB | GLU | A | 211 | 180.272 | 171.970 | 18.751 | 1.00 | 15.25 | A |
| ATOM C | 742 | CG | GLU | A | 211 | 180.147 | 170.462 | 18.927 | 1.00 | 16.63 | A |
| ATOM C | 743 | CD | GLU | A | 211 | 179.379 | 169.783 | 17.784 | 1.00 | 23.31 | A |
| ATOM O | 744 | OE1 | GLU | A | 211 | 179.201 | 168.531 | 17.837 | 1.00 | 22.59 | A |
| ATOM O | 745 | OE2 | GLU | A | 211 | 178.954 | 170.500 | 16.829 | 1.00 | 22.15 | A |
| ATOM C | 746 | C | GLU | A | 211 | 182.424 | 171.723 | 20.009 | 1.00 | 14.58 | A |
| ATOM O | 747 | O | GLU | A | 211 | 183.296 | 171.561 | 19.156 | 1.00 | 11.41 | A |
| ATOM N | 748 | N | TYR | A | 212 | 182.466 | 171.133 | 21.196 | 1.00 | 13.40 | A |
| ATOM C | 749 | CA | TYR | A | 212 | 183.555 | 170.247 | 21.589 | 1.00 | 15.01 | A |
| ATOM C | 750 | CB | TYR | A | 212 | 183.541 | 170.072 | 23.108 | 1.00 | 16.32 | A |
| ATOM C | 751 | CG | TYR | A | 212 | 184.387 | 168.938 | 23.679 | 1.00 | 18.16 | A |
| ATOM C | 752 | CD1 | TYR | A | 212 | 185.744 | 168.820 | 23.391 | 1.00 | 15.83 | A |
| ATOM C | 753 | CE1 | TYR | A | 212 | 186.528 | 167.845 | 24.013 | 1.00 | 16.35 | A |
| ATOM C | 754 | CD2 | TYR | A | 212 | 183.829 | 168.042 | 24.601 | 1.00 | 18.49 | A |
| ATOM C | 755 | CE2 | TYR | A | 212 | 184.590 | 167.074 | 25.223 | 1.00 | 16.40 | A |
| ATOM C | 756 | CZ | TYR | A | 212 | 185.934 | 166.973 | 24.934 | 1.00 | 19.22 | A |
| ATOM O | 757 | OH | TYR | A | 212 | 186.655 | 165.995 | 25.594 | 1.00 | 15.81 | A |
| ATOM C | 758 | C | TYR | A | 212 | 183.463 | 168.889 | 20.909 | 1.00 | 15.14 | A |
| ATOM O | 759 | O | TYR | A | 212 | 182.414 | 168.259 | 20.920 | 1.00 | 14.66 | A |
| ATOM N | 760 | N | ALA | A | 213 | 184.561 | 168.451 | 20.297 | 1.00 | 15.74 | A |
| ATOM C | 761 | CA | ALA | A | 213 | 184.623 | 167.142 | 19.636 | 1.00 | 14.66 | A |
| ATOM C | 762 | CB | ALA | A | 213 | 185.341 | 167.270 | 18.302 | 1.00 | 16.59 | A |
| ATOM C | 763 | C | ALA | A | 213 | 185.417 | 166.270 | 20.615 | 1.00 | 16.61 | A |
| ATOM O | 764 | O | ALA | A | 213 | 186.638 | 166.352 | 20.682 | 1.00 | 16.21 | A |
| ATOM N | 765 | N | PRO | A | 214 | 184.724 | 165.418 | 21.380 | 1.00 | 15.97 | A |
| ATOM C | 766 | CD | PRO | A | 214 | 183.323 | 165.077 | 21.082 | 1.00 | 15.58 | A |
| ATOM C | 767 | CA | PRO | A | 214 | 185.258 | 164.515 | 22.398 | 1.00 | 15.31 | A |
| ATOM C | 768 | CB | PRO | A | 214 | 184.000 | 163.837 | 22.963 | 1.00 | 14.60 | A |
| ATOM C | 769 | CG | PRO | A | 214 | 182.844 | 164.624 | 22.417 | 1.00 | 17.26 | A |
| ATOM C | 770 | C | PRO | A | 214 | 186.319 | 163.472 | 22.000 | 1.00 | 17.90 | A |
| ATOM O | 771 | O | PRO | A | 214 | 187.172 | 163.152 | 22.829 | 1.00 | 18.00 | A |
| ATOM N | 772 | N | LEU | A | 215 | 186.274 | 162.922 | 20.777 | 1.00 | 14.95 | A |
| ATOM C | 773 | CA | LEU | A | 215 | 187.214 | 161.880 | 20.362 | 1.00 | 14.66 | A |
| ATOM C | 774 | CB | LEU | A | 215 | 186.453 | 160.710 | 19.692 | 1.00 | 16.00 | A |
| ATOM C | 775 | CG | LEU | A | 215 | 185.316 | 160.072 | 20.536 | 1.00 | 22.00 | A |
| ATOM C | 776 | CD1 | LEU | A | 215 | 184.664 | 158.818 | 19.898 | 1.00 | 17.85 | A |
| ATOM C | 777 | CD2 | LEU | A | 215 | 185.906 | 159.717 | 21.877 | 1.00 | 20.55 | A |
| ATOM C | 778 | C | LEU | A | 215 | 188.415 | 162.306 | 19.505 | 1.00 | 14.59 | A |
| ATOM O | 779 | O | LEU | A | 215 | 189.093 | 161.477 | 18.905 | 1.00 | 14.29 | A |
| ATOM N | 780 | N | GLY | A | 216 | 188.691 | 163.603 | 19.455 | 1.00 | 16.74 | A |
| ATOM C | 781 | CA | GLY | A | 216 | 189.836 | 164.070 | 18.694 | 1.00 | 14.83 | A |
| ATOM C | 782 | C | GLY | A | 216 | 189.701 | 164.060 | 17.184 | 1.00 | 13.54 | A |
| ATOM O | 783 | O | GLY | A | 216 | 188.597 | 164.008 | 16.656 | 1.00 | 12.48 | A |
| ATOM N | 784 | N | THR | A | 217 | 190.843 | 164.091 | 16.497 | 1.00 | 12.97 | A |
| ATOM C | 785 | CA | THR | A | 217 | 190.867 | 164.136 | 15.044 | 1.00 | 14.08 | A |
| ATOM C | 786 | CB | THR | A | 217 | 192.005 | 164.998 | 14.503 | 1.00 | 14.49 | A |
| ATOM O | 787 | OG1 | THR | A | 217 | 193.242 | 164.310 | 14.733 | 1.00 | 16.68 | A |
| ATOM C | 788 | CG2 | THR | A | 217 | 192.043 | 166.347 | 15.167 | 1.00 | 10.84 | A |
| ATOM C | 789 | C | THR | A | 217 | 191.060 | 162.811 | 14.347 | 1.00 | 16.46 | A |
| ATOM O | 790 | O | THR | A | 217 | 191.741 | 161.910 | 14.849 | 1.00 | 15.02 | A |
| ATOM N | 791 | N | VAL | A | 218 | 190.481 | 162.721 | 13.153 | 1.00 | 16.27 | A |
| ATOM C | 792 | CA | VAL | A | 218 | 190.616 | 161.533 | 12.340 | 1.00 | 15.99 | A |
| ATOM C | 793 | CB | VAL | A | 218 | 189.802 | 161.683 | 11.038 | 1.00 | 16.57 | A |
| ATOM C | 794 | CG1 | VAL | A | 218 | 190.263 | 160.649 | 9.999 | 1.00 | 18.56 | A |
| ATOM C | 795 | CG2 | VAL | A | 218 | 188.339 | 161.504 | 11.349 | 1.00 | 11.27 | A |
| ATOM C | 796 | C | VAL | A | 218 | 192.117 | 161.366 | 12.051 | 1.00 | 16.41 | A |
| ATOM O | 797 | O | VAL | A | 218 | 192.611 | 160.255 | 11.896 | 1.00 | 15.84 | A |
| ATOM N | 798 | N | TYR | A | 219 | 192.826 | 162.487 | 12.008 | 1.00 | 15.85 | A |
| ATOM C | 799 | CA | TYR | A | 219 | 194.261 | 162.509 | 11.789 | 1.00 | 16.76 | A |
| ATOM C | 800 | CB | TYR | A | 219 | 194.771 | 163.937 | 11.908 | 1.00 | 18.52 | A |
| ATOM C | 801 | CG | TYR | A | 219 | 196.259 | 164.024 | 11.800 | 1.00 | 19.33 | A |
| ATOM C | 802 | CD1 | TYR | A | 219 | 196.870 | 163.870 | 10.570 | 1.00 | 17.55 | A |
| ATOM C | 803 | CE1 | TYR | A | 219 | 198.249 | 163.929 | 10.450 | 1.00 | 23.59 | A |
| ATOM C | 804 | CD2 | TYR | A | 219 | 197.065 | 164.236 | 12.936 | 1.00 | 21.27 | A |
| ATOM C | 805 | CE2 | TYR | A | 219 | 198.454 | 164.289 | 12.834 | 1.00 | 20.92 | A |
| ATOM C | 806 | CZ | TYR | A | 219 | 199.032 | 164.136 | 11.584 | 1.00 | 24.53 | A |
| ATOM O | 807 | OH | TYR | A | 219 | 200.392 | 164.188 | 11.448 | 1.00 | 28.22 | A |
| ATOM C | 808 | C | TYR | A | 219 | 194.999 | 161.661 | 12.826 | 1.00 | 18.49 | A |
| ATOM O | 809 | O | TYR | A | 219 | 195.910 | 160.888 | 12.494 | 1.00 | 18.32 | A |
| ATOM N | 810 | N | ARG | A | 220 | 194.617 | 161.836 | 14.086 | 1.00 | 16.66 | A |
| ATOM C | 811 | CA | ARG | A | 220 | 195.247 | 161.100 | 15.154 | 1.00 | 19.76 | A |
| ATOM C | 812 | CB | ARG | A | 220 | 194.848 | 161.674 | 16.514 | 1.00 | 22.26 | A |
| ATOM C | 813 | CG | ARG | A | 220 | 195.514 | 160.949 | 17.669 | 1.00 | 27.26 | A |
| ATOM C | 814 | CD | ARG | A | 220 | 197.039 | 161.124 | 17.660 | 1.00 | 29.11 | A |
| ATOM N | 815 | NE | ARG | A | 220 | 197.689 | 160.200 | 18.595 | 1.00 | 32.84 | A |
| ATOM C | 816 | CZ | ARG | A | 220 | 199.003 | 160.005 | 18.702 | 1.00 | 35.53 | A |
| ATOM N | 817 | NH1 | ARG | A | 220 | 199.867 | 160.669 | 17.931 | 1.00 | 39.53 | A |
| ATOM N | 818 | NH2 | ARG | A | 220 | 199.449 | 159.129 | 19.594 | 1.00 | 36.11 | A |
| ATOM C | 819 | C | ARG | A | 220 | 194.908 | 159.608 | 15.088 | 1.00 | 19.85 | A |
| ATOM O | 820 | O | ARG | A | 220 | 195.768 | 158.748 | 15.322 | 1.00 | 19.84 | A |
| ATOM N | 821 | N | GLU | A | 221 | 193.661 | 159.309 | 14.746 | 1.00 | 19.44 | A |
| ATOM C | 822 | CA | GLU | A | 221 | 193.192 | 157.935 | 14.628 | 1.00 | 21.14 | A |
| ATOM C | 823 | CB | GLU | A | 221 | 191.670 | 157.930 | 14.386 | 1.00 | 22.94 | A |
| ATOM C | 824 | CG | GLU | A | 221 | 190.968 | 156.594 | 14.519 | 1.00 | 27.24 | A |
| ATOM C | 825 | CD | GLU | A | 221 | 191.078 | 155.998 | 15.918 | 1.00 | 30.92 | A |
| ATOM O | 826 | OE1 | GLU | A | 221 | 190.736 | 154.798 | 16.073 | 1.00 | 35.57 | A |
| ATOM O | 827 | OE2 | GLU | A | 221 | 191.502 | 156.716 | 16.857 | 1.00 | 31.40 | A |
| ATOM C | 828 | C | GLU | A | 221 | 193.930 | 157.268 | 13.465 | 1.00 | 22.03 | A |
| ATOM O | 829 | O | GLU | A | 221 | 194.201 | 156.061 | 13.493 | 1.00 | 21.79 | A |
| ATOM N | 830 | N | LEU | A | 222 | 194.257 | 158.060 | 12.442 | 1.00 | 20.91 | A |
| ATOM C | 831 | CA | LEU | A | 222 | 194.969 | 157.533 | 11.287 | 1.00 | 18.01 | A |
| ATOM C | 832 | CB | LEU | A | 222 | 194.838 | 158.485 | 10.079 | 1.00 | 17.83 | A |
| ATOM C | 833 | CG | LEU | A | 222 | 195.181 | 157.923 | 8.676 | 1.00 | 21.87 | A |
| ATOM C | 834 | CD1 | LEU | A | 222 | 194.366 | 156.652 | 8.358 | 1.00 | 17.44 | A |
| ATOM C | 835 | CD2 | LEU | A | 222 | 194.892 | 159.007 | 7.634 | 1.00 | 19.79 | A |
| ATOM C | 836 | C | LEU | A | 222 | 196.439 | 157.261 | 11.639 | 1.00 | 16.91 | A |
| ATOM O | 837 | O | LEU | A | 222 | 197.012 | 156.317 | 11.119 | 1.00 | 16.89 | A |
| ATOM N | 838 | N | GLN | A | 223 | 197.062 | 158.066 | 12.503 | 1.00 | 16.66 | A |
| ATOM C | 839 | CA | GLN | A | 223 | 198.446 | 157.769 | 12.900 | 1.00 | 17.24 | A |
| ATOM C | 840 | CB | GLN | A | 223 | 199.022 | 158.808 | 13.850 | 1.00 | 19.18 | A |
| ATOM C | 841 | CG | GLN | A | 223 | 199.250 | 160.205 | 13.317 | 1.00 | 28.34 | A |
| ATOM C | 842 | CD | GLN | A | 223 | 199.954 | 161.058 | 14.355 | 1.00 | 31.88 | A |
| ATOM O | 843 | OE1 | GLN | A | 223 | 199.437 | 161.241 | 15.474 | 1.00 | 31.86 | A |
| ATOM N | 844 | NE2 | GLN | A | 223 | 201.149 | 161.584 | 14.005 | 1.00 | 34.43 | A |
| ATOM C | 845 | C | GLN | A | 223 | 198.456 | 156.448 | 13.688 | 1.00 | 21.01 | A |
| ATOM O | 846 | O | GLN | A | 223 | 199.360 | 155.630 | 13.523 | 1.00 | 18.77 | A |
| ATOM N | 847 | N | LYS | A | 224 | 197.464 | 156.277 | 14.570 | 1.00 | 21.30 | A |
| ATOM C | 848 | CA | LYS | A | 224 | 197.357 | 155.099 | 15.419 | 1.00 | 21.07 | A |
| ATOM C | 849 | CB | LYS | A | 224 | 196.258 | 155.300 | 16.492 | 1.00 | 24.80 | A |
| ATOM C | 850 | CG | LYS | A | 224 | 196.479 | 156.522 | 17.399 | 1.00 | 26.43 | A |
| ATOM C | 851 | CD | LYS | A | 224 | 195.427 | 156.642 | 18.505 | 1.00 | 30.49 | A |
| ATOM C | 852 | CE | LYS | A | 224 | 195.834 | 157.727 | 19.507 | 1.00 | 31.44 | A |
| ATOM N | 853 | NZ | LYS | A | 224 | 194.917 | 157.868 | 20.668 | 1.00 | 33.32 | A |
| ATOM C | 854 | C | LYS | A | 224 | 197.088 | 153.812 | 14.647 | 1.00 | 20.29 | A |
| ATOM O | 855 | O | LYS | A | 224 | 197.799 | 152.829 | 14.821 | 1.00 | 19.90 | A |
| ATOM N | 856 | N | LEU | A | 225 | 196.059 | 153.800 | 13.806 | 1.00 | 20.45 | A |
| ATOM C | 857 | CA | LEU | A | 225 | 195.753 | 152.603 | 13.039 | 1.00 | 18.46 | A |
| ATOM C | 858 | CB | LEU | A | 225 | 194.272 | 152.588 | 12.615 | 1.00 | 20.03 | A |
| ATOM C | 859 | CG | LEU | A | 225 | 193.224 | 152.691 | 13.740 | 1.00 | 24.89 | A |
| ATOM C | 860 | CD1 | LEU | A | 225 | 191.784 | 152.656 | 13.213 | 1.00 | 20.57 | A |
| ATOM C | 861 | CD2 | LEU | A | 225 | 193.462 | 151.543 | 14.674 | 1.00 | 26.58 | A |
| ATOM C | 862 | C | LEU | A | 225 | 196.631 | 152.464 | 11.780 | 1.00 | 19.83 | A |
| ATOM O | 863 | O | LEU | A | 225 | 196.694 | 151.381 | 11.186 | 1.00 | 20.09 | A |
| ATOM N | 864 | N | SER | A | 226 | 197.296 | 153.545 | 11.375 | 1.00 | 18.33 | A |
| ATOM C | 865 | CA | SER | A | 226 | 198.137 | 153.578 | 10.158 | 1.00 | 21.51 | A |
| ATOM C | 866 | CB | SER | A | 226 | 199.160 | 152.426 | 10.124 | 1.00 | 21.48 | A |
| ATOM O | 867 | OG | SER | A | 226 | 200.116 | 152.533 | 11.177 | 1.00 | 27.63 | A |
| ATOM C | 868 | C | SER | A | 226 | 197.295 | 153.542 | 8.871 | 1.00 | 20.48 | A |
| ATOM O | 869 | O | SER | A | 226 | 197.566 | 154.284 | 7.927 | 1.00 | 19.41 | A |
| ATOM N | 870 | N | LYS | A | 227 | 196.290 | 152.664 | 8.838 | 1.00 | 20.02 | A |
| ATOM C | 871 | CA | LYS | A | 227 | 195.358 | 152.524 | 7.697 | 1.00 | 19.86 | A |
| ATOM C | 872 | CB | LYS | A | 227 | 195.668 | 151.280 | 6.876 | 1.00 | 24.72 | A |
| ATOM C | 873 | CG | LYS | A | 227 | 196.854 | 151.364 | 5.990 | 1.00 | 29.64 | A |
| ATOM C | 874 | CD | LYS | A | 227 | 197.148 | 149.990 | 5.419 | 1.00 | 35.21 | A |
| ATOM C | 875 | CE | LYS | A | 227 | 198.330 | 150.088 | 4.465 | 1.00 | 41.32 | A |
| ATOM N | 876 | NZ | LYS | A | 227 | 198.843 | 148.758 | 4.016 | 1.00 | 43.82 | A |
| ATOM C | 877 | C | LYS | A | 227 | 193.970 | 152.303 | 8.274 | 1.00 | 16.62 | A |
| ATOM O | 878 | O | LYS | A | 227 | 193.852 | 151.881 | 9.411 | 1.00 | 18.80 | A |
| ATOM N | 879 | N | PHE | A | 228 | 192.930 | 152.584 | 7.499 | 1.00 | 14.54 | A |
| ATOM C | 880 | CA | PHE | A | 228 | 191.548 | 152.342 | 7.932 | 1.00 | 15.70 | A |
| ATOM C | 881 | CB | PHE | A | 228 | 190.665 | 153.590 | 7.729 | 1.00 | 12.09 | A |
| ATOM C | 882 | CG | PHE | A | 228 | 190.958 | 154.697 | 8.679 | 1.00 | 13.97 | A |
| ATOM C | 883 | CD1 | PHE | A | 228 | 190.564 | 156.005 | 8.400 | 1.00 | 14.03 | A |
| ATOM C | 884 | CD2 | PHE | A | 228 | 191.629 | 154.439 | 9.860 | 1.00 | 13.49 | A |
| ATOM C | 885 | CE1 | PHE | A | 228 | 190.844 | 157.046 | 9.299 | 1.00 | 12.37 | A |
| ATOM C | 886 | CE2 | PHE | A | 228 | 191.912 | 155.466 | 10.767 | 1.00 | 14.79 | A |
| ATOM C | 887 | CZ | PHE | A | 228 | 191.520 | 156.772 | 10.487 | 1.00 | 11.50 | A |
| ATOM C | 888 | C | PHE | A | 228 | 191.051 | 151.227 | 7.016 | 1.00 | 15.70 | A |
| ATOM O | 889 | O | PHE | A | 228 | 191.399 | 151.212 | 5.833 | 1.00 | 16.89 | A |
| ATOM N | 890 | N | ASP | A | 229 | 190.261 | 150.290 | 7.536 | 1.00 | 16.61 | A |
| ATOM C | 891 | CA | ASP | A | 229 | 189.753 | 149.221 | 6.666 | 1.00 | 18.23 | A |
| ATOM C | 892 | CB | ASP | A | 229 | 189.261 | 148.005 | 7.478 | 1.00 | 17.42 | A |
| ATOM C | 893 | CG | ASP | A | 229 | 188.092 | 148.325 | 8.369 | 1.00 | 20.41 | A |
| ATOM O | 894 | OD1 | ASP | A | 229 | 187.161 | 149.024 | 7.930 | 1.00 | 28.20 | A |
| ATOM O | 895 | OD2 | ASP | A | 229 | 188.072 | 147.859 | 9.511 | 1.00 | 26.24 | A |
| ATOM C | 896 | C | ASP | A | 229 | 188.624 | 149.817 | 5.810 | 1.00 | 16.57 | A |
| ATOM O | 897 | O | ASP | A | 229 | 188.322 | 151.006 | 5.939 | 1.00 | 17.37 | A |
| ATOM N | 898 | N | GLU | A | 230 | 187.998 | 149.020 | 4.952 | 1.00 | 15.33 | A |
| ATOM C | 899 | CA | GLU | A | 230 | 186.947 | 149.560 | 4.084 | 1.00 | 17.43 | A |
| ATOM C | 900 | CB | GLU | A | 230 | 186.571 | 148.541 | 3.002 | 1.00 | 16.24 | A |
| ATOM C | 901 | CG | GLU | A | 230 | 187.743 | 148.066 | 2.153 | 1.00 | 23.01 | A |
| ATOM C | 902 | CD | GLU | A | 230 | 187.313 | 147.497 | 0.794 | 1.00 | 27.22 | A |
| ATOM O | 903 | OE1 | GLU | A | 230 | 186.584 | 146.478 | 0.753 | 1.00 | 28.49 | A |
| ATOM O | 904 | OE2 | GLU | A | 230 | 187.711 | 148.085 | -0.244 | 1.00 | 29.45 | A |
| ATOM C | 905 | C | GLU | A | 230 | 185.680 | 150.062 | 4.783 | 1.00 | 16.70 | A |
| ATOM O | 906 | O | GLU | A | 230 | 185.141 | 151.099 | 4.419 | 1.00 | 13.33 | A |
| ATOM N | 907 | N | GLN | A | 231 | 185.209 | 149.326 | 5.782 | 1.00 | 16.53 | A |
| ATOM C | 908 | CA | GLN | A | 231 | 184.003 | 149.711 | 6.512 | 1.00 | 17.02 | A |
| ATOM C | 909 | CB | GLN | A | 231 | 183.728 | 148.656 | 7.596 | 1.00 | 15.32 | A |
| ATOM C | 910 | CG | GLN | A | 231 | 182.370 | 148.737 | 8.289 | 1.00 | 17.56 | A |
| ATOM C | 911 | CD | GLN | A | 231 | 182.297 | 149.841 | 9.359 | 1.00 | 22.59 | A |
| ATOM O | 912 | OE1 | GLN | A | 231 | 183.298 | 150.112 | 10.075 | 1.00 | 21.11 | A |
| ATOM N | 913 | NE2 | GLN | A | 231 | 181.106 | 150.478 | 9.494 | 1.00 | 19.70 | A |
| ATOM C | 914 | C | GLN | A | 231 | 184.195 | 151.124 | 7.120 | 1.00 | 17.00 | A |
| ATOM O | 915 | O | GLN | A | 231 | 183.381 | 152.024 | 6.890 | 1.00 | 18.40 | A |
| ATOM N | 916 | N | ARG | A | 232 | 185.281 | 151.308 | 7.873 | 1.00 | 17.36 | A |
| ATOM C | 917 | CA | ARG | A | 232 | 185.607 | 152.593 | 8.509 | 1.00 | 17.63 | A |
| ATOM C | 918 | CB | ARG | A | 232 | 186.905 | 152.465 | 9.307 | 1.00 | 19.87 | A |
| ATOM C | 919 | CG | ARG | A | 232 | 187.205 | 153.647 | 10.172 | 1.00 | 20.99 | A |
| ATOM C | 920 | CD | ARG | A | 232 | 186.795 | 153.359 | 11.597 | 1.00 | 28.38 | A |
| ATOM N | 921 | NE | ARG | A | 232 | 187.588 | 154.189 | 12.487 | 1.00 | 32.23 | A |
| ATOM C | 922 | CZ | ARG | A | 232 | 188.247 | 153.745 | 13.545 | 1.00 | 31.41 | A |
| ATOM N | 923 | NH1 | ARG | A | 232 | 188.213 | 152.459 | 13.873 | 1.00 | 29.13 | A |
| ATOM N | 924 | NH2 | ARG | A | 232 | 188.963 | 154.604 | 14.257 | 1.00 | 35.98 | A |
| ATOM C | 925 | C | ARG | A | 232 | 185.770 | 153.747 | 7.507 | 1.00 | 14.94 | A |
| ATOM O | 926 | O | ARG | A | 232 | 185.356 | 154.866 | 7.761 | 1.00 | 12.98 | A |
| ATOM N | 927 | N | THR | A | 233 | 186.403 | 153.457 | 6.384 | 1.00 | 13.16 | A |
| ATOM C | 928 | CA | THR | A | 233 | 186.652 | 154.446 | 5.361 | 1.00 | 13.60 | A |
| ATOM C | 929 | CB | THR | A | 233 | 187.719 | 153.902 | 4.340 | 1.00 | 14.55 | A |
| ATOM O | 930 | OG1 | THR | A | 233 | 188.937 | 153.623 | 5.048 | 1.00 | 14.87 | A |
| ATOM C | 931 | CG2 | THR | A | 233 | 188.016 | 154.908 | 3.231 | 1.00 | 13.20 | A |
| ATOM C | 932 | C | THR | A | 233 | 185.339 | 154.863 | 4.686 | 1.00 | 13.47 | A |
| ATOM O | 933 | O | THR | A | 233 | 185.090 | 156.056 | 4.508 | 1.00 | 12.81 | A |
| ATOM N | 934 | N | ALA | A | 234 | 184.481 | 153.901 | 4.353 | 1.00 | 11.54 | A |
| ATOM C | 935 | CA | ALA | A | 234 | 183.199 | 154.225 | 3.713 | 1.00 | 12.90 | A |
| ATOM C | 936 | CB | ALA | A | 234 | 182.531 | 152.967 | 3.184 | 1.00 | 6.31 | A |
| ATOM C | 937 | C | ALA | A | 234 | 182.254 | 154.953 | 4.675 | 1.00 | 15.13 | A |
| ATOM O | 938 | O | ALA | A | 234 | 181.498 | 155.843 | 4.272 | 1.00 | 18.25 | A |
| ATOM N | 939 | N | THR | A | 235 | 182.297 | 154.579 | 5.945 | 1.00 | 13.08 | A |
| ATOM C | 940 | CA | THR | A | 235 | 181.450 | 155.221 | 6.909 | 1.00 | 11.34 | A |
| ATOM C | 941 | CB | THR | A | 235 | 181.581 | 154.543 | 8.289 | 1.00 | 13.04 | A |
| ATOM O | 942 | OG1 | THR | A | 235 | 181.181 | 153.167 | 8.177 | 1.00 | 13.03 | A |
| ATOM C | 943 | CG2 | THR | A | 235 | 180.679 | 155.235 | 9.304 | 1.00 | 9.30 | A |
| ATOM C | 944 | C | THR | A | 235 | 181.813 | 156.701 | 6.976 | 1.00 | 12.76 | A |
| ATOM O | 945 | O | THR | A | 235 | 180.930 | 157.553 | 6.894 | 1.00 | 14.21 | A |
| ATOM N | 946 | N | TYR | A | 236 | 183.104 | 157.003 | 7.115 | 1.00 | 12.83 | A |
| ATOM C | 947 | CA | TYR | A | 236 | 183.586 | 158.392 | 7.157 | 1.00 | 14.72 | A |
| ATOM C | 948 | CB | TYR | A | 236 | 185.096 | 158.451 | 7.371 | 1.00 | 16.17 | A |
| ATOM C | 949 | CG | TYR | A | 236 | 185.572 | 158.175 | 8.772 | 1.00 | 16.12 | A |
| ATOM C | 950 | CD1 | TYR | A | 236 | 184.785 | 158.491 | 9.876 | 1.00 | 16.03 | A |
| ATOM C | 951 | CE1 | TYR | A | 236 | 185.237 | 158.261 | 11.162 | 1.00 | 16.90 | A |
| ATOM C | 952 | CD2 | TYR | A | 236 | 186.830 | 157.626 | 8.993 | 1.00 | 13.79 | A |
| ATOM C | 953 | CE2 | TYR | A | 236 | 187.290 | 157.397 | 10.266 | 1.00 | 15.98 | A |
| ATOM C | 954 | CZ | TYR | A | 236 | 186.490 | 157.709 | 11.347 | 1.00 | 19.04 | A |
| ATOM O | 955 | OH | TYR | A | 236 | 186.935 | 157.420 | 12.609 | 1.00 | 22.33 | A |
| ATOM C | 956 | C | TYR | A | 236 | 183.291 | 159.154 | 5.862 | 1.00 | 16.50 | A |
| ATOM O | 957 | O | TYR | A | 236 | 182.873 | 160.311 | 5.898 | 1.00 | 15.39 | A |
| ATOM N | 958 | N | ILE | A | 237 | 183.536 | 158.517 | 4.718 | 1.00 | 14.99 | A |
| ATOM C | 959 | CA | ILE | A | 237 | 183.274 | 159.176 | 3.456 | 1.00 | 15.34 | A |
| ATOM C | 960 | CB | ILE | A | 237 | 183.656 | 158.295 | 2.242 | 1.00 | 15.72 | A |
| ATOM C | 961 | CG2 | ILE | A | 237 | 183.331 | 159.027 | 0.960 | 1.00 | 17.81 | A |
| ATOM C | 962 | CG1 | ILE | A | 237 | 185.157 | 157.993 | 2.241 | 1.00 | 13.11 | A |
| ATOM C | 963 | CD1 | ILE | A | 237 | 186.062 | 159.254 | 2.278 | 1.00 | 13.67 | A |
| ATOM C | 964 | C | ILE | A | 237 | 181.795 | 159.542 | 3.379 | 1.00 | 17.56 | A |
| ATOM O | 965 | O | ILE | A | 237 | 181.442 | 160.638 | 2.953 | 1.00 | 18.55 | A |
| ATOM N | 966 | N | THR | A | 238 | 180.935 | 158.626 | 3.804 | 1.00 | 17.36 | A |
| ATOM C | 967 | CA | THR | A | 238 | 179.489 | 158.853 | 3.816 | 1.00 | 14.99 | A |
| ATOM C | 968 | CB | THR | A | 238 | 178.760 | 157.600 | 4.330 | 1.00 | 13.08 | A |
| ATOM O | 969 | OG1 | THR | A | 238 | 179.059 | 156.502 | 3.464 | 1.00 | 16.26 | A |
| ATOM C | 970 | CG2 | THR | A | 238 | 177.257 | 157.817 | 4.354 | 1.00 | 13.58 | A |
| ATOM C | 971 | C | THR | A | 238 | 179.069 | 160.037 | 4.696 | 1.00 | 14.23 | A |
| ATOM O | 972 | O | THR | A | 238 | 178.214 | 160.829 | 4.307 | 1.00 | 14.27 | A |
| ATOM N | 973 | N | GLU | A | 239 | 179.649 | 160.149 | 5.888 | 1.00 | 11.63 | A |
| ATOM C | 974 | CA | GLU | A | 239 | 179.285 | 161.242 | 6.781 | 1.00 | 13.23 | A |
| ATOM C | 975 | CB | GLU | A | 239 | 179.913 | 161.025 | 8.170 | 1.00 | 14.00 | A |
| ATOM C | 976 | CG | GLU | A | 239 | 179.443 | 159.721 | 8.804 | 1.00 | 18.38 | A |
| ATOM C | 977 | CD | GLU | A | 239 | 180.121 | 159.365 | 10.124 | 1.00 | 24.00 | A |
| ATOM O | 978 | OE1 | GLU | A | 239 | 181.366 | 159.259 | 10.187 | 1.00 | 24.67 | A |
| ATOM O | 979 | OE2 | GLU | A | 239 | 179.385 | 159.158 | 11.114 | 1.00 | 32.52 | A |
| ATOM C | 980 | C | GLU | A | 239 | 179.767 | 162.548 | 6.149 | 1.00 | 13.27 | A |
| ATOM O | 981 | O | GLU | A | 239 | 179.028 | 163.525 | 6.073 | 1.00 | 12.45 | A |
| ATOM N | 982 | N | LEU | A | 240 | 181.005 | 162.538 | 5.668 | 1.00 | 11.05 | A |
| ATOM C | 983 | CA | LEU | A | 240 | 181.566 | 163.707 | 5.049 | 1.00 | 13.29 | A |
| ATOM C | 984 | CB | LEU | A | 240 | 183.043 | 163.465 | 4.738 | 1.00 | 15.79 | A |
| ATOM C | 985 | CG | LEU | A | 240 | 183.894 | 164.638 | 4.235 | 1.00 | 16.19 | A |
| ATOM C | 986 | CD1 | LEU | A | 240 | 184.031 | 165.720 | 5.279 | 1.00 | 19.17 | A |
| ATOM C | 987 | CD2 | LEU | A | 240 | 185.267 | 164.108 | 3.884 | 1.00 | 19.04 | A |
| ATOM C | 988 | C | LEU | A | 240 | 180.798 | 164.081 | 3.773 | 1.00 | 13.39 | A |
| ATOM O | 989 | O | LEU | A | 240 | 180.513 | 165.256 | 3.548 | 1.00 | 14.19 | A |
| ATOM N | 990 | N | ALA | A | 241 | 180.443 | 163.091 | 2.955 | 1.00 | 11.67 | A |
| ATOM C | 991 | CA | ALA | A | 241 | 179.726 | 163.372 | 1.711 | 1.00 | 12.50 | A |
| ATOM C | 992 | CB | ALA | A | 241 | 179.577 | 162.108 | 0.873 | 1.00 | 9.22 | A |
| ATOM C | 993 | C | ALA | A | 241 | 178.367 | 164.004 | 1.979 | 1.00 | 14.45 | A |
| ATOM O | 994 | O | ALA | A | 241 | 177.923 | 164.878 | 1.222 | 1.00 | 14.36 | A |
| ATOM N | 995 | N | ASN | A | 242 | 177.714 | 163.562 | 3.053 | 1.00 | 14.82 | A |
| ATOM C | 996 | CA | ASN | A | 242 | 176.420 | 164.109 | 3.447 | 1.00 | 16.37 | A |
| ATOM C | 997 | CB | ASN | A | 242 | 175.822 | 163.329 | 4.619 | 1.00 | 20.05 | A |
| ATOM C | 998 | CG | ASN | A | 242 | 175.213 | 162.005 | 4.210 | 1.00 | 22.16 | A |
| ATOM O | 999 | OD1 | ASN | A | 242 | 175.027 | 161.133 | 5.055 | 1.00 | 23.63 | A |
| ATOM N | 1000 | ND2 | ASN | A | 242 | 174.883 | 161.850 | 2.922 | 1.00 | 24.84 | A |
| ATOM C | 1001 | C | ASN | A | 242 | 176.623 | 165.540 | 3.918 | 1.00 | 16.87 | A |
| ATOM O | 1002 | O | ASN | A | 242 | 175.804 | 166.429 | 3.636 | 1.00 | 13.50 | A |
| ATOM N | 1003 | N | ALA | A | 243 | 177.713 | 165.747 | 4.656 | 1.00 | 13.07 | A |
| ATOM C | 1004 | CA | ALA | A | 243 | 178.019 | 167.065 | 5.190 | 1.00 | 13.28 | A |
| ATOM C | 1005 | CB | ALA | A | 243 | 179.204 | 166.982 | 6.137 | 1.00 | 13.95 | A |
| ATOM C | 1006 | C | ALA | A | 243 | 178.292 | 168.063 | 4.072 | 1.00 | 14.76 | A |
| ATOM O | 1007 | O | ALA | A | 243 | 177.751 | 169.173 | 4.080 | 1.00 | 11.54 | A |
| ATOM N | 1008 | N | LEU | A | 244 | 179.126 | 167.651 | 3.113 | 1.00 | 15.34 | A |
| ATOM C | 1009 | CA | LEU | A | 244 | 179.487 | 168.477 | 1.955 | 1.00 | 14.00 | A |
| ATOM C | 1010 | CB | LEU | A | 244 | 180.572 | 167.766 | 1.134 | 1.00 | 12.82 | A |
| ATOM C | 1011 | CG | LEU | A | 244 | 181.958 | 167.607 | 1.791 | 1.00 | 13.66 | A |
| ATOM C | 1012 | CD1 | LEU | A | 244 | 182.862 | 166.784 | 0.883 | 1.00 | 10.60 | A |
| ATOM C | 1013 | CD2 | LEU | A | 244 | 182.570 | 168.990 | 2.068 | 1.00 | 10.59 | A |
| ATOM C | 1014 | C | LEU | A | 244 | 178.245 | 168.764 | 1.078 | 1.00 | 13.84 | A |
| ATOM O | 1015 | O | LEU | A | 244 | 178.079 | 169.862 | 0.545 | 1.00 | 9.94 | A |
| ATOM N | 1016 | N | SER | A | 245 | 177.363 | 167.776 | 0.949 | 1.00 | 11.65 | A |
| ATOM C | 1017 | CA | SER | A | 245 | 176.162 | 167.951 | 0.149 | 1.00 | 12.45 | A |
| ATOM C | 1018 | CB | SER | A | 245 | 175.403 | 166.623 | 0.101 | 1.00 | 11.63 | A |
| ATOM O | 1019 | OG | SER | A | 245 | 174.265 | 166.724 | -0.729 | 1.00 | 18.58 | A |
| ATOM C | 1020 | C | SER | A | 245 | 175.284 | 169.090 | 0.716 | 1.00 | 12.58 | A |
| ATOM O | 1021 | O | SER | A | 245 | 174.753 | 169.925 | -0.028 | 1.00 | 13.38 | A |
| ATOM N | 1022 | N | TYR | A | 246 | 175.156 | 169.126 | 2.038 | 1.00 | 10.82 | A |
| ATOM C | 1023 | CA | TYR | A | 246 | 174.388 | 170.146 | 2.719 | 1.00 | 12.87 | A |
| ATOM C | 1024 | CB | TYR | A | 246 | 174.337 | 169.838 | 4.232 | 1.00 | 10.92 | A |
| ATOM C | 1025 | CG | TYR | A | 246 | 173.941 | 171.010 | 5.076 | 1.00 | 13.12 | A |
| ATOM C | 1026 | CD1 | TYR | A | 246 | 172.615 | 171.398 | 5.169 | 1.00 | 11.79 | A |
| ATOM C | 1027 | CE1 | TYR | A | 246 | 172.249 | 172.526 | 5.886 | 1.00 | 15.75 | A |
| ATOM C | 1028 | CD2 | TYR | A | 246 | 174.905 | 171.782 | 5.727 | 1.00 | 14.34 | A |
| ATOM C | 1029 | CE2 | TYR | A | 246 | 174.548 | 172.925 | 6.452 | 1.00 | 17.65 | A |
| ATOM C | 1030 | CZ | TYR | A | 246 | 173.209 | 173.288 | 6.526 | 1.00 | 18.09 | A |
| ATOM O | 1031 | OH | TYR | A | 246 | 172.806 | 174.411 | 7.231 | 1.00 | 20.69 | A |
| ATOM C | 1032 | C | TYR | A | 246 | 175.068 | 171.506 | 2.474 | 1.00 | 14.69 | A |
| ATOM O | 1033 | O | TYR | A | 246 | 174.399 | 172.522 | 2.263 | 1.00 | 16.54 | A |
| ATOM N | 1034 | N | CYS | A | 247 | 176.398 | 171.506 | 2.526 | 1.00 | 12.79 | A |
| ATOM C | 1035 | CA | CYS | A | 247 | 177.217 | 172.695 | 2.312 | 1.00 | 15.14 | A |
| ATOM C | 1036 | CB | CYS | A | 247 | 178.697 | 172.339 | 2.453 | 1.00 | 18.30 | A |
| ATOM S | 1037 | SG | CYS | A | 247 | 179.389 | 172.397 | 4.097 | 1.00 | 20.02 | A |
| ATOM C | 1038 | C | CYS | A | 247 | 177.033 | 173.298 | 0.930 | 1.00 | 14.64 | A |
| ATOM O | 1039 | O | CYS | A | 247 | 176.944 | 174.517 | 0.765 | 1.00 | 13.32 | A |
| ATOM N | 1040 | N | HIS | A | 248 | 177.018 | 172.424 | -0.063 | 1.00 | 12.96 | A |
| ATOM C | 1041 | CA | HIS | A | 248 | 176.867 | 172.853 | -1.435 | 1.00 | 14.92 | A |
| ATOM C | 1042 | CB | HIS | A | 248 | 177.246 | 171.711 | -2.361 | 1.00 | 14.58 | A |
| ATOM C | 1043 | CG | HIS | A | 248 | 178.698 | 171.388 | -2.305 | 1.00 | 15.75 | A |
| ATOM C | 1044 | CD2 | HIS | A | 248 | 179.651 | 171.777 | -1.427 | 1.00 | 14.69 | A |
| ATOM N | 1045 | ND1 | HIS | A | 248 | 179.331 | 170.596 | -3.236 | 1.00 | 18.18 | A |
| ATOM C | 1046 | CE1 | HIS | A | 248 | 180.614 | 170.512 | -2.934 | 1.00 | 15.22 | A |
| ATOM N | 1047 | NE2 | HIS | A | 248 | 180.632 | 171.220 | -1.840 | 1.00 | 14.77 | A |
| ATOM C | 1048 | C | HIS | A | 248 | 175.474 | 173.338 | -1.717 | 1.00 | 16.00 | A |
| ATOM O | 1049 | O | HIS | A | 248 | 175.285 | 174.228 | -2.538 | 1.00 | 16.44 | A |
| ATOM N | 1050 | N | SER | A | 249 | 174.503 | 172.744 | -1.028 | 1.00 | 14.49 | A |
| ATOM C | 1051 | CA | SER | A | 249 | 173.125 | 173.148 | -1.197 | 1.00 | 15.32 | A |
| ATOM C | 1052 | CB | SER | A | 249 | 172.201 | 172.233 | -0.392 | 1.00 | 11.40 | A |
| ATOM O | 1053 | OG | SER | A | 249 | 172.281 | 172.527 | 0.985 | 1.00 | 16.48 | A |
| ATOM C | 1054 | C | SER | A | 249 | 173.055 | 174.597 | -0.696 | 1.00 | 16.09 | A |
| ATOM O | 1055 | O | SER | A | 249 | 172.139 | 175.350 | -1.034 | 1.00 | 18.04 | A |
| ATOM N | 1056 | N | LYS | A | 250 | 174.031 | 174.988 | 0.118 | 1.00 | 15.99 | A |
| ATOM C | 1057 | CA | LYS | A | 250 | 174.072 | 176.363 | 0.599 | 1.00 | 15.13 | A |
| ATOM C | 1058 | CB | LYS | A | 250 | 174.405 | 176.441 | 2.107 | 1.00 | 13.57 | A |
| ATOM C | 1059 | CG | LYS | A | 250 | 173.350 | 175.850 | 3.040 | 1.00 | 15.19 | A |
| ATOM C | 1060 | CD | LYS | A | 250 | 171.930 | 176.203 | 2.629 | 1.00 | 19.05 | A |
| ATOM C | 1061 | CE | LYS | A | 250 | 170.943 | 175.150 | 3.131 | 1.00 | 24.43 | A |
| ATOM N | 1062 | NZ | LYS | A | 250 | 169.492 | 175.372 | 2.776 | 1.00 | 28.76 | A |
| ATOM C | 1063 | C | LYS | A | 250 | 175.106 | 177.133 | -0.210 | 1.00 | 11.97 | A |
| ATOM O | 1064 | O | LYS | A | 250 | 175.375 | 178.286 | .057 | 1.00 | 14.99 | A |
| ATOM N | 1065 | N | ARG | A | 251 | 175.686 | 176.478 | -1.208 | 1.00 | 14.14 | A |
| ATOM C | 1066 | CA | ARG | A | 251 | 176.690 | 177.099 | -2.079 | 1.00 | 13.45 | A |
| ATOM C | 1067 | CB | ARG | A | 251 | 176.080 | 178.305 | -2.792 | 1.00 | 14.05 | A |
| ATOM C | 1068 | CG | ARG | A | 251 | 174.976 | 177.936 | -3.779 | 1.00 | 21.06 | A |
| ATOM C | 1069 | CD | ARG | A | 251 | 175.441 | 178.112 | -5.227 | 1.00 | 27.95 | A |
| ATOM N | 1070 | NE | ARG | A | 251 | 175.547 | 176.844 | -5.943 | 1.00 | 31.22 | A |
| ATOM C | 1071 | CZ | ARG | A | 251 | 176.391 | 176.615 | -6.949 | 1.00 | 31.35 | A |
| ATOM N | 1072 | NH1 | ARG | A | 251 | 177.214 | 177.570 | -7.368 | 1.00 | 28.63 | A |
| ATOM N | 1073 | NH2 | ARG | A | 251 | 176.427 | 175.422 | -7.526 | 1.00 | 31.96 | A |
| ATOM C | 1074 | C | ARG | A | 251 | 177.974 | 177.493 | -1.362 | 1.00 | 12.30 | A |
| ATOM O | 1075 | O | ARG | A | 251 | 178.618 | 178.472 | -1.703 | 1.00 | 12.69 | A |
| ATOM N | 1076 | N | VAL | A | 252 | 178.344 | 176.714 | -0.363 | 1.00 | 11.12 | A |
| ATOM C | 1077 | CA | VAL | A | 252 | 179.569 | 176.971 | 0.368 | 1.00 | 12.45 | A |
| ATOM C | 1078 | CB | VAL | A | 252 | 179.338 | 176.810 | 1.885 | 1.00 | 12.23 | A |
| ATOM C | 1079 | CG1 | VAL | A | 252 | 180.651 | 176.509 | 2.587 | 1.00 | 9.08 | A |
| ATOM C | 1080 | CG2 | VAL | A | 252 | 178.681 | 178.059 | 2.446 | 1.00 | 10.54 | A |
| ATOM C | 1081 | C | VAL | A | 252 | 180.604 | 175.940 | -0.083 | 1.00 | 15.27 | A |
| ATOM O | 1082 | O | VAL | A | 252 | 180.267 | 174.774 | -0.265 | 1.00 | 12.78 | A |
| ATOM N | 1083 | N | ILE | A | 253 | 181.840 | 176.373 | -0.326 | 1.00 | 13.72 | A |
| ATOM C | 1084 | CA | ILE | A | 253 | 182.872 | 175.419 | -0.680 | 1.00 | 10.89 | A |
| ATOM C | 1085 | CB | ILE | A | 253 | 183.670 | 175.788 | -1.957 | 1.00 | 11.67 | A |
| ATOM C | 1086 | CG2 | ILE | A | 253 | 184.426 | 174.535 | -2.441 | 1.00 | 5.83 | A |
| ATOM C | 1087 | CG1 | ILE | A | 253 | 182.733 | 176.323 | -3.059 | 1.00 | 10.28 | A |
| ATOM C | 1088 | CD1 | ILE | A | 253 | 183.439 | 176.812 | -4.329 | 1.00 | 3.57 | A |
| ATOM C | 1089 | C | ILE | A | 253 | 183.831 | 175.453 | 0.498 | 1.00 | 13.01 | A |
| ATOM O | 1090 | O | ILE | A | 253 | 184.347 | 176.524 | 0.849 | 1.00 | 13.11 | A |
| ATOM N | 1091 | N | HIS | A | 254 | 184.050 | 174.288 | 1.113 | 1.00 | 13.19 | A |
| ATOM C | 1092 | CA | HIS | A | 254 | 184.943 | 174.161 | 2.259 | 1.00 | 12.95 | A |
| ATOM C | 1093 | CB | HIS | A | 254 | 184.868 | 172.745 | 2.843 | 1.00 | 12.70 | A |
| ATOM C | 1094 | CG | HIS | A | 254 | 185.587 | 172.606 | 4.160 | 1.00 | 12.92 | A |
| ATOM C | 1095 | CD2 | HIS | A | 254 | 185.206 | 172.914 | 5.423 | 1.00 | 9.91 | A |
| ATOM N | 1096 | ND1 | HIS | A | 254 | 186.898 | 172.180 | 4.257 | 1.00 | 13.73 | A |
| ATOM C | 1097 | CE1 | HIS | A | 254 | 187.290 | 172.229 | 5.515 | 1.00 | 10.14 | A |
| ATOM N | 1098 | NE2 | HIS | A | 254 | 186.282 | 172.671 | 6.243 | 1.00 | 11.29 | A |
| ATOM C | 1099 | C | HIS | A | 254 | 186.383 | 174.507 | 1.889 | 1.00 | 13.04 | A |
| ATOM O | 1100 | O | HIS | A | 254 | 186.971 | 175.438 | 2.447 | 1.00 | 14.41 | A |
| ATOM N | 1101 | N | ARG | A | 255 | 186.935 | 173.743 | 0.953 | 1.00 | 12.07 | A |
| ATOM C | 1102 | CA | ARG | A | 255 | 188.291 | 173.910 | 0.438 | 1.00 | 12.35 | A |
| ATOM C | 1103 | CB | ARG | A | 255 | 188.494 | 175.341 | -0.050 | 1.00 | 13.05 | A |
| ATOM C | 1104 | CG | ARG | A | 255 | 187.553 | 175.664 | -1.200 | 1.00 | 15.96 | A |
| ATOM C | 1105 | CD | ARG | A | 255 | 187.942 | 176.885 | -1.997 | 1.00 | 11.42 | A |
| ATOM N | 1106 | NE | ARG | A | 255 | 188.045 | 178.075 | -1.183 | 1.00 | 13.42 | A |
| ATOM C | 1107 | CZ | ARG | A | 255 | 188.194 | 179.295 | -1.690 | 1.00 | 17.97 | A |
| ATOM N | 1108 | NH1 | ARG | A | 255 | 188.248 | 179.453 | -3.012 | 1.00 | 12.59 | A |
| ATOM N | 1109 | NH2 | ARG | A | 255 | 188.308 | 180.348 | -0.880 | 1.00 | 11.08 | A |
| ATOM C | 1110 | C | ARG | A | 255 | 189.479 | 173.491 | 1.328 | 1.00 | 14.82 | A |
| ATOM O | 1111 | O | ARG | A | 255 | 190.628 | 173.656 | 0.914 | 1.00 | 16.45 | A |
| ATOM N | 1112 | N | ASP | A | 256 | 189.237 | 172.959 | 2.528 | 1.00 | 13.90 | A |
| ATOM C | 1113 | CA | ASP | A | 256 | 190.355 | 172.522 | 3.364 | 1.00 | 14.13 | A |
| ATOM C | 1114 | CB | ASP | A | 256 | 190.779 | 173.631 | 4.341 | 1.00 | 15.61 | A |
| ATOM C | 1115 | CG | ASP | A | 256 | 192.185 | 173.408 | 4.930 | 1.00 | 17.71 | A |
| ATOM O | 1116 | OD1 | ASP | A | 256 | 193.119 | 172.950 | 4.217 | 1.00 | 18.23 | A |
| ATOM O | 1117 | OD2 | ASP | A | 256 | 192.364 | 173.712 | 6.130 | 1.00 | 20.01 | A |
| ATOM C | 1118 | C | ASP | A | 256 | 190.021 | 171.231 | 4.094 | 1.00 | 14.83 | A |
| ATOM O | 1119 | O | ASP | A | 256 | 190.209 | 171.088 | 5.300 | 1.00 | 17.90 | A |
| ATOM N | 1120 | N | ILE | A | 257 | 189.522 | 170.285 | 3.321 | 1.00 | 14.83 | A |
| ATOM C | 1121 | CA | ILE | A | 257 | 189.170 | 168.978 | 3.809 | 1.00 | 15.89 | A |
| ATOM C | 1122 | CB | ILE | A | 257 | 188.209 | 168.318 | 2.821 | 1.00 | 16.68 | A |
| ATOM C | 1123 | CG2 | ILE | A | 257 | 188.096 | 166.834 | 3.106 | 1.00 | 18.21 | A |
| ATOM C | 1124 | CG1 | ILE | A | 257 | 186.851 | 169.010 | 2.909 | 1.00 | 19.11 | A |
| ATOM C | 1125 | CD1 | ILE | A | 257 | 185.939 | 168.636 | 1.771 | 1.00 | 26.98 | A |
| ATOM C | 1126 | C | ILE | A | 257 | 190.461 | 168.170 | 3.888 | 1.00 | 16.84 | A |
| ATOM O | 1127 | O | ILE | A | 257 | 191.180 | 168.019 | 2.890 | 1.00 | 19.23 | A |
| ATOM N | 1128 | N | LYS | A | 258 | 190.759 | 167.673 | 5.079 | 1.00 | 14.42 | A |
| ATOM C | 1129 | CA | LYS | A | 258 | 191.948 | 166.861 | 5.321 | 1.00 | 13.61 | A |
| ATOM C | 1130 | CB | LYS | A | 258 | 193.218 | 167.696 | 5.165 | 1.00 | 11.12 | A |
| ATOM C | 1131 | CG | LYS | A | 258 | 193.288 | 168.912 | 6.021 | 1.00 | 10.35 | A |
| ATOM C | 1132 | CD | LYS | A | 258 | 194.685 | 169.397 | 5.941 | 1.00 | 11.98 | A |
| ATOM C | 1133 | CE | LYS | A | 258 | 194.946 | 170.539 | 6.844 | 1.00 | 10.31 | A |
| ATOM N | 1134 | NZ | LYS | A | 258 | 196.316 | 171.028 | 6.523 | 1.00 | 13.12 | A |
| ATOM C | 1135 | C | LYS | A | 258 | 191.828 | 166.275 | 6.728 | 1.00 | 12.82 | A |
| ATOM O | 1136 | O | LYS | A | 258 | 191.093 | 166.807 | 7.565 | 1.00 | 12.94 | A |
| ATOM N | 1137 | N | PRO | A | 259 | 192.547 | 165.180 | 7.011 | 1.00 | 13.48 | A |
| ATOM C | 1138 | CD | PRO | A | 259 | 193.607 | 164.582 | 6.181 | 1.00 | 11.93 | A |
| ATOM C | 1139 | CA | PRO | A | 259 | 192.487 | 164.525 | 8.329 | 1.00 | 14.07 | A |
| ATOM C | 1140 | CB | PRO | A | 259 | 193.641 | 163.517 | 8.267 | 1.00 | 14.89 | A |
| ATOM C | 1141 | CG | PRO | A | 259 | 193.714 | 163.197 | 6.778 | 1.00 | 14.98 | A |
| ATOM C | 1142 | C | PRO | A | 259 | 192.527 | 165.413 | 9.583 | 1.00 | 15.36 | A |
| ATOM O | 1143 | O | PRO | A | 259 | 191.773 | 165.171 | 10.520 | 1.00 | 15.88 | A |
| ATOM N | 1144 | N | CLU | A | 260 | 193.388 | 166.428 | 9.593 | 1.00 | 15.22 | A |
| ATOM C | 1145 | CA | GLU | A | 260 | 193.516 | 167.340 | 10.734 | 1.00 | 19.96 | A |
| ATOM C | 1146 | CB | GLU | A | 260 | 194.770 | 168.225 | 10.563 | 1.00 | 22.41 | A |
| ATOM C | 1147 | CG | GLU | A | 260 | 196.090 | 167.457 | 10.516 | 1.00 | 29.11 | A |
| ATOM C | 1148 | CD | GLU | A | 260 | 196.610 | 167.140 | 9.092 | 1.00 | 32.88 | A |
| ATOM O | 1149 | OE1 | GLU | A | 260 | 195.826 | 166.656 | 8.235 | 1.00 | 33.12 | A |
| ATOM O | 1150 | OE2 | GLU | A | 260 | 197.823 | 167.360 | 8.841 | 1.00 | 35.49 | A |
| ATOM C | 1151 | C | GLU | A | 260 | 192.280 | 168.252 | 10.924 | 1.00 | 18.80 | A |
| ATOM O | 1152 | O | GLU | A | 260 | 192.140 | 168.905 | 11.957 | 1.00 | 18.71 | A |
| ATOM N | 1153 | N | ASN | A | 261 | 191.401 | 168.309 | 9.922 | 1.00 | 16.14 | A |
| ATOM C | 1154 | CA | ASN | A | 261 | 190.206 | 169.139 | 10.014 | 1.00 | 14.12 | A |
| ATOM C | 1155 | CB | ASN | A | 261 | 190.084 | 170.036 | 8.799 | 1.00 | 12.74 | A |
| ATOM C | 1156 | CG | ASN | A | 261 | 191.068 | 171.176 | 8.824 | 1.00 | 12.37 | A |
| ATOM O | 1157 | OD1 | ASN | A | 261 | 191.719 | 171.446 | 9.840 | 1.00 | 11.65 | A |
| ATOM N | 1158 | ND2 | ASN | A | 261 | 191.178 | 171.868 | 7.705 | 1.00 | 11.18 | A |
| ATOM C | 1159 | C | ASN | A | 261 | 188.944 | 168.329 | 10.159 | 1.00 | 14.87 | A |
| ATOM O | 1160 | O | ASN | A | 261 | 187.844 | 168.855 | 10.027 | 1.00 | 14.12 | A |
| ATOM N | 1161 | N | LEU | A | 262 | 189.117 | 167.037 | 10.428 | 1.00 | 16.20 | A |
| ATOM C | 1162 | CA | LEU | A | 262 | 187.996 | 166.127 | 10.596 | 1.00 | 13.58 | A |
| ATOM C | 1163 | CB | LEU | A | 262 | 188.128 | 164.954 | 9.627 | 1.00 | 13.22 | A |
| ATOM C | 1164 | CG | LEU | A | 262 | 188.164 | 165.406 | 8.162 | 1.00 | 15.01 | A |
| ATOM C | 1165 | CD1 | LEU | A | 262 | 188.502 | 164.230 | 7.232 | 1.00 | 8.10 | A |
| ATOM C | 1166 | CD2 | LEU | A | 262 | 186.807 | 166.049 | 7.834 | 1.00 | 10.56 | A |
| ATOM C | 1167 | C | LEU | A | 262 | 188.027 | 165.633 | 12.031 | 1.00 | 15.43 | A |
| ATOM O | 1168 | O | LEU | A | 262 | 188.999 | 165.008 | 12.459 | 1.00 | 17.59 | A |
| ATOM N | 1169 | N | LEU | A | 263 | 186.968 | 165.915 | 12.777 | 1.00 | 11.89 | A |
| ATOM C | 1170 | CA | LEU | A | 263 | 186.926 | 165.505 | 14.154 | 1.00 | 10.46 | A |
| ATOM C | 1171 | CB | LEU | A | 263 | 186.657 | 166.725 | 15.005 | 1.00 | 13.46 | A |
| ATOM C | 1172 | CG | LEU | A | 263 | 187.584 | 167.921 | 14.699 | 1.00 | 14.28 | A |
| ATOM C | 1173 | CD1 | LEU | A | 263 | 187.208 | 169.055 | 15.634 | 1.00 | 15.02 | A |
| ATOM C | 1174 | CD2 | LEU | A | 263 | 189.050 | 167.563 | 14.865 | 1.00 | 12.28 | A |
| ATOM C | 1175 | C | LEU | A | 263 | 185.905 | 164.407 | 14.409 | 1.00 | 12.75 | A |
| ATOM O | 1176 | O | LEU | A | 263 | 185.068 | 164.112 | 13.553 | 1.00 | 13.83 | A |
| ATOM N | 1177 | N | LEU | A | 264 | 185.989 | 163.780 | 15.576 | 1.00 | 13.44 | A |
| ATOM C | 1178 | CA | LEU | A | 264 | 185.076 | 162.693 | 15.919 | 1.00 | 15.08 | A |
| ATOM C | 1179 | CB | LEU | A | 264 | 185.856 | 161.421 | 16.298 | 1.00 | 15.27 | A |
| ATOM C | 1180 | CG | LEU | A | 264 | 186.808 | 160.821 | 15.263 | 1.00 | 16.94 | A |
| ATOM C | 1181 | CD1 | LEU | A | 264 | 187.393 | 159.499 | 15.783 | 1.00 | 20.04 | A |
| ATOM C | 1182 | CD2 | LEU | A | 264 | 186.043 | 160.590 | 13.944 | 1.00 | 16.69 | A |
| ATOM C | 1183 | C | LEU | A | 264 | 184.190 | 163.103 | 17.078 | 1.00 | 14.83 | A |
| ATOM O | 1184 | O | LEU | A | 264 | 184.669 | 163.613 | 18.096 | 1.00 | 13.07 | A |
| ATOM N | 1185 | N | GLY | A | 265 | 182.890 | 162.881 | 16.907 | 1.00 | 14.85 | A |
| ATOM C | 1186 | CA | GLY | A | 265 | 181.943 | 163.218 | 17.947 | 1.00 | 15.69 | A |
| ATOM C | 1187 | C | GLY | A | 265 | 181.844 | 162.127 | 18.999 | 1.00 | 17.48 | A |
| ATOM O | 1188 | O | GLY | A | 265 | 182.597 | 161.144 | 18.989 | 1.00 | 19.40 | A |
| ATOM N | 1189 | N | SER | A | 266 | 180.888 | 162.306 | 19.899 | 1.00 | 17.64 | A |
| ATOM C | 1190 | CA | SER | A | 266 | 180.641 | 161.398 | 21.007 | 1.00 | 18.83 | A |
| ATOM C | 1191 | CB | SER | A | 266 | 179.417 | 161.895 | 21.766 | 1.00 | 17.49 | A |
| ATOM O | 1192 | OG | SER | A | 266 | 179.347 | 161.294 | 23.037 | 1.00 | 31.06 | A |
| ATOM C | 1193 | C | SER | A | 266 | 180.457 | 159.923 | 20.615 | 1.00 | 16.59 | A |
| ATOM O | 1194 | O | SER | A | 266 | 180.771 | 159.025 | 21.389 | 1.00 | 16.64 | A |
| ATOM N | 1195 | N | ALA | A | 267 | 179.960 | 159.675 | 19.412 | 1.00 | 15.97 | A |
| ATOM C | 1196 | CA | ALA | A | 267 | 179.731 | 158.314 | 18.939 | 1.00 | 17.67 | A |
| ATOM C | 1197 | CB | ALA | A | 267 | 178.288 | 158.163 | 18.456 | 1.00 | 14.65 | A |
| ATOM C | 1198 | C | ALA | A | 267 | 180.670 | 157.930 | 17.815 | 1.00 | 18.92 | A |
| ATOM O | 1199 | O | ALA | A | 267 | 180.353 | 157.040 | 17.025 | 1.00 | 21.00 | A |
| ATOM N | 1200 | N | GLY | A | 268 | 181.812 | 158.604 | 17.716 | 1.00 | 21.24 | A |
| ATOM C | 1201 | CA | GLY | A | 268 | 182.741 | 158.278 | 16.648 | 1.00 | 22.38 | A |
| ATOM C | 1202 | C | GLY | A | 268 | 182.289 | 158.773 | 15.282 | 1.00 | 21.15 | A |
| ATOM O | 1203 | O | GLY | A | 268 | 182.809 | 158.323 | 14.266 | 1.00 | 20.78 | A |
| ATOM N | 1204 | N | GLU | A | 269 | 181.324 | 159.690 | 15.249 | 1.00 | 19.71 | A |
| ATOM C | 1205 | CA | GLU | A | 269 | 180.831 | 160.239 | 13.983 | 1.00 | 18.83 | A |
| ATOM C | 1206 | CB | GLU | A | 269 | 179.386 | 160.746 | 14.107 | 1.00 | 20.11 | A |
| ATOM C | 1207 | CG | GLU | A | 269 | 179.239 | 162.020 | 14.944 | 1.00 | 27.28 | A |
| ATOM C | 1208 | CD | GLU | A | 269 | 178.874 | 161.722 | 16.385 | 1.00 | 29.04 | A |
| ATOM O | 1209 | OE1 | GLU | A | 269 | 179.762 | 161.324 | 17.177 | 1.00 | 30.97 | A |
| ATOM O | 1210 | OE2 | GLU | A | 269 | 177.677 | 161.868 | 16.711 | 1.00 | 33.63 | A |
| ATOM C | 1211 | C | GLU | A | 269 | 181.702 | 161.409 | 13.549 | 1.00 | 17.27 | A |
| ATOM O | 1212 | O | GLU | A | 269 | 182.088 | 162.249 | 14.368 | 1.00 | 15.38 | A |
| ATOM N | 1213 | N | LEU | A | 270 | 181.994 | 161.461 | 12.253 | 1.00 | 14.97 | A |
| ATOM C | 1214 | CA | LEU | A | 270 | 182.822 | 162.515 | 11.689 | 1.00 | 15.39 | A |
| ATOM C | 1215 | CB | LEU | A | 270 | 183.169 | 162.167 | 10.229 | 1.00 | 21.78 | A |
| ATOM C | 1216 | CG | LEU | A | 270 | 184.100 | 163.071 | 9.408 | 1.00 | 22.18 | A |
| ATOM C | 1217 | CD1 | LEU | A | 270 | 185.484 | 162.839 | 9.938 | 1.00 | 26.73 | A |
| ATOM C | 1218 | CD2 | LEU | A | 270 | 184.109 | 162.725 | 7.944 | 1.00 | 27.39 | A |
| ATOM C | 1219 | C | LEU | A | 270 | 182.129 | 163.876 | 11.722 | 1.00 | 13.13 | A |
| ATOM O | 1220 | O | LEU | A | 270 | 180.932 | 163.973 | 11.490 | 1.00 | 12.45 | A |
| ATOM N | 1221 | N | LYS | A | 271 | 182.895 | 164.918 | 12.027 | 1.00 | 13.69 | A |
| ATOM C | 1222 | CA | LYS | A | 271 | 182.395 | 166.296 | 12.025 | 1.00 | 13.80 | A |
| ATOM C | 1223 | CB | LYS | A | 271 | 182.201 | 166.814 | 13.450 | 1.00 | 13.22 | A |
| ATOM C | 1224 | CG | LYS | A | 271 | 180.846 | 166.378 | 14.025 | 1.00 | 16.86 | A |
| ATOM C | 1225 | CD | LYS | A | 271 | 180.790 | 166.428 | 15.509 | 1.00 | 17.04 | A |
| ATOM C | 1226 | CE | LYS | A | 271 | 179.494 | 165.823 | 15.967 | 1.00 | 15.79 | A |
| ATOM N | 1227 | NZ | LYS | A | 271 | 178.379 | 166.692 | 15.545 | 1.00 | 20.81 | A |
| ATOM C | 1228 | C | LYS | A | 271 | 183.392 | 167.151 | 11.270 | 1.00 | 15.22 | A |
| ATOM O | 1229 | O | LYS | A | 271 | 184.586 | 167.152 | 11.582 | 1.00 | 16.95 | A |
| ATOM N | 1230 | N | ILE | A | 272 | 182.919 | 167.842 | 10.238 | 1.00 | 15.55 | A |
| ATOM C | 1231 | CA | ILE | A | 272 | 183.803 | 168.702 | 9.462 | 1.00 | 15.61 | A |
| ATOM C | 1232 | CB | ILE | A | 272 | 183.143 | 169.220 | 8.163 | 1.00 | 19.61 | A |
| ATOM C | 1233 | CG2 | ILE | A | 272 | 184.054 | 170.269 | 7.530 | 1.00 | 18.20 | A |
| ATOM C | 1234 | CG1 | ILE | A | 272 | 182.850 | 168.069 | 7.203 | 1.00 | 22.81 | A |
| ATOM C | 1235 | CD1 | ILE | A | 272 | 182.068 | 168.504 | 5.967 | 1.00 | 25.62 | A |
| ATOM C | 1236 | C | ILE | A | 272 | 184.051 | 169.926 | 10.303 | 1.00 | 13.27 | A |
| ATOM O | 1237 | O | ILE | A | 272 | 183.136 | 170.422 | 10.945 | 1.00 | 12.04 | A |
| ATOM N | 1238 | N | ALA | A | 273 | 185.270 | 170.428 | 10.294 | 1.00 | 11.17 | A |
| ATOM C | 1239 | CA | ALA | A | 273 | 185.568 | 171.623 | 11.069 | 1.00 | 13.73 | A |
| ATOM C | 1240 | CB | ALA | A | 273 | 186.266 | 171.248 | 12.376 | 1.00 | 10.14 | A |
| ATOM C | 1241 | C | ALA | A | 273 | 186.472 | 172.498 | 10.225 | 1.00 | 16.38 | A |
| ATOM O | 1242 | O | ALA | A | 273 | 186.688 | 172.208 | 9.047 | 1.00 | 16.96 | A |
| ATOM N | 1243 | N | ASP | A | 274 | 186.986 | 173.568 | 10.834 | 1.00 | 17.04 | A |
| ATOM C | 1244 | CA | ASP | A | 274 | 187.913 | 174.500 | 10.200 | 1.00 | 15.18 | A |
| ATOM C | 1245 | CB | ASP | A | 274 | 189.288 | 173.805 | 10.074 | 1.00 | 17.02 | A |
| ATOM C | 1246 | CG | ASP | A | 274 | 190.447 | 174.786 | 9.800 | 1.00 | 19.27 | A |
| ATOM O | 1247 | OD1 | ASP | A | 274 | 190.190 | 175.999 | 9.635 | 1.00 | 22.45 | A |
| ATOM O | 1248 | OD2 | ASP | A | 274 | 191.624 | 174.338 | 9.753 | 1.00 | 17.87 | A |
| ATOM C | 1249 | C | ASP | A | 274 | 187.434 | 175.064 | 8.838 | 1.00 | 16.14 | A |
| ATOM O | 1250 | O | ASP | A | 274 | 187.846 | 174.604 | 7.776 | 1.00 | 12.68 | A |
| ATOM N | 1251 | N | PHE | A | 275 | 186.559 | 176.065 | 8.881 | 1.00 | 15.86 | A |
| ATOM C | 1252 | CA | PHE | A | 275 | 186.074 | 176.688 | 7.666 | 1.00 | 14.12 | A |
| ATOM C | 1253 | CB | PHE | A | 275 | 184.602 | 177.108 | 7.835 | 1.00 | 13.16 | A |
| ATOM C | 1254 | CG | PHE | A | 275 | 183.640 | 175.948 | 7.721 | 1.00 | 12.72 | A |
| ATOM C | 1255 | CD1 | PHE | A | 275 | 183.540 | 174.994 | 8.746 | 1.00 | 11.42 | A |
| ATOM C | 1256 | CD2 | PHE | A | 275 | 182.936 | 175.727 | 6.535 | 1.00 | 11.44 | A |
| ATOM C | 1257 | CE1 | PHE | A | 275 | 182.768 | 173.843 | 8.581 | 1.00 | 10.04 | A |
| ATOM C | 1258 | CE2 | PHE | A | 275 | 182.157 | 174.568 | 6.367 | 1.00 | 12.57 | A |
| ATOM C | 1259 | CZ | PHE | A | 275 | 182.079 | 173.631 | 7.396 | 1.00 | 11.15 | A |
| ATOM C | 1260 | C | PHE | A | 275 | 186.966 | 177.868 | 7.289 | 1.00 | 15.87 | A |
| ATOM O | 1261 | O | PHE | A | 275 | 186.541 | 178.780 | 6.568 | 1.00 | 15.07 | A |
| ATOM N | 1262 | N | GLY | A | 276 | 188.221 | 177.820 | 7.751 | 1.00 | 15.09 | A |
| ATOM C | 1263 | CA | GLY | A | 276 | 189.184 | 178.875 | 7.452 | 1.00 | 17.20 | A |
| ATOM C | 1264 | C | GLY | A | 276 | 189.322 | 179.285 | 5.983 | 1.00 | 16.44 | A |
| ATOM O | 1265 | O | GLY | A | 276 | 189.368 | 180.482 | 5.665 | 1.00 | 17.13 | A |
| ATOM N | 1266 | N | TRP | A | 277 | 189.378 | 178.307 | 5.082 | 1.00 | 13.73 | A |
| ATOM C | 1267 | CA | TRP | A | 277 | 189.514 | 178.596 | 3.664 | 1.00 | 14.40 | A |
| ATOM C | 1268 | CB | TRP | A | 277 | 190.568 | 177.667 | 3.071 | 1.00 | 20.13 | A |
| ATOM C | 1269 | CG | TRP | A | 277 | 191.860 | 177.753 | 3.789 | 1.00 | 22.40 | A |
| ATOM C | 1270 | CD2 | TRP | A | 277 | 192.691 | 178.904 | 3.893 | 1.00 | 23.73 | A |
| ATOM C | 1271 | CE2 | TRP | A | 277 | 193.788 | 178.558 | 4.701 | 1.00 | 26.05 | A |
| ATOM C | 1272 | CE3 | TRP | A | 277 | 192.617 | 180.198 | 3.375 | 1.00 | 25.94 | A |
| ATOM C | 1273 | CD1 | TRP | A | 277 | 192.463 | 176.777 | 4.517 | 1.00 | 23.25 | A |
| ATOM N | 1274 | NE1 | TRP | A | 277 | 193.625 | 177.250 | 5.074 | 1.00 | 24.53 | A |
| ATOM C | 1275 | CZ2 | TRP | A | 277 | 194.806 | 179.467 | 5.015 | 1.00 | 27.86 | A |
| ATOM C | 1276 | CZ3 | TRP | A | 277 | 193.631 | 181.106 | 3.686 | 1.00 | 28.74 | A |
| ATOM C | 1277 | CH2 | TRP | A | 277 | 194.711 | 180.733 | 4.496 | 1.00 | 26.68 | A |
| ATOM C | 1278 | C | TRP | A | 277 | 188.202 | 178.467 | 2.888 | 1.00 | 13.46 | A |
| ATOM O | 1279 | O | TRP | A | 277 | 188.190 | 178.362 | 1.664 | 1.00 | 14.44 | A |
| ATOM N | 1280 | N | SER | A | 278 | 187.097 | 178.481 | 3.605 | 1.00 | 14.67 | A |
| ATOM C | 1281 | CA | SER | A | 278 | 185.809 | 178.360 | 2.973 | 1.00 | 16.81 | A |
| ATOM C | 1282 | CB | SER | A | 278 | 184.785 | 177.937 | 4.009 | 1.00 | 18.65 | A |
| ATOM O | 1283 | OG | SER | A | 278 | 185.042 | 176.588 | 4.334 | 1.00 | 31.56 | A |
| ATOM C | 1284 | C | SER | A | 278 | 185.338 | 179.620 | 2.269 | 1.00 | 15.59 | A |
| ATOM O | 1285 | O | SER | A | 278 | 185.887 | 180.700 | 2.456 | 1.00 | 13.40 | A |
| ATOM N | 1286 | N | VAL | A | 279 | 184.330 | 179.468 | 1.427 | 1.00 | 12.47 | A |
| ATOM C | 1287 | CA | VAL | A | 279 | 183.790 | 180.622 | 0.750 | 1.00 | 15.04 | A |
| ATOM C | 1288 | CB | VAL | A | 279 | 184.631 | 181.010 | -0.524 | 1.00 | 14.76 | A |
| ATOM C | 1289 | CG1 | VAL | A | 279 | 184.436 | 179.985 | -1.646 | 1.00 | 10.49 | A |
| ATOM C | 1290 | CG2 | VAL | A | 279 | 184.256 | 182.419 | -0.971 | 1.00 | 9.51 | A |
| ATOM C | 1291 | C | VAL | A | 279 | 182.347 | 180.399 | 0.352 | 1.00 | 15.66 | A |
| ATOM O | 1292 | O | VAL | A | 279 | 181.902 | 179.259 | 0.162 | 1.00 | 16.74 | A |
| ATOM N | 1293 | N | HIS | A | 280 | 181.604 | 181.491 | 0.276 | 1.00 | 14.84 | A |
| ATOM C | 1294 | CA | HIS | A | 280 | 180.224 | 181.389 | -0.133 | 1.00 | 17.48 | A |
| ATOM C | 1295 | CB | HIS | A | 280 | 179.332 | 182.330 | 0.694 | 1.00 | 14.36 | A |
| ATOM C | 1296 | CG | HIS | A | 280 | 177.885 | 182.230 | 0.336 | 1.00 | 16.95 | A |
| ATOM C | 1297 | CD2 | HIS | A | 280 | 177.154 | 181.176 | -0.102 | 1.00 | 16.10 | A |
| ATOM N | 1298 | ND1 | HIS | A | 280 | 177.045 | 183.323 | 0.300 | 1.00 | 15.76 | A |
| ATOM C | 1299 | CE1 | HIS | A | 280 | 175.864 | 182.946 | -0.154 | 1.00 | 17.22 | A |
| ATOM N | 1300 | NE2 | HIS | A | 280 | 175.903 | 181.648 | -0.407 | 1.00 | 18.56 | A |
| ATOM C | 1301 | C | HIS | A | 280 | 180.236 | 181.772 | -1.622 | 1.00 | 17.74 | A |
| ATOM O | 1302 | O | HIS | A | 280 | 180.439 | 182.942 | -1.989 | 1.00 | 16.22 | A |
| ATOM N | 1303 | N | ALA | A | 281 | 180.048 | 180.773 | -2.480 | 1.00 | 15.79 | A |
| ATOM C | 1304 | CA | ALA | A | 281 | 180.066 | 180.984 | -3.930 | 1.00 | 17.35 | A |
| ATOM C | 1305 | CB | ALA | A | 281 | 181.103 | 180.047 | -4.565 | 1.00 | 15.43 | A |
| ATOM C | 1306 | C | ALA | A | 281 | 178.693 | 180.779 | -4.610 | 1.00 | 18.31 | A |
| ATOM O | 1307 | O | ALA | A | 281 | 178.489 | 179.809 | -5.311 | 1.00 | 18.09 | A |
| ATOM N | 1308 | N | PRO | A | 282 | 177.754 | 181.726 | -4.412 | 1.00 | 20.27 | A |
| ATOM C | 1309 | CD | PRO | A | 282 | 177.983 | 182.946 | -3.597 | 1.00 | 20.44 | A |
| ATOM C | 1310 | CA | PRO | A | 282 | 176.381 | 181.701 | -4.974 | 1.00 | 21.92 | A |
| ATOM C | 1311 | CB | PRO | A | 282 | 175.770 | 183.032 | -4.483 | 1.00 | 21.47 | A |
| ATOM C | 1312 | CG | PRO | A | 282 | 176.575 | 183.413 | -3.327 | 1.00 | 21.85 | A |
| ATOM C | 1313 | C | PRO | A | 282 | 176.326 | 181.581 | -6.480 | 1.00 | 22.24 | A |
| ATOM O | 1314 | O | PRO | A | 282 | 175.611 | 180.729 | -6.987 | 1.00 | 24.89 | A |
| ATOM N | 1315 | N | SER | A | 283 | 177.088 | 182.439 | -7.163 | 1.00 | 24.90 | A |
| ATOM C | 1316 | CA | SER | A | 283 | 177.131 | 182.464 | -8.629 | 1.00 | 28.30 | A |
| ATOM C | 1317 | CB | SER | A | 283 | 176.711 | 183.837 | -9.202 | 1.00 | 26.31 | A |
| ATOM O | 1318 | OG | SER | A | 283 | 175.659 | 184.461 | -8.492 | 1.00 | 30.72 | A |
| ATOM C | 1319 | C | SER | A | 283 | 178.454 | 182.133 | -9.327 | 1.00 | 27.14 | A |
| ATOM O | 1320 | O | SER | A | 283 | 178.563 | 181.133 | -10.033 | 1.00 | 31.35 | A |
| ATOM N | 1321 | N | SER | A | 284 | 179.434 | 183.011 | -9.147 | 1.00 | 25.92 | A |
| ATOM C | 1322 | CA | SER | A | 284 | 180.749 | 182.937 | -9.789 | 1.00 | 26.41 | A |
| ATOM C | 1323 | CB | SER | A | 284 | 181.476 | 184.252 | -9.583 | 1.00 | 26.11 | A |
| ATOM O | 1324 | OG | SER | A | 284 | 180.590 | 185.288 | -9.231 | 1.00 | 32.06 | A |
| ATOM C | 1325 | C | SER | A | 284 | 181.720 | 181.869 | -9.346 | 1.00 | 24.70 | A |
| ATOM O | 1326 | O | SER | A | 284 | 181.617 | 181.347 | -8.254 | 1.00 | 26.06 | A |
| ATOM N | 1327 | N | ARG | A | 285 | 182.717 | 181.636 | -10.192 | 1.00 | 23.60 | A |
| ATOM C | 1328 | CA | ARG | A | 285 | 183.782 | 180.701 | -9.910 | 1.00 | 25.00 | A |
| ATOM C | 1329 | CB | ARG | A | 285 | 184.337 | 180.169 | -11.225 | 1.00 | 25.95 | A |
| ATOM C | 1330 | CG | ARG | A | 285 | 183.367 | 179.201 | -11.935 | 1.00 | 31.00 | A |
| ATOM C | 1331 | CD | ARG | A | 285 | 184.154 | 178.152 | -12.746 | 1.00 | 38.12 | A |
| ATOM N | 1332 | NE | ARG | A | 285 | 184.419 | 178.543 | -14.135 | 1.00 | 42.96 | A |
| ATOM C | 1333 | CZ | ARG | A | 285 | 185.202 | 177.856 | -14.971 | 1.00 | 45.91 | A |
| ATOM N | 1334 | NH1 | ARG | A | 285 | 185.808 | 176.744 | -14.560 | 1.00 | 46.53 | A |
| ATOM N | 1335 | NH2 | ARG | A | 285 | 185.364 | 178.267 | -16.225 | 1.00 | 46.52 | A |
| ATOM C | 1336 | C | ARG | A | 285 | 184.869 | 181.436 | -9.074 | 1.00 | 23.19 | A |
| ATOM O | 1337 | O | ARG | A | 285 | 184.791 | 182.652 | -8.873 | 1.00 | 23.63 | A |
| ATOM N | 1338 | N | ARG | A | 286 | 185.871 | 180.720 | -8.578 | 1.00 | 22.25 | A |
| ATOM C | 1339 | CA | ARG | A | 286 | 186.898 | 181.369 | -7.770 | 1.00 | 18.11 | A |
| ATOM C | 1340 | CB | ARG | A | 286 | 186.781 | 180.826 | -6.344 | 1.00 | 16.41 | A |
| ATOM C | 1341 | CG | ARG | A | 286 | 185.467 | 181.252 | -5.671 | 1.00 | 17.03 | A |
| ATOM C | 1342 | CD | ARG | A | 286 | 185.642 | 182.655 | -5.179 | 1.00 | 16.99 | A |
| ATOM N | 1343 | NE | ARG | A | 286 | 184.443 | 183.221 | -4.632 | 1.00 | 25.38 | A |
| ATOM C | 1344 | CZ | ARG | A | 286 | 184.398 | 184.417 | -4.072 | 1.00 | 26.94 | A |
| ATOM N | 1345 | NH1 | ARG | A | 286 | 185.509 | 185.138 | -3.995 | 1.00 | 28.48 | A |
| ATOM N | 1346 | NH2 | ARG | A | 286 | 183.244 | 184.894 | -3.605 | 1.00 | 30.62 | A |
| ATOM C | 1347 | C | ARG | A | 286 | 188.309 | 181.189 | -8.339 | 1.00 | 19.47 | A |
| ATOM O | 1348 | O | ARG | A | 286 | 188.537 | 180.305 | -9.174 | 1.00 | 17.62 | A |
| ATOM N | 1349 | N | TPO | A | 287 | 189.226 | 182.041 | -7.916 | 1.00 | 21.77 | A |
| ATOM C | 1350 | CA | TPO | A | 287 | 190.558 | 181.974 | -8.377 | 1.00 | 21.28 | A |
| ATOM C | 1351 | CB | TPO | A | 287 | 190.775 | 183.306 | -9.113 | 1.00 | 25.22 | A |
| ATOM C | 1352 | CG2 | TPO | A | 287 | 189.942 | 183.372 | -10.421 | 1.00 | 22.17 | A |
| ATOM O | 1353 | OG1 | TPO | A | 287 | 190.484 | 184.557 | -8.448 | 1.00 | 33.29 | A |
| ATOM P | 1354 | P | TPO | A | 287 | 191.582 | 185.763 | -8.444 | 1.00 | 33.70 | A |
| ATOM O | 1355 | O1P | TPO | A | 287 | 190.902 | 186.991 | -9.195 | 1.00 | 41.17 | A |
| ATOM O | 1356 | O2P | TPO | A | 287 | 192.847 | 185.199 | -9.239 | 1.00 | 37.62 | A |
| ATOM O | 1357 | O3P | TPO | A | 287 | 191.990 | 186.067 | -6.912 | 1.00 | 41.70 | A |
| ATOM C | 1358 | C | TPO | A | 287 | 191.612 | 181.717 | -7.265 | 1.00 | 16.99 | A |
| ATOM O | 1359 | O | TPO | A | 287 | 192.886 | 181.536 | -7.600 | 1.00 | 25.86 | A |
| ATOM N | 1360 | N | TPO | A | 288 | 191.139 | 181.614 | -6.033 | 1.00 | 16.91 | A |
| ATOM C | 1361 | CA | TPO | A | 288 | 192.066 | 181.441 | -4.938 | 1.00 | 16.31 | A |
| ATOM C | 1362 | CB | TPO | A | 288 | 191.262 | 181.750 | -3.672 | 1.00 | 18.77 | A |
| ATOM C | 1363 | CG2 | TPO | A | 288 | 192.190 | 181.949 | -2.512 | 1.00 | 16.50 | A |
| ATOM O | 1364 | OG1 | TPO | A | 288 | 190.694 | 183.015 | -3.936 | 1.00 | 23.64 | A |
| ATOM P | 1365 | P | TPO | A | 288 | 189.163 | 183.188 | -3.329 | 1.00 | 18.56 | A |
| ATOM O | 1366 | O1P | TPO | A | 288 | 189.234 | 182.723 | -1.818 | 1.00 | 19.25 | A |
| ATOM O | 1367 | O2P | TPO | A | 288 | 188.217 | 182.242 | -4.154 | 1.00 | 24.67 | A |
| ATOM O | 1368 | O3P | TPO | A | 288 | 188.820 | 184.760 | -3.320 | 1.00 | 24.29 | A |
| ATOM C | 1369 | C | TPO | A | 288 | 192.828 | 180.109 | -4.873 | 1.00 | 17.62 | A |
| ATOM O | 1370 | O | TPO | A | 288 | 192.132 | 178.986 | -5.036 | 1.00 | 16.48 | A |
| ATOM N | 1371 | N | LEU | A | 289 | 194.152 | 180.122 | -4.825 | 1.00 | 19.62 | A |
| ATOM C | 1372 | CA | LEU | A | 289 | 194.793 | 178.875 | -4.419 | 1.00 | 20.29 | A |
| ATOM . C | 1373 | CB | LEU | A | 289 | 196.229 | 178.759 | -4.993 | 1.00 | 21.71 | A |
| ATOM C | 1374 | CG | LEU | A | 289 | 196.977 | 177.458 | -4.598 | 1.00 | 25.59 | A |
| ATOM C | 1375 | CD1 | LEU | A | 289 | 196.506 | 176.298 | -5.488 | 1.00 | 30.17 | A |
| ATOM C | 1376 | CD2 | LEU | A | 289 | 198.475 | 177.596 | -4.769 | 1.00 | 26.69 | A |
| ATOM C | 1377 | C | LEU | A | 289 | 194.833 | 178.800 | -2.900 | 1.00 | 17.60 | A |
| ATOM O | 1378 | O | LEU | A | 289 | 195.428 | 179.646 | -2.272 | 1.00 | 20.61 | A |
| ATOM N | 1379 | N | CYS | A | 290 | 194.191 | 177.810 | -2.307 | 1.00 | 16.97 | A |
| ATOM C | 1380 | CA | CYS | A | 290 | 194.204 | 177.679 | -0.844 | 1.00 | 18.91 | A |
| ATOM C | 1381 | CB | CYS | A | 290 | 193.102 | 178.535 | -0.201 | 1.00 | 16.86 | A |
| ATOM S | 1382 | SG | CYS | A | 290 | 191.434 | 178.234 | -0.820 | 1.00 | 22.10 | A |
| ATOM C | 1383 | C | CYS | A | 290 | 194.003 | 176.211 | -0.453 | 1.00 | 17.81 | A |
| ATOM O | 1384 | O | CYS | A | 290 | 193.554 | 175.403 | -1.266 | 1.00 | 15.28 | A |
| ATOM N | 1385 | N | GLY | A | 291 | 194.340 | 175.862 | 0.786 | 1.00 | 19.11 | A |
| ATOM C | 1386 | CA | GLY | A | 291 | 194.203 | 174.484 | 1.227 | 1.00 | 18.98 | A |
| ATOM C | 1387 | C | GLY | A | 291 | 195.536 | 173.896 | 1.644 | 1.00 | 19.71 | A |
| ATOM O | 1388 | O | GLY | A | 291 | 196.458 | 174.606 | 2.047 | 1.00 | 21.01 | A |
| ATOM N | 1389 | N | THR | A | 292 | 195.626 | 172.584 | 1.523 | 1.00 | 18.60 | A |
| ATOM C | 1390 | CA | THR | A | 292 | 196.800 | 171.837 | 1.903 | 1.00 | 16.87 | A |
| ATOM C | 1391 | CB | THR | A | 292 | 196.378 | 170.657 | 2.760 | 1.00 | 19.75 | A |
| ATOM O | 1392 | OG1 | THR | A | 292 | 195.457 | 171.126 | 3.752 | 1.00 | 25.57 | A |
| ATOM C | 1393 | CG2 | THR | A | 292 | 197.563 | 169.991 | 3.407 | 1.00 | 18.64 | A |
| ATOM C | 1394 | C | THR | A | 292 | 197.453 | 171.308 | 0.652 | 1.00 | 17.38 | A |
| ATOM O | 1395 | O | THR | A | 292 | 196.772 | 170.864 | -0.269 | 1.00 | 16.20 | A |
| ATOM N | 1396 | N | LEU | A | 293 | 198.780 | 171.347 | 0.631 | 1.00 | 18.38 | A |
| ATOM C | 1397 | CA | LEU | A | 293 | 199.545 | 170.868 | -0.511 | 1.00 | 18.67 | A |
| ATOM C | 1398 | CB | LEU | A | 293 | 201.011 | 170.747 | -0.102 | 1.00 | 18.33 | A |
| ATOM C | 1399 | CG | LEU | A | 293 | 201.947 | 170.217 | -1.188 | 1.00 | 19.70 | A |
| ATOM C | 1400 | CD1 | LEU | A | 293 | 201.997 | 171.229 | -2.316 | 1.00 | 20.14 | A |
| ATOM C | 1401 | CD2 | LEU | A | 293 | 203.332 | 169.962 | -0.603 | 1.00 | 18.52 | A |
| ATOM C | 1402 | C | LEU | A | 293 | 199.063 | 169.533 | -1.126 | 1.00 | 19.06 | A |
| ATOM O | 1403 | O | LEU | A | 293 | 198.654 | 169.496 | -2.287 | 1.00 | 16.78 | A |
| ATOM N | 1404 | N | ASP | A | 294 | 199.106 | 168.447 | -0.355 | 1.00 | 16.52 | A |
| ATOM C | 1405 | CA | ASP | A | 294 | 198.701 | 167.143 | -0.868 | 1.00 | 15.08 | A |
| ATOM C | 1406 | CB | ASP | A | 294 | 199.029 | 166.048 | 0.164 | 1.00 | 19.73 | A |
| ATOM C | 1407 | CG | ASP | A | 294 | 200.527 | 165.669 | 0.175 | 1.00 | 23.92 | A |
| ATOM O | 1408 | OD1 | ASP | A | 294 | 201.065 | 165.254 | -0.891 | 1.00 | 23.94 | A |
| ATOM O | 1409 | OD2 | ASP | A | 294 | 201.161 | 165.783 | 1.245 | 1.00 | 24.71 | A |
| ATOM C | 1410 | C | ASP | A | 294 | 197.239 | 167.026 | -1.309 | 1.00 | 14.50 | A |
| ATOM O | 1411 | O | ASP | A | 294 | 196.875 | 166.084 | -2.011 | 1.00 | 11.76 | A |
| ATOM N | 1412 | N | TYR | A | 295 | 196.411 | 167.996 | -0.923 | 1.00 | 13.78 | A |
| ATOM C | 1413 | CA | TYR | A | 295 | 194.993 | 167.963 | -1.267 | 1.00 | 12.35 | A |
| ATOM C | 1414 | CB | TYR | A | 295 | 194.156 | 168.157 | -0.001 | 1.00 | 12.74 | A |
| ATOM C | 1415 | CG | TYR | A | 295 | 194.061 | 166.933 | 0.868 | 1.00 | 16.79 | A |
| ATOM C | 1416 | CD1 | TYR | A | 295 | 195.179 | 166.449 | 1.561 | 1.00 | 19.00 | A |
| ATOM C | 1417 | CE1 | TYR | A | 295 | 195.119 | 165.265 | 2.307 | 1.00 | 18.70 | A |
| ATOM C | 1418 | CD2 | TYR | A | 295 | 192.871 | 166.216 | 0.947 | 1.00 | 16.93 | A |
| ATOM C | 1419 | CE2 | TYR | A | 295 | 192.792 | 165.035 | 1.682 | 1.00 | 21.69 | A |
| ATOM C | 1420 | CZ | TYR | A | 295 | 193.916 | 164.559 | 2.357 | 1.00 | 22.19 | A |
| ATOM O | 1421 | OH | TYR | A | 295 | 193.805 | 163.362 | 3.046 | 1.00 | 26.24 | A |
| ATOM C | 1422 | C | TYR | A | 295 | 194.542 | 168.963 | -2.336 | 1.00 | 10.66 | A |
| ATOM O | 1423 | O | TYR | A | 295 | 193.387 | 168.955 | -2.741 | 1.00 | 11.85 | A |
| ATOM N | 1424 | N | LEU | A | 296 | 195.460 | 169.797 | -2.805 | 1.00 | 10.92 | A |
| ATOM C | 1425 | CA | LEU | A | 296 | 195.119 | 170.800 | -3.807 | 1.00 | 13.78 | A |
| ATOM C | 1426 | CB | LEU | A | 296 | 196.328 | 171.679 | -4.144 | 1.00 | 11.44 | A |
| ATOM C | 1427 | CG | LEU | A | 296 | 196.931 | 172.488 | -2.995 | 1.00 | 15.78 | A |
| ATOM C | 1428 | CD1 | LEU | A | 296 | 198.287 | 173.081 | -3.411 | 1.00 | 13.74 | A |
| ATOM C | 1429 | CD2 | LEU | A | 296 | 195.954 | 173.585 | -2.582 | 1.00 | 15.05 | A |
| ATOM C | 1430 | C | LEU | A | 296 | 194.591 | 170.206 | -5.108 | 1.00 | 14.17 | A |
| ATOM O | 1431 | O | LEU | A | 296 | 195.144 | 169.255 | -5.648 | 1.00 | 12.20 | A |
| ATOM N | 1432 | N | PRO | A | 297 | 193.504 | 170.783 | -5.631 | 1.00 | 13.56 | A |
| ATOM C | 1433 | CD | PRO | A | 297 | 192.676 | 171.841 | -5.015 | 1.00 | 13.83 | A |
| ATOM C | 1434 | CA | PRO | A | 297 | 192.921 | 170.302 | -6.893 | 1.00 | 12.38 | A |
| ATOM C | 1435 | CB | PRO | A | 297 | 191.516 | 170.923 | -6.872 | 1.00 | 14.10 | A |
| ATOM C | 1436 | CG | PRO | A | 297 | 191.744 | 172.233 | -6.155 | 1.00 | 13.16 | A |
| ATOM C | 1437 | C | PRO | A | 297 | 193.772 | 170.826 | -8.083 | 1.00 | 14.86 | A |
| ATOM O | 1438 | O | PRO | A | 297 | 194.361 | 171.906 | -7.993 | 1.00 | 14.20 | A |
| ATOM N | 1439 | N | PRO | A | 298 | 193.830 | 170.080 | -9.213 | 1.00 | 14.90 | A |
| ATOM C | 1440 | CD | PRO | A | 298 | 193.074 | 168.838 | -9.478 | 1.00 | 13.08 | A |
| ATOM C | 1441 | CA | PRO | A | 298 | 194.606 | 170.479 | -10.403 | 1.00 | 12.06 | A |
| ATOM C | 1442 | CB | PRO | A | 298 | 194.228 | 169.428 | -11.450 | 1.00 | 12.23 | A |
| ATOM C | 1443 | CG | PRO | A | 298 | 193.813 | 168.254 | -10.653 | 1.00 | 11.96 | A |
| ATOM C | 1444 | C | PRO | A | 298 | 194.250 | 171.874 | -10.894 | 1.00 | 12.75 | A |
| ATOM O | 1445 | O | PRO | A | 298 | 195.124 | 172.677 | -11.218 | 1.00 | 12.47 | A |
| ATOM N | 1446 | N | GLU | A | 299 | 192.954 | 172.154 | -10.954 | 1.00 | 12.43 | A |
| ATOM C | 1447 | CA | GLU | A | 299 | 192.495 | 173.447 | -11.427 | 1.00 | 15.93 | A |
| ATOM C | 1448 | CB | GLU | A | 299 | 190.956 | 173.520 | -11.439 | 1.00 | 15.99 | A |
| ATOM C | 1449 | CG | GLU | A | 299 | 190.283 | 173.211 | -10.098 | 1.00 | 20.28 | A |
| ATOM C | 1450 | CD | GLU | A | 299 | 189.893 | 171.729 | -9.951 | 1.00 | 22.71 | A |
| ATOM O | 1451 | OE1 | GLU | A | 299 | 190.741 | 170.854 | -10.262 | 1.00 | 22.72 | A |
| ATOM O | 1452 | OE2 | GLU | A | 299 | 188.743 | 171.450 | -9.525 | 1.00 | 19.63 | A |
| ATOM C | 1453 | C | GLU | A | 299 | 193.068 | 174.609 | -10.615 | 1.00 | 17.99 | A |
| ATOM O | 1454 | O | GLU | A | 299 | 193.217 | 175.706 | -11.147 | 1.00 | 21.24 | A |
| ATOM N | 1455 | N | MET | A | 300 | 193.385 | 174.401 | -9.340 | 1.00 | 17.80 | A |
| ATOM C | 1456 | CA | MET | A | 300 | 193.949 | 175.497 | -8.541 | 1.00 | 21.17 | A |
| ATOM C | 1457 | CB | MET | A | 300 | 193.781 | 175.248 | -7.031 | 1.00 | 21.87 | A |
| ATOM C | 1458 | CG | MET | A | 300 | 192.562 | 175.935 | -6.417 | 1.00 | 24.58 | A |
| ATOM S | 1459 | SD | MET | A | 300 | 192.223 | 175.516 | -4.665 | 1.00 | 23.96 | A |
| ATOM C | 1460 | CE | MET | A | 300 | 193.835 | 175.427 | -4.097 | 1.00 | 25.92 | A |
| ATOM C | 1461 | C | MET | A | 300 | 195.429 | 175.697 | -8.845 | 1.00 | 22.52 | A |
| ATOM O | 1462 | O | MET | A | 300 | 195.900 | 176.818 | -9.034 | 1.00 | 17.97 | A |
| ATOM N | 1463 | N | ILE | A | 301 | 196.153 | 174.592 | -8.905 | 1.00 | 25.70 | A |
| ATOM C | 1464 | CA | ILE | A | 301 | 197.573 | 174.627 | -9.160 | 1.00 | 28.05 | A |
| ATOM C | 1465 | CB | ILE | A | 301 | 198.162 | 173.192 | -9.173 | 1.00 | 31.23 | A |
| ATOM C | 1466 | CG2 | ILE | A | 301 | 199.640 | 173.228 | -9.502 | 1.00 | 33.00 | A |
| ATOM C | 1467 | CG1 | ILE | A | 301 | 197.977 | 172.525 | -7.813 | 1.00 | 34.03 | A |
| ATOM C | 1468 | CD1 | ILE | A | 301 | 198.227 | 171.019 | -7.871 | 1.00 | 39.59 | A |
| ATOM C | 1469 | C | ILE | A | 301 | 197.857 | 175.289 | -10.501 | 1.00 | 28.39 | A |
| ATOM O | 1470 | O | ILE | A | 301 | 198.736 | 176.140 | -10.608 | 1.00 | 28.70 | A |
| ATOM N | 1471 | N | GLU | A | 302 | 197.091 | 174.902 | -11.513 | 1.00 | 29.15 | A |
| ATOM C | 1472 | CA | GLU | A | 302 | 197.260 | 175.411 | -12.864 | 1.00 | 27.63 | A |
| ATOM C | 1473 | CB | GLU | A | 302 | 196.614 | 174.449 | -13.847 | 1.00 | 28.51 | A |
| ATOM C | 1474 | CG | GLU | A | 302 | 197.309 | 173.128 | -13.932 | 1.00 | 31.90 | A |
| ATOM C | 1475 | CD | GLU | A | 302 | 196.442 | 172.074 | -14.604 | 1.00 | 34.88 | A |
| ATOM O | 1476 | OE1 | GLU | A | 302 | 195.546 | 172.441 | -15.420 | 1.00 | 35.44 | A |
| ATOM O | 1477 | OE2 | GLU | A | 302 | 196.663 | 170.878 | -14.322 | 1.00 | 33.88 | A |
| ATOM C | 1478 | C | GLU | A | 302 | 196.759 | 176.817 | -13.160 | 1.00 | 26.56 | A |
| ATOM O | 1479 | O | GLU | A | 302 | 196.730 | 177.216 | -14.323 | 1.00 | 25.45 | A |
| ATOM N | 1480 | N | GLY | A | 303 | 196.351 | 177.553 | -12.130 | 1.00 | 26.68 | A |
| ATOM C | 1481 | CA | GLY | A | 303 | 195.891 | 178.915 | -12.333 | 1.00 | 24.63 | A |
| ATOM C | 1482 | C | GLY | A | 303 | 194.576 | 179.072 | -13.077 | 1.00 | 26.17 | A |
| ATOM O | 1483 | O | GLY | A | 303 | 194.333 | 180.080 | -13.739 | 1.00 | 25.60 | A |
| ATOM N | 1484 | N | ARG | A | 304 | 193.718 | 178.068 | -12.976 | 1.00 | 27.24 | A |
| ATOM C | 1485 | CA | ARG | A | 304 | 192.409 | 178.095 | -13.617 | 1.00 | 26.83 | A |
| ATOM C | 1486 | CB | ARG | A | 304 | 192.068 | 176.721 | -14.229 | 1.00 | 26.35 | A |
| ATOM C | 1487 | CG | ARG | A | 304 | 192.726 | 176.410 | -15.584 | 1.00 | 35.40 | A |
| ATOM C | 1488 | CD | ARG | A | 304 | 191.737 | 176.691 | -16.749 | 1.00 | 43.36 | A |
| ATOM N | 1489 | NE | ARG | A | 304 | 192.316 | 176.665 | -18.095 | 1.00 | 47.14 | A |
| ATOM C | 1490 | CZ | ARG | A | 304 | 191.602 | 176.827 | -19.210 | 1.00 | 51.59 | A |
| ATOM N | 1491 | NH1 | ARG | A | 304 | 190.290 | 177.025 | -19.121 | 1.00 | 54.10 | A |
| ATOM N | 1492 | NH2 | ARG | A | 304 | 192.189 | 176.794 | -20.413 | 1.00 | 53.71 | A |
| ATOM C | 1493 | C | ARG | A | 304 | 191.386 | 178.429 | -12.536 | 1.00 | 27.81 | A |
| ATOM O | 1494 | O | ARG | A | 304 | 191.684 | 178.391 | -11.326 | 1.00 | 29.71 | A |
| ATOM N | 1495 | N | MET | A | 305 | 190.178 | 178.746 | -12.969 | 1.00 | 25.68 | A |
| ATOM C | 1496 | CA | MET | A | 305 | 189.112 | 179.048 | -12.038 | 1.00 | 26.17 | A |
| ATOM C | 1497 | CB | MET | A | 305 | 188.027 | 179.807 | -12.757 | 1.00 | 30.29 | A |
| ATOM C | 1498 | CG | MET | A | 305 | 188.517 | 181.155 | -13.154 | 1.00 | 35.60 | A |
| ATOM S | 1499 | SD | MET | A | 305 | 187.192 | 182.156 | -13.740 | 1.00 | 46.48 | A |
| ATOM C | 1500 | CE | MET | A | 305 | 187.949 | 182.896 | -15.291 | 1.00 | 45.95 | A |
| ATOM C | 1501 | C | MET | A | 305 | 188.544 | 177.772 | -11.466 | 1.00 | 23.39 | A |
| ATOM O | 1502 | O | MET | A | 305 | 188.653 | 176.721 | -12.082 | 1.00 | 23.54 | A |
| ATOM N | 1503 | N | HIS | A | 306 | 187.934 | 177.852 | -10.290 | 1.00 | 21.16 | A |
| ATOM C | 1504 | CA | HIS | A | 306 | 187.363 | 176.655 | -9.703 | 1.00 | 17.32 | A |
| ATOM C | 1505 | CB | HIS | A | 306 | 188.312 | 176.035 | -8.671 | 1.00 | 15.79 | A |
| ATOM C | 1506 | CG | HIS | A | 306 | 188.498 | 176.853 | -7.431 | 1.00 | 14.36 | A |
| ATOM C | 1507 | CD2 | HIS | A | 306 | 187.895 | 176.788 | -6.218 | 1.00 | 12.29 | A |
| ATOM N | 1508 | ND1 | HIS | A | 306 | 189.411 | 177.880 | -7.347 | 1.00 | 15.91 | A |
| ATOM C | 1509 | CE1 | HIS | A | 306 | 189.366 | 178.411 | -6.139 | 1.00 | 13.45 | A |
| ATOM N | 1510 | NE2 | HIS | A | 306 | 188.453 | 177.767 | -5.434 | 1.00 | 10.38 | A |
| ATOM C | 1511 | C | HIS | A | 306 | 186.000 | 176.913 | -9.078 | 1.00 | 18.65 | A |
| ATOM O | 1512 | O | HIS | A | 306 | 185.528 | 178.063 | -9.040 | 1.00 | 15.46 | A |
| ATOM N | 1513 | N | ASP | A | 307 | 185.376 | 175.822 | -8.622 | 1.00 | 18.40 | A |
| ATOM C | 1514 | CA | ASP | A | 307 | 184.057 | 175.824 | -7.986 | 1.00 | 20.30 | A |
| ATOM C | 1515 | CB | ASP | A | 307 | 182.969 | 175.631 | -9.035 | 1.00 | 24.34 | A |
| ATOM C | 1516 | CG | ASP | A | 307 | 183.174 | 174.374 | -9.865 | 1.00 | 30.83 | A |
| ATOM O | 1517 | OD1 | ASP | A | 307 | 183.553 | 173.321 | -9.286 | 1.00 | 33.25 | A |
| ATOM O | 1518 | OD2 | ASP | A | 307 | 182.949 | 174.430 | -11.098 | 1.00 | 33.88 | A |
| ATOM C | 1519 | C | ASP | A | 307 | 183.948 | 174.721 | -6.917 | 1.00 | 19.00 | A |
| ATOM O | 1520 | O | ASP | A | 307 | 184.973 | 174.176 | -6.486 | 1.00 | 21.60 | A |
| ATOM N | 1521 | N | GLU | A | 308 | 182.720 | 174.368 | -6.519 | 1.00 | 18.46 | A |
| ATOM C | 1522 | CA | GLU | A | 308 | 182.507 | 173.358 | -5.479 | 1.00 | 17.19 | A |
| ATOM C | 1523 | CB | GLU | A | 308 | 181.014 | 173.257 | -5.075 | 1.00 | 19.78 | A |
| ATOM C | 1524 | CG | GLU | A | 308 | 180.067 | 172.849 | -6.190 | 1.00 | 29.00 | A |
| ATOM C | 1525 | CD | GLU | A | 308 | 178.600 | 172.827 | -5.777 | 1.00 | 33.58 | A |
| ATOM O | 1526 | OE1 | GLU | A | 308 | 178.121 | 173.800 | -5.139 | 1.00 | 38.06 | A |
| ATOM O | 1527 | OE2 | GLU | A | 308 | 177.910 | 171.836 | -6.115 | 1.00 | 41.55 | A |
| ATOM C | 1528 | C | GLU | A | 308 | 183.048 | 171.977 | -5.820 | 1.00 | 14.99 | A |
| ATOM O | 1529 | O | GLU | A | 308 | 183.135 | 171.106 | -4.946 | 1.00 | 14.14 | A |
| ATOM N | 1530 | N | LYS | A | 309 | 183.452 | 171.780 | -7.066 | 1.00 | 12.83 | A |
| ATOM C | 1531 | CA | LYS | A | 309 | 183.997 | 170.488 | -7.449 | 1.00 | 16.26 | A |
| ATOM C | 1532 | CB | LYS | A | 309 | 184.061 | 170.376 | -8.972 | 1.00 | 18.82 | A |
| ATOM C | 1533 | CG | LYS | A | 309 | 182.731 | 170.086 | -9.593 | 1.00 | 21.29 | A |
| ATOM C | 1534 | CD | LYS | A | 309 | 182.215 | 168.769 | -9.041 | 1.00 | 29.52 | A |
| ATOM C | 1535 | CE | LYS | A | 309 | 181.020 | 168.263 | -9.842 | 1.00 | 30.17 | A |
| ATOM N | 1536 | NZ | LYS | A | 309 | 180.649 | 166.912 | -9.358 | 1.00 | 35.57 | A |
| ATOM C | 1537 | C | LYS | A | 309 | 185.370 | 170.202 | -6.826 | 1.00 | 16.49 | A |
| ATOM O | 1538 | O | LYS | A | 309 | 185.816 | 169.051 | -6.804 | 1.00 | 15.73 | A |
| ATOM N | 1539 | N | VAL | A | 310 | 186.025 | 171.233 | -6.291 | 1.00 | 15.44 | A |
| ATOM C | 1540 | CA | VAL | A | 310 | 187.319 | 171.020 | -5.662 | 1.00 | 13.90 | A |
| ATOM C | 1541 | CB | VAL | A | 310 | 188.051 | 172.358 | -5.281 | 1.00 | 15.52 | A |
| ATOM C | 1542 | CG1 | VAL | A | 310 | 188.197 | 173.247 | -6.503 | 1.00 | 7.36 | A |
| ATOM C | 1543 | CG2 | VAL | A | 310 | 187.332 | 173.065 | -4.151 | 1.00 | 12.70 | A |
| ATOM C | 1544 | C | VAL | A | 310 | 187.132 | 170.159 | -4.410 | 1.00 | 17.23 | A |
| ATOM O | 1545 | O | VAL | A | 310 | 188.008 | 169.357 | -4.083 | 1.00 | 19.39 | A |
| ATOM N | 1546 | N | ASP | A | 311 | 186.013 | 170.306 | -3.700 | 1.00 | 14.90 | A |
| ATOM C | 1547 | CA | ASP | A | 311 | 185.804 | 169.452 | -2.541 | 1.00 | 14.20 | A |
| ATOM C | 1548 | CB | ASP | A | 311 | 184.574 | 169.876 | -1.727 | 1.00 | 16.81 | A |
| ATOM C | 1549 | CG | ASP | A | 311 | 184.778 | 171.192 | -0.974 | 1.00 | 16.28 | A |
| ATOM O | 1550 | OD1 | ASP | A | 311 | 185.950 | 171.565 | -0.731 | 1.00 | 12.12 | A |
| ATOM O | 1551 | OD2 | ASP | A | 311 | 183.756 | 171.832 | -0.617 | 1.00 | 13.33 | A |
| ATOM C | 1552 | C | ASP | A | 311 | 185.640 | 167.983 | -2.979 | 1.00 | 14.98 | A |
| ATOM O | 1553 | O | ASP | A | 311 | 185.953 | 167.090 | -2.202 | 1.00 | 16.71 | A |
| ATOM N | 1554 | N | LEU | A | 312 | 185.162 | 167.723 | -4.205 | 1.00 | 13.32 | A |
| ATOM C | 1555 | CA | LEU | A | 312 | 185.019 | 166.342 | -4.703 | 1.00 | 13.83 | A |
| ATOM C | 1556 | CB | LEU | A | 312 | 184.238 | 166.279 | -6.028 | 1.00 | 12.75 | A |
| ATOM C | 1557 | CG | LEU | A | 312 | 182.708 | 166.216 | -6.008 | 1.00 | 16.02 | A |
| ATOM C | 1558 | CD1 | LEU | A | 312 | 182.269 | 164.959 | -5.273 | 1.00 | 14.12 | A |
| ATOM C | 1559 | CD2 | LEU | A | 312 | 182.118 | 167.482 | -5.336 | 1.00 | 16.09 | A |
| ATOM C | 1560 | C | LEU | A | 312 | 186.399 | 165.749 | -4.951 | 1.00 | 13.94 | A |
| ATOM O | 1561 | O | LEU | A | 312 | 186.616 | 164.546 | -4.837 | 1.00 | 15.77 | A |
| ATOM N | 1562 | N | TRP | A | 313 | 187.338 | 166.594 | -5.329 | 1.00 | 15.19 | A |
| ATOM C | 1563 | CA | TRP | A | 313 | 188.673 | 166.093 | -5.553 | 1.00 | 13.67 | A |
| ATOM C | 1564 | CB | TRP | A | 313 | 189.505 | 167.131 | -6.287 | 1.00 | 10.13 | A |
| ATOM C | 1565 | CG | TRP | A | 313 | 190.948 | 166.889 | -6.208 | 1.00 | 9.91 | A |
| ATOM C | 1566 | CD2 | TRP | A | 313 | 191.775 | 166.296 | -7.214 | 1.00 | 11.32 | A |
| ATOM C | 1567 | CE2 | TRP | A | 313 | 193.109 | 166.370 | -6.750 | 1.00 | 10.99 | A |
| ATOM C | 1568 | CE3 | TRP | A | 313 | 191.523 | 165.729 | -8.471 | 1.00 | 8.98 | A |
| ATOM C | 1569 | CD1 | TRP | A | 313 | 191.784 | 167.266 | -5.199 | 1.00 | 10.59 | A |
| ATOM N | 1570 | NE1 | TRP | A | 313 | 193.083 | 166.960 | -5.513 | 1.00 | 9.03 | A |
| ATOM C | 1571 | CZ2 | TRP | A | 313 | 194.190 | 165.890 | -7.494 | 1.00 | 10.17 | A |
| ATOM C | 1572 | CZ3 | TRP | A | 313 | 192.598 | 165.254 | -9.214 | 1.00 | 13.43 | A |
| ATOM C | 1573 | CH2 | TRP | A | 313 | 193.923 | 165.344 | -8.723 | 1.00 | 12.05 | A |
| ATOM C | 1574 | C | TRP | A | 313 | 189.258 | 165.776 | -4.183 | 1.00 | 12.20 | A |
| ATOM O | 1575 | O | TRP | A | 313 | 189.803 | 164.704 | -3.983 | 1.00 | 11.38 | A |
| ATOM N | 1576 | N | SER | A | 314 | 189.111 | 166.700 | -3.236 | 1.00 | 13.68 | A |
| ATOM C | 1577 | CA | SER | A | 314 | 189.636 | 166.492 | -1.893 | 1.00 | 16.07 | A |
| ATOM C | 1578 | CB | SER | A | 314 | 189.229 | 167.643 | -0.984 | 1.00 | 17.53 | A |
| ATOM O | 1579 | OG | SER | A | 314 | 190.261 | 168.612 | -0.972 | 1.00 | 25.41 | A |
| ATOM C | 1580 | C | SER | A | 314 | 189.172 | 165.168 | -1.283 | 1.00 | 16.32 | A |
| ATOM O | 1581 | O | SER | A | 314 | 189.942 | 164.475 | -0.628 | 1.00 | 15.29 | A |
| ATOM N | 1582 | N | LEU | A | 315 | 187.907 | 164.838 | -1.517 | 1.00 | 15.55 | A |
| ATOM C | 1583 | CA | LEU | A | 315 | 187.311 | 163.618 | -1.029 | 1.00 | 15.03 | A |
| ATOM C | 1584 | CB | LEU | A | 315 | 185.822 | 163.597 | -1.392 | 1.00 | 15.51 | A |
| ATOM C | 1585 | CG | LEU | A | 315 | 184.951 | 162.599 | -0.624 | 1.00 | 17.84 | A |
| ATOM C | 1586 | CD1 | LEU | A | 315 | 184.877 | 163.015 | 0.840 | 1.00 | 17.24 | A |
| ATOM C | 1587 | CD2 | LEU | A | 315 | 183.555 | 162.551 | -1.222 | 1.00 | 15.53 | A |
| ATOM C | 1588 | C | LEU | A | 315 | 188.031 | 162.402 | -1.626 | 1.00 | 15.76 | A |
| ATOM O | 1589 | O | LEU | A | 315 | 188.237 | 161.399 | -0.935 | 1.00 | 16.26 | A |
| ATOM N | 1590 | N | GLY | A | 316 | 188.414 | 162.480 | -2.899 | 1.00 | 13.03 | A |
| ATOM C | 1591 | CA | GLY | A | 316 | 189.109 | 161.357 | -3.507 | 1.00 | 12.73 | A |
| ATOM C | 1592 | C | GLY | A | 316 | 190.486 | 161.200 | -2.878 | 1.00 | 13.41 | A |
| ATOM O | 1593 | O | GLY | A | 316 | 190.933 | 160.092 | -2.576 | 1.00 | 11.78 | A |
| ATOM N | 1594 | N | VAL | A | 317 | 191.167 | 162.329 | -2.702 | 1.00 | 12.49 | A |
| ATOM C | 1595 | CA | VAL | A | 317 | 192.481 | 162.351 | -2.089 | 1.00 | 11.35 | A |
| ATOM C | 1596 | CB | VAL | A | 317 | 193.034 | 163.812 | -1.984 | 1.00 | 11.65 | A |
| ATOM C | 1597 | CG1 | VAL | A | 317 | 194.231 | 163.859 | -1.079 | 1.00 | 10.54 | A |
| ATOM C | 1598 | CG2 | VAL | A | 317 | 193.431 | 164.323 | -3.360 | 1.00 | 8.84 | A |
| ATOM C | 1599 | C | VAL | A | 317 | 192.362 | 161.759 | -0.680 | 1.00 | 12.97 | A |
| ATOM O | 1600 | O | VAL | A | 317 | 193.152 | 160.887 | -0.280 | 1.00 | 11.80 | A |
| ATOM N | 1601 | N | LEU | A | 318 | 191.355 | 162.229 | 0.052 | 1.00 | 10.99 | A |
| ATOM C | 1602 | CA | LEU | A | 318 | 191.097 | 161.785 | 1.408 | 1.00 | 12.40 | A |
| ATOM C | 1603 | CB | LEU | A | 318 | 189.958 | 162.618 | 2.007 | 1.00 | 15.26 | A |
| ATOM C | 1604 | CG | LEU | A | 318 | 189.652 | 162.340 | 3.475 | 1.00 | 15.97 | A |
| ATOM C | 1605 | CD1 | LEU | A | 318 | 190.731 | 162.990 | 4.377 | 1.00 | 14.54 | A |
| ATOM C | 1606 | CD2 | LEU | A | 318 | 188.310 | 162.873 | 3.783 | 1.00 | 13.89 | A |
| ATOM C | 1607 | C | LEU | A | 318 | 190.760 | 160.286 | 1.527 | 1.00 | 12.64 | A |
| ATOM O | 1608 | O | LEU | A | 318 | 191.232 | 159.610 | 2.437 | 1.00 | 10.92 | A |
| ATOM N | 1609 | N | CYS | A | 319 | 189.936 | 159.778 | 0.617 | 1.00 | 12.91 | A |
| ATOM C | 1610 | CA | CYS | A | 319 | 189.547 | 158.372 | 0.623 | 1.00 | 15.63 | A |
| ATOM C | 1611 | CB | CYS | A | 319 | 188.512 | 158.092 | -0.479 | 1.00 | 20.57 | A |
| ATOM S | 1612 | SG | CYS | A | 319 | 187.933 | 156.363 | -0.531 | 1.00 | 23.04 | A |
| ATOM C | 1613 | C | CYS | A | 319 | 190.757 | 157.490 | 0.394 | 1.00 | 15.66 | A |
| ATOM O | 1614 | O | CYS | A | 319 | 190.906 | 156.439 | 1.025 | 1.00 | 15.68 | A |
| ATOM N | 1615 | N | TYR | A | 320 | 191.614 | 157.922 | -0.527 | 1.00 | 13.11 | A |
| ATOM C | 1616 | CA | TYR | A | 320 | 192.833 | 157.194 | -0.827 | 1.00 | 12.42 | A |
| ATOM C | 1617 | CB | TYR | A | 320 | 193.560 | 157.870 | -1.999 | 1.00 | 12.90 | A |
| ATOM C | 1618 | CG | TYR | A | 320 | 194.879 | 157.235 | -2.378 | 1.00 | 11.79 | A |
| ATOM C | 1619 | CD1 | TYR | A | 320 | 196.014 | 157.423 | -1.594 | 1.00 | 11.63 | A |
| ATOM C | 1620 | CE1 | TYR | A | 320 | 197.213 | 156.803 | -1.906 | 1.00 | 13.39 | A |
| ATOM C | 1621 | CD2 | TYR | A | 320 | 194.978 | 156.414 | -3.495 | 1.00 | 13.02 | A |
| ATOM C | 1622 | CE2 | TYR | A | 320 | 196.167 | 155.790 | -3.822 | 1.00 | 14.81 | A |
| ATOM C | 1623 | CZ | TYR | A | 320 | 197.287 | 155.980 | -3.023 | 1.00 | 16.61 | A |
| ATOM O | 1624 | OH | TYR | A | 320 | 198.459 | 155.299 | -3.325 | 1.00 | 14.17 | A |
| ATOM C | 1625 | C | TYR | A | 320 | 193.727 | 157.138 | 0.428 | 1.00 | 13.58 | A |
| ATOM O | 1626 | O | TYR | A | 320 | 194.217 | 156.062 | 0.797 | 1.00 | 12.75 | A |
| ATOM N | 1627 | N | GLU | A | 321 | 193.922 | 158.287 | 1.083 | 1.00 | 12.98 | A |
| ATOM C | 1628 | CA | GLU | A | 321 | 194.752 | 158.359 | 2.289 | 1.00 | 13.87 | A |
| ATOM C | 1629 | CB | GLU | A | 321 | 194.858 | 159.798 | 2.815 | 1.00 | 15.03 | A |
| ATOM C | 1630 | CG | GLU | A | 321 | 196.109 | 160.011 | 3.685 | 1.00 | 22.02 | A |
| ATOM C | 1631 | CD | GLU | A | 321 | 196.204 | 161.394 | 4.326 | 1.00 | 27.52 | A |
| ATOM O | 1632 | OE1 | GLU | A | 321 | 195.889 | 162.413 | 3.676 | 1.00 | 30.94 | A |
| ATOM O | 1633 | OE2 | GLU | A | 321 | 196.621 | 161.470 | 5.494 | 1.00 | 31.35 | A |
| ATOM C | 1634 | C | GLU | A | 321 | 194.218 | 157.470 | 3.416 | 1.00 | 14.01 | A |
| ATOM O | 1635 | O | GLU | A | 321 | 194.986 | 156.877 | 4.166 | 1.00 | 14.13 | A |
| ATOM N | 1636 | N | PHE | A | 322 | 192.899 | 157.384 | 3.536 | 1.00 | 13.55 | A |
| ATOM C | 1637 | CA | PHE | A | 322 | 192.291 | 156.568 | 4.576 | 1.00 | 12.09 | A |
| ATOM C | 1638 | CB | PHE | A | 322 | 190.765 | 156.710 | 4.570 | 1.00 | 6.70 | A |
| ATOM C | 1639 | CG | PHE | A | 322 | 190.272 | 157.975 | 5.176 | 1.00 | 6.79 | A |
| ATOM C | 1640 | CD1 | PHE | A | 322 | 191.144 | 158.836 | 5.838 | 1.00 | 7.89 | A |
| ATOM C | 1641 | CD2 | PHE | A | 322 | 188.933 | 158.312 | 5.106 | 1.00 | 8.09 | A |
| ATOM C | 1642 | CE1 | PHE | A | 322 | 190.688 | 160.011 | 6.416 | 1.00 | 6.90 | A |
| ATOM C | 1643 | CE2 | PHE | A | 322 | 188.464 | 159.497 | 5.689 | 1.00 | 10.00 | A |
| ATOM C | 1644 | CZ | PHE | A | 322 | 189.346 | 160.340 | 6.345 | 1.00 | 8.24 | A |
| ATOM C | 1645 | C | PHE | A | 322 | 192.629 | 155.107 | 4.398 | 1.00 | 12.56 | A |
| ATOM O | 1646 | O | PHE | A | 322 | 192.888 | 154.401 | 5.375 | 1.00 | 14.57 | A |
| ATOM N | 1647 | N | LEU | A | 323 | 192.629 | 154.663 | 3.147 | 1.00 | 11.23 | A |
| ATOM C | 1648 | CA | LEU | A | 323 | 192.876 | 153.269 | 2.830 | 1.00 | 10.81 | A |
| ATOM C | 1649 | CB | LEU | A | 323 | 192.181 | 152.923 | 1.510 | 1.00 | 11.58 | A |
| ATOM C | 1650 | CG | LEU | A | 323 | 190.650 | 152.911 | 1.467 | 1.00 | 11.82 | A |
| ATOM C | 1651 | CD1 | LEU | A | 323 | 190.149 | 152.693 | 0.033 | 1.00 | 1.00 | A |
| ATOM C | 1652 | CD2 | LEU | A | 323 | 190.158 | 151.799 | 2.429 | 1.00 | 8.83 | A |
| ATOM C | 1653 | C | LEU | A | 323 | 194.3 | 2.888 | 2.725 | 1.00 | 13.31 | A |
| ATOM O | 1654 | O | LEU | A | 323 | 194.734 | 151.769 | 3.072 | 1.00 | 13.08 | A |
| ATOM N | 1655 | N | VAL | A | 324 | 195.144 | 153.832 | 2.253 | 1.00 | 13.79 | A |
| ATOM C | 1656 | CA | VAL | A | 324 | 196.552 | 153.589 | 2.028 | 1.00 | 13.43 | A |
| ATOM C | 1657 | CB | VAL | A | 324 | 196.958 | 154.241 | 0.703 | 1.00 | 13.53 | A |
| ATOM C | 1658 | CG1 | VAL | A | 324 | 198.387 | 153.909 | 0.365 | 1.00 | 10.33 | A |
| ATOM C | 1659 | CG2 | VAL | A | 324 | 196.009 | 153.788 | -0.384 | 1.00 | 10.31 | A |
| ATOM C | 1660 | C | VAL | A | 324 | 197.439 | 154.085 | 3.153 | 1.00 | 15.36 | A |
| ATOM O | 1661 | O | VAL | A | 324 | 198.496 | 153.525 | 3.419 | 1.00 | 15.10 | A |
| ATOM N | 1662 | N | GLY | A | 325 | 197.019 | 155.146 | 3.819 | 1.00 | 16.58 | A |
| ATOM C | 1663 | CA | GLY | A | 325 | 197.834 | 155.647 | 4.902 | 1.00 | 18.98 | A |
| ATOM C | 1664 | C | GLY | A | 325 | 198.687 | 156.819 | 4.459 | 1.00 | 22.31 | A |
| ATOM O | 1665 | O | GLY | A | 325 | 199.484 | 157.330 | 5.245 | 1.00 | 22.91 | A |
| ATOM N | 1666 | N | LYS | A | 326 | 198.556 | 157.238 | 3.204 | 1.00 | 22.34 | A |
| ATOM C | 1667 | CA | LYS | A | 326 | 199.299 | 158.399 | 2.743 | 1.00 | 20.73 | A |
| ATOM C | 1668 | CB | LYS | A | 326 | 200.775 | 158.057 | 2.468 | 1.00 | 22.54 | A |
| ATOM C | 1669 | CG | LYS | A | 326 | 201.002 | 157.016 | 1.405 | 1.00 | 26.76 | A |
| ATOM C | 1670 | CD | LYS | A | 326 | 202.478 | 156.728 | 1.181 | 1.00 | 29.37 | A |
| ATOM C | 1671 | CE | LYS | A | 326 | 202.635 | 155.634 | 0.108 | 1.00 | 36.99 | A |
| ATOM N | 1672 | NZ | LYS | A | 326 | 204.059 | 155.235 | -0.146 | 1.00 | 40.73 | A |
| ATOM C | 1673 | C | LYS | A | 326 | 198.627 | 158.927 | 1.496 | 1.00 | 18.10 | A |
| ATOM O | 1674 | O | LYS | A | 326 | 198.050 | 158.173 | 0.745 | 1.00 | 18.75 | A |
| ATOM N | 1675 | N | PRO | A | 327 | 198.657 | 160.254 | 1.290 | 1.00 | 19.06 | A |
| ATOM C | 1676 | CD | PRO | A | 327 | 199.416 | 161.265 | 2.050 | 1.00 | 18.18 | A |
| ATOM C | 1677 | CA | PRO | A | 327 | 198.046 | 160.877 | 0.111 | 1.00 | 16.77 | A |
| ATOM C | 1678 | CB | PRO | A | 327 | 198.349 | 162.358 | 0.331 | 1.00 | 18.27 | A |
| ATOM C | 1679 | CG | PRO | A | 327 | 199.652 | 162.318 | 0.997 | 1.00 | 17.79 | A |
| ATOM C | 1680 | C | PRO | A | 327 | 198.719 | 160.334 | -1.156 | 1.00 | 15.47 | A |
| ATOM O | 1681 | O | PRO | A | 327 | 199.920 | 160.074 | -1.165 | 1.00 | 17.98 | A |
| ATOM N | 1682 | N | PRO | A | 328 | 197.967 | 160.221 | -2.256 | 1.00 | 15.81 | A |
| ATOM C | 1683 | CD | PRO | A | 328 | 196.629 | 160.830 | -2.419 | 1.00 | 13.52 | A |
| ATOM C | 1684 | CA | PRO | A | 328 | 198.467 | 159.699 | -3.540 | 1.00 | 13.75 | A |
| ATOM C | 1685 | CB | PRO | A | 328 | 197.183 | 159.511 | -4.345 | 1.00 | 15.58 | A |
| ATOM C | 1686 | CG | PRO | A | 328 | 196.384 | 160.712 | -3.922 | 1.00 | 15.99 | A |
| ATOM C | 1687 | C | PRO | A | 328 | 199.517 | 160.502 | -4.312 | 1.00 | 14.29 | A |
| ATOM O | 1688 | O | PRO | A | 328 | 200.226 | 159.947 | -5.150 | 1.00 | 15.51 | A |
| ATOM N | 1689 | N | PHE | A | 329 | 199.627 | 161.798 | -4.039 | 1.00 | 15.34 | A |
| ATOM C | 1690 | CA | PHE | A | 329 | 200.600 | 162.627 | -4.758 | 1.00 | 16.02 | A |
| ATOM C | 1691 | CB | PHE | A | 329 | 199.880 | 163.832 | -5.357 | 1.00 | 11.57 | A |
| ATOM C | 1692 | CG | PHE | A | 329 | 198.677 | 163.456 | -6.171 | 1.00 | 10.41 | A |
| ATOM C | 1693 | CD1 | PHE | A | 329 | 198.831 | 162.822 | -7.393 | 1.00 | 7.22 | A |
| ATOM C | 1694 | CD2 | PHE | A | 329 | 197.389 | 163.646 | -5.672 | 1.00 | 10.88 | A |
| ATOM C | 1695 | CE1 | PHE | A | 329 | 197.722 | 162.365 | -8.117 | 1.00 | 11.91 | A |
| ATOM C | 1696 | CE2 | PHE | A | 329 | 196.267 | 163.194 | -6.387 | 1.00 | 9.29 | A |
| ATOM C | 1697 | CZ | PHE | A | 329 | 196.435 | 162.548 | -7.611 | 1.00 | 11.49 | A |
| ATOM C | 1698 | C | PHE | A | 329 | 201.774 | 163.073 | -3.896 | 1.00 | 18.50 | A |
| ATOM O | 1699 | O | PHE | A | 329 | 202.501 | 163.999 | -4.242 | 1.00 | 17.82 | A |
| ATOM N | 1700 | N | GLU | A | 330 | 201.968 | 162.411 | -2.765 | 1.00 | 20.96 | A |
| ATOM C | 1701 | CA | GLU | A | 330 | 203.061 | 162.781 | -1.872 | 1.00 | 25.68 | A |
| ATOM C | 1702 | CB | GLU | A | 330 | 203.042 | 161.868 | -0.653 | 1.00 | 28.63 | A |
| ATOM C | 1703 | CG | GLU | A | 330 | 203.889 | 162.292 | 0.527 | 1.00 | 35.48 | A |
| ATOM C | 1704 | CD | GLU | A | 330 | 203.618 | 161.381 | 1.725 | 1.00 | 40.14 | A |
| ATOM O | 1705 | OE1 | GLU | A | 330 | 203.825 | 161.798 | 2.890 | 1.00 | 43.93 | A |
| ATOM O | 1706 | OE2 | GLU | A | 330 | 203.186 | 160.230 | 1.490 | 1.00 | 43.19 | A |
| ATOM C | 1707 | C | GLU | A | 330 | 204.404 | 162.666 | -2.593 | 1.00 | 25.36 | A |
| ATOM O | 1708 | O | GLU | A | 330 | 204.712 | 161.627 | -3.177 | 1.00 | 25.32 | A |
| ATOM N | 1709 | N | ALA | A | 331 | 205.195 | 163.733 | -2.551 | 1.00 | 23.31 | A |
| ATOM C | 1710 | CA | ALA | A | 331 | 206.506 | 163.745 | -3.184 | 1.00 | 23.23 | A |
| ATOM C | 1711 | CB | ALA | A | 331 | 206.432 | 164.479 | -4.500 | 1.00 | 19.73 | A |
| ATOM C | 1712 | C | ALA | A | 331 | 207.503 | 164.429 | -2.239 | 1.00 | 24.76 | A |
| ATOM O | 1713 | O | ALA | A | 331 | 207.109 | 165.040 | -1.249 | 1.00 | 22.52 | A |
| ATOM N | 1714 | N | ASN | A | 332 | 208.794 | 164.320 | -2.542 | 1.00 | 27.89 | A |
| ATOM C | 1715 | CA | ASN | A | 332 | 209.822 | 164.922 | -1.692 | 1.00 | 30.47 | A |
| ATOM C | 1716 | CB | ASN | A | 332 | 211.174 | 164.246 | -1.975 | 1.00 | 32.87 | A |
| ATOM C | 1717 | CG | ASN | A | 332 | 211.190 | 162.794 | -1.504 | 1.00 | 37.45 | A |
| ATOM O | 1718 | OD1 | ASN | A | 332 | 210.716 | 162.504 | -0.401 | 1.00 | 36.73 | A |
| ATOM N | 1719 | ND2 | ASN | A | 332 | 211.729 | 161.883 | -2.321 | 1.00 | 37.38 | A |
| ATOM C | 1720 | C | ASN | A | 332 | 209.942 | 166.462 | -1.771 | 1.00 | 29.91 | A |
| ATOM O | 1721 | O | ASN | A | 332 | 210.601 | 167.073 | -0.928 | 1.00 | 29.72 | A |
| ATOM N | 1722 | N | THR | A | 333 | 209.303 | 167.092 | -2.754 | 1.00 | 27.34 | A |
| ATOM C | 1723 | CA | THR | A | 333 | 209.369 | 168.544 | -2.881 | 1.00 | 25.63 | A |
| ATOM C | 1724 | CB | THR | A | 333 | 210.411 | 168.968 | -3.919 | 1.00 | 27.29 | A |
| ATOM O | 1725 | OG1 | THR | A | 333 | 209.862 | 168.766 | -5.232 | 1.00 | 25.31 | A |
| ATOM C | 1726 | CG2 | THR | A | 333 | 211.683 | 168.122 | -3.769 | 1.00 | 28.40 | A |
| ATOM C | 1727 | C | THR | A | 333 | 208.021 | 169.099 | -3.341 | 1.00 | 24.25 | A |
| ATOM O | 1728 | O | THR | A | 333 | 207.168 | 168.355 | -3.832 | 1.00 | 24.65 | A |
| ATOM N | 1729 | N | TYR | A | 334 | 207.839 | 170.406 | -3.177 | 1.00 | 22.97 | A |
| ATOM C | 1730 | CA | TYR | A | 334 | 206.610 | 171.081 | -3.584 | 1.00 | 24.27 | A |
| ATOM C | 1731 | CB | TYR | A | 334 | 206.670 | 172.557 | -3.243 | 1.00 | 26.40 | A |
| ATOM C | 1732 | CG | TYR | A | 334 | 206.122 | 172.899 | -1.885 | 1.00 | 28.73 | A |
| ATOM C | 1733 | CD1 | TYR | A | 334 | 206.846 | 172.639 | -0.725 | 1.00 | 29.53 | A |
| ATOM C | 1734 | CE1 | TYR | A | 334 | 206.322 | 172.945 | 0.537 | 1.00 | 32.11 | A |
| ATOM C | 1735 | CD2 | TYR | A | 334 | 204.863 | 173.476 | -1.759 | 1.00 | 28.50 | A |
| ATOM C | 1736 | CE2 | TYR | A | 334 | 204.329 | 173.794 | -0.497 | 1.00 | 29.43 | A |
| ATOM C | 1737 | CZ | TYR | A | 334 | 205.072 | 173.518 | 0.645 | 1.00 | 31.88 | A |
| ATOM O | 1738 | OH | TYR | A | 334 | 204.597 | 173.783 | 1.910 | 1.00 | 34.80 | A |
| ATOM C | 1739 | C | TYR | A | 334 | 206.379 | 170.979 | -5.088 | 1.00 | 25.01 | A |
| ATOM O | 1740 | O | TYR | A | 334 | 205.267 | 170.712 | -5.567 | 1.00 | 24.09 | A |
| ATOM N | 1741 | N | GLN | A | 335 | 207.445 | 171.221 | -5.831 | 1.00 | 24.15 | A |
| ATOM C | 1742 | CA | GLN | A | 335 | 207.401 | 171.183 | -7.272 | 1.00 | 26.26 | A |
| ATOM C | 1743 | CB | GLN | A | 335 | 208.763 | 171.616 | -7.820 | 1.00 | 30.16 | A |
| ATOM C | 1744 | CG | GLN | A | 335 | 208.690 | 172.407 | -9.123 | 1.00 | 37.20 | A |
| ATOM C | 1745 | CD | GLN | A | 335 | 209.938 | 173.249 | -9.353 | 1.00 | 44.29 | A |
| ATOM O | 1746 | OE1 | GLN | A | 335 | 211.075 | 172.716 | -9.361 | 1.00 | 45.86 | A |
| ATOM N | 1747 | NE2 | GLN | A | 335 | 209.749 | 174.581 | -9.540 | 1.00 | 45.65 | A |
| ATOM C | 1748 | C | GLN | A | 335 | 207.014 | 169.785 | -7.766 | 1.00 | 24.06 | A |
| ATOM O | 1749 | O | GLN | A | 335 | 206.182 | 169.643 | -8.661 | 1.00 | 20.60 | A |
| ATOM N | 1750 | N | GLU | A | 336 | 207.596 | 168.746 | -7.179 | 1.00 | 23.21 | A |
| ATOM C | 1751 | CA | GLU | A | 336 | 207.243 | 167.407 | -7.624 | 1.00 | 22.61 | A |
| ATOM C | 1752 | CB | GLU | A | 336 | 208.183 | 166.360 | -7.022 | 1.00 | 19.74 | A |
| ATOM C | 1753 | CG | GLU | A | 336 | 207.807 | 164.932 | -7.350 | 1.00 | 27.97 | A |
| ATOM C | 1754 | CD | GLU | A | 336 | 207.852 | 164.603 | -8.845 | 1.00 | 35.28 | A |
| ATOM O | 1755 | OE1 | GLU | A | 336 | 207.231 | 163.579 | -9.236 | 1.00 | 38.15 | A |
| ATOM O | 1756 | OE2 | GLU | A | 336 | 208.508 | 165.350 | -9.628 | 1.00 | 39.74 | A |
| ATOM C | 1757 | C | GLU | A | 336 | 205.775 | 167.056 | -7.327 | 1.00 | 23.14 | A |
| ATOM O | 1758 | O | GLU | A | 336 | 205.138 | 166.363 | -8.124 | 1.00 | 23.03 | A |
| ATOM N | 1759 | N | THR | A | 337 | 205.241 | 167.517 | -6.196 | 1.00 | 21.09 | A |
| ATOM C | 1760 | CA | THR | A | 337 | 203.849 | 167.229 | -5.868 | 1.00 | 21.21 | A |
| ATOM C | 1761 | CB | THR | A | 337 | 203.541 | 167.589 | -4.380 | 1.00 | 22.66 | A |
| ATOM O | 1762 | OG1 | THR | A | 337 | 204.334 | 166.744 | -3.537 | 1.00 | 24.71 | A |
| ATOM C | 1763 | CG2 | THR | A | 337 | 202.072 | 167.366 | -4.030 | 1.00 | 16.15 | A |
| ATOM C | 1764 | C | THR | A | 337 | 202.918 | 167.944 | -6.855 | 1.00 | 19.71 | A |
| ATOM O | 1765 | O | THR | A | 337 | 201.978 | 167.329 | -7.346 | 1.00 | 21.88 | A |
| ATOM N | 1766 | N | TYR | A | 338 | 203.191 | 169.210 | -7.171 | 1.00 | 17.76 | A |
| ATOM C | 1767 | CA | TYR | A | 338 | 202.404 | 169.982 | -8.151 | 1.00 | 17.94 | A |
| ATOM C | 1768 | CB | TYR | A | 338 | 203.051 | 171.348 | -8.416 | 1.00 | 19.72 | A |
| ATOM C | 1769 | CG | TYR | A | 338 | 202.924 | 172.309 | -7.282 | 1.00 | 21.47 | A |
| ATOM C | 1770 | CD1 | TYR | A | 338 | 203.927 | 173.231 | -7.014 | 1.00 | 26.12 | A |
| ATOM C | 1771 | CE1 | TYR | A | 338 | 203.780 | 174.172 | -6.003 | 1.00 | 31.99 | A |
| ATOM C | 1772 | CD2 | TYR | A | 338 | 201.775 | 172.333 | -6.506 | 1.00 | 23.72 | A |
| ATOM C | 1773 | CE2 | TYR | A | 338 | 201.609 | 173.256 | -5.498 | 1.00 | 28.25 | A |
| ATOM C | 1774 | CZ | TYR | A | 338 | 202.605 | 174.184 | -5.255 | 1.00 | 32.26 | A |
| ATOM O | 1775 | OH | TYR | A | 338 | 202.398 | 175.197 | -4.343 | 1.00 | 37.49 | A |
| ATOM C | 1776 | C | TYR | A | 338 | 202.342 | 169.248 | -9.480 | 1.00 | 17.24 | A |
| ATOM O | 1777 | O | TYR | A | 338 | 201.303 | 169.202 | -10.143 | 1.00 | 17.93 | A |
| ATOM N | 1778 | N | LYS | A | 339 | 203.492 | 168.704 | -9.869 | 1.00 | 18.06 | A |
| ATOM C | 1779 | CA | LYS | A | 339 | 203.635 | 167.967 | -11.112 | 1.00 | 15.13 | A |
| ATOM C | 1780 | CB | LYS | A | 339 | 205.087 | 167.521 | -11.318 | 1.00 | 15.66 | A |
| ATOM C | 1781 | CG | LYS | A | 339 | 205.282 | 166,761 | -12.647 | 1.00 | 25.11 | A |
| ATOM C | 1782 | CD | LYS | A | 339 | 206.675 | 166.164 | -12.838 | 1.00 | 28.61 | A |
| ATOM C | 1783 | CE | LYS | A | 339 | 207.736 | 167.232 | -12.973 | 1.00 | 30.48 | A |
| ATOM N | 1784 | NZ | LYS | A | 339 | 209.081 | 166.590 | -13.128 | 1.00 | 37.06 | A |
| ATOM C | 1785 | C | LYS | A | 339 | 202.734 | 166.747 | -11.110 | 1.00 | 15.66 | A |
| ATOM O | 1786 | O | LYS | A | 339 | 201.999 | 166.520 | -12.062 | 1.00 | 16.79 | A |
| ATOM N | 1787 | N | ARG | A | 340 | 202.788 | 165.963 | -10.038 | 1.00 | 14.01 | A |
| ATOM C | 1788 | CA | ARG | A | 340 | 201.980 | 164.754 | -9.941 | 1.00 | 17.25 | A |
| ATOM C | 1789 | CB | ARG | A | 340 | 202.444 | 163.917 | -8.749 | 1.00 | 17.92 | A |
| ATOM C | 1790 | CG | ARG | A | 340 | 203.675 | 163.112 | -9.058 | 1.00 | 20.94 | A |
| ATOM C | 1791 | CD | ARG | A | 340 | 204.273 | 162.632 | -7.805 | 1.00 | 26.11 | A |
| ATOM N | 1792 | NE | ARG | A | 340 | 205.579 | 162.024 | -8.000 | 1.00 | 30.74 | A |
| ATOM C | 1793 | CZ | ARG | A | 340 | 206.247 | 161.430 | -7.014 | 1.00 | 34.14 | A |
| ATOM N | 1794 | NH1 | ARG | A | 340 | 205.697 | 161.387 | -5.805 | 1.00 | 36.45 | A |
| ATOM N | 1795 | NH2 | ARG | A | 340 | 207.447 | 160.886 | -7.219 | 1.00 | 32.82 | A |
| ATOM C | 1796 | C | ARG | A | 340 | 200.467 | 164.980 | -9.876 | 1.00 | 16.32 | A |
| ATOM O | 1797 | O | ARG | A | 340 | 199.692 | 164.177 | -10.393 | 1.00 | 13.54 | A |
| ATOM N | 1798 | N | ILE | A | 341 | 200.052 | 166.068 | -9.242 | 1.00 | 16.44 | A |
| ATOM C | 1799 | CA | ILE | A | 341 | 198.637 | 166.396 | -9.147 | 1.00 | 15.90 | A |
| ATOM C | 1800 | CB | ILE | A | 341 | 198.425 | 167.544 | -8.133 | 1.00 | 14.61 | A |
| ATOM C | 1801 | CG2 | ILE | A | 341 | 197.031 | 168.173 | -8.284 | 1.00 | 10.21 | A |
| ATOM C | 1802 | CG1 | ILE | A | 341 | 198.636 | 166.991 | -6.722 | 1.00 | 9.86 | A |
| ATOM C | 1803 | CD1 | ILE | A | 341 | 198.865 | 168.066 | -5.710 | 1.00 | 10.30 | A |
| ATOM C | 1804 | C | ILE | A | 341 | 198.139 | 166.813 | -10.524 | 1.00 | 16.94 | A |
| ATOM O | 1805 | O | ILE | A | 341 | 197.099 | 166.346 | -11.003 | 1.00 | 15.49 | A |
| ATOM N | 1806 | N | SER | A | 342 | 198.911 | 167.677 | -11.165 | 1.00 | 17.49 | A |
| ATOM C | 1807 | CA | SER | A | 342 | 198.556 | 168.185 | -12.479 | 1.00 | 19.50 | A |
| ATOM C | 1808 | CB | SER | A | 342 | 199.638 | 169.153 | -12.927 | 1.00 | 19.12 | A |
| ATOM O | 1809 | OG | SER | A | 342 | 199.178 | 169.899 | -14.023 | 1.00 | 28.84 | A |
| ATOM C | 1810 | C | SER | A | 342 | 198.385 | 167.065 | -13.518 | 1.00 | 18.65 | A |
| ATOM O | 1811 | O | SER | A | 342 | 197.544 | 167.157 | -14.414 | 1.00 | 17.15 | A |
| ATOM N | 1812 | N | ARG | A | 343 | 199.209 | 166.027 | -13.391 | 1.00 | 16.76 | A |
| ATOM C | 1813 | CA | ARG | A | 343 | 199.187 | 164.881 | -14.291 | 1.00 | 16.82 | A |
| ATOM C | 1814 | CB | ARG | A | 343 | 200.563 | 164.214 | -14.356 | 1.00 | 19.40 | A |
| ATOM C | 1815 | CG | ARG | A | 343 | 201.744 | 165.034 | -14.873 | 1.00 | 20.85 | A |
| ATOM C | 1816 | CD | ARG | A | 343 | 202.986 | 164.139 | -14.768 | 1.00 | 22.63 | A |
| ATOM N | 1817 | NE | ARG | A | 343 | 204.181 | 164.667 | -15.420 | 1.00 | 29.67 | A |
| ATOM C | 1818 | CZ | ARG | A | 343 | 205.296 | 163.964 | -15.619 | 1.00 | 29.30 | A |
| ATOM N | 1819 | NH1 | ARG | A | 343 | 205.362 | 162.702 | -15.211 | 1.00 | 27.60 | A |
| ATOM N | 1820 | NH2 | ARG | A | 343 | 206.337 | 164.521 | -16.239 | 1.00 | 30.90 | A |
| ATOM C | 1821 | C | ARG | A | 343 | 198.213 | 163.833 | -13.749 | 1.00 | 17.95 | A |
| ATOM O | 1822 | O | ARG | A | 343 | 197.789 | 162.927 | -14.470 | 1.00 | 18.61 | A |
| ATOM N | 1823 | N | VAL | A | 344 | 197.880 | 163.963 | -12.465 | 1.00 | 16.47 | A |
| ATOM C | 1824 | CA | VAL | A | 344 | 197.014 | 163.027 | -11.767 | 1.00 | 15.15 | A |
| ATOM C | 1825 | CB | VAL | A | 344 | 195.644 | 162.882 | -12.427 | 1.00 | 14.04 | A |
| ATOM C | 1826 | CG1 | VAL | A | 344 | 194.791 | 161.919 | -11.603 | 1.00 | 14.73 | A |
| ATOM C | 1827 | CG2 | VAL | A | 344 | 194.949 | 164.217 | -12.503 | 1.00 | 14.41 | A |
| ATOM C | 1828 | C | VAL | A | 344 | 197.751 | 161.702 | -11.847 | 1.00 | 19.48 | A |
| ATOM O | 1829 | O | VAL | A | 344 | 197.240 | 160.697 | -12.362 | 1.00 | 17.59 | A |
| ATOM N | 1830 | N | GLU | A | 345 | 198.970 | 161.733 | -11.320 | 1.00 | 20.63 | A |
| ATOM C | 1831 | CA | GLU | A | 345 | 199.883 | 160.600 | -11.314 | 1.00 | 21.68 | A |
| ATOM C | 1832 | CB | GLU | A | 345 | 201.297 | 161.116 | -11.635 | 1.00 | 22.57 | A |
| ATOM C | 1833 | CG | GLU | A | 345 | 202.291 | 160.066 | -12.115 | 1.00 | 29.02 | A |
| ATOM C | 1834 | CD | GLU | A | 345 | 203.609 | 160.693 | -12.596 | 1.00 | 34.43 | A |
| ATOM O | 1835 | OE1 | GLU | A | 345 | 203.572 | 161.826 | -13.143 | 1.00 | 41.54 | A |
| ATOM O | 1836 | OE2 | GLU | A | 345 | 204.677 | 160.062 | -12.448 | 1.00 | 34.88 | A |
| ATOM C | 1837 | C | GLU | A | 345 | 199.906 | 159.839 | -9.990 | 1.00 | 21.02 | A |
| ATOM O | 1838 | O | GLU | A | 345 | 200.516 | 160.289 | -9.009 | 1.00 | 22.86 | A |
| ATOM N | 1839 | N | PHE | A | 346 | 199.260 | 158.679 | -9.953 | 1.00 | 20.71 | A |
| ATOM C | 1840 | CA | PHE | A | 346 | 199.268 | 157.868 | -8.730 | 1.00 | 20.54 | A |
| ATOM C | 1841 | CB | PHE | A | 346 | 198.272 | 158.454 | -7.705 | 1.00 | 19.53 | A |
| ATOM C | 1842 | CG | PHE | A | 346 | 196.835 | 158.138 | -8.013 | 1.00 | 21.11 | A |
| ATOM C | 1843 | CD1 | PHE | A | 346 | 196.203 | 157.064 | -7.403 | 1.00 | 22.02 | A |
| ATOM C | 1844 | CD2 | PHE | A | 346 | 196.130 | 158.888 | -8.936 | 1.00 | 18.36 | A |
| ATOM C | 1845 | CE1 | PHE | A | 346 | 194.878 | 156.744 | -7.713 | 1.00 | 24.10 | A |
| ATOM C | 1846 | CE2 | PHE | A | 346 | 194.807 | 158.574 | -9.253 | 1.00 | 23.07 | A |
| ATOM C | 1847 | CZ | PHE | A | 346 | 194.181 | 157.500 | -8.640 | 1.00 | 22.64 | A |
| ATOM C | 1848 | C | PHE | A | 346 | 198.893 | 156.424 | -9.065 | 1.00 | 19.41 | A |
| ATOM O | 1849 | O | PHE | A | 346 | 198.376 | 156.142 | -10.153 | 1.00 | 21.26 | A |
| ATOM N | 1850 | N | THR | A | 347 | 199.158 | 155.512 | -8.137 | 1.00 | 17.51 | A |
| ATOM C | 1851 | CA | THR | A | 347 | 198.809 | 154.113 | -8.335 | 1.00 | 15.60 | A |
| ATOM C | 1852 | CB | THR | A | 347 | 200.042 | 153.259 | -8.736 | 1.00 | 17.00 | A |
| ATOM O | 1853 | OG1 | THR | A | 347 | 201.113 | 153.494 | -7.812 | 1.00 | 20.54 | A |
| ATOM C | 1854 | CG2 | THR | A | 347 | 200.506 | 153.617 | -10.145 | 1.00 | 14.88 | A |
| ATOM C | 1855 | C | THR | A | 347 | 198.245 | 153.623 | -7.026 | 1.00 | 14.01 | A |
| ATOM O | 1856 | O | THR | A | 347 | 198.322 | 154.324 | -6.030 | 1.00 | 14.17 | A |
| ATOM N | 1857 | N | PHE | A | 348 | 197.659 | 152.434 | -7.029 | 1.00 | 14.30 | A |
| ATOM C | 1858 | CA | PHE | A | 348 | 197.097 | 151.875 | -5.809 | 1.00 | 15.62 | A |
| ATOM C | 1859 | CB | PHE | A | 348 | 195.673 | 151.335 | -6.022 | 1.00 | 13.71 | A |
| ATOM C | 1860 | CG | PHE | A | 348 | 194.678 | 152.345 | -6.553 | 1.00 | 14.90 | A |
| ATOM C | 1861 | CD1 | PHE | A | 348 | 194.476 | 152.496 | -7.926 | 1.00 | 14.48 | A |
| ATOM C | 1862 | CD2 | PHE | A | 348 | 193.915 | 153.115 | -5.678 | 1.00 | 14.25 | A |
| ATOM C | 1863 | CE1 | PHE | A | 348 | 193.520 | 153.399 | -8.421 | 1.00 | 13.33 | A |
| ATOM C | 1864 | CE2 | PHE | A | 348 | 192.956 | 154.020 | -6.160 | 1.00 | 12.61 | A |
| ATOM C | 1865 | CZ | PHE | A | 348 | 192.758 | 154.162 | -7.530 | 1.00 | 13.12 | A |
| ATOM C | 1866 | C | PHE | A | 348 | 197.936 | 150.690 | -5.379 | 1.00 | 17.39 | A |
| ATOM O | 1867 | O | PHE | A | 348 | 198.493 | 149.986 | -6.222 | 1.00 | 17.79 | A |
| ATOM N | 1868 | N | PRO | A | 349 | 198.077 | 150.478 | -4.060 | 1.00 | 19.22 | A |
| ATOM C | 1869 | CD | PRO | A | 349 | 197.716 | 151.428 | -2.984 | 1.00 | 20.64 | A |
| ATOM C | 1870 | CA | PRO | A | 349 | 198.843 | 149.326 | -3.550 | 1.00 | 18.28 | A |
| ATOM C | 1871 | CB | PRO | A | 349 | 198.881 | 149.550 | -2.034 | 1.00 | 19.47 | A |
| ATOM C | 1872 | CG | PRO | A | 349 | 198.663 | 151.042 | -1.874 | 1.00 | 21.78 | A |
| ATOM C | 1873 | C | PRO | A | 349 | 197.913 | 148.144 | -3.906 | 1.00 | 18.89 | A |
| ATOM O | 1874 | O | PRO | A | 349 | 196.765 | 148.365 | -4.282 | 1.00 | 18.06 | A |
| ATOM N | 1875 | N | ASP | A | 350 | 198.360 | 146.901 | -3.778 | 1.00 | 22.60 | A |
| ATOM C | 1876 | CA | ASP | A | 350 | 197.488 | 145.780 | -4.137 | 1.00 | 24.29 | A |
| ATOM C | 1877 | CB | ASP | A | 350 | 198.312 | 144.494 | -4.298 | 1.00 | 28.79 | A |
| ATOM C | 1878 | CG | ASP | A | 350 | 199.378 | 144.608 | -5.404 | 1.00 | 33.91 | A |
| ATOM O | 1879 | OD1 | ASP | A | 350 | 200.001 | 143.575 | -5.741 | 1.00 | 33.53 | A |
| ATOM O | 1880 | OD2 | ASP | A | 350 | 199.591 | 145.733 | -5.928 | 1.00 | 35.29 | A |
| ATOM C | 1881 | C | ASP | A | 350 | 196.293 | 145.489 | -3.238 | 1.00 | 23.99 | A |
| ATOM O | 1882 | O | ASP | A | 350 | 195.352 | 144.835 | -3.671 | 1.00 | 23.48 | A |
| ATOM N | 1883 | N | PHE | A | 351 | 196.279 | 145.964 | -2.003 | 1.00 | 22.20 | A |
| ATOM C | 1884 | CA | PHE | A | 351 | 195.125 | 145.636 | -1.171 | 1.00 | 23.75 | A |
| ATOM C | 1885 | CB | PHE | A | 351 | 195.510 | 145.662 | 0.300 | 1.00 | 22.72 | A |
| ATOM C | 1886 | CG | PHE | A | 351 | 196.082 | 146.956 | 0.738 | 1.00 | 20.40 | A |
| ATOM C | 1887 | CD1 | PHE | A | 351 | 197.451 | 147.151 | 0.756 | 1.00 | 17.28 | A |
| ATOM C | 1888 | CD2 | PHE | A | 351 | 195.245 | 147.994 | 1.111 | 1.00 | 19.08 | A |
| ATOM C | 1889 | CE1 | PHE | A | 351 | 197.983 | 148.379 | 1.142 | 1.00 | 21.72 | A |
| ATOM C | 1890 | CE2 | PHE | A | 351 | 195.761 | 149.217 | 1.497 | 1.00 | 19.31 | A |
| ATOM C | 1891 | CZ | PHE | A | 351 | 197.128 | 149.417 | 1.515 | 1.00 | 20.15 | A |
| ATOM C | 1892 | C | PHE | A | 351 | 193.883 | 146.509 | -1.382 | 1.00 | 24.86 | A |
| ATOM O | 1893 | O | PHE | A | 351 | 192.826 | 146.215 | -0.820 | 1.00 | 26.36 | A |
| ATOM N | 1894 | N | VAL | A | 352 | 193.999 | 147.575 | -2.174 | 1.00 | 22.98 | A |
| ATOM C | 1895 | CA | VAL | A | 352 | 192.845 | 148.431 | -2.428 | 1.00 | 22.53 | A |
| ATOM C | 1896 | CB | VAL | A | 352 | 193.297 | 149.810 | -2.976 | 1.00 | 19.91 | A |
| ATOM C | 1897 | CG1 | VAL | A | 352 | 192.105 | 150.680 | -3.293 | 1.00 | 15.94 | A |
| ATOM C | 1898 | CG2 | VAL | A | 352 | 194.164 | 150.489 | -1.936 | 1.00 | 17.58 | A |
| ATOM C | 1899 | C | VAL | A | 352 | 191.941 | 147.702 | -3.416 | 1.00 | 22.85 | A |
| ATOM O | 1900 | O | VAL | A | 352 | 192.351 | 147.406 | -4.541 | 1.00 | 25.56 | A |
| ATOM N | 1901 | N | THR | A | 353 | 190.722 | 147.393 | -2.985 | 1.00 | 20.20 | A |
| ATOM C | 1902 | CA | THR | A | 353 | 189.786 | 146.668 | -3.833 | 1.00 | 20.47 | A |
| ATOM C | 1903 | CB | THR | A | 353 | 188.530 | 146.264 | -3.059 | 1.00 | 18.44 | A |
| ATOM O | 1904 | OG1 | THR | A | 353 | 187.777 | 147.445 | -2.740 | 1.00 | 19.14 | A |
| ATOM C | 1905 | CG2 | THR | A | 353 | 188.915 | 145.544 | -1.786 | 1.00 | 15.00 | A |
| ATOM C | 1906 | C | THR | A | 353 | 189.331 | 147.452 | -5.049 | 1.00 | 23.02 | A |
| ATOM O | 1907 | O | THR | A | 353 | 189.525 | 148.665 | -5.144 | 1.00 | 21.93 | A |
| ATOM N | 1908 | N | GLU | A | 354 | 188.685 | 146.733 | -5.960 | 1.00 | 23.98 | A |
| ATOM C | 1909 | CA | GLU | A | 354 | 188.175 | 147.274 | -7.208 | 1.00 | 25.43 | A |
| ATOM C | 1910 | CB | GLU | A | 354 | 187.543 | 146.130 | -8.001 | 1.00 | 30.21 | A |
| ATOM C | 1911 | CG | GLU | A | 354 | 186.780 | 146.540 | -9.261 | 1.00 | 37.82 | A |
| ATOM C | 1912 | CD | GLU | A | 354 | 186.031 | 145.351 | -9.890 | 1.00 | 41.79 | A |
| ATOM O | 1913 | OE1 | GLU | A | 354 | 185.501 | 145.472 | -11.025 | 1.00 | 43.51 | A |
| ATOM O | 1914 | OE2 | GLU | A | 354 | 185.974 | 144.281 | -9.224 | 1.00 | 44.80 | A |
| ATOM C | 1915 | C | GLU | A | 354 | 187.175 | 148.424 | -7.055 | 1.00 | 24.34 | A |
| ATOM O | 1916 | O | GLU | A | 354 | 187.182 | 149.386 | -7.840 | 1.00 | 24.85 | A |
| ATOM N | 1917 | N | GLY | A | 355 | 186.298 | 148.303 | -6.065 | 1.00 | 20.96 | A |
| ATOM C | 1918 | CA | GLY | A | 355 | 185.289 | 149.314 | -5.842 | 1.00 | 13.82 | A |
| ATOM C | 1919 | C | GLY | A | 355 | 185.908 | 150.595 | -5.333 | 1.00 | 16.94 | A |
| ATOM O | 1920 | O | GLY | A | 355 | 185.529 | 151.694 | -5.760 | 1.00 | 13.60 | A |
| ATOM N | 1921 | N | ALA | A | 356 | 186.868 | 150.473 | -4.425 | 1.00 | 15.45 | A |
| ATOM C | 1922 | CA | ALA | A | 356 | 187.501 | 151.674 | -3.909 | 1.00 | 19.10 | A |
| ATOM C | 1923 | CB | ALA | A | 356 | 188.475 | 151.326 | -2.761 | 1.00 | 18.64 | A |
| ATOM C | 1924 | C | ALA | A | 356 | 188.235 | 152.381 | -5.068 | 1.00 | 19.08 | A |
| ATOM O | 1925 | O | ALA | A | 356 | 188.203 | 153.604 | -5.162 | 1.00 | 16.84 | A |
| ATOM N | 1926 | N | ARG | A | 357 | 188.874 | 151.611 | -5.951 | 1.00 | 18.44 | A |
| ATOM C | 1927 | CA | ARG | A | 357 | 189.586 | 152.184 | -7.100 | 1.00 | 18.13 | A |
| ATOM C | 1928 | CB | ARG | A | 357 | 190.349 | 151.113 | -7.881 | 1.00 | 16.00 | A |
| ATOM C | 1929 | CG | ARG | A | 357 | 191.447 | 150.447 | -7.112 | 1.00 | 15.75 | A |
| ATOM C | 1930 | CD | ARG | A | 357 | 192.125 | 149.414 | -7.941 | 1.00 | 14.24 | A |
| ATOM N | 1931 | NE | ARG | A | 357 | 193.120 | 148.721 | -7.139 | 1.00 | 18.00 | A |
| ATOM C | 1932 | CZ | ARG | A | 357 | 194.249 | 148.203 | -7.607 | 1.00 | 18.74 | A |
| ATOM N | 1933 | NH1 | ARG | A | 357 | 194.553 | 148.285 | -8.898 | 1.00 | 21.62 | A |
| ATOM N | 1934 | NH2 | ARG | A | 357 | 195.097 | 147.622 | -6.768 | 1.00 | 20.49 | A |
| ATOM C | 1935 | C | ARG | A | 357 | 188.627 | 152.856 | -8.062 | 1.00 | 17.28 | A |
| ATOM O | 1936 | O | ARG | A | 357 | 188.949 | 153.878 | -8.637 | 1.00 | 16.53 | A |
| ATOM N | 1937 | N | ASP | A | 358 | 187.454 | 152.272 | -8.261 | 1.00 | 17.73 | A |
| ATOM C | 1938 | CA | ASP | A | 358 | 186.511 | 152.887 | -9.176 | 1.00 | 17.93 | A |
| ATOM C | 1939 | CB | ASP | A | 358 | 185.267 | 152.024 | -9.389 | 1.00 | 21.16 | A |
| ATOM C | 1940 | CG | ASP | A | 358 | 184.269 | 152.688 | -10.327 | 1.00 | 23.93 | A |
| ATOM O | 1941 | OD1 | ASP | A | 358 | 184.508 | 152.671 | -11.559 | 1.00 | 29.93 | A |
| ATOM O | 1942 | OD2 | ASP | A | 358 | 183.266 | 153.257 | -9.840 | 1.00 | 22.82 | A |
| ATOM C | 1943 | C | ASP | A | 358 | 186.081 | 154.234 | -8.611 | 1.00 | 17.83 | A |
| ATOM O | 1944 | O | ASP | A | 358 | 185.996 | 155.206 | -9.348 | 1.00 | 20.18 | A |
| ATOM N | 1945 | N | LEU | A | 359 | 185.823 | 154.299 | -7.303 | 1.00 | 16.69 | A |
| ATOM C | 1946 | CA | LEU | A | 359 | 185.389 | 155.543 | -6.680 | 1.00 | 15.23 | A |
| ATOM C | 1947 | CB | LEU | A | 359 | 184.873 | 155.292 | -5.250 | 1.00 | 13.90 | A |
| ATOM C | 1948 | CG | LEU | A | 359 | 184.459 | 156.568 | -4.482 | 1.00 | 15.76 | A |
| ATOM C | 1949 | CD1 | LEU | A | 359 | 183.222 | 157.164 | -5.105 | 1.00 | 14.16 | A |
| ATOM C | 1950 | CD2 | LEU | A | 359 | 184.210 | 156.266 | -3.027 | 1.00 | 16.19 | A |
| ATOM C | 1951 | C | LEU | A | 359 | 186.478 | 156.623 | -6.646 | 1.00 | 15.38 | A |
| ATOM O | 1952 | O | LEU | A | 359 | 186.234 | 157.740 | -7.071 | 1.00 | 16.61 | A |
| ATOM N | 1953 | N | ILE | A | 360 | 187.661 | 156.300 | -6.132 | 1.00 | 15.08 | A |
| ATOM C | 1954 | CA | ILE | A | 360 | 188.737 | 157.280 | -6.054 | 1.00 | 16.09 | A |
| ATOM C | 1955 | CB | ILE | A | 360 | 189.972 | 156.694 | -5.305 | 1.00 | 13.18 | A |
| ATOM C | 1956 | CG2 | ILE | A | 360 | 191.132 | 157.645 | -5.348 | 1.00 | 8.95 | A |
| ATOM C | 1957 | CG1 | ILE | A | 360 | 189.613 | 156.445 | -3.843 | 1.00 | 11.43 | A |
| ATOM C | 1958 | CD1 | ILE | A | 360 | 190.486 | 155.406 | -3.211 | 1.00 | 15.13 | A |
| ATOM C | 1959 | C | ILE | A | 360 | 189.126 | 157.752 | -7.457 | 1.00 | 18.67 | A |
| ATOM O | 1960 | O | ILE | A | 360 | 189.433 | 158.935 | -7.652 | 1.00 | 19.76 | A |
| ATOM N | 1961 | N | SER | A | 361 | 189.099 | 156.841 | -8.427 | 1.00 | 17.86 | A |
| ATOM C | 1962 | CA | SER | A | 361 | 189.437 | 157.193 | -9.792 | 1.00 | 17.51 | A |
| ATOM C | 1963 | CB | SER | A | 361 | 189.511 | 155.952 | -10.682 | 1.00 | 18.17 | A |
| ATOM O | 1964 | OG | SER | A | 361 | 190.686 | 155.223 | -10.384 | 1.00 | 20.30 | A |
| ATOM C | 1965 | C | SER | A | 361 | 188.438 | 158.163 | -10.382 | 1.00 | 18.63 | A |
| ATOM O | 1966 | O | SER | A | 361 | 188.805 | 159.011 | -11.209 | 1.00 | 19.26 | A |
| ATOM N | 1967 | N | ARG | A | 362 | 187.175 | 158.051 | -9.977 | 1.00 | 18.68 | A |
| ATOM C | 1968 | CA | ARG | A | 362 | 186.156 | 158.973 | -10.492 | 1.00 | 17.96 | A |
| ATOM C | 1969 | CB | ARG | A | 362 | 184.740 | 158.464 | -10.227 | 1.00 | 18.15 | A |
| ATOM C | 1970 | CG | ARG | A | 362 | 184.389 | 157.189 | -10.948 | 1.00 | 21.87 | A |
| ATOM C | 1971 | CD | ARG | A | 362 | 182.950 | 156.762 | -10.710 | 1.00 | 24.92 | A |
| ATOM N | 1972 | NE | ARG | A | 362 | 182.669 | 155.532 | -11.449 | 1.00 | 31.73 | A |
| ATOM C | 1973 | CZ | ARG | A | 362 | 182.150 | 155.461 | -12.682 | 1.00 | 35.25 | A |
| ATOM N | 1974 | NH1 | ARG | A | 362 | 181.805 | 156.557 | -13.358 | 1.00 | 34.91 | A |
| ATOM N | 1975 | NH2 | ARG | A | 362 | 182.052 | 154.276 | -13.281 | 1.00 | 35.12 | A |
| ATOM C | 1976 | C | ARG | A | 362 | 186.287 | 160.332 | -9.817 | 1.00 | 16.66 | A |
| ATOM O | 1977 | O | ARG | A | 362 | 186.086 | 161.367 | -10.452 | 1.00 | 21.59 | A |
| ATOM N | 1978 | N | LEU | A | 363 | 186.602 | 160.348 | -8.527 | 1.00 | 15.31 | A |
| ATOM C | 1979 | CA | LEU | A | 363 | 186.726 | 161.620 | -7.814 | 1.00 | 15.39 | A |
| ATOM C | 1980 | CB | LEU | A | 363 | 186.758 | 161.376 | -6.295 | 1.00 | 13.54 | A |
| ATOM C | 1981 | CG | LEU | A | 363 | 185.452 | 160.880 | -5.638 | 1.00 | 14.63 | A |
| ATOM C | 1982 | CD1 | LEU | A | 363 | 185.723 | 160.358 | -4.216 | 1.00 | 12.73 | A |
| ATOM C | 1983 | CD2 | LEU | A | 363 | 184.423 | 162.005 | -5.613 | 1.00 | 7.80 | A |
| ATOM C | 1984 | C | LEU | A | 363 | 187.989 | 162.357 | -8.252 | 1.00 | 16.50 | A |
| ATOM O | 1985 | O | LEU | A | 363 | 188.033 | 163.593 | -8.252 | 1.00 | 16.60 | A |
| ATOM N | 1986 | N | LEU | A | 364 | 189.008 | 161.597 | -8.656 | 1.00 | 17.23 | A |
| ATOM C | 1987 | CA | LEU | A | 364 | 190.285 | 162.182 | -9.051 | 1.00 | 20.15 | A |
| ATOM C | 1988 | CB | LEU | A | 364 | 191.419 | 161.335 | -8.467 | 1.00 | 18.29 | A |
| ATOM C | 1989 | CG | LEU | A | 364 | 191.504 | 161.377 | -6.926 | 1.00 | 20.11 | A |
| ATOM C | 1990 | CD1 | LEU | A | 364 | 192.737 | 160.607 | -6.509 | 1.00 | 16.10 | A |
| ATOM C | 1991 | CD2 | LEU | A | 364 | 191.576 | 162.820 | -6.413 | 1.00 | 16.30 | A |
| ATOM C | 1992 | C | LEU | A | 364 | 190.474 | 162.393 | -10.557 | 1.00 | 19.92 | A |
| ATOM O | 1993 | O | LEU | A | 364 | 191.511 | 162.052 | -11.143 | 1.00 | 20.12 | A |
| ATOM N | 1994 | N | LYS | A | 365 | 189.450 | 162.967 | -11.169 | 1.00 | 19.36 | A |
| ATOM C | 1995 | CA | LYS | A | 365 | 189.461 | 163.275 | -12.581 | 1.00 | 19.00 | A |
| ATOM C | 1996 | CB | LYS | A | 365 | 188.039 | 163.289 | -13.117 | 1.00 | 22.47 | A |
| ATOM C | 1997 | CG | LYS | A | 365 | 187.417 | 161.929 | -13.243 | 1.00 | 23.42 | A |
| ATOM C | 1998 | CD | LYS | A | 365 | 188.039 | 161.224 | -14.400 | 1.00 | 23.31 | A |
| ATOM C | 1999 | CE | LYS | A | 365 | 187.320 | 159.930 | -14.693 | 1.00 | 24.89 | A |
| ATOM N | 2000 | NZ | LYS | A | 365 | 187.970 | 159.249 | -15.851 | 1.00 | 30.35 | A |
| ATOM C | 2001 | C | LYS | A | 365 | 190.039 | 164.670 | -12.715 | 1.00 | 18.75 | A |
| ATOM O | 2002 | O | LYS | A | 365 | 189.654 | 165.568 | -11.956 | 1.00 | 15.64 | A |
| ATOM N | 2003 | N | HIS | A | 366 | 190.949 | 164.850 | -13.676 | 1.00 | 17.07 | A |
| ATOM C | 2004 | CA | HIS | A | 366 | 191.568 | 166.150 | -13.920 | 1.00 | 17.25 | A |
| ATOM C | 2005 | CB | HIS | A | 366 | 192.611 | 166.038 | -15.043 | 1.00 | 15.66 | A |
| ATOM C | 2006 | CG | HIS | A | 366 | 193.302 | 167.331 | -15.352 | 1.00 | 18.71 | A |
| ATOM C | 2007 | CD2 | HIS | A | 366 | 194.437 | 167.877 | -14.849 | 1.00 | 17.17 | A |
| ATOM N | 2008 | ND1 | HIS | A | 366 | 192.763 | 168.283 | -16.194 | 1.00 | 19.79 | A |
| ATOM C | 2009 | CE1 | HIS | A | 366 | 193.530 | 169.361 | -16.187 | 1.00 | 18.47 | A |
| ATOM N | 2010 | NE2 | HIS | A | 366 | 194.552 | 169.141 | -15.378 | 1.00 | 18.20 | A |
| ATOM C | 2011 | C | HIS | A | 366 | 190.493 | 167.186 | -14.291 | 1.00 | 17.88 | A |
| ATOM O | 2012 | O | HIS | A | 366 | 190.553 | 168.334 | -13.864 | 1.00 | 14.94 | A |
| ATOM N | 2013 | N | ASN | A | 367 | 189.500 | 166.769 | -15.071 | 1.00 | 19.37 | A |
| ATOM C | 2014 | CA | ASN | A | 367 | 188.436 | 167.676 | -15.490 | 1.00 | 22.11 | A |
| ATOM C | 2015 | CB | ASN | A | 367 | 187.841 | 167.205 | -16.823 | 1.00 | 24.11 | A |
| ATOM C | 2016 | CG | ASN | A | 367 | 186.929 | 168.246 | -17.452 | 1.00 | 29.29 | A |
| ATOM O | 2017 | OD1 | ASN | A | 367 | 185.990 | 168.718 | -16.827 | 1.00 | 34.30 | A |
| ATOM N | 2018 | ND2 | ASN | A | 367 | 187.209 | 168.611 | -18.688 | 1.00 | 31.42 | A |
| ATOM C | 2019 | C | ASN | A | 367 | 187.348 | 167.721 | -14.412 | 1.00 | 22.00 | A |
| ATOM O | 2020 | O | ASN | A | 367 | 186.644 | 166.739 | -14.198 | 1.00 | 21.37 | A |
| ATOM N | 2021 | N | PRO | A | 368 | 187.174 | 168.881 | -13.751 | 1.00 | 22.13 | A |
| ATOM C | 2022 | CD | PRO | A | 368 | 187.763 | 170.174 | -14.151 | 1.00 | 21.11 | A |
| ATOM C | 2023 | CA | PRO | A | 368 | 186.181 | 169.073 | -12.682 | 1.00 | 21.45 | A |
| ATOM C | 2024 | CB | PRO | A | 368 | 186.292 | 170.571 | -12.377 | 1.00 | 23.08 | A |
| ATOM C | 2025 | CG | PRO | A | 368 | 187.705 | 170.942 | -12.864 | 1.00 | 19.70 | A |
| ATOM C | 2026 | C | PRO | A | 368 | 184.755 | 168.638 | -13.047 | 1.00 | 22.70 | A |
| ATOM O | 2027 | O | PRO | A | 368 | 184.016 | 168.091 | -12.209 | 1.00 | 21.09 | A |
| ATOM N | 2028 | N | SER | A | 369 | 184.386 | 168.865 | -14.303 | 1.00 | 21.10 | A |
| ATOM C | 2029 | CA | SER | A | 369 | 183.068 | 168.492 | -14.818 | 1.00 | 25.15 | A |
| ATOM C | 2030 | CB | SER | A | 369 | 182.919 | 168.982 | -16.263 | 1.00 | 25.61 | A |
| ATOM O | 2031 | OG | SER | A | 369 | 183.115 | 170.391 | -16.341 | 1.00 | 37.93 | A |
| ATOM C | 2032 | C | SER | A | 369 | 182.809 | 166.986 | -14.785 | 1.00 | 23.28 | A |
| ATOM O | 2033 | O | SER | A | 369 | 181.685 | 166.555 | -14.591 | 1.00 | 26.91 | A |
| ATOM N | 2034 | N | GLN | A | 370 | 183.848 | 166.196 | -14.995 | 1.00 | 20.71 | A |
| ATOM C | 2035 | CA | GLN | A | 370 | 183.744 | 164.749 | -15.009 | 1.00 | 21.53 | A |
| ATOM C | 2036 | CB | GLN | A | 370 | 184.922 | 164.172 | -15.785 | 1.00 | 24.23 | A |
| ATOM C | 2037 | CG | GLN | A | 370 | 184.674 | 164.178 | -17.279 | 1.00 | 31.18 | A |
| ATOM C | 2038 | CD | GLN | A | 370 | 185.948 | 164.183 | -18.045 | 1.00 | 38.83 | A |
| ATOM O | 2039 | OE1 | GLN | A | 370 | 186.879 | 163.380 | -17.767 | 1.00 | 41.58 | A |
| ATOM N | 2040 | NE2 | GLN | A | 370 | 186.033 | 165.098 | -19.038 | 1.00 | 43.83 | A |
| ATOM C | 2041 | C | GLN | A | 370 | 183.688 | 164.073 | -13.665 | 1.00 | 20.69 | A |
| ATOM O | 2042 | O | GLN | A | 370 | 183.444 | 162.864 | -13.600 | 1.00 | 19.42 | A |
| ATOM N | 2043 | N | ARG | A | 371 | 183.940 | 164.804 | -12.585 | 1.00 | 18.88 | A |
| ATOM C | 2044 | CA | ARG | A | 371 | 183.906 | 164.189 | -11.252 | 1.00 | 21.24 | A |
| ATOM C | 2045 | CB | ARG | A | 371 | 184.612 | 165.072 | -10.231 | 1.00 | 17.55 | A |
| ATOM C | 2046 | CG | ARG | A | 371 | 186.104 | 165.158 | -10.394 | 1.00 | 15.36 | A |
| ATOM C | 2047 | CD | ARG | A | 371 | 186.643 | 166.286 | -9.564 | 1.00 | 11.86 | A |
| ATOM N | 2048 | NE | ARG | A | 371 | 187.913 | 166.739 | -10.108 | 1.00 | 12.57 | A |
| ATOM _{C} | 2049 | CZ | ARG | A | 371 | 188.440 | 167.951 | -9.938 | 1.00 | 14.14 | A |
| ATOM N | 2050 | NH1 | ARG | A | 371 | 187.817 | 168.878 | -9.216 | 1.00 | 10.91 | A |
| ATOM N | 2051 | NH2 | ARG | A | 371 | 189.583 | 168.257 | -10.545 | 1.00 | 9.48 | A |
| ATOM C | 2052 | C | ARG | A | 371 | 182.440 | 164.038 | -10.854 | 1.00 | 22.37 | A |
| ATOM O | 2053 | O | ARG | A | 371 | 181.601 | 164.841 | -11.176 | 1.00 | 22.50 | A |
| ATOM N | 2054 | N | PRO | A | 372 | 182.141 | 162.957 | -10.111 | 1.00 | 23.64 | A |
| ATOM C | 2055 | CD | PRO | A | 372 | 183.165 | 162.051 | -9.512 | 1.00 | 23.75 | A |
| ATOM C | 2056 | CA | PRO | A | 372 | 180.757 | 162.663 | -9.653 | 1.00 | 21.80 | A |
| ATOM C | 2057 | CB | PRO | A | 372 | 180.923 | 161.269 | -9.007 | 1.00 | 22.29 | A |
| ATOM C | 2058 | CG | PRO | A | 372 | 182.365 | 161.227 | -8.548 | 1.00 | 22.92 | A |
| ATOM C | 2059 | C | PRO | A | 372 | 180.160 | 163.726 | -8.739 | 1.00 | 21.72 | A |
| ATOM O | 2060 | O | PRO | A | 372 | 180.875 | 164.579 | -8.217 | 1.00 | 23.11 | A |
| ATOM N | 2061 | N | MET | A | 373 | 178.840 | 163.740 | -8.654 | 1.00 | 20.67 | A |
| ATOM C | 2062 | CA | MET | A | 373 | 178.153 | 164.668 | -7.731 | 1.00 | 19.68 | A |
| ATOM C | 2063 | CB | MET | A | 373 | 176.674 | 164.841 | -8.137 | 1.00 | 25.32 | A |
| ATOM C | 2064 | CG | MET | A | 373 | 176.470 | 165.598 | -9.397 | 1.00 | 29.58 | A |
| ATOM S | 2065 | SD | MET | A | 373 | 177.274 | 167.224 | -9.216 | 1.00 | 44.08 | A |
| ATOM C | 2066 | CE | MET | A | 373 | 175.850 | 168.314 | -8.571 | 1.00 | 34.35 | A |
| ATOM C | 2067 | C | MET | A | 373 | 178.231 | 163.942 | -6.368 | 1.00 | 18.77 | A |
| ATOM O | 2068 | O | MET | A | 373 | 178.518 | 162.750 | -6.337 | 1.00 | 14.31 | A |
| ATOM N | 2069 | N | LEU | A | 374 | 177.985 | 164.639 | -5.257 | 1.00 | 16.82 | A |
| ATOM C | 2070 | CA | LEU | A | 374 | 178.041 | 163.984 | -3.959 | 1.00 | 17.05 | A |
| ATOM C | 2071 | CB | LEU | A | 374 | 177.959 | 165.010 | -2.820 | 1.00 | 15.61 | A |
| ATOM C | 2072 | CG | LEU | A | 374 | 179.224 | 165.888 | -2.751 | 1.00 | 17.98 | A |
| ATOM C | 2073 | CD1 | LEU | A | 374 | 178.940 | 167.269 | -2.145 | 1.00 | 8.94 | A |
| ATOM C | 2074 | CD2 | LEU | A | 374 | 180.290 | 165.125 | -1.973 | 1.00 | 12.26 | A |
| ATOM C | 2075 | C | LEU | A | 374 | 176.934 | 162.915 | -3.837 | 1.00 | 18.65 | A |
| ATOM O | 2076 | O | LEU | A | 374 | 177.122 | 161.885 | -3.201 | 1.00 | 19.21 | A |
| ATOM N | 2077 | N | ARG | A | 375 | 175.804 | 163.167 | -4.504 | 1.00 | 18.78 | A |
| ATOM C | 2078 | CA | ARG | A | 375 | 174.657 | 162.248 | -4.510 | 1.00 | 20.13 | A |
| ATOM C | 2079 | CB | ARG | A | 375 | 173.504 | 162.813 | -5.210 | 1.00 | 21.87 | A |
| ATOM C | 2080 | C | ARG | A | 375 | 175.033 | 160.942 | -5.178 | 1.00 | 21.00 | A |
| ATOM O | 2081 | O | ARG | A | 375 | 174.457 | 159.916 | -4.909 | 1.00 | 21.05 | A |
| ATOM N | 2082 | N | GLU | A | 376 | 175.970 | 161.009 | -6.106 | 1.00 | 23.11 | A |
| ATOM C | 2083 | CA | GLU | A | 376 | 176.419 | 159.788 | -6.763 | 1.00 | 23.55 | A |
| ATOM C | 2084 | CB | GLU | A | 376 | 177.142 | 160.139 | -8.053 | 1.00 | 24.82 | A |
| ATOM C | 2085 | CG | GLU | A | 376 | 176.400 | 161.110 | -8.977 | 1.00 | 32.25 | A |
| ATOM C | 2086 | CD | GLU | A | 376 | 177.239 | 161.561 | -10.206 | 1.00 | 34.95 | A |
| ATOM O | 2087 | OE1 | GLU | A | 376 | 177.030 | 162.670 | -10.770 | 1.00 | 37.06 | A |
| ATOM O | 2088 | OE2 | GLU | A | 376 | 178.110 | 160.783 | -10.630 | 1.00 | 37.87 | A |
| ATOM C | 2089 | C | GLU | A | 376 | 177.380 | 158.962 | -5.852 | 1.00 | 23.36 | A |
| ATOM O | 2090 | O | GLU | A | 376 | 177.448 | 157.712 | -5.874 | 1.00 | 25.32 | A |
| ATOM N | 2091 | N | VAL | A | 377 | 178.204 | 159.683 | -5.113 | 1.00 | 20.65 | A |
| ATOM C | 2092 | CA | VAL | A | 377 | 179.168 | 159.065 | -4.241 | 1.00 | 19.06 | A |
| ATOM C | 2093 | CB | VAL | A | 377 | 180.021 | 160.117 | -3.550 | 1.00 | 20.25 | A |
| ATOM C | 2094 | CG1 | VAL | A | 377 | 180.772 | 159.513 | -2.415 | 1.00 | 14.07 | A |
| ATOM C | 2095 | CG2 | VAL | A | 377 | 180.949 | 160.762 | -4.587 | 1.00 | 16.78 | A |
| ATOM C | 2096 | C | VAL | A | 377 | 178.386 | 158.284 | -3.229 | 1.00 | 20.23 | A |
| ATOM O | 2097 | O | VAL | A | 377 | 178.647 | 157.099 | -3.037 | 1.00 | 20.59 | A |
| ATOM N | 2098 | N | LEU | A | 378 | 177.392 | 158.937 | -2.620 | 1.00 | 20.07 | A |
| ATOM C | 2099 | CA | LEU | A | 378 | 176.526 | 158.314 | -1.619 | 1.00 | 20.48 | A |
| ATOM C | 2100 | CB | LEU | A | 378 | 175.514 | 159.346 | -1.107 | 1.00 | 19.33 | A |
| ATOM C | 2101 | CG | LEU | A | 378 | 175.794 | 160.087 | 0.237 | 1.00 | 25.33 | A |
| ATOM C | 2102 | CD1 | LEU | A | 378 | 177.197 | 159.794 | 0.722 | 1.00 | 23.60 | A |
| ATOM C | 2103 | CD2 | LEU | A | 378 | 175.575 | 161.610 | 0.073 | 1.00 | 20.05 | A |
| ATOM C | 2104 | C | LEU | A | 378 | 175.812 | 157.030 | -2.120 | 1.00 | 22.64 | A |
| ATOM O | 2105 | O | LEU | A | 378 | 175.497 | 156.130 | -1.318 | 1.00 | 21.93 | A |
| ATOM N | 2106 | N | GLU | A | 379 | 175.612 | 156.930 | -3.435 | 1.00 | 19.85 | A |
| ATOM C | 2107 | CA | GLU | A | 379 | 174.972 | 155.756 | -4.020 | 1.00 | 24.21 | A |
| ATOM C | 2108 | CB | GLU | A | 379 | 174.038 | 156.118 | -5.171 | 1.00 | 29.18 | A |
| ATOM C | 2109 | CG | GLU | A | 379 | 173.003 | 157.140 | -4.872 | 1.00 | 37.87 | A |
| ATOM C | 2110 | CD | GLU | A | 379 | 172.172 | 157.458 | -6.104 | 1.00 | 44.63 | A |
| ATOM O | 2111 | OE1 | GLU | A | 379 | 172.774 | 157.586 | -7.214 | 1.00 | 47.26 | A |
| ATOM O | 2112 | OE2 | GLU | A | 379 | 170.924 | 157.587 | -5.961 | 1.00 | 46.30 | A |
| ATOM C | 2113 | C | GLU | A | 379 | 175.989 | 154.817 | -4.623 | 1.00 | 22.35 | A |
| ATOM O | 2114 | O | GLU | A | 379 | 175.607 | 153.788 | -5.180 | 1.00 | 22.07 | A |
| ATOM N | 2115 | N | HIS | A | 380 | 177.268 | 155.165 | -4.555 | 1.00 | 20.53 | A |
| ATOM C | 2116 | CA | HIS | A | 380 | 178.278 | 154.301 | -5.145 | 1.00 | 20.63 | A |
| ATOM C | 2117 | CB | HIS | A | 380 | 179.683 | 154.909 | -4.995 | 1.00 | 15.41 | A |
| ATOM C | 2118 | CG | HIS | A | 380 | 180.717 | 154.184 | -5.800 | 1.00 | 17.05 | A |
| ATOM C | 2119 | CD2 | HIS | A | 380 | 181.309 | 154.493 | -6.982 | 1.00 | 16.91 | A |
| ATOM N | 2120 | ND1 | HIS | A | 380 | 181.129 | 152.901 | -5.498 | 1.00 | 16.78 | A |
| ATOM C | 2121 | CE1 | HIS | A | 380 | 181.918 | 152.453 | -6.459 | 1.00 | 16.66 | A |
| ATOM N | 2122 | NE2 | HIS | A | 380 | 182.042 | 153.399 | -7.372 | 1.00 | 15.37 | A |
| ATOM C | 2123 | C | HIS | A | 380 | 178.209 | 152.904 | -4.509 | 1.00 | 20.19 | A |
| ATOM O | 2124 | O | HIS | A | 380 | 178.129 | 152.774 | -3.293 | 1.00 | 21.40 | A |
| ATOM N | 2125 | N | PRO | A | 381 | 178.231 | 151.843 | -5.333 | 1.00 | 21.11 | A |
| ATOM C | 2126 | CD | PRO | A | 381 | 178.446 | 151.912 | -6.792 | 1.00 | 20.62 | A |
| ATOM C | 2127 | CA | PRO | A | 381 | 178.165 | 150.440 | -4.874 | 1.00 | 22.07 | A |
| ATOM C | 2128 | CB | PRO | A | 381 | 178.512 | 149.635 | -6.139 | 1.00 | 23.24 | A |
| ATOM C | 2129 | CG | PRO | A | 381 | 177.998 | 150.510 | -7.246 | 1.00 | 25.34 | A |
| ATOM C | 2130 | C | PRO | A | 381 | 179.103 | 150.109 | -3.708 | 1.00 | 20.71 | A |
| ATOM O | 2131 | O | PRO | A | 381 | 178.722 | 149.416 | -2.762 | 1.00 | 22.12 | A |
| ATOM N | 2132 | N | TRP | A | 382 | 180.330 | 150.607 | -3.783 | 1.00 | 19.08 | A |
| ATOM C | 2133 | CA | TRP | A | 382 | 181.307 | 150.367 | -2.733 | 1.00 | 18.09 | A |
| ATOM C | 2134 | CB | TRP | A | 382 | 182.667 | 150.885 | -3.160 | 1.00 | 16.26 | A |
| ATOM C | 2135 | CG | TRP | A | 382 | 183.757 | 150.595 | -2.180 | 1.00 | 17.75 | A |
| ATOM C | 2136 | CD2 | TRP | A | 382 | 184.370 | 151.524 | -1.271 | 1.00 | 17.24 | A |
| ATOM C | 2137 | CE2 | TRP | A | 382 | 185.366 | 150.816 | -0.559 | 1.00 | 16.47 | A |
| ATOM C | 2138 | CE3 | TRP | A | 382 | 184.165 | 152.882 | -0.983 | 1.00 | 17.55 | A |
| ATOM C | 2139 | CD1 | TRP | A | 382 | 184.389 | 149.398 | -1.986 | 1.00 | 14.76 | A |
| ATOM N | 2140 | NE1 | TRP | A | 382 | 185.358 | 149.524 | -1.022 | 1.00 | 16.65 | A |
| ATOM C | 2141 | CZ2 | TRP | A | 382 | 186.168 | 151.422 | 0.421 | 1.00 | 15.85 | A |
| ATOM C | 2142 | CZ3 | TRP | A | 382 | 184.965 | 153.487 | -0.002 | 1.00 | 19.06 | A |
| ATOM C | 2143 | CH2 | TRP | A | 382 | 185.951 | 152.754 | 0.687 | 1.00 | 17.35 | A |
| ATOM C | 2144 | C | TRP | A | 382 | 180.898 | 151.042 | -1.431 | 1.00 | 18.11 | A |
| ATOM O | 2145 | O | TRP | A | 382 | 181.045 | 150.467 | -0.352 | 1.00 | 19.08 | A |
| ATOM N | 2146 | N | ILE | A | 383 | 180.391 | 152.266 | -1.533 | 1.00 | 18.03 | A |
| ATOM C | 2147 | CA | ILE | A | 383 | 179.963 | 153.003 | -0.354 | 1.00 | 17.34 | A |
| ATOM C | 2148 | CB | ILE | A | 383 | 179.585 | 154.460 | -0.709 | 1.00 | 15.70 | A |
| ATOM C | 2149 | CG2 | ILE | A | 383 | 178.985 | 155.178 | 0.495 | 1.00 | 13.48 | A |
| ATOM C | 2150 | CG1 | ILE | A | 383 | 180.841 | 155.212 | -1.132 | 1.00 | 8.79 | A |
| ATOM C | 2151 | CD1 | ILE | A | 383 | 181.809 | 155.455 | 0.019 | 1.00 | 11.19 | A |
| ATOM C | 2152 | C | ILE | A | 383 | 178.790 | 152.312 | 0.324 | 1.00 | 19.72 | A |
| ATOM O | 2153 | O | ILE | A | 383 | 178.851 | 152.044 | 1.523 | 1.00 | 21.11 | A |
| ATOM N | 2154 | N | THR | A | 384 | 177.739 | 151.994 | -0.428 | 1.00 | 18.67 | A |
| ATOM C | 2155 | CA | THR | A | 384 | 176.585 | 151.336 | 0.180 | 1.00 | 21.04 | A |
| ATOM C | 2156 | CB | THR | A | 384 | 175.352 | 151.287 | -0.784 | 1.00 | 22.27 | A |
| ATOM O | 2157 | OG1 | THR | A | 384 | 175.657 | 150.457 | -1.912 | 1.00 | 23.10 | A |
| ATOM C | 2158 | CG2 | THR | A | 384 | 174.969 | 152.706 | -1.271 | 1.00 | 15.14 | A |
| ATOM C | 2159 | C | THR | A | 384 | 176.868 | 149.921 | 0.721 | 1.00 | 20.77 | A |
| ATOM O | 2160 | O | THR | A | 384 | 176.220 | 149.496 | 1.683 | 1.00 | 24.40 | A |
| ATOM N | 2161 | N | ALA | A | 385 | 177.835 | 149.206 | 0.138 | 1.00 | 18.85 | A |
| ATOM C | 2162 | CA | ALA | A | 385 | 178.195 | 147.848 | 0.598 | 1.00 | 16.58 | A |
| ATOM C | 2163 | CB | ALA | A | 385 | 178.996 | 147.119 | -0.481 | 1.00 | 8.89 | A |
| ATOM C | 2164 | C | ALA | A | 385 | 179.021 | 147.877 | 1.890 | 1.00 | 19.30 | A |
| ATOM O | 2165 | O | ALA | A | 385 | 178.987 | 146.950 | 2.699 | 1.00 | 17.69 | A |
| ATOM N | 2166 | N | ASN | A | 386 | 179.748 | 148.966 | 2.104 | 1.00 | 23.78 | A |
| ATOM C | 2167 | CA | ASN | A | 386 | 180.614 | 149.059 | 3.274 | 1.00 | 24.44 | A |
| ATOM C | 2168 | CB | ASN | A | 386 | 182.039 | 149.240 | 2.783 | 1.00 | 22.82 | A |
| ATOM C | 2169 | CG | ASN | A | 386 | 182.509 | 148.051 | 1.997 | 1.00 | 22.92 | A |
| ATOM O | 2170 | OD1 | ASN | A | 386 | 182.581 | 146.947 | 2.539 | 1.00 | 23.56 | A |
| ATOM N | 2171 | ND2 | ASN | A | 386 | 182.807 | 148.251 | 0.706 | 1.00 | 20.68 | A |
| ATOM C | 2172 | C | ASN | A | 386 | 180.305 | 150.133 | 4.291 | 1.00 | 25.32 | A |
| ATOM O | 2173 | O | ASN | A | 386 | 180.891 | 150.175 | 5.366 | 1.00 | 25.70 | A |
| ATOM N | 2174 | N | SER | A | 387 | 179.385 | 151.009 | 3.950 | 1.00 | 28.67 | A |
| ATOM C | 2175 | CA | SER | A | 387 | 179.063 | 152.111 | 4.823 | 1.00 | 30.65 | A |
| ATOM C | 2176 | CB | SER | A | 387 | 178.507 | 153.232 | 3.955 | 1.00 | 29.51 | A |
| ATOM O | 2177 | OG | SER | A | 387 | 178.208 | 154.364 | 4.724 | 1.00 | 35.78 | A |
| ATOM C | 2178 | C | SER | A | 387 | 178.063 | 151.687 | 5.890 | 1.00 | 32.67 | A |
| ATOM O | 2179 | O | SER | A | 387 | 177.162 | 150.889 | 5.535 | 1.00 | 35.02 | A |
| ATOM O | 2180 | OXT | SER | A | 387 | 178.173 | 152.164 | 7.055 | 1.00 | 35.90 | A |
| ATOM C | 2181 | CB | SER | B | 7 | 187.273 | 195.899 | 10.921 | 1.00 | 30.72 | B |
| ATOM O | 2182 | OG | SER | B | 7 | 185.919 | 195.490 | 11.062 | 1.00 | 28.92 | B |
| ATOM C | 2183 | C | SER | B | 7 | 187.728 | 193.792 | 12.227 | 1.00 | 32.69 | B |
| ATOM O | 2184 | O | SER | B | 7 | 187.932 | 192.918 | 11.331 | 1.00 | 33.35 | B |
| ATOM N | 2185 | N | SER | B | 7 | 189.583 | 195.368 | 11.576 | 1.00 | 34.34 | B |
| ATOM C | 2186 | CA | SER | B | 7 | 188.150 | 195.268 | 12.001 | 1.00 | 32.37 | B |
| ATOM N | 2187 | N | TYR | B | 8 | 187.139 | 193.527 | 13.402 | 1.00 | 28.10 | B |
| ATOM C | 2188 | CA | TYR | B | 8 | 186.671 | 192.188 | 13.775 | 1.00 | 25.10 | B |
| ATOM C | 2189 | CB | TYR | B | 8 | 187.465 | 191.681 | 14.999 | 1.00 | 22.64 | B |
| ATOM C | 2190 | CG | TYR | B | 8 | 188.962 | 191.579 | 14.731 | 1.00 | 22.03 | B |
| ATOM C | 2191 | CD1 | TYR | B | 8 | 189.806 | 192.667 | 14.982 | 1.00 | 19.79 | B |
| ATOM C | 2192 | CE1 | TYR | B | 8 | 191.153 | 192.623 | 14.642 | 1.00 | 19.33 | B |
| ATOM C | 2193 | CD2 | TYR | B | 8 | 189.524 | 190.435 | 14.128 | 1.00 | 18.09 | B |
| ATOM C | 2194 | CE2 | TYR | B | 8 | 190.881 | 190.391 | 13.775 | 1.00 | 18.91 | B |
| ATOM C | 2195 | CZ | TYR | B | 8 | 191.686 | 191.488 | 14.038 | 1.00 | 20.94 | B |
| ATOM O | 2196 | OH | TYR | B | 8 | 193.020 | 191.444 | 13.712 | 1.00 | 23.31 | B |
| ATOM C | 2197 | C | TYR | B | 8 | 185.151 | 192.143 | 14.045 | 1.00 | 24.33 | B |
| ATOM O | 2198 | O | TYR | B | 8 | 184.677 | 191.422 | 14.936 | 1.00 | 23.49 | B |
| ATOM N | 2199 | N | SER | B | 9 | 184.410 | 192.931 | 13.263 | 1.00 | 24.49 | B |
| ATOM C | 2200 | CA | SER | B | 9 | 182.945 | 193.017 | 13.314 | 1.00 | 24.46 | B |
| ATOM C | 2201 | CB | SER | B | 9 | 182.455 | 194.444 | 13.006 | 1.00 | 23.56 | B |
| ATOM O | 2202 | OG | SER | B | 9 | 182.756 | 195.336 | 14.059 | 1.00 | 29.98 | B |
| ATOM C | 2203 | C | SER | B | 9 | 182.426 | 192.085 | 12.229 | 1.00 | 22.36 | B |
| ATOM O | 2204 | O | SER | B | 9 | 182.469 | 192.408 | 11.035 | 1.00 | 22.10 | B |
| ATOM N | 2205 | N | TYR | B | 10 | 181.926 | 190.938 | 12.653 | 1.00 | 20.97 | B |
| ATOM C | 2206 | CA | TYR | B | 10 | 181.434 | 189.932 | 11.728 | 1.00 | 21.80 | B |
| ATOM C | 2207 | CB | TYR | B | 10 | 182.099 | 188.582 | 12.063 | 1.00 | 21.23 | B |
| ATOM C | 2208 | CG | TYR | B | 10 | 183.618 | 188.617 | 12.082 | 1.00 | 23.30 | B |
| ATOM C | 2209 | CD1 | TYR | B | 10 | 184.345 | 187.694 | 12.836 | 1.00 | 24.95 | B |
| ATOM C | 2210 | CE1 | TYR | B | 10 | 185.744 | 187.683 | 12.816 | 1.00 | 25.40 | B |
| ATOM C | 2211 | CD2 | TYR | B | 10 | 184.331 | 189.542 | 11.311 | 1.00 | 23.55 | B |
| ATOM C | 2212 | CE2 | TYR | B | 10 | 185.725 | 189.541 | 11.285 | 1.00 | 24.34 | B |
| ATOM C | 2213 | CZ | TYR | B | 10 | 186.421 | 188.600 | 12.036 | 1.00 | 26.19 | B |
| ATOM O | 2214 | OH | TYR | B | 10 | 187.800 | 188.526 | 11.956 | 1.00 | 31.92 | B |
| ATOM C | 2215 | C | TYR | B | 10 | 179.918 | 189.807 | 11.832 | 1.00 | 21.84 | B |
| ATOM O | 2216 | O | TYR | B | 10 | 179.334 | 190.098 | 12.872 | 1.00 | 22.91 | B |
| ATOM N | 2217 | N | ASP | B | 11 | 179.283 | 189.372 | 10.754 | 1.00 | 19.80 | B |
| ATOM C | 2218 | CA | ASP | B | 11 | 177.842 | 189.173 | 10.751 | 1.00 | 19.63 | B |
| ATOM C | 2219 | CB | ASP | B | 11 | 177.308 | 189.274 | 9.310 | 1.00 | 21.09 | B |
| ATOM C | 2220 | CG | ASP | B | 11 | 175.778 | 189.141 | 9.217 | 1.00 | 23.91 | B |
| ATOM O | 2221 | OD1 | ASP | B | 11 | 175.093 | 188.957 | 10.256 | 1.00 | 24.67 | B |
| ATOM O | 2222 | OD2 | ASP | B | 11 | 175.264 | 189.221 | 8.077 | 1.00 | 22.90 | B |
| ATOM C | 2223 | C | ASP | B | 11 | 177.653 | 187.756 | 11.297 | 1.00 | 19.67 | B |
| ATOM O | 2224 | O | ASP | B | 11 | 177.383 | 186.820 | 10.565 | 1.00 | 20.37 | B |
| ATOM N | 2225 | N | ALA | B | 12 | 177.844 | 187.593 | 12.590 | 1.00 | 19.26 | B |
| ATOM C | 2226 | CA | ALA | B | 12 | 177.696 | 186.285 | 13.174 | 1.00 | 19.72 | B |
| ATOM C | 2227 | CB | ALA | B | 12 | 178.993 | 185.498 | 13.013 | 1.00 | 20.60 | B |
| ATOM C | 2228 | C | ALA | B | 12 | 177.337 | 186.502 | 14.640 | 1.00 | 20.60 | B |
| ATOM O | 2229 | O | ALA | B | 12 | 177.450 | 187.632 | 15.149 | 1.00 | 20.41 | B |
| ATOM N | 2230 | N | PRO | B | 13 | 176.918 | 185.448 | 15.350 | 1.00 | 20.13 | B |
| ATOM C | 2231 | CD | PRO | B | 13 | 176.769 | 184.055 | 14.907 | 1.00 | 17.56 | B |
| ATOM C | 2232 | CA | PRO | B | 13 | 176.545 | 185.608 | 16.761 | 1.00 | 16.88 | B |
| ATOM C | 2233 | CB | PRO | B | 13 | 175.959 | 184.246 | 17.137 | 1.00 | 16.25 | B |
| ATOM C | 2234 | CG | PRO | B | 13 | 175.670 | 183.563 | 15.811 | 1.00 | 18.64 | B |
| ATOM C | 2235 | C | PRO | B | 13 | 177.663 | 186.014 | 17.728 | 1.00 | 17.09 | B |
| ATOM O | 2236 | O | PRO | B | 13 | 178.797 | 185.573 | 17.590 | 1.00 | 17.90 | B |
| ATOM N | 2237 | N | SER | B | 14 | 177.334 | 186.839 | 18.720 | 1.00 | 17.14 | B |
| ATOM C | 2238 | CA | SER | B | 14 | 178.294 | 187.258 | 19.739 | 1.00 | 20.00 | B |
| ATOM C | 2239 | CB | SER | B | 14 | 179.094 | 188.499 | 19.294 | 1.00 | 19.42 | B |
| ATOM O | 2240 | OG | SER | B | 14 | 178.288 | 189.384 | 18.544 | 1.00 | 30.47 | B |
| ATOM C | 2241 | C | SER | B | 14 | 177.587 | 187.518 | 21.069 | 1.00 | 18.32 | B |
| ATOM O | 2242 | O | SER | B | 14 | 178.199 | 187.968 | 22.056 | 1.00 | 14.51 | B |
| ATOM N | 2243 | N | ASP | B | 15 | 176.297 | 187.215 | 21.076 | 1.00 | 18.48 | B |
| ATOM C | 2244 | CA | ASP | B | 15 | 175.445 | 187.376 | 22.248 | 1.00 | 23.79 | B |
| ATOM C | 2245 | CB | ASP | B | 15 | 173.991 | 187.526 | 21.783 | 1.00 | 30.49 | B |
| ATOM C | 2246 | CG | ASP | B | 15 | 173.676 | 188.932 | 21.226 | 1.00 | 39.83 | B |
| ATOM O | 2247 | OD1 | ASP | B | 15 | 174.558 | 189.605 | 20.616 | 1.00 | 44.43 | B |
| ATOM O | 2248 | OD2 | ASP | B | 15 | 172.510 | 189.378 | 21.401 | 1.00 | 45.45 | B |
| ATOM C | 2249 | C | ASP | B | 15 | 175.569 | 186.172 | 23.199 | 1.00 | 21.79 | B |
| ATOM O | 2250 | O | ASP | B | 15 | 175.594 | 185.035 | 22.754 | 1.00 | 22.39 | B |
| ATOM N | 2251 | N | PHE | B | 16 | 175.660 | 186.432 | 24.500 | 1.00 | 21.47 | B |
| ATOM C | 2252 | CA | PHE | B | 16 | 175.756 | 185.373 | 25.514 | 1.00 | 22.21 | B |
| ATOM C | 2253 | CB | PHE | B | 16 | 175.700 | 185.993 | 26.930 | 1.00 | 19.46 | B |
| ATOM C | 2254 | CG | PHE | B | 16 | 175.637 | 184.978 | 28.025 | 1.00 | 18.34 | B |
| ATOM C | 2255 | CD1 | PHE | B | 16 | 176.789 | 184.362 | 28.491 | 1.00 | 18.98 | B |
| ATOM C | 2256 | CD2 | PHE | B | 16 | 174.417 | 184.569 | 28.528 | 1.00 | 15.51 | B |
| ATOM C | 2257 | CE1 | PHE | B | 16 | 176.716 | 183.339 | 29.435 | 1.00 | 18.59 | B |
| ATOM C | 2258 | CE2 | PHE | B | 16 | 174.340 | 183.552 | 29.469 | 1.00 | 15.01 | B |
| ATOM C | 2259 | CZ | PHE | B | 16 | 175.488 | 182.939 | 29.921 | 1.00 | 15.05 | B |
| ATOM C | 2260 | C | PHE | B | 16 | 174.542 | 184.448 | 25.301 | 1.00 | 21.16 | B |
| ATOM O | 2261 | O | PHE | B | 16 | 173.442 | 184.938 | 25.058 | 1.00 | 21.78 | B |
| ATOM N | 2262 | N | ILE | B | 17 | 174.739 | 183.135 | 25.370 | 1.00 | 19.97 | B |
| ATOM C | 2263 | CA | ILE | B | 17 | 173.652 | 182.163 | 25.182 | 1.00 | 20.86 | B |
| ATOM C | 2264 | CB | ILE | B | 17 | 173.965 | 181.144 | 24.009 | 1.00 | 21.47 | B |
| ATOM C | 2265 | CG2 | ILE | B | 17 | 172.988 | 179.964 | 24.011 | 1.00 | 11.76 | B |
| ATOM C | 2266 | CG1 | ILE | B | 17 | 173.957 | 181.844 | 22.654 | 1.00 | 16.04 | B |
| ATOM C | 2267 | CD1 | ILE | B | 17 | 174.760 | 181.051 | 21.631 | 1.00 | 18.42 | B |
| ATOM C | 2268 | C | ILE | B | 17 | 173.478 | 181.318 | 26.439 | 1.00 | 21.56 | B |
| ATOM O | 2269 | O | ILE | B | 17 | 174.462 | 180.965 | 27.086 | 1.00 | 23.11 | B |
| ATOM N | 2270 | N | ASN | B | 18 | 172.227 | 181.002 | 26.778 | 1.00 | 24.87 | B |
| ATOM C | 2271 | CA | ASN | B | 18 | 171.893 | 180.143 | 27.928 | 1.00 | 26.79 | B |
| ATOM C | 2272 | CB | ASN | B | 18 | 170.485 | 180.437 | 28.427 | 1.00 | 26.34 | B |
| ATOM C | 2273 | CG | ASN | B | 18 | 170.052 | 179.482 | 29.543 | 1.00 | 30.28 | B |
| ATOM O | 2274 | OD1 | ASN | B | 18 | 170.753 | 178.519 | 29.870 | 1.00 | 30.01 | B |
| ATOM N | 2275 | ND2 | ASN | B | 18 | 168.893 | 179.747 | 30.125 | 1.00 | 28.50 | B |
| ATOM C | 2276 | C | ASN | B | 18 | 171.911 | 178.718 | 27.373 | 1.00 | 28.06 | B |
| ATOM O | 2277 | O | ASN | B | 18 | 170.947 | 178.305 | 26.737 | 1.00 | 31.09 | B |
| ATOM N | 2278 | N | PHE | B | 19 | 172.969 | 177.950 | 27.616 | 1.00 | 30.32 | B |
| ATOM C | 2279 | CA | PHE | B | 19 | 173.027 | 176.599 | 27.050 | 1.00 | 32.46 | B |
| ATOM C | 2280 | CB | PHE | B | 19 | 174.460 | 176.061 | 27.076 | 1.00 | 27.50 | B |
| ATOM C | 2281 | CG | PHE | B | 19 | 175.430 | 176.837 | 26.214 | 1.00 | 22.59 | B |
| ATOM C | 2282 | CD1 | PHE | B | 19 | 176.332 | 177.737 | 26.785 | 1.00 | 20.42 | B |
| ATOM C | 2283 | CD2 | PHE | B | 19 | 175.426 | 176.682 | 24.831 | 1.00 | 21.89 | B |
| ATOM C | 2284 | CE1 | PHE | B | 19 | 177.223 | 178.454 | 25.989 | 1.00 | 19.77 | B |
| ATOM C | 2285 | CE2 | PHE | B | 19 | 176.312 | 177.398 | 24.021 | 1.00 | 18.13 | B |
| ATOM C | 2286 | CZ | PHE | B | 19 | 177.206 | 178.291 | 24.605 | 1.00 | 19.50 | B |
| ATOM C | 2287 | C | PHE | B | 19 | 172.086 | 175.529 | 27.618 | 1.00 | 36.90 | B |
| ATOM O | 2288 | O | PHE | B | 19 | 172.063 | 174.397 | 27.124 | 1.00 | 41.31 | B |
| ATOM N | 2289 | N | SER | B | 20 | 171.317 | 175.844 | 28.646 | 1.00 | 41.32 | B |
| ATOM C | 2290 | CA | SER | B | 20 | 170.402 | 174.824 | 29.142 | 1.00 | 46.46 | B |
| ATOM C | 2291 | CB | SER | B | 20 | 170.317 | 174.921 | 30.652 | 1.00 | 47.60 | B |
| ATOM O | 2292 | OG | SER | B | 20 | 170.456 | 176.275 | 31.032 | 1.00 | 49.62 | B |
| ATOM C | 2293 | C | SER | B | 20 | 169.029 | 175.021 | 28.496 | 1.00 | 48.43 | B |
| ATOM O | 2294 | O | SER | B | 20 | 168.337 | 174.060 | 28.170 | 1.00 | 50.43 | B |
| ATOM N | 2295 | N | SER | B | 21 | 168.645 | 176.275 | 28.286 | 1.00 | 50.08 | B |
| ATOM C | 2296 | CA | SER | B | 21 | 167.349 | 176.574 | 27.676 | 1.00 | 50.65 | B |
| ATOM C | 2297 | CB | SER | B | 21 | 166.742 | 177.810 | 28.317 | 1.00 | 49.82 | B |
| ATOM O | 2298 | OG | SER | B | 21 | 167.467 | 178.945 | 27.866 | 1.00 | 50.01 | B |
| ATOM C | 2299 | C | SER | B | 21 | 167.581 | 176.880 | 26.204 | 1.00 | 51.89 | B |
| ATOM O | 2300 | O | SER | B | 21 | 166.843 | 176.336 | 25.325 | 1.00 | 51.53 | B |
| ATOM O | 2301 | OXT | SER | B | 21 | 168.482 | 177.716 | 25.953 | 1.00 | 53.54 | B |
| ATOM C | 2302 | CB | ASN | C | 30 | 165.336 | 177.781 | 10.155 | 1.00 | 41.18 | C |
| ATOM C | 2303 | CG | ASN | C | 30 | 164.486 | 178.568 | 9.178 | 1.00 | 46.58 | C |
| ATOM O | 2304 | OD1 | ASN | C | 30 | 164.828 | 179.693 | 8.808 | 1.00 | 49.62 | C |
| ATOM N | 2305 | ND2 | ASN | C | 30 | 163.363 | 177.973 | 8.740 | 1.00 | 49.11 | C |
| ATOM C | 2306 | C | ASN | C | 30 | 167.301 | 179.381 | 10.314 | 1.00 | 36.00 | C |
| ATOM O | 2307 | O | ASN | C | 30 | 167.202 | 179.731 | 9.143 | 1.00 | 34.36 | C |
| ATOM N | 2308 | N | ASN | C | 30 | 165.267 | 179.744 | 11.676 | 1.00 | 37.72 | C |
| ATOM C | 2309 | CA | ASN | C | 30 | 166.154 | 178.688 | 11.078 | 1.00 | 38.33 | C |
| ATOM N | 2310 | N | ILE | C | 31 | 168.406 | 179.537 | 11.026 | 1.00 | 32.89 | C |
| ATOM C | 2311 | CA | ILE | C | 31 | 169.605 | 180.229 | 10.589 | 1.00 | 28.57 | C |
| ATOM C | 2312 | CB | ILE | C | 31 | 170.635 | 180.011 | 11.676 | 1.00 | 31.08 | C |
| ATOM C | 2313 | CG2 | ILE | C | 31 | 171.873 | 180.840 | 11.426 | 1.00 | 30.71 | C |
| ATOM C | 2314 | CG1 | ILE | C | 31 | 169.991 | 180.405 | 13.012 | 1.00 | 34.10 | C |
| ATOM C | 2315 | CD1 | ILE | C | 31 | 169.738 | 181.909 | 13.147 | 1.00 | 41.37 | C |
| ATOM C | 2316 | C | ILE | C | 31 | 170.254 | 180.041 | 9.214 | 1.00 | 25.67 | C |
| ATOM O | 2317 | O | ILE | C | 31 | 170.816 | 180.996 | 8.677 | 1.00 | 20.90 | C |
| ATOM N | 2318 | N | ASP | C | 32 | 170.188 | 178.856 | 8.623 | 1.00 | 23.52 | C |
| ATOM C | 2319 | CA | ASP | C | 32 | 170.872 | 178.678 | 7.351 | 1.00 | 24.02 | C |
| ATOM C | 2320 | CB | ASP | C | 32 | 171.126 | 177.197 | 7.086 | 1.00 | 27.28 | C |
| ATOM C | 2321 | CG | ASP | C | 32 | 169.858 | 176.423 | 6.805 | 1.00 | 32.74 | C |
| ATOM O | 2322 | OD1 | ASP | C | 32 | 168.852 | 177.009 | 6.338 | 1.00 | 36.15 | C |
| ATOM O | 2323 | OD2 | ASP | C | 32 | 169.884 | 175.202 | 7.039 | 1.00 | 33.71 | C |
| ATOM C | 2324 | C | ASP | C | 32 | 170.289 | 179.324 | 6.104 | 1.00 | 23.19 | C |
| ATOM O | 2325 | O | ASP | C | 32 | 170.804 | 179.126 | 5.001 | 1.00 | 22.55 | C |
| ATOM N | 2326 | N | SER | C | 33 | 169.223 | 180.090 | 6.271 | 1.00 | 21.46 | C |
| ATOM C | 2327 | CA | SER | C | 33 | 168.608 | 180.781 | 5.136 | 1.00 | 22.57 | C |
| ATOM C | 2328 | CB | SER | C | 33 | 167.205 | 181.244 | 5.499 | 1.00 | 23.28 | C |
| ATOM O | 2329 | OG | SER | C | 33 | 166.326 | 180.142 | 5.639 | 1.00 | 33.59 | C |
| ATOM C | 2330 | C | SER | C | 33 | 169.448 | 182.002 | 4.805 | 1.00 | 19.49 | C |
| ATOM O | 2331 | O | SER | C | 33 | 169.273 | 182.639 | 3.770 | 1.00 | 20.33 | C |
| ATOM N | 2332 | N | TRP | C | 34 | 170.344 | 182.330 | 5.728 | 1.00 | 18.04 | C |
| ATOM C | 2333 | CA | TRP | C | 34 | 171.254 | 183.466 | 5.621 | 1.00 | 16.02 | C |
| ATOM C | 2334 | CB | TRP | C | 34 | 172.234 | 183.422 | 6.792 | 1.00 | 15.82 | C |
| ATOM C | 2335 | CG | TRP | C | 34 | 173.240 | 184.531 | 6.836 | 1.00 | 15.40 | C |
| ATOM C | 2336 | CD2 | TRP | C | 34 | 174.599 | 184.462 | 6.404 | 1.00 | 13.15 | C |
| ATOM C | 2337 | CE2 | TRP | C | 34 | 175.180 | 185.735 | 6.640 | 1.00 | 14.42 | C |
| ATOM C | 2338 | CE3 | TRP | C | 34 | 175.387 | 183.451 | 5.843 | 1.00 | 10.87 | C |
| ATOM C | 2339 | CD1 | TRP | C | 34 | 173.052 | 185.801 | 7.302 | 1.00 | 13.49 | C |
| ATOM N | 2340 | ME1 | TRP | C | 34 | 174.212 | 186.530 | 7.189 | 1.00 | 11.42 | C |
| ATOM C | 2341 | CZ2 | TRP | C | 34 | 176.523 | 186.024 | 6.330 | 1.00 | 14.91 | C |
| ATOM C | 2342 | CZ3 | TRP | C | 34 | 176.727 | 183.738 | 5.538 | 1.00 | 12.26 | C |
| ATOM C | 2343 | CH2 | TRP | C | 34 | 177.278 | 185.016 | 5.785 | 1.00 | 10.61 | C |
| ATOM C | 2344 | C | TRP | C | 34 | 172.022 | 183.499 | 4.299 | 1.00 | 15.62 | C |
| ATOM O | 2345 | O | TRP | C | 34 | 172.251 | 184.579 | 3.735 | 1.00 | 16.88 | C |
| ATOM N | 2346 | N | PHE | C | 35 | 172.435 | 182.324 | 3.819 | 1.00 | 13.45 | C |
| ATOM C | 2347 | CA | PHE | C | 35 | 173.191 | 182.222 | 2.567 | 1.00 | 12.33 | C |
| ATOM C | 2348 | CB | PHE | C | 35 | 173.739 | 180.801 | 2.344 | 1.00 | 8.78 | C |
| ATOM C | 2349 | CG | PHE | C | 35 | 174.597 | 180.304 | 3.456 | 1.00 | 9.30 | C |
| ATOM C | 2350 | CD1 | PHE | C | 35 | 174.042 | 179.566 | 4.495 | 1.00 | 9.43 | C |
| ATOM C | 2351 | CD2 | PHE | C | 35 | 175.960 | 180.610 | 3.500 | 1.00 | 9.38 | C |
| ATOM C | 2352 | CE1 | PHE | C | 35 | 174.822 | 179.141 | 5.565 | 1.00 | 6.69 | C |
| ATOM C | 2353 | CE2 | PHE | C | 35 | 176.749 | 180.190 | 4.569 | 1.00 | 8.62 | C |
| ATOM C | 2354 | CZ | PHE | C | 35 | 176.174 | 179.455 | 5.602 | 1.00 | 10.55 | C |
| ATOM C | 2355 | C | PHE | C | 35 | 172.367 | 182.623 | 1.351 | 1.00 | 13.51 | C |
| ATOM O | 2356 | O | PHE | C | 35 | 172.779 | 183.502 | 0.597 | 1.00 | 13.63 | C |
| ATOM N | 2357 | N | ALA | C | 36 | 171.207 | 181.997 | 1.153 | 1.00 | 14.52 | C |
| ATOM C | 2358 | CA | ALA | C | 36 | 170.392 | 182.349 | -0.008 | 1.00 | 18.05 | C |
| ATOM C | 2359 | CB | ALA | C | 36 | 169.246 | 181.349 | -0.209 | 1.00 | 13.28 | C |
| ATOM C | 2360 | C | ALA | C | 36 | 169.844 | 183.775 | 0.103 | 1.00 | 18.08 | C |
| ATOM O | 2361 | O | ALA | C | 36 | 169.797 | 184.500 | -0.899 | 1.00 | 20.25 | C |
| ATOM N | 2362 | N | GLU | C | 37 | 169.458 | 184.192 | 1.307 | 1.00 | 18.61 | C |
| ATOM C | 2363 | CA | GLU | C | 37 | 168.905 | 185.536 | 1.505 | 1.00 | 21.91 | C |
| ATOM C | 2364 | CB | GLU | C | 37 | 168.391 | 185.686 | 2.933 | 1.00 | 22.41 | C |
| ATOM C | 2365 | CG | GLU | C | 37 | 167.103 | 184.901 | 3.240 | 1.00 | 24.75 | C |
| ATOM C | 2366 | CD | GLU | C | 37 | 166.619 | 185.133 | 4.681 | 1.00 | 30.55 | C |
| ATOM O | 2367 | OE1 | GLU | C | 37 | 167.473 | 185.511 | 5.529 | 1.00 | 31.00 | C |
| ATOM O | 2368 | OE2 | GLU | C | 37 | 165.414 | 184.932 | 4.976 | 1.00 | 30.11 | C |
| ATOM C | 2369 | C | GLU | C | 37 | 169.913 | 186.649 | 1.194 | 1.00 | 22.74 | C |
| ATOM O | 2370 | O | GLU | C | 37 | 169.561 | 187.754 | 0.791 | 1.00 | 22.40 | C |
| ATOM N | 2371 | N | LYS | C | 38 | 171.181 | 186.332 | 1.375 | 1.00 | 25.80 | C |
| ATOM C | 2372 | CA | LYS | C | 38 | 172.269 | 187.252 | 1.114 | 1.00 | 24.79 | C |
| ATOM C | 2373 | CB | LYS | C | 38 | 173.438 | 186.831 | 1.982 | 1.00 | 27.15 | C |
| ATOM C | 2374 | CG | LYS | C | 38 | 174.708 | 187.488 | 1.649 | 1.00 | 30.16 | C |
| ATOM C | 2375 | CD | LYS | C | 38 | 175.776 | 187.057 | 2.622 | 1.00 | 31.96 | C |
| ATOM C | 2376 | CE | LYS | C | 38 | 177.007 | 187.927 | 2.391 | 1.00 | 35.28 | C |
| ATOM N | 2377 | NZ | LYS | C | 38 | 178.028 | 187.735 | 3.456 | 1.00 | 40.31 | C |
| ATOM C | 2378 | C | LYS | C | 38 | 172.625 | 187.194 | -0.373 | 1.00 | 25.05 | C |
| ATOM O | 2379 | O | LYS | C | 38 | 173.062 | 188.177 | -0.969 | 1.00 | 23.54 | C |
| ATOM N | 2380 | N | ALA | C | 39 | 172.418 | 186.032 | -0.975 | 1.00 | 23.76 | C |
| ATOM C | 2381 | CA | ALA | C | 39 | 172.687 | 185.849 | -2.400 | 1.00 | 23.35 | C |
| ATOM C | 2382 | CB | ALA | C | 39 | 172.656 | 184.343 | -2.762 | 1.00 | 20.98 | C |
| ATOM C | 2383 | C | ALA | C | 39 | 171.642 | 186.576 | -3.245 | 1.00 | 24.77 | C |
| ATOM O | 2384 | O | ALA | C | 39 | 171.950 | 187.112 | -4.318 | 1.00 | 24.28 | C |
| ATOM N | 2385 | N | ASN | C | 40 | 170.403 | 186.592 | -2.754 | 1.00 | 23.38 | C |
| ATOM C | 2386 | CA | ASN | C | 40 | 169.306 | 187.210 | -3.480 | 1.00 | 23.68 | C |
| ATOM C | 2387 | CB | ASN | C | 40 | 167.993 | 186.526 | -3.137 | 1.00 | 18.03 | C |
| ATOM C | 2388 | CG | ASN | C | 40 | 168.024 | 185.035 | -3.395 | 1.00 | 17.89 | C |
| ATOM O | 2389 | OD1 | ASN | C | 40 | 168.646 | 184.553 | -4.351 | 1.00 | 18.06 | C |
| ATOM N | 2390 | ND2 | ASN | C | 40 | 167.325 | 184.287 | -2.545 | 1.00 | 16.76 | C |
| ATOM C | 2391 | C | ASN | C | 40 | 169.122 | 188.701 | -3.279 | 1.00 | 25.17 | C |
| ATOM O | 2392 | O | ASN | C | 40 | 168.413 | 189.332 | -4.052 | 1.00 | 27.11 | C |
| ATOM N | 2393 | N | LEU | C | 41 | 169.731 | 189.268 | -2.246 | 1.00 | 26.95 | C |
| ATOM C | 2394 | CA | LEU | C | 41 | 169.600 | 190.709 | -1.965 | 1.00 | 29.31 | C |
| ATOM C | 2395 | CB | LEU | C | 41 | 170.380 | 191.047 | -0.705 | 1.00 | 29.88 | C |
| ATOM C | 2396 | CG | LEU | C | 41 | 169.816 | 192.308 | -0.057 | 1.00 | 30.53 | C |
| ATOM C | 2397 | CD1 | LEU | C | 41 | 168.319 | 192.148 | 0.221 | 1.00 | 30.41 | C |
| ATOM C | 2398 | CD2 | LEU | C | 41 | 170.580 | 192.558 | 1.209 | 1.00 | 29.64 | C |
| ATOM C | 2399 | C | LEU | C | 41 | 170.091 | 191.589 | -3.120 | 1.00 | 29.84 | C |
| ATOM O | 2400 | O | LEU | C | 41 | 171.051 | 191.230 | -3.787 | 1.00 | 30.13 | C |
| ATOM N | 2401 | N | GLU | C | 42 | 169.487 | 192.763 | -3.312 | 1.00 | 32.17 | C |
| ATOM C | 2402 | CA | GLU | C | 42 | 169.851 | 193.627 | -4.439 | 1.00 | 35.33 | C |
| ATOM C | 2403 | CB | GLU | C | 42 | 168.583 | 193.847 | -5.286 | 1.00 | 33.50 | C |
| ATOM C | 2404 | CG | GLU | C | 42 | 167.819 | 192.560 | -5.616 | 1.00 | 32.30 | C |
| ATOM C | 2405 | CD | GLU | C | 42 | 166.473 | 192.784 | -6.334 | 1.00 | 34.80 | C |
| ATOM O | 2406 | OE1 | GLU | C | 42 | 165.892 | 193.892 | -6.241 | 1.00 | 34.22 | C |
| ATOM O | 2407 | OE2 | GLU | C | 42 | 165.980 | 191.829 | -6.987 | 1.00 | 33.99 | C |
| ATOM C | 2408 | C | GLU | C | 42 | 170.537 | 194.977 | -4.190 | 1.00 | 37.36 | C |
| ATOM O | 2409 | O | GLU | C | 42 | 170.487 | 195.539 | -3.109 | 1.00 | 38.22 | C |
| ATOM N | 2410 | N | ASN | C | 43 | 171.220 | 195.457 | -5.221 | 1.00 | 43.15 | C |
| ATOM C | 2411 | CA | ASN | C | 43 | 171.863 | 196.790 | -5.266 | 1.00 | 48.74 | C |
| ATOM C | 2412 | CB | ASN | C | 43 | 172.611 | 197.184 | -3.967 | 1.00 | 48.84 | C |
| ATOM C | 2413 | CG | ASN | C | 43 | 173.769 | 196.255 | -3.632 | 1.00 | 50.97 | C |
| ATOM O | 2414 | OD1 | ASN | C | 43 | 173.749 | 195.054 | -3.970 | 1.00 | 50.34 | C |
| ATOM N | 2415 | ND2 | ASN | C | 43 | 174.793 | 196.804 | -2.939 | 1.00 | 51.10 | C |
| ATOM C | 2416 | C | ASN | C | 43 | 172.809 | 196.704 | -6.455 | 1.00 | 52.67 | C |
| ATOM O | 2417 | O | ASN | C | 43 | 172.493 | 197.379 | -7.493 | 1.00 | 54.34 | C |
| ATOM O | 2418 | OXT | ASN | C | 43 | 173.808 | 195.931 | -6.344 | 1.00 | 55.98 | C |
| ATOM P | 2419 | PB | ADP | S | 531 | 193.788 | 175.824 | 12.432 | 1.00 | 20.87 | S |
| ATOM O | 2420 | O1B | ADP | S | 531 | 193.884 | 176.352 | 13.792 | 1.00 | 32.28 | S |
| ATOM O | 2421 | O2B | ADP | S | 531 | 193.566 | 176.837 | 11.394 | 1.00 | 26.12 | S |
| ATOM O | 2422 | O3B | ADP | S | 531 | 194.979 | 174.908 | 12.132 | 1.00 | 31.89 | S |
| ATOM P | 2423 | PA | ADP | S | 531 | 191.748 | 174.063 | 13.545 | 1.00 | 18.35 | S |
| ATOM O | 2424 | O1A | ADP | S | 531 | 190.611 | 174.901 | 14.032 | 1.00 | 26.99 | S |
| ATOM O | 2425 | O2A | ADP | S | 531 | 191.357 | 172.755 | 12.954 | 1.00 | 26.80 | S |
| ATOM O | 2426 | O3A | ADP | S | 531 | 192.532 | 174.874 | 12.450 | 1.00 | 28.14 | S |
| ATOM O | 2427 | O5* | ADP | S | 531 | 192.642 | 173.906 | 14.839 | 1.00 | 22.21 | S |
| ATOM C | 2428 | C5* | ADP | S | 531 | 193.768 | 172.965 | 14.869 | 1.00 | 20.71 | S |
| ATOM C | 2429 | C4* | ADP | S | 531 | 193.594 | 171.623 | 15.640 | 1.00 | 17.30 | S |
| ATOM O | 2430 | O4* | ADP | S | 531 | 192.923 | 171.842 | 16.897 | 1.00 | 18.08 | S |
| ATOM C | 2431 | C3* | ADP | S | 531 | 192.751 | 170.494 | 14.997 | 1.00 | 17.80 | S |
| ATOM O | 2432 | O3* | ADP | S | 531 | 193.561 | 169.824 | 14.038 | 1.00 | 18.78 | S |
| ATOM C | 2433 | C2* | ADP | S | 531 | 192.369 | 169.659 | 16.218 | 1.00 | 19.78 | S |
| ATOM O | 2434 | O2* | ADP | S | 531 | 193.423 | 168.749 | 16.615 | 1.00 | 19.10 | S |
| ATOM C | 2435 | C1* | ADP | S | 531 | 192.152 | 170.704 | 17.312 | 1.00 | 18.01 | S |
| ATOM N | 2436 | N9 | ADP | S | 531 | 190.711 | 171.169 | 17.445 | 1.00 | 18.12 | S |
| ATOM C | 2437 | C8 | ADP | S | 531 | 190.169 | 172.237 | 16.808 | 1.00 | 20.72 | S |
| ATOM N | 2438 | N7 | ADP | S | 531 | 188.877 | 172.431 | 17.108 | 1.00 | 19.94 | S |
| ATOM C | 2439 | C5 | ADP | S | 531 | 188.596 | 171.408 | 17.961 | 1.00 | 15.87 | S |
| ATOM C | 2440 | C6 | ADP | S | 531 | 187.377 | 171.071 | 18.652 | 1.00 | 14.49 | S |
| ATOM N | 2441 | N6 | ADP | S | 531 | 186.260 | 171.696 | 18.563 | 1.00 | 9.66 | S |
| ATOM N | 2442 | N1 | ADP | S | 531 | 187.474 | 169.935 | 19.454 | 1.00 | 13.01 | S |
| ATOM C | 2443 | C2 | ADP | S | 531 | 188.642 | 169.198 | 19.595 | 1.00 | 14.56 | S |
| ATOM N | 2444 | N3 | ADP | S | 531 | 189.773 | 169.512 | 18.982 | 1.00 | 17.25 | S |
| ATOM C | 2445 | C4 | ADP | S | 531 | 189.716 | 170.610 | 18.162 | 1.00 | 16.09 | S |
| ATOM MG+2 | 2446 | MG | MG | X | 1 | 192.801 | 173.013 | 10.897 | 1.00 | 18.98 | X |
| ATOM MG+2 | 2447 | MG | MG | X | 2 | 192.933 | 178.461 | 10.215 | 1.00 | 21.17 | X |
| ATOM MG+2 | 2448 | MG | MG | X | 3 | 174.135 | 172.090 | -6.081 | 1.00 | 37.43 | X |
| ATOM S | 2449 | S | S04 | Y | 1 | 175.520 | 167.060 | -4.810 | 1.00 | 40.27 | Y |
| ATOM O | 2450 | O1 | SO4 | Y | 1 | 175.005 | 168.134 | -3.755 | 1.00 | 40.61 | Y |
| ATOM O | 2451 | 02 | SO4 | Y | 1 | 176.918 | 167.395 | -5.118 | 1.00 | 40.85 | Y |
| ATOM O | 2452 | 03 | SO4 | Y | 1 | 175.333 | 165.874 | -4.319 | 1.00 | 45.29 | Y |
| ATOM O | 2453 | 04 | SO4 | Y | 1 | 174.705 | 167.387 | -6.003 | 1.00 | 46.53 | Y |
| ATOM S | 2454 | S | SO4 | Y | 2 | 196.317 | 160.442 | 22.149 | 1.00 | 52.91 | Y |
| ATOM O | 2455 | O1 | SO4 | Y | 2 | 194.902 | 160.702 | 22.904 | 1.00 | 53.25 | Y |
| ATOM O | 2456 | 02 | SO4 | Y | 2 | 197.156 | 161.615 | 22.484 | 1.00 | 52.23 | Y |
| ATOM O | 2457 | 03 | SO4 | Y | 2 | 196.808 | 159.261 | 22.506 | 1.00 | 50.45 | Y |
| ATOM O | 2458 | 04 | SO4 | Y | 2 | 195.932 | 160.567 | 20.717 | 1.00 | 53.17 | Y |
| ATOM S | 2459 | S | SO4 | Y | 3 | 184.237 | 187.281 | -0.473 | 1.00 | 69.64 | Y |
| ATOM O | 2460 | 01 | SO4 | Y | 3 | 182.905 | 188.209 | -0.613 | 1.00 | 70.60 | Y |
| ATOM O | 2461 | 02 | SO4 | Y | 3 | 185.274 | 188.143 | 0.172 | 1.00 | 70.08 | Y |
| ATOM O | 2462 | 03 | SO4 | Y | 3 | 183.925 | 186.181 | 0.170 | 1.00 | 69.68 | Y |
| ATOM O | 2463 | 04 | S04 | Y | 3 | 184.641 | 187.119 | -1.915 | 1.00 | 70.92 | Y |
| ATOM O | 2464 | OH2 | WAT | W | 1 | 179.030 | 185.642 | -6.293 | 1.00 | 8.84 | W |
| ATOM O | 2465 | OH2 | WAT | W | 2 | 194.313 | 179.202 | -8.444 | 1.00 | 16.79 | W |
| ATOM O | 2466 | OH2 | WAT | W | 3 | 192.921 | 180.168 | 8.084 | 1.00 | 30.46 | W |
| ATOM O | 2467 | OH2 | WAT | W | 4 | 187.994 | 175.656 | 4.804 | 1.00 | 16.00 | W |
| ATOM O | 2468 | OH2 | WAT | W | 5 | 178.455 | 169.305 | -5.499 | 1.00 | 13.87 | W |
| ATOM O | 2469 | OH2 | WAT | W | 6 | 197.111 | 180.066 | 22.244 | 1.00 | 15.19 | W |
| ATOM O | 2470 | OH2 | WAT | W | 7 | 180.414 | 171.384 | 22.814 | 1.00 | 8.30 | W |
| ATOM O | 2471 | OH2 | WAT | W | 8 | 188.179 | 184.543 | -6.390 | 1.00 | 10.24 | W |
| ATOM O | 2472 | OH2 | WAT | W | 9 | 188.183 | 181.649 | 3.509 | 1.00 | 23.15 | W |
| ATOM O | 2473 | OH2 | WAT | W | 10 | 185.065 | 157.114 | 14.496 | 1.00 | 16.39 | W |
| ATOM O | 2474 | OH2 | WAT | W | 11 | 192.854 | 158.543 | 18.441 | 1.00 | 20.42 | W |
| ATOM O | 2475 | OH2 | WAT | W | 12 | 194.144 | 171.703 | 11.618 | 1.00 | 13.61 | W |
| ATOM O | 2476 | OH2 | WAT | W | 13 | 194.572 | 183.197 | -8.077 | 1.00 | 36.64 | W |
| ATOM O | 2477 | OH2 | WAT | W | 14 | 198.254 | 147.344 | -7.377 | 1.00 | 23.99 | W |
| ATOM O | 2478 | OH2 | WAT | W | 15 | 174.141 | 170.073 | -2.678 | 1.00 | 18.36 | W |
| ATOM O | 2479 | OH2 | WAT | W | 16 | 197.136 | 162.247 | 7.860 | 1.00 | 20.24 | W |
| ATOM O | 2480 | OH2 | WAT | W | 17 | 178.742 | 175.122 | -2.821 | 1.00 | 12.41 | W |
| ATOM O | 2481 | OH2 | WAT | W | 18 | 200.365 | 168.406 | 2.165 | 1.00 | 9.98 | W |
| ATOM O | 2482 | OH2 | WAT | W | 19 | 168.522 | 176.594 | 9.704 | 1.00 | 27.47 | W |
| ATOM O | 2483 | OH2 | WAT | W | 20 | 193.215 | 179.995 | 11.973 | 1.00 | 17.15 | W |
| ATOM O | 2484 | OH2 | WAT | W | 21 | 188.165 | 173.468 | 14.493 | 1.00 | 18.83 | W |
| ATOM O | 2485 | OH2 | WAT | W | 22 | 178.977 | 189.493 | 5.006 | 1.00 | 25.99 | W |
| ATOM O | 2486 | OH2 | WAT | W | 23 | 194.904 | 178.835 | 14.332 | 1.00 | 9.84 | W |
| ATOM O | 2487 | OH2 | WAT | W | 24 | 172.594 | 187.824 | 25.336 | 1.00 | 22.88 | W |
| ATOM O | 2488 | OH2 | WAT | W | 25 | 186.612 | 173.366 | -9.877 | 1.00 | 19.22 | W |
| ATOM O | 2489 | OH2 | WAT | W | 26 | 176.840 | 183.702 | 20.193 | 1.00 | 22.57 | W |
| ATOM O | 2490 | OH2 | WAT | W | 27 | 176.801 | 160.388 | 11.646 | 1.00 | 15.90 | W |
| ATOM O | 2491 | OH2 | WAT | W | 28 | 178.487 | 174.788 | 11.702 | 1.00 | 18.10 | W |
| ATOM O | 2492 | OH2 | WAT | W | 29 | 181.155 | 186.952 | 32.619 | 1.00 | 32.00 | W |
| ATOM O | 2493 | OH2 | WAT | W | 30 | 209.304 | 163.564 | -16.281 | 1.00 | 27.44 | W |
| ATOM O | 2494 | OH2 | WAT | W | 31 | 203.827 | 165.930 | -0.838 | 1.00 | 16.37 | W |
| ATOM O | 2495 | OH2 | WAT | W | 32 | 183.937 | 190.638 | 21.333 | 1.00 | 17.02 | W |
| ATOM O | 2496 | OH2 | WAT | W | 33 | 190.362 | 181.451 | 8.363 | 1.00 | 23.84 | W |
| ATOM O | 2497 | OH2 | WAT | W | 34 | 201.524 | 183.136 | 11.412 | 1.00 | 27.78 | W |
| ATOM O | 2498 | OH2 | WAT | W | 35 | 176.401 | 172.283 | 12.285 | 1.00 | 22.44 | W |
| ATOM O | 2499 | OH2 | WAT | W | 36 | 191.486 | 178.801 | -8.556 | 1.00 | 14.47 | W |
| ATOM O | 2500 | OH2 | WAT | W | 37 | 193.706 | 178.975 | 16.555 | 1.00 | 28.73 | W |
| ATOM O | 2501 | OH2 | WAT | W | 38 | 200.711 | 191.015 | 5.492 | 1.00 | 22.39 | W |
| ATOM O | 2502 | OH2 | WAT | W | 39 | 198.698 | 163.980 | -2.087 | 1.00 | 16.88 | W |
| ATOM O | 2503 | OH2 | WAT | W | 40 | 186.096 | 174.714 | 13.402 | 1.00 | 14.25 | W |
| ATOM O | 2504 | OH2 | WAT | W | 41 | 189.561 | 189.228 | 27.405 | 1.00 | 21.42 | W |
| ATOM O | 2505 | OH2 | WAT | W | 42 | 185.742 | 175.020 | -12.633 | 1.00 | 32.61 | W |
| ATOM O | 2506 | OH2 | WAT | W | 43 | 189.284 | 166.218 | 21.436 | 1.00 | 18.57 | W |
| ATOM O | 2507 | OH2 | WAT | W | 44 | 189.806 | 150.396 | 10.582 | 1.00 | 17.00 | W |
| ATOM O | 2508 | OH2 | WAT | W | 45 | 182.606 | 183.843 | 1.498 | 1.00 | 23.82 | W |
| ATOM O | 2509 | OH2 | WAT | W | 46 | 203.088 | 159.272 | -4.093 | 1.00 | 20.75 | W |
| ATOM O | 2510 | OH2 | WAT | W | 47 | 197.775 | 190.097 | 18.980 | 1.00 | 22.00 | W |
| ATOM O | 2511 | OH2 | WAT | W | 48 | 193.113 | 164.352 | 18.292 | 1.00 | 18.52 | W |
| ATOM O | 2512 | OH2 | WAT | W | 49 | 188.303 | 192.170 | 8.139 | 1.00 | 29.33 | W |
| ATOM O | 2513 | OH2 | WAT | W | 50 | 178.988 | 188.073 | 29.292 | 1.00 | 22.03 | W |
| ATOM O | 2514 | OH2 | WAT | W | 51 | 179.041 | 176.835 | -4.879 | 1.00 | 28.93 | W |
| ATOM O | 2515 | OH2 | WAT | W | 52 | 177.094 | 169.275 | 14.745 | 1.00 | 17.83 | W |
| ATOM O | 2516 | OH2 | WAT | W | 53 | 173.359 | 158.848 | 5.717 | 1.00 | 26.69 | W |
| ATOM O | 2517 | OH2 | WAT | W | 54 | 184.713 | 145.517 | -1.099 | 1.00 | 25.66 | W |
| ATOM O | 2518 | OH2 | WAT | W | 55 | 197.989 | 162.673 | 24.709 | 1.00 | 31.35 | W |
| ATOM O | 2519 | OH2 | WAT | W | 56 | 190.279 | 149.132 | -0.366 | 1.00 | 23.37 | W |
| ATOM O | 2520 | OH2 | WAT | W | 57 | 175.195 | 156.308 | 1.515 | 1.00 | 25.31 | W |
| ATOM O | 2521 | OH2 | WAT | W | 58 | 189.320 | 149.244 | 12.871 | 1.00 | 39.74 | W |
| ATOM O | 2522 | OH2 | WAT | W | 59 | 191.345 | 160.562 | 17.366 | 1.00 | 19.61 | W |
| ATOM O | 2523 | OH2 | WAT | W | 60 | 209.300 | 189.438 | 10.191 | 1.00 | 32.59 | W |
| ATOM O | 2524 | OH2 | WAT | W | 61 | 176.961 | 195.154 | 23.343 | 1.00 | 26.84 | W |
| ATOM O | 2525 | OH2 | WAT | W | 62 | 194.908 | 178.479 | 10.038 | 1.00 | 12.42 | W |
| ATOM O | 2526 | OH2 | WAT | W | 63 | 192.932 | 176.681 | 8.727 | 1.00 | 17.49 | W |
| ATOM O | 2527 | OH2 | WAT | W | 64 | 191.151 | 178.650 | 10.287 | 1.00 | 25.58 | W |
| ATOM O | 2528 | OH2 | WAT | W | 65 | 182.000 | 183.023 | -13.348 | 1.00 | 15.88 | W |
| ATOM O | 2529 | OH2 | WAT | W | 66 | 174.844 | 179.377 | 29.234 | 1.00 | 21.85 | W |
| ATOM O | 2530 | OH2 | WAT | W | 67 | 192.666 | 150.025 | 4.037 | 1.00 | 24.70 | W |
| ATOM O | 2531 | OH2 | WAT | W | 68 | 191.227 | 167.117 | 19.848 | 1.00 | 27.52 | W |
| ATOM O | 2532 | OH2 | WAT | W | 69 | 195.798 | 166.787 | -4.678 | 1.00 | 16.05 | W |
| ATOM O | 2533 | OH2 | WAT | W | 70 | 188.683 | 164.953 | 24.250 | 1.00 | 16.65 | W |
| ATOM O | 2534 | OH2 | WAT | W | 71 | 202.921 | 187.953 | 5.807 | 1.00 | 25.48 | W |
| ATOM O | 2535 | OH2 | WAT | W | 72 | 173.656 | 180.838 | -1.296 | 1.00 | 19.16 | W |
| ATOM O | 2536 | OH2 | WAT | W | 73 | 178.223 | 170.722 | 21.753 | 1.00 | 28.96 | W |
| ATOM O | 2537 | OH2 | WAT | W | 74 | 200.047 | 185.273 | 4.691 | 1.00 | 36.63 | W |
| ATOM O | 2538 | OH2 | WAT | W | 75 | 199.421 | 160.492 | 21.694 | 1.00 | 54.41 | W |
| ATOM O | 2539 | OH2 | WAT | W | 76 | 174.343 | 151.575 | -4.090 | 1.00 | 24.22 | W |
| ATOM O | 2540 | OH2 | WAT | W | 77 | 199.217 | 156.232 | 8.084 | 1.00 | 24.50 | W |
| ATOM O | 2541 | OH2 | WAT | W | 78 | 186.693 | 195.451 | 15.184 | 1.00 | 30.28 | W |
| ATOM O | 2542 | OH2 | WAT | W | 79 | 204.072 | 167.051 | -16.474 | 1.00 | 30.81 | W |
| ATOM O | 2543 | OH2 | WAT | W | 80 | 189.729 | 170.883 | 13.071 | 1.00 | 24.00 | W |
| ATOM O | 2544 | OH2 | WAT | W | 81 | 193.562 | 167.033 | 18.576 | 1.00 | 34.40 | W |
| ATOM O | 2545 | OH2 | WAT | W | 82 | 188.055 | 177.150 | -16.174 | 1.00 | 39.31 | W |
| ATOM O | 2546 | OH2 | WAT | W | 83 | 209.167 | 161.801 | -3.764 | 1.00 | 29.76 | W |
| ATOM O | 2547 | OH2 | WAT | W | 84 | 189.954 | 187.203 | -3.104 | 1.00 | 30.21 | W |
| ATOM O | 2548 | OH2 | WAT | W | 85 | 166.356 | 175.210 | 9.226 | 1.00 | 35.64 | W |
| ATOM O | 2549 | OH2 | WAT | W | 86 | 209.038 | 162.643 | -13.951 | 1.00 | 29.71 | W |
| ATOM O | 2550 | OH2 | WAT | W | 87 | 179.913 | 164.861 | 20.417 | 1.00 | 23.03 | W |
| ATOM O | 2551 | OH2 | WAT | W | 88 | 176.985 | 177.272 | 13.711 | 1.00 | 28.39 | W |
| ATOM O | 2552 | OH2 | WAT | W | 89 | 197.775 | 160.663 | 10.355 | 1.00 | 30.90 | W |
| ATOM O | 2553 | OH2 | WAT | W | 90 | 180.418 | 156.423 | -15.983 | 1.00 | 38.69 | W |
| ATOM O | 2554 | OH2 | WAT | W | 91 | 197.603 | 165.048 | 7.104 | 1.00 | 39.85 | W |
| ATOM O | 2555 | OH2 | WAT | W | 92 | 201.038 | 146.684 | -3.627 | 1.00 | 28.72 | W |
| ATOM O | 2556 | OH2 | WAT | W | 93 | 191.798 | 173.307 | -1.526 | 1.00 | 20.55 | W |
| ATOM O | 2557 | OH2 | WAT | W | 94 | 195.433 | 186.638 | 2.426 | 1.00 | 32.36 | W |
| ATOM O | 2558 | OH2 | WAT | W | 95 | 185.689 | 149.073 | 10.194 | 1.00 | 32.44 | W |
| ATOM O | 2559 | OH2 | WAT | W | 96 | 181.725 | 186.361 | 4.468 | 1.00 | 44.13 | W |
| ATOM O | 2560 | OH2 | WAT | W | 97 | 190.638 | 181.376 | 1.418 | 1.00 | 40.50 | W |
| ATOM O | 2561 | OH2 | WAT | W | 98 | 203.221 | 164.983 | 2.765 | 1.00 | 30.78 | W |
| ATOM O | 2562 | OH2 | WAT | W | 99 | 191.430 | 148.459 | 1.931 | 1.00 | 29.87 | W |
| ATOM O | 2563 | OH2 | WAT | W | 100 | 172.186 | 178.856 | -0.480 | 1.00 | 35.57 | W |
| ATOM O | 2564 | OH2 | WAT | W | 101 | 195.541 | 178.234 | 1.979 | 1.00 | 25.17 | W |
| ATOM O | 2565 | OH2 | WAT | W | 102 | 170.598 | 187.029 | 21.078 | 1.00 | 24.60 | W |
| ATOM O | 2566 | OH2 | WAT | W | 103 | 175.607 | 172.274 | -8.036 | 1.00 | 36.47 | W |
| ATOM O | 2567 | OH2 | WAT | W | 104 | 168.429 | 188.864 | -6.367 | 1.00 | 22.94 | W |
| ATOM O | 2568 | OH2 | WAT | W | 105 | 186.340 | 190.812 | 24.666 | 1.00 | 32.99 | W |
| ATOM O | 2569 | OH2 | WAT | W | 106 | 201.816 | 192.555 | 4.023 | 1.00 | 28.31 | W |
| ATOM O | 2570 | OH2 | WAT | W | 107 | 202.504 | 159.814 | -7.441 | 1.00 | 22.58 | W |
| ATOM O | 2571 | OH2 | WAT | W | 108 | 174.542 | 185.298 | -11.131 | 1.00 | 31.96 | W |
| ATOM O | 2572 | OH2 | WAT | W | 109 | 175.457 | 179.403 | 15.662 | 1.00 | 39.35 | W |
| ATOM O | 2573 | OH2 | WAT | W | 110 | 196.564 | 193.700 | 7.818 | 1.00 | 32.38 | W |
| ATOM O | 2574 | OH2 | WAT | W | 111 | 173.232 | 181.808 | -8.349 | 1.00 | 19.06 | W |
| ATOM O | 2575 | OH2 | WAT | W | 112 | 182.827 | 187.517 | -4.364 | 1.00 | 45.61 | W |
| ATOM O | 2576 | OH2 | WAT | W | 113 | 189.838 | 194.231 | 7.217 | 1.00 | 42.16 | W |
| ATOM O | 2577 | OH2 | WAT | W | 114 | 191.170 | 159.628 | -12.340 | 1.00 | 35.35 | W |
| ATOM O | 2578 | OH2 | WAT | W | 115 | 191.207 | 184.866 | -0.535 | 1.00 | 34.38 | W |
| ATOM O | 2579 | OH2 | WAT | W | 116 | 183.572 | 197.288 | 15.706 | 1.00 | 39.58 | W |
| ATOM O | 2580 | OH2 | WAT | W | 117 | 199.841 | 150.695 | 13.176 | 1.00 | 35.40 | W |
| ATOM O | 2581 | OH2 | WAT | W | 118 | 174.316 | 180.622 | 18.227 | 1.00 | 29.36 | W |
| ATOM O | 2582 | OH2 | WAT | W | 119 | 174.539 | 169.945 | 15.478 | 1.00 | 36.75 | W |
| ATOM O | 2583 | OH2 | WAT | W | 120 | 186.965 | 189.198 | 27.316 | 1.00 | 33.73 | W |
| ATOM O | 2584 | OH2 | WAT | W | 121 | 185.505 | 146.137 | -4.208 | 1.00 | 30.91 | W |
| ATOM O | 2585 | OH2 | WAT | W | 122 | 168.783 | 176.131 | 0.277 | 1.00 | 37.59 | W |
| ATOM O | 2586 | OH2 | WAT | W | 123 | 179.830 | 187.974 | 34.894 | 1.00 | 39.81 | W |
| ATOM O | 2587 | OH2 | WAT | W | 124 | 194.391 | 191.022 | 26.003 | 1.00 | 35.26 | W |
| ATOM O | 2588 | OH2 | WAT | W | 125 | 175.707 | 190.116 | 18.448 | 1.00 | 37.47 | W |
| ATOM O | 2589 | OH2 | WAT | W | 126 | 172.799 | 187.052 | 31.328 | 1.00 | 38.58 | W |
| ATOM O | 2590 | OH2 | WAT | W | 127 | 173.867 | 181.212 | 14.618 | 1.00 | 28.93 | W |
| ATOM O | 2591 | OH2 | WAT | W | 128 | 169.850 | 183.554 | 9.734 | 1.00 | 31.19 | W |
| ATOM O | 2592 | OH2 | WAT | W | 129 | 201.846 | 186.034 | 10.890 | 1.00 | 39.65 | W |
| ATOM O | 2593 | OH2 | WAT | W | 130 | 192.261 | 183.101 | 8.973 | 1.00 | 35.20 | W |
| ATOM O | 2594 | OH2 | WAT | W | 131 | 195.036 | 155.601 | 22.286 | 1.00 | 43.08 | W |
| ATOM O | 2595 | OH2 | WAT | W | 132 | 188.136 | 149.463 | -10.689 | 1.00 | 31.69 | W |
| ATOM O | 2596 | OH2 | WAT | W | 133 | 193.611 | 166.439 | 22.911 | 1.00 | 37.73 | W |
| ATOM O | 2597 | OH2 | WAT | W | 134 | 169.159 | 198.181 | -6.371 | 1.00 | 34.05 | W |
| ATOM O | 2598 | OH2 | WAT | W | 135 | 173.141 | 166.101 | 3.246 | 1.00 | 37.73 | W |
| ATOM O | 2599 | OH2 | WAT | W | 136 | 196.411 | 181.887 | 24.452 | 1.00 | 31.18 | W |
| ATOM O | 2600 | OH2 | WAT | W | 137 | 166.875 | 190.046 | -8.389 | 1.00 | 35.44 | W |
| ATOM O | 2601 | OH2 | WAT | W | 138 | 168.310 | 173.985 | 5.026 | 1.00 | 36.83 | W |
| ATOM O | 2602 | OH2 | WAT | W | 139 | 191.553 | 162.337 | -15.173 | 1.00 | 30.34 | W |
| ATOM O | 2603 | OH2 | WAT | W | 140 | 196.789 | 179.956 | 0.077 | 1.00 | 34.96 | W |
| ATOM O | 2604 | OH2 | WAT | W | 141 | 204.362 | 177.082 | -3.998 | 1.00 | 42.74 | W |
| ATOM O | 2605 | OH2 | WAT | W | 142 | 178.237 | 157.118 | 15.427 | 1.00 | 37.51 | W |
| ATOM O | 2606 | OH2 | WAT | W | 143 | 180.703 | 166.918 | 18.919 | 1.00 | 22.25 | W |
| ATOM O | 2607 | OH2 | WAT | W | 144 | 190.076 | 196.775 | 13.865 | 1.00 | 38.81 | W |

## Claims

1. A crystal of phosphorylated human Aurora-A kinase fragment comprising amino acid residues 122-403 complexed with amino acid residues 1-43 of human TPX2, wherein said crystal diffracts to at least 3 angstrom resolution and has a crystal stability within 5% of its unit cell dimensions.

2. The crystal according to claim 1, having the coordinates as listed in Table B.

3. The crystal according to claim 1 or 2, said crystal belonging to the orthorhombic space group P2₁2₁2₁ and having the unit cell dimensions in angstroms: a = 59.63 ± 5%, b = 81.72 ± 5%, c = 70.38 ± 5%.

4. The crystal according to any one of claims 1 to 3, having a Aurora-A ligand binding site defined by the structure coordinates of Aurora-A amino acids Q127, W128, R126, L159, F157, E170, L169, V206, Y199, H187, R179, L178, V182, Y199, L188, l184, V252, K250, P282, H280 according to Table B.

5. A molecule or molecular complexe comprising at least a part of the ligand binding site defined by structure coordinates of Aurora-A amino acids Q127, W128, R126, L159, F157, E170, L169, V206, Y199, H187, R179, L178, V182, Y199, L188, I184, V252, K250, P282, H280 according to Table B, or a mutant or homologue thereof.

6. A machine-readable data storage medium comprising a data storage material encoded with machine readable data, wherein the data is defined by the structure coordinates of phosphorylated human Aurora-A kinase complexed with amino acid residues 1-43 of human TPX2 according to Table B or a homologue of said complex, wherein said homologue comprises backbone atoms that have a root mean square deviation from the backbone atoms of the complex of not more than 3.0 A.

7. A binding site in Aurora-A, or a homologue or mutant thereof, for an AR modulator in which a portion of said ligand is in van der Walls contact or hydrogen bonding contact with any portion or all of residues Q127, W128, R126, L159, F157, E170, L169, V206, Y199, H187, R179, L178, V182, Y199, L188, I184, V252, K250, P282, H280 of Aurora-A according to Table B.

8. The binding site according to claim 7, wherein the homologue or mutant has 25%-95% identity to residues Q127, W128, R126, L159, F157, E170, L169, V206, Y199, H187, R179, L178, V182, Y199, L188, I184, V252, K250, P282, H280 of Aurora-A according toTable B.

9. A method for identifying a compound that modulates Aurora-A kinase activity, the method comprising any combination of steps of :
a) modeling test compounds that fit spatially into the Aurora-A binding site as defined by structure coordinates according to Table B;
b) using said structure coordinates or binding site as set forth in claim 7 to identify structural and chemical features;
c) employing identified structural or chemical features to design or select compounds as potential Aurora-A modulators;
d) employing the three-dimensional structural model or the ligand binding site to design or select compounds as potential Aurora-A modulators;
e) synthesizing the potential Aurora-A modulators;
f) screening the potential Aurora-A modulators in an assay **characterized by** binding of a test compound to the Aurora-A; and
g) modifying or replacing one or more amino acids from Aurora-A selected from the group consisting of Q127, W128, R126, L159, F157, E170, L169, V206, Y199, H187, R179, L178, V182, Y199, L188, I184, V252, K250, P282, H280 of Aurora-A according to Table B.

10. An Aurora-A modulator identified by the method of claim 9.

11. An allosteric inhibitor of Aurora-A, at least a portion of which binds with any portion or all of residues Q127, W128, R126, L159, F157, E170, L169, V206, Y199, H187, R179, L178, V182, Y199, L188, I184, V252, K250, P282, H280 of Aurora-A according to Table B.

12. The allosteric inhibitor of claim 11, wherein binding is van der Walls contact or hydrogen bonding contact.

13. Indole and indene derivatives of formula (I) 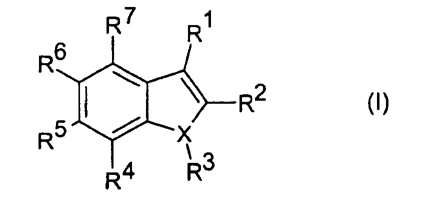 wherein
R¹ represents hydrogen, alkylene-NHR⁸, alkylene-OR⁸, or alkylene-SR⁸;
R² represents hydrogen, alkylene-NHR⁸, alkylene-OR⁸, or alkylene-SR⁸;
R³ represents hydrogen, alkyl, alkylene-R⁹, alkenylene-R⁹, alkynylene-R⁹, or arylene-R⁹;
R⁴ represents hydrogen;
R⁵ represents hydrogen, alkyl, OR¹⁰, NHR¹⁰, SR¹⁰, alkylene-R¹⁰, alkenylene-R¹⁰, alkynylene-R¹⁰, or arylene-R¹⁰;
R⁶ represents hydrogen, alkyl, OR¹⁰, NHR¹⁰, SR¹⁰, alkylene-R¹⁰, alkenylene-R¹⁰, alkynylene-R¹⁰, or arylene-R¹⁰;
R⁷ represents hydrogen;
R⁸ represents hydrogen, CO-alkyl, (aa)ₘasp(aa)ₙ, (aa)ₘglu(aa)ₙ, or (aa)ₘcys(aa)ₙ;
R⁹ represents NH-alkyl, N(alkyl)₂, N⁺(alkyl)₃, aryl, or heteroaryl;
R¹⁰ represents hydrogen, aryl, or substituted aryl;
X represents a nitrogen atom or CH;
aa represents an amino acid radical; and
n is zero or an integer of 1 to 10;
m is zero or an integer of 1 to 10,
provided that R¹ and R² are not both hydrogen and that R⁵ and R⁶ are not both hydrogen,
and optical isomers, physiologically acceptable salts and prodrugs thereof.

14. The compound according to claim 13, wherein one of residues R¹ and R², preferably R², is hydrogen and the other, preferably R¹, represents alkylene-NHR⁸.

15. The compound according to claim 13 or 14, wherein R⁸ is a radical of the formula (II) 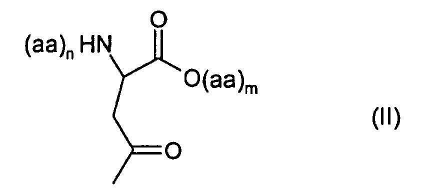 wherein
aa represents an amino acid radical;
n is zero or an integer of 1 to 10; and
m is zero or an integer of 1 to 10.

16. The compound according to any one of claims 13 to 15, wherein R³ is a radical of the formula (IV)

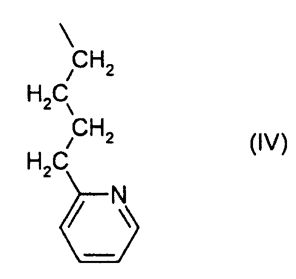

17. The compound according to any one of claims 13 to 16, wherein R⁵ and/or R⁶ represent OR¹⁰, wherein R¹⁰ is defined as in claim 13.

18. The compound according to any one of claims 13 to 17, wherein R¹⁰ is aryl which may be substituted with 1, 2 or 3 substituents independently selected from the group consisting of hydroxy -OPO₃H₂, -CH₂PO₃H₂, -CF₂PO₃H₂, -COOH, -CH(COOH)₂, -OPO₃(R¹¹)₂, -CH₂OPO₃(R¹¹)₂, -CF₂PO₃(R¹¹)₂, -COOR¹¹, and -CH(COOR¹¹)₂, wherein R¹¹ is a radical that is cleavable *in vivo.*

19. The compound according to claim 18, wherein R¹¹ represents alkyl, CH₂OCO-alkyl, and C₂H₄-S-CO-alkyl.

20. The compound according to any one of claims 13 to 19, wherein R⁵ and/or R⁶ are/is the radical of formula (V)

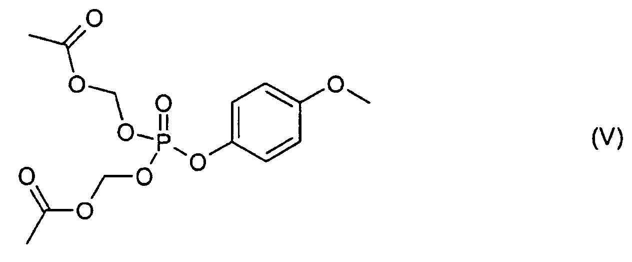

21. The compound according to claim 13, having the formula (Ia) 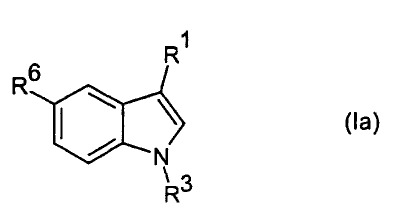 wherein
R¹, R³ and R⁶ are defined as in any one of claims 13 to 20.

22. The indole derivative of formula (1) 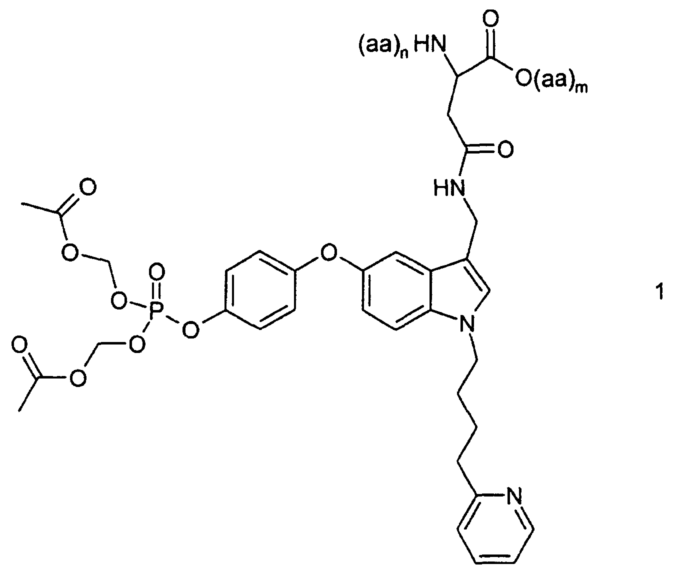 wherein
aa, n and m are defined as in claim 13,
and optical isomers and physiologically acceptable salts thereof.

23. The Aurora-A modulator of claim 10, the allosteric inhibitor of claims 11 or 12, or the indole or indene derivative of any one of claims 13 to 22 for use in therapy.

24. Pharmaceutical composition, comprising at least one Aurora-A modulator of claim 10, at least one allosteric inhibitor of claims 11 or 12, or at least one indole or indene derivative of any one of claims 13 to 22, optionally in combination with a pharmaceutically acceptable excipient.

25. The use of an Aurora-A modulator of claim 10, of an allosteric inhibitor of claims 11 or 12, or of an indole or indene derivative of any one of claims 13 to 22 in the manufacture of a medicament for treating cancer.

26. The use according to claim 25, wherein the cancer is a breast or colon carcinoma.


### Annex to the application documents - subsequently filed sequences listing

SEQUENCE LISTING

<110>  EMBL

<120>  Crystals of an Aurora-A TPX2 Complex, TPX2 binding site of Aurora-A,
Aurora-A ligands and their use

<130>  M/44278

<140>  EP03023136.9
<141>  2003-10-10

<160>  11

<170>  PatentIn version 3.1

<210>  1
<211>  43
<212>  PRT
<213>  Homo sapiens

<400>  1

Met Ser Gln Val Lys Ser Ser Tyr Ser Tyr Asp Ala Pro Ser Asp Phe
1               5                   10                  15

Ile Asn Phe Ser Ser Leu Asp Asp Glu Gly Asp Thr Gln Asn Ile Asp
            20                  25                  30

Ser Trp Phe Glu Glu Lys Ala Asn Leu Glu Asn
        35                  40

<210>  2
<211>  39
<212>  PRT
<213>  Xenopus sp.

<400>  2

Met Glu Asp Thr Gln Asp Thr Tyr Ser Tyr Asp Ala Pro Ser Ile Phe
1               5                   10                  15

Asn Phe Ser Ser Phe His Glu Asp His Asn Ala Asp Ser Trp Phe Asp
            20                  25                  30

Gln Val Thr Asn Ala Glu Asn
        35

<210>  3
<211>  45
<212>  PRT
<213>  Fugu rubripes

<400>  3

Met Ala Glu Ser Asn Phe Asp Ala Asp Ala Glu Leu Tyr Glu Tyr Asp
1               5                   10                  15

Ala Pro Ser Glu Val Val Asp Leu Lys Glu Leu Gln Asp Val Glu Gly
            20                  25                  30

Asp Asp Lys Trp Phe Glu Glu Gln Ala Leu Gly Val Asp
        35                  40                  45

<210>  4
<211>  282
<212>  PRT
<213>  Homo sapiens

<400>  4

Glu Ser Lys Lys Arg Gln Trp Ala Leu Glu Asp Phe Glu Ile Gly Arg
1               5                   10                  15

Pro Leu Gly Lys Gly Lys Phe Gly Asn Val Tyr Leu Ala Arg Glu Lys
            20                  25                  30

Gln Ser Lys Phe Ile Leu Ala Leu Lys Val Leu Phe Lys Ala Gln Leu
        35                  40                  45

Glu Lys Ala Gly Val Glu His Gln Leu Arg Arg Glu Val Glu Ile Gln
    50                  55                  60

Ser His Leu Arg His Pro Asn Ile Leu Arg Leu Tyr Gly Tyr Phe His
65                  70                  75                  80

Asp Ala Thr Arg Val Tyr Leu Ile Leu Glu Tyr Ala Pro Leu Gly Thr
                85                  90                  95

Val Tyr Arg Glu Leu Gln Lys Leu Ser Lys Phe Asp Glu Gln Arg Thr
            100                 105                 110

Ala Thr Tyr Ile Thr Glu Leu Ala Asn Ala Leu Ser Tyr Cys His Ser
        115                 120                 125

Lys Arg Val Ile His Arg Asp Ile Lys Pro Glu Asn Leu Leu Leu Gly
    130                 135                 140

Ser Ala Gly Glu Leu Lys Ile Ala Asp Phe Gly Trp Ser Val His Ala
145                 150                 155                 160

Pro Ser Ser Arg Arg Thr Thr Leu Cys Gly Thr Leu Asp Tyr Leu Pro
                165                 170                 175

Pro Glu Met Ile Glu Gly Arg Met His Asp Glu Lys Val Asp Leu Trp
            180                 185                 190

Ser Leu Gly Val Leu Cys Tyr Glu Phe Leu Val Gly Lys Pro Pro Phe
        195                 200                 205

Glu Ala Asn Thr Tyr Gln Glu Thr Tyr Lys Arg Ile Ser Arg Val Glu
    210                 215                 220

Phe Thr Phe Pro Asp Phe Val Thr Glu Gly Ala Arg Asp Leu Ile Ser
225                 230                 235                 240

Arg Leu Leu Lys His Asn Pro Ser Gln Arg Pro Met Leu Arg Glu Val
                245                 250                 255

Leu Glu His Pro Trp Ile Thr Ala Asn Ser Ser Lys Pro Ser Asn Cys
            260                 265                 270

Gln Asn Lys Glu Ser Ala Ser Lys Gln Ser
        275                 280

<210>  5
<211>  279
<212>  PRT
<213>  Xenopus sp.

<400>  5

Glu Gly Lys Lys Lys Gln Trp Cys Leu Glu Asp Phe Glu Ile Gly Arg
1               5                   10                  15

Pro Leu Gly Lys Gly Lys Phe Gly Asn Val Tyr Leu Ala Arg Glu Arg
            20                  25                  30

Glu Ser Lys Phe Ile Leu Ala Leu Lys Val Leu Phe Lys Ser Gln Leu
        35                  40                  45

Glu Lys Ala Gly Val Glu His Gln Leu Arg Arg Glu Val Glu Ile Gln
    50                  55                  60

Ser His Leu Arg His Pro Asn Ile Leu Arg Leu Tyr Gly Tyr Phe His
65                  70                  75                  80

Asp Ala Ser Arg Val Tyr Leu Ile Leu Asp Tyr Ala Pro Gly Gly Glu
                85                  90                  95

Leu Phe Arg Glu Leu Gln Lys Cys Thr Arg Phe Asp Asp Gln Arg Ser
            100                 105                 110

Ala Met Tyr Ile Lys Gln Leu Ala Glu Ala Leu Leu Tyr Cys His Ser
        115                 120                 125

Lys Lys Val Ile His Arg Asp Ile Lys Pro Glu Asn Leu Leu Leu Gly
    130                 135                 140

Ser Asn Gly Glu Leu Lys Ile Ala Asp Phe Gly Trp Ser Val His Ala
145                 150                 155                 160

Pro Ser Ser Arg Arg Thr Thr Leu Cys Gly Thr Leu Asp Tyr Leu Pro
                165                 170                 175

Pro Glu Met Ile Glu Gly Arg Met His Asp Glu Thr Val Asp Leu Trp
            180                 185                 190

Ser Leu Gly Val Leu Cys Tyr Glu Phe Leu Val Gly Lys Pro Pro Phe
        195                 200                 205

Glu Thr Asp Thr His Gln Glu Thr Tyr Arg Arg Ile Ser Lys Val Glu
    210                 215                 220

Phe Gln Tyr Pro Pro Tyr Val Ser Glu Glu Ala Arg Asp Leu Val Ser
225                 230                 235                 240

Lys Leu Leu Lys His Asn Pro Asn His Arg Leu Pro Leu Lys Gly Val
                245                 250                 255

Leu Glu His Pro Trp Ile Ile Lys Asn Ser Gln Leu Lys Lys Lys Asp
            260                 265                 270

Glu Pro Leu Pro Gly Ala Gln
        275

<210>  6
<211>  267
<212>  PRT
<213>  Fugu rubripes

<400>  6

Arg Arg Arg Trp Ser Leu Glu Asn Phe Asp Ile Gly Arg Pro Leu Gly
1               5                   10                  15

Lys Gly Lys Phe Gly Asn Val Tyr Leu Ala Arg Glu Arg Gln Ser Arg
            20                  25                  30

Phe Ile Leu Ala Leu Lys Val Leu Phe Lys Lys Gln Leu Glu Lys Ala
        35                  40                  45

Gly Val Glu His Gln Leu Arg Arg Glu Val Glu Ile Gln Ser His Leu
    50                  55                  60

Arg His Pro Asn Ile Leu Arg Leu Tyr Gly Tyr Phe His Asp Pro Ser
65                  70                  75                  80

Arg Val Tyr Leu Ile Leu Glu Phe Ala Pro Lys Gly Glu Leu Tyr Gly
                85                  90                  95

Glu Leu Gln Arg Cys Gly Ser Phe Pro Glu Glu Arg Ser Ala Thr Tyr
            100                 105                 110

Ile Met Glu Leu Ala Asp Ala Leu Asn Tyr Cys His Ser Lys Lys Val
        115                 120                 125

Ile His Arg Asp Ile Lys Pro Glu Asn Leu Leu Leu Gly Ala Asn Gly
    130                 135                 140

Glu Leu Lys Ile Ala Asp Phe Gly Trp Ser Val His Thr Pro Ser Ser
145                 150                 155                 160

Arg Arg Ser Thr Leu Cys Gly Thr Leu Asp Tyr Leu Pro Pro Glu Met
                165                 170                 175

Ile Glu Gly Lys Thr His Asp Glu Lys Val Asp Leu Trp Ser Leu Gly
            180                 185                 190

Val Leu Cys Tyr Glu Phe Leu Val Gly Lys Pro Pro Phe Glu Ala Lys
        195                 200                 205

Thr His Glu Glu Thr Tyr Arg Arg Ile Ser Arg Val Glu Tyr Thr Tyr
    210                 215                 220

Pro Ala His Thr Asn Ile Ser Asp Gly Ala Lys Asp Leu Val Ser Arg
225                 230                 235                 240

Leu Leu Lys His Asn Pro Met Gln Arg Leu Pro Val Gln Gly Val Leu
                245                 250                 255

Ala His Pro Trp Val Val Glu Arg Ser Thr Lys
            260                 265

<210>  7
<211>  269
<212>  PRT
<213>  Drosophila sp.

<400>  7

Gln Lys Pro Lys Lys Thr Trp Glu Leu Asn Asn Phe Asp Ile Gly Arg
1               5                   10                  15

Leu Leu Gly Arg Gly Lys Phe Gly Asn Val Tyr Leu Ala Arg Glu Lys
            20                  25                  30

Glu Ser Gln Phe Val Val Ala Leu Lys Val Leu Phe Lys Arg Gln Ile
        35                  40                  45

Gly Glu Ser Asn Val Glu His Gln Val Arg Arg Glu Ile Glu Ile Gln
    50                  55                  60

Ser His Leu Arg His Pro His Ile Leu Arg Leu Tyr Ala Tyr Phe His
65                  70                  75                  80

Asp Asp Val Arg Ile Tyr Leu Ile Leu Glu Tyr Ala Pro Gln Gly Thr
                85                  90                  95

Leu Phe Asn Ala Leu Gln Ala Gln Pro Met Lys Arg Phe Asp Glu Arg
            100                 105                 110

Gln Ser Ala Thr Tyr Ile Gln Ala Leu Cys Ser Ala Leu Leu Tyr Leu
        115                 120                 125

His Glu Arg Asp Ile Ile His Arg Asp Ile Lys Pro Glu Asn Leu Leu
    130                 135                 140

Leu Gly His Lys Gly Val Leu Lys Ile Ala Asp Phe Gly Trp Ser Val
145                 150                 155                 160

His Glu Pro Asn Ser Met Arg Met Thr Leu Cys Gly Thr Val Asp Tyr
                165                 170                 175

Leu Pro Pro Glu Met Val Gln Gly Lys Pro His Thr Lys Asn Val Asp
            180                 185                 190

Leu Trp Ser Leu Gly Val Leu Cys Phe Glu Leu Leu Val Gly His Ala
        195                 200                 205

Pro Phe Tyr Ser Lys Asn Tyr Asp Glu Thr Tyr Lys Lys Ile Leu Lys
    210                 215                 220

Val Asp Tyr Lys Leu Pro Glu His Ile Ser Lys Ala Ala Ser His Leu
225                 230                 235                 240

Ile Ser Lys Leu Leu Val Leu Asn Pro Gln His Arg Leu Pro Leu Asp
                245                 250                 255

Gln Val Met Val His Pro Trp Ile Leu Ala His Thr Gln

260                 265

<210>  8
<211>  284
<212>  PRT
<213>  Caenorhabditis elegans

<400>  8

Ala Arg Glu Glu Ser Cys Trp Ser Leu Asp Asp Phe Asp Val Gly Arg
1               5                   10                  15

Pro Leu Gly Lys Gly Lys Phe Gly Asn Val Phe Ile Ser Arg Glu Lys
            20                  25                  30

Lys Thr Lys Arg Ile Ile Ala Leu Lys Val Leu Phe Lys Thr Gln Leu
        35                  40                  45

Leu Gln Leu Gly Val Ser His Gln Leu Lys Arg Glu Ile Glu Ile Gln
    50                  55                  60

Tyr His Leu Arg His Pro Asn Ile Leu Thr Leu Tyr Gly Tyr Phe His
65                  70                  75                  80

Asp Asp Lys Arg Val Phe Val Ile Leu Asp Tyr Ala Ser Arg Gly Glu
                85                  90                  95

Leu Phe Asn Val Leu Gln Ser Gln Pro Gly His Lys Val Asn Glu Val
            100                 105                 110

Ile Ala Gly Arg Phe Val Arg Gln Leu Ala Asn Ala Leu His Tyr Cys
        115                 120                 125

His Ser Lys Gly Val Ile His Arg Asp Ile Lys Pro Glu Asn Leu Leu
    130                 135                 140

Leu Asp Ser Lys Leu Asn Leu Lys Leu Ala Asp Phe Gly Trp Ser Val
145                 150                 155                 160

Val Ala Asp His Ser Lys Arg His Thr Leu Cys Gly Thr Met Asp Tyr
                165                 170                 175

Leu Ala Pro Glu Met Val Ser Asn Gln Pro His Asp Phe Asn Val Asp
            180                 185                 190

Ile Trp Ala Ile Gly Ile Leu Leu Phe Glu Met Leu Val Gly Tyr Ala
        195                 200                 205

Pro Phe Ala Asn Gln Thr Gly Asp Lys Leu Ile Ala Arg Ile Lys Glu

210                 215                 220

Cys Lys Ile Tyr Ile Pro Ser Val Val Thr Asp Gly Ala Ala Ser Leu
225                 230                 235                 240

Ile Asn Ala Ile Ile Lys Lys Glu Pro Gln Glu Arg Leu Pro Leu Val
                245                 250                 255

Asp Ile Met Ala His Pro Trp Ile Lys Glu Met Lys Gln Arg Glu Asp
            260                 265                 270

Ile Glu Val Pro Leu Phe Ile Ser Thr Leu Thr Lys
        275                 280

<210>  9
<211>  279
<212>  PRT
<213>  Homo sapiens

<400>  9

Asp Ile Leu Thr Arg His Phe Thr Ile Asp Asp Phe Glu Ile Gly Arg
1               5                   10                  15

Pro Leu Gly Lys Gly Lys Phe Gly Asn Val Tyr Leu Ala Arg Glu Lys
            20                  25                  30

Lys Ser His Phe Ile Val Ala Leu Lys Val Leu Phe Lys Ser Gln Ile
        35                  40                  45

Glu Lys Glu Gly Val Glu His Gln Leu Arg Arg Glu Ile Glu Ile Gln
    50                  55                  60

Ala His Leu His His Pro Asn Ile Leu Arg Leu Tyr Asn Tyr Phe Tyr
65                  70                  75                  80

Asp Arg Arg Arg Ile Tyr Leu Ile Leu Glu Tyr Ala Pro Arg Gly Glu
                85                  90                  95

Leu Tyr Lys Glu Leu Gln Lys Ser Cys Thr Phe Asp Glu Gln Arg Thr
            100                 105                 110

Ala Thr Ile Met Glu Glu Leu Ala Asp Ala Leu Met Tyr Cys His Gly
        115                 120                 125

Lys Lys Val Ile His Arg Asp Ile Lys Pro Glu Asn Leu Leu Leu Gly
    130                 135                 140

Leu Lys Gly Glu Leu Lys Ile Ala Asp Phe Gly Trp Ser Val His Ala

145                 150                 155                 160

Pro Ser Leu Arg Arg Lys Thr Met Cys Gly Thr Leu Asp Tyr Leu Pro
                165                 170                 175

Pro Glu Met Ile Glu Gly Arg Met His Asn Glu Lys Val Asp Leu Trp
            180                 185                 190

Cys Ile Gly Val Leu Cys Tyr Glu Leu Leu Val Gly Asn Pro Pro Phe
        195                 200                 205

Glu Ser Ala Ser His Asn Glu Thr Tyr Arg Arg Ile Val Lys Val Asp
    210                 215                 220

Leu Lys Phe Pro Ala Ser Val Pro Thr Gly Ala Gln Asp Leu Ile Ser
225                 230                 235                 240

Lys Leu Leu Arg His Asn Pro Ser Glu Arg Leu Pro Leu Ala Gln Val
                245                 250                 255

Ser Ala His Pro Trp Val Arg Ala Asn Ser Arg Arg Val Leu Pro Pro
            260                 265                 270

Ser Ala Leu Gln Ser Val Ala
        275

<210>  10
<211>  280
<212>  PRT
<213>  Xenopus sp.

<400>  10

Glu Met Pro Lys Arg Lys Phe Thr Ile Asp Asp Phe Asp Ile Gly Arg
1               5                   10                  15

Pro Leu Gly Lys Gly Lys Phe Gly Asn Val Tyr Leu Ala Arg Glu Lys
            20                  25                  30

Gln Asn Lys Phe Ile Met Ala Leu Lys Val Leu Phe Lys Ser Gln Leu
        35                  40                  45

Glu Lys Glu Gly Val Glu His Gln Leu Arg Arg Glu Ile Glu Ile Gln
    50                  55                  60

Ser His Leu Arg His Pro Asn Ile Leu Arg Met Tyr Asn Tyr Phe His
65                  70                  75                  80

Asp Arg Lys Arg Ile Tyr Leu Met Leu Glu Phe Ala Pro Arg Gly Glu

85                  90                  95

Leu Tyr Lys Glu Leu Gln Lys His Gly Arg Phe Asp Glu Gln Arg Ser
            100                 105                 110

Ala Thr Phe Met Glu Glu Leu Ala Asp Ala Leu His Tyr Cys His Glu
        115                 120                 125

Arg Lys Val Ile His Arg Asp Ile Lys Pro Glu Asn Leu Leu Met Gly
    130                 135                 140

Tyr Lys Gly Glu Leu Lys Ile Ala Asp Phe Gly Trp Ser Val His Ala
145                 150                 155                 160

Pro Ser Leu Arg Arg Arg Thr Met Cys Gly Thr Leu Asp Tyr Leu Pro
                165                 170                 175

Pro Glu Met Ile Glu Gly Lys Thr His Asp Glu Lys Val Asp Leu Trp
            180                 185                 190

Cys Ala Gly Val Leu Cys Tyr Glu Phe Leu Val Gly Met Pro Pro Phe
        195                 200                 205

Asp Ser Pro Ser His Thr Glu Thr His Arg Arg Ile Val Asn Val Asp
    210                 215                 220

Leu Lys Phe Pro Pro Phe Leu Ser Asp Gly Ser Lys Asp Leu Ile Ser
225                 230                 235                 240

Lys Leu Leu Arg Tyr His Pro Pro Gln Arg Leu Pro Leu Lys Gly Val
                245                 250                 255

Met Glu His Pro Trp Val Lys Ala Asn Ser Arg Arg Val Leu Pro Pro
            260                 265                 270

Val Tyr Gln Ser Thr Gln Ser Lys
        275                 280

<210>  11
<211>  286
<212>  PRT
<213>  Unknown

<220>
<223>  Vertebrate cAPK

<400>  11

Thr Pro Ser Gln Asn Thr Ala Gln Leu Asp Gln Phe Asp Arg Ile Lys
1               5                   10                  15

Thr Leu Gly Thr Gly Ser Phe Gly Arg Val Met Leu Val Lys His Lys
            20                  25                  30

Glu Ser Gly Asn His Tyr Ala Met Lys Ile Leu Asp Lys Gln Lys Val
        35                  40                  45

Val Lys Leu Lys Gln Ile Glu His Thr Leu Asn Glu Lys Arg Ile Leu
    50                  55                  60

Gln Ala Val Asn Phe Pro Phe Leu Val Lys Leu Glu Phe Ser Phe Lys
65                  70                  75                  80

Asp Asn Ser Asn Leu Tyr Met Val Met Glu Tyr Val Ala Gly Gly Glu
                85                  90                  95

Met Phe Ser His Leu Arg Arg Ile Gly Arg Phe Ser Glu Pro His Ala
            100                 105                 110

Arg Phe Tyr Ala Ala Gln Ile Val Leu Thr Phe Glu Tyr Leu His Ser
        115                 120                 125

Leu Asp Leu Ile Tyr Arg Asp Leu Lys Pro Glu Asn Leu Leu Ile Asp
    130                 135                 140

Gln Gln Gly Tyr Ile Gln Val Thr Asp Phe Gly Phe Ala Lys Arg Val
145                 150                 155                 160

Lys Gly Arg Thr Trp Thr Leu Cys Gly Thr Pro Glu Tyr Leu Ala Pro
                165                 170                 175

Glu Ile Ile Leu Ser Lys Gly Tyr Asn Lys Ala Val Asp Trp Trp Ala
            180                 185                 190

Leu Gly Val Leu Ile Tyr Glu Met Ala Ala Gly Tyr Pro Pro Phe Phe
        195                 200                 205

Ala Asp Glu Pro Ile Gln Ile Tyr Glu Lys Ile Val Ser Gly Lys Val
    210                 215                 220

Arg Phe Pro Ser His Phe Ser Ser Asp Leu Lys Asp Leu Leu Arg Asn
225                 230                 235                 240

Leu Leu Gln Val Asp Leu Thr Lys Arg Phe Gly Asn Leu Lys Asn Gly
                245                 250                 255

Val Asn Asp Ile Lys Asn His Lys Trp Phe Ala Thr Thr Asp Trp Ile

            260                 265                 270
Ala Ile Tyr Gln Arg Lys Val Glu Ala Pro Phe Ile Pro Lys
        275                 280                 285